# FASCICULE DE BREVET EUROPEEN

(11) **EP 2 291 352 B1**
(45) Date de publication et mention de la délivrance du brevet: **13.09.2017**
(21) Numéro de dépôt: 09733789.3
(22) Date de dépôt: 16.04.2009
(51) Int. Cl.: C07C 335/26, C07C 275/54, C07D 209/08, C07D 209/14, C07D 213/81, C07D 235/14, C07D 307/68, C07D 317/68, C07D 319/18, C07D 333/38, C07D 471/04, C07D 513/04, A61K 31/17, A61P 3/10, A61P 25/00

(54) **N-ACYLTHIOUREES ET N-ACYLUREES INHIBITEURS DE LA VOIE DE SIGNALISATION DES PROTEINES HEDGEHOG**
N-ACYLTHIOHARNSTOFF- UND N-ACYLHARNSTOFFVERBINDUNGEN ALS INHIBITOREN DES HEDGEHOG-PROTEIN-SIGNALPFADES
N-ACYLTHIOUREA AND N-ACYLUREA INHIBITORS OF THE HEDGEHOG PROTEIN SIGNALLING PATHWAY

(30) Priorité: 24.04.2008 FR 0802302
(43) Date de publication de la demande: 09.03.2011
(73) Titulaire: Centre National de la Recherche Scientifique, 75016 Paris (FR); Université de Strasbourg, 67081 Strasbourg Cedex (FR)
(72) Inventeur: RUAT, Martial, F-91400 Orsay (FR); FAURE, Hélène, F-91190 Gif-sur-Yvette (FR); TRAIFFORT, Elisabeth, F-75013 Paris (FR); SCHOENFELDER, Angèle, F-67450 Lampertheim (FR); MANN, André, F-67540 Ostwald (FR); TADDEI, Maurizio, I-53035 Monteriggioni (IT); SOLINAS, Antonio, 07100 Sassari (SS) (IT); MANETTI, Fabrizio, I-53019 Castelnuovo Berardenga (IT)
(74) Mandataire: Gevers & Orès
(86) Numéro de dépôt international: PCT/FR2009/000442
(87) Numéro de publication internationale: WO 2009/130422

(56) Documents cités:
- EP-A1- 1 695 966
- GB-A- 2 134 518
- US-A1- 2003 187 043
- US-A1- 2004 087 659
- US-A1- 2004 138 465
- US-A1- 2004 152 743
- US-A1- 2005 014 822
- US-A1- 2005 085 519
- US-B1- 6 521 643
- M MONTES, ET AL.: "Receptor-based virtual ligand screening for the identification of novel CDC25 phosphatase inhibitors" JOURNAL OF CHEMICAL INFORMATION AND MODELING, vol. 48, no. 1, janvier 2008 (2008-01), pages 157-165, XP002553275 AMERICAN CHEMICAL SOCIETY, WASHINGTON, DC ISSN: 1549-9596
- JIAN LI, ET AL.: "Discovering novel chemical inhibitors of human cyclophilin A: virtual screening, synthesis, and bioassay" BIOORGANIC & MEDICINAL CHEMISTRY, vol. 14, no. 7, 22 novembre 2005 (2005-11-22), pages 2209-2224, XP002553276 ELSEVIER SCIENCE, OXFORD, GB ISSN: 0968-0896
- T. KLABUNDE, ET AL.: "Acyl ureas as human liver glycogen phosphorylase inhibitors for the treatment of type 2 diabetes", JOURNAL OF MEDICINAL CHEMISTRY, vol. 48, no. 20, 9 September 2005 (2005-09-09), pages 6178-6193, XP002508279, American Chemical Society, Washington, DC, US ISSN: 0022-2623, DOI: 10.1021/jm049034y [retrieved on 2005-09-09]

## Description

La présente invention est relative à l'utilisation de dérivés d'acyl-thiourées ou d'acyl-urées pour le traitement de pathologies impliquant un dysfonctionnement tissulaire lié à une dérégulation de la voie de signalisation des protéines Hedgehog, ainsi qu'à de nouveaux dérivés d'acyl-thiourées ou d'acyl-urées en tant que tels, à leur utilisation comme médicament, et aux compositions pharmaceutiques les contenant.

La molécule de signalisation Hedgehog (Hh) est une protéine auto-protéolytique sécrétée qui active la voie de signalisation des protéines Hedgehog, une voie de signalisation qui joue un rôle fondamental dans la morphogenèse de nombreux tissus, en particulier dans la formation de l'endoderme et de l'axe embryonnaire, le développement du cerveau et des follicules pileux, ainsi que dans la prolifération cellulaire, et serait impliquée dans le maintien et la réparation tissulaire chez l'adulte (Pour une revue voir : Ingham et al., Genes Dev., 2001, 15, 3059-3087 ; Marti et al., Trends Neurosci., 2002, 25, 89-96 ; Weschler et al., Annu. Rev. Neurosci., 2001, 24, 385-428).

La protéine Hedgehog et la voie de transduction associée, initialement mises en évidence chez la drosophile, sont conservées chez les vertébrés et les invertébrées. Une seul homologue de Hh est présent chez la Drosophile, alors que trois homologues de Hh : Sonic (Shh), Indian (Ihh) et Desert (Dhh) sont présents chez les mammifères. Parmi ces trois homologues, Shh a été la plus étudiée du fait de son profil d'expression étendu durant le développement. Shh participe à la ventralisation du tube neural en spécifiant le phénotype précoce de plusieurs types neuronaux le long de la ligne médiane ventrale (motoneurones de la moelle épinière, neurones dopaminergiques ou cholinergiques) et en induisant la génération des précurseurs oligodendrocytaires à partir de la moelle épinière ventrale. Par ailleurs, Shh induit la survie des neurones gabaergiques et dopaminergiques, oriente le devenir des précurseurs sérotoninergiques et prévient la mort des neurones dopaminergiques provoquée par la toxine MPP. Il induit enfin la prolifération des précurseurs des cellules granulaires dans le cervelet post-natal précoce. Les autres membres de la famille Hedgehog, participent quant à eux, respectivement au développement du tissu osseux (Ihh), des testicules et des nerfs périphériques (Dhh). En outre, les résultats obtenus avec Shh s'appliquent également à Dhh et Ihh.

Le rôle régulateur de la voie de signalisation des protéines Hedgehog durant le développement embryonnaire a été largement étudié : Hh a été associée aux processus de maintien et de réparation du tissu normal, à la régulation spatiotemporelle de la prolifération et de la différenciation, permettant ainsi aux tissus en développement d'atteindre leur taille correcte avec les types cellulaires appropriés et des degrés appropriés de vascularisation et d'innervation. Le rôle essentiel de la fonction de signalisation de Hh est démontré par les conséquences dramatiques des défauts dans cette voie de signalisation chez le foetus humain, comme l'holoprosencéphalie observée avec les mutants de Sonic Hedgehog.

Plus récemment la voie Shh a été identifiée dans le cerveau adulte, où la forme active amino-terminale de la molécule est exprimée dans de nombreuses régions du système nerveux mature, à un niveau plus élevé que celui rencontré au cours de la période post-natale précoce (Traiffort et al., Eur. J. Neurosci., 1999, 11, 3199-3214 et 2001, 14, 839-850). Bien que les rôles de Shh chez l'adulte ne soient pas complètement élucidés, il est d'abord apparu, à l'image d'autres molécules neurotrophiques, comme un facteur capable de promouvoir la survie et le maintien du phénotype des cellules du système nerveux (Reilly et al., Mol. Cell. Neurosci., 2002, 19, 88-96 ; Charytoniuk et al., Eur. J. Neurosci., 2002, 16, 2351-2357). Dans des conditions pathologiques, telles qu'un modèle de la maladie de Parkinson ou un modèle de neuropathie périphérique, Shh est capable, de préserver les projections axonales des neurones dopaminergiques dans le striatum ou d'améliorer le temps nécessaire à la récupération motrice consécutive à l'écrasement du nerf sciatique (Tsuboi et al., Exp. Neurol., 2002, 173, 95-104 ; Pepinski et al., J. Pharm. Sci., 2002, 91, 371-387).

Les protéines Hh sont synthétisées sous la forme de précurseurs immatures d'environ 45 kDa qui sont soumis à un clivage intramoléculaire catalysé par la région C-terminale du précurseur. Ce clivage produit un fragment C-terminal de 25 kDa sans fonction supplémentaire connue et en un fragment amino-terminal actif de 19 kDa (dénommée HhNp pour *N-terminal processed domain*) lié à son extrémité C-terminale à une molécule de cholestérol, suffisante pour toutes les activités de signalisation connues des protéines Hedgehog.

La voie de signalisation des protéines Hedgehog comprend trois composants principaux ; le ligand de Hh, un circuit de récepteur transmembranaire, composé du régulateur négatif Patched (Ptc) et de l'activateur Smoothed (Smo) et un complexe cytoplasmique qui régule les effecteurs transcriptionnels.

La réponse cellulaire au morphogène Hedgehog est contrôlée par les produits d'expression du gène *Patched (Ptc),* un gène suppresseur de tumeurs, et du proto-oncogène *Smoothened (Smo)* ; toutefois, le mécanisme exact de la régulation de la voie Hedgehog n'est pas complètement élucidé. Chez les mammifères, il existe deux gènes Patched codant respectivement pour Ptc1 et Ptc2, des glycoprotéines à 12 domaines transmembranaires, homologues à des transporteurs bactériens. Le produit du gène *Smo* qui code pour une protéine de la famille des récepteurs couplés aux protéines G, ne possède aucun ligand endogène connu. En l'absence de Hedgehog, Ptc bloquerait l'activité constitutive de Smo. La liaison de Hedgehog à Ptc lèverait cette inhibition et permettrait la transduction du signal par l'intermédiaire de Smo. Le mécanisme de régulation de l'activité de Smo par Ptc chez les mammifères, pourrait faire intervenir une molécule transportée par Ptc et interagissant avec Smo (Taipale et al., Nature, 2002, 418, 892-896). L'activation des facteurs de transcription Gli est impliquée dans la cascade d'événements résultant de l'activité de Smo. La protéine transmembranaire de type I, HIP (Hedgehog Intercating Protein), constitue un autre récepteur des molécules Hedgehog qu'il lie avec une affinité comparable à celle de Ptc ; HIP a été proposé comme un régulateur négatif de la voie (Ingham *et al.,* précité ; Ho et al., Curr. Opin. Neurobiol., 2002, 12, 57-63 ; Taipale et al., Nature, 2001, 411, 349-354). En outre, les produits du gène *dispatched* (*disp*), notamment DispA seraient impliqués dans le relargage et l'accumulation dans le milieu extracellulaire des protéines Hedgehog sous forme soluble (Ma et al., Cell, 2002, 111, 63-75).

Des dysfonctionnements de la voie de signalisation Shh ont été associés à de nombreux cancers, en particulier à la suite de la caractérisation de *Ptc* comme gène suppresseur de tumeur. En effet, des mutations inactivatrices de *Ptc* sont associées au Syndrome de Gorlin ou naevomatose basocellulaire, une maladie autosomale dominante caractérisée par des malformations cranofaciales et cérébrales, mais surtout par une incidence élevée de diverses tumeurs, plus particulièrement des carcinomes basocellulaires au niveau cutané et des médulloblastomes, au niveau cérébral. Des souris hétérozygotes pour le gène *Ptc* développent des tumeurs du cervelet suggérant qu'une modification de la voie Shh est à l'origine de ces tumeurs (Goodrich et al., Science, 1997, 277, 1109-1113).

Des mutations des gènes *Ptc* ou *Smo* humains sont également observées dans des tumeurs primitives neuroectodermales du système nerveux central, principalement des médulloblastomes (30 % des cas), mais aussi dans des formes sporadiques de carcinomes basocellulaires (40 % et 20 %, respectivement pour *Ptc* et *Smo*). En outre, des mutations de Shh (H133Y) sont également associées à des carcinomes basocellulaires. Les mutations de Smo qui concernent principalement deux acides aminés situés dans le septième domaine hydrophobe du récepteur (W535L et S533N), induisent une activation constitutive de la voie qui échappe au contrôle négatif de Ptc. En revanche, celles de Ptc conduisent à une diminution de l'inhibition qu'il exerce sur Smo en l'absence de Shh. Dans les deux cas, une activation de la voie Shh en résulte et conduit à une puissante activité mitogénique démontrée dans des cultures de précurseurs de cellules granulaires de cervelet en développement, et à un blocage de l'étape terminale de différenciation de ces neuroblastes (Traiffort et al., Eur. J ; Neurosci., 1999, précité ; Charytoniuk et al., J. Physiol. Paris, 2002, 96, 9-16 ; Dahmane et al., Development, 1999, 126, 3089-3100 ; Wallace et al., Curr. Biol., 1999, 9, 445-448 ; Weshler-Reya et al., Neuron., 1999, 22, 103-114). De même, l'expression de Smo portant une de ces mutations dans des souris transgéniques, conduit à la présence de carcinomes basocellulaires, ce qui démontre l'implication directe de Smo dans le développement de ces tumeurs (Xie et al., Nature, 1998, 391, 90-92).

En dehors des carcinomes basocellulaires et des médulloblastomes, d'autres types de tumeurs ont été associés à un défaut de la voie de signalisation Hedgehog ; la localisation de ces tumeurs est étroitement corrélée aux sites d'expression des composantes de la voie au cours du développement embryonnaire. A titre d'exemple non-limitatif on peut citer : des cancers du sein et des méningiomes associés à des mutations de Ptc, des glioblastomes associés à des mutations de Gli, des cancers gastro-intestinaux, notamment les cancers primaires de l'estomac, des cancers de la prostate, des fibromes et des dermoïdes ovariens, des rhabdomyosarcomes, des cancers du poumon à petites cellules, des carcinomes oraux à cellules squameuses. Récemment, Shh a été associée au psoriasis.

Du fait du rôle crucial de la voie de signalisation des protéines Hedgehog dans de nombreux processus physiologiques et par conséquent de l'importance des pathologies liées à son dysfonctionnement, les composantes de cette voie telles que les protéines Smoothened, Patched (Patched 1 et Patched 2), les protéines Dispatched (Dispatched 1 et Dispatched 2) ou bien encore la protéine HIP représentent des cibles pour la mise au point de nouvelles molécules capables de moduler (activer ou inhiber) cette voie et donc de réguler positivement ou négativement le développement [prolifération, différenciation, migration, survie (apoptose)] et/ou l'activité de cellules différenciées et de cellules souches, *in vitro* et/ou *in vivo* chez l'embryon ou chez l'adulte.

De telles molécules sont utiles dans le traitement des tumeurs associées à une hyperactivation de la voie Hedgehog : les tumeurs du tissu nerveux (médulloblastomes, tumeurs primitives neuroectodermiques, glioblastomes, méningiomes et oligodendrogliomes), les tumeurs cutanées (carcinomes basocellulaires, trichoépithéliomes), les tumeurs des tissus musculaires et osseux (rhabdomyosarcomes, ostéosarcomes) et les tumeurs d'autres tissus (rein, vessie).

De telles molécules sont également utiles dans le traitement des pathologies de type neuro-dégénératif nécessitant un blocage de la voie Hedgehog (maladie de Parkinson, Chorée de Huntington, maladie d'Alzheimer, sclérose en plaques, maladie du motoneurone), et des maladies dans lesquelles le blocage de la voie de signalisation Hedgehog pourrait être bénéfique comme le diabète.

De telles molécules sont également utiles dans le traitement médical ou chirurgical (chirurgie plastique ou réparatrice, greffe de tissus ou d'organes) de nombreuses pathologies aiguës, sub-aiguës ou chroniques, génétiques ou acquises - impliquant un dysfonctionnement tissulaire lié à une dérégulation de la voie Hedgehog - pour induire la formation, la régénération, la réparation et/ou l'augmentation de l'activité de tissus tels que de manière non-limitative : le tissu nerveux [système nerveux central (cerveau) et périphérique (neurones sensoriels, moteurs, sympathiques)], l'os, le cartilage, les testicules, le foie, la rate, l'intestin, le pancréas, les reins, les muscles lisses et squelettiques, le coeur, les poumons, la peau et le système pileux, les muqueuses, les cellules sanguines et les cellules du système immunitaire. A titre d'exemple non-limitatif de ces pathologies, on peut citer notamment les neuropathies et les maladies neuromusculaires associées, le diabète, l'alopécie, les brûlures, les ulcérations (peau et muqueuse) et les troubles de la spermatogenèse.

Différentes molécules, capables de moduler l'activité de la voie Hedgehog, ont été identifiées :
- les protéines Hedgehog et des polypeptides dérivés (fragments, variants...), notamment des antagonistes des protéines Hedgehog (Demande Internationale PCT WO 01/98344 au nom de BIOGEN) ; du fait de leur taille, ces protéines et les polypeptides dérivés ne peuvent pas passer la barrière hématoencéphalique et ne peuvent donc pas être administrés par voie systémique, notamment pour le traitement des tumeurs cérébrales liées à une hyperactivation de la voie de signalisation des protéines Hedgehog. En outre, de telles molécules sont difficiles à produire et à purifier et sont peu stables ;
- des molécules organiques hétérocycliques (Demande Internationale PCT WO 01/74344 au nom de CURIS et Chen et al., PNAS, 2002, 99, 14071-14076) ;
- des molécules hétérocycliques azotées (Demandes Internationales PCT WO 01/19800, WO 01/26644 et WO 02/30421 au nom de CURIS et Kamenetsky et al., J. Biol., 2002, 1, 1-19) ; et
- des stéroïdes végétaux dérivés de *Veratrum* spp (jervine, cyclopamine et cycloposine) et de *Solanum* spp. (solanidine), substitués en position 16, 17 ou 18 par une amine ou un dérivé d'amine, et du cholestérol : Brevet américain US 6,432,970 et Demandes Internationales PCT WO 99/52534 et WO 01/27135 au nom de JOHNS HOPKINS UNIVERSITY SCHOOL OF MEDICINE ; Brevet américain US 6,291,516 au nom de CURIS ; Demande Internationale PCT WO 00/41545 au nom de ONTOGENY ; Demande Internationale PCT WO 02/30462 au nom de CURIS ; Talpale et al., Nature, 2000, 406, 1005-1009 ; Berman et al., Science, 2002, 297, 1559-1561. Toutefois, la cyclopamine est un agent tératogène à l'origine d'holoprasencéphalie et de cyclopie pour l'embryon chez les mammifères et l'absence de toxicité, pour les mammifères, des autres composés dérivés de stéroïdes végétaux n'a pas encore été démontrée ;
- la mifepristone (17β-hydroxy 11β-(4-diméthylamino phényl) 17α-(prop-1-ynyl)estra-4,9-dièn-3-one), également dénommée RU-486 ou RU-38486 (Brevet français FR 03 00646 au nom du CNRS) pour laquelle une activité inhibitrice de l'activité de la voie de signalisation des protéines Hedgehog a été démontrée.

Il ressort de ce qui précède qu'il n'existe actuellement pas de molécule efficace pour le traitement des états pathologiques nécessitant une inhibition de l'activité de la voie de signalisation des protéines Hedgehog dont l'absence de toxicité ait été vérifiée par des essais cliniques chez l'Homme.

En outre, la demande US 2005/0085519 décrit des dérivés urées et thiourées en tant qu'inhibiteurs de la voie Hedgehog. Cependant, ces composés sont différents de ceux de la présente invention. Par ailleurs, la demande EP 1 695 966 décrit des dérivés urées dans le cadre du traitement de la maladie d'Alzheimer, la maladie de Parkinson et la maladie de Huntington. Cependant, ces composés sont différents de ceux de la présente invention utilisés dans le cadre des maladies précitées.

En conséquence, les Inventeurs se sont donné pour but de pourvoir à de nouveaux composés inhibiteurs de la voie de signalisation des protéines Hedgehog qui répondent mieux aux besoins de la pratique, notamment en ce qu'ils sont simples à synthétiser et potentiellement utilisables en thérapie humaine.

Cet objectif est atteint par les composés de formule **(I)** qui sont décrits ci-après dans la mesure où ces molécules présentent l'avantage de comporter des fonctions de type acyl-thiourée ou acyl-urée qui s'obtiennent à partir de matières premières aisément disponibles. De plus les thiourées se transforment en urées par une simple étape d'oxydation ; l'obtention de l'ensemble des composés de formule **(I)** est ainsi très commode.

L'objet de la présente invention est défini par les revendications 1 à 20.

En conséquence, la présente description a pour objet l'utilisation pour la fabrication d'un médicament pour le traitement des tumeurs associées à une hyperactivation de la voie de signalisation des protéines Hedgehog ou des pathologies de type neuro-dégénératif des composés de formule **(I)** suivante : 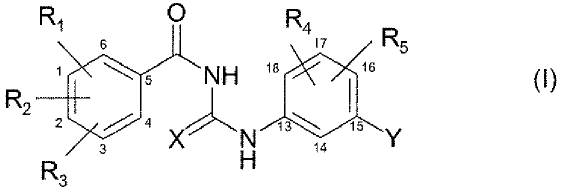 dans laquelle :
- R₁, R₂ et R₃, identiques ou différents et indépendamment les uns des autres, représentent un atome d'hydrogène ou d'halogène, un radical hydroxyle, un groupement alkyle, perfluoroalkyle, alcoxy, alkylthio, nitrile, un alcoxy substitué, ou un hétérocycle fusionné obetnue à partir de deux des radicaux R₁, R₂ et R₃, adjacents, pouvant former, conjointement avec les atomes de carbone du cycle phényle auxquels ils sont liés, un hétérocycle fusionné,
- X représente un atome de soufre ou d'oxygène,
- Y représente un groupement hétéroaryle mono- ou polycyclique, substitué ou non substitué, un groupement -NH-(C=O)-R₆, -(C=O)-NH-R₆ ou -NH-(C=O)-NH-R₆ dans lequel R₆ représente un groupement aryle non substitué ; un groupement aryle comportant un ou plusieurs substituants choisis parmi un atome d'halogène et un radical alkyle, alcoxy ou mono- ou dialkylamino ; un groupement hétéroaryle mono- ou polycyclique ; un radical alkyle linéaire ou ramifié ; un groupement hydrocarboné mono- ou polycyclique saturé ou insaturé ;
- R₄ et R₅, identiques ou différents et indépendamment l'un de l'autre, représentent un atome d'hydrogène ou d'halogène, un groupe alcoxy, alkylthio, alkyle, perfluoroalkyle, nitrile ou nitro.

De préférence :
* R₄ et/ou R₅ représentent un groupement nitro,
* deux des R₁, R₂ et R₃, adjacents, forment, conjointement avec les atomes de carbone du cycle phényle auxquels ils sont liés, un hétérocycle fusionné tel que par exemple un benzodioxole, un oxindole, une benzoxazolone ou une quinoline,
lorsque Y représente un groupement -NH-(C=O)-R₆.

De manière avantageuse, lorsque Y représente un hétérocycle substitué ou non substitué, ledit hétérocycle est un hétérocycle polycyclique. De manière encore plus préférée, ledit hétérocycle polycyclique est choisi parmi les groupements indole, benzoimidazole, imidazopyridine et imidazothiazole.

Ainsi que cela est démontré dans les exemples qui illustrent la présente description, les composés de formule **(I)** conformes à la description présente une activité inhibitrice de la voie de signalisation des protéines Hedgehog et sont donc utiles pour le traitement des tumeurs associées à une hyperactivation de la voie de signalisation des protéines Hedgehog ou des pathologies de type neuro-dégénératif.

Les composés de formule **(I)** conformes à la présente description peuvent être divisés en sous-unités A, B, C, (ou C' ou C") et D (ou D' ou D") et représentés par les formules **(I-a)**, **(I**-**a')**, **(I-b)**, **(I-b')**, **(I-c)**, **(I-c')**, **(I-d)** et **(I-d')** suivantes : 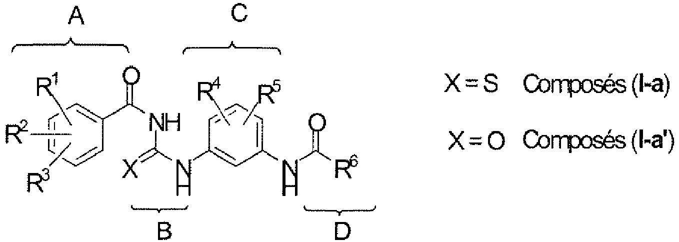 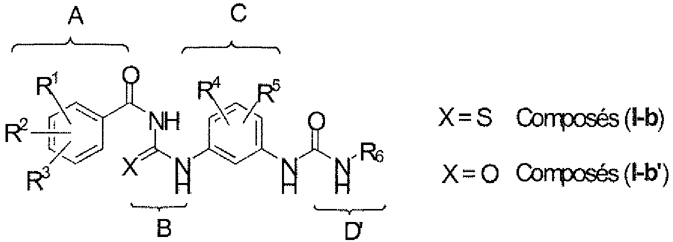 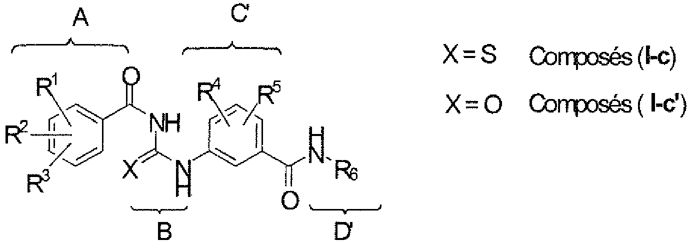 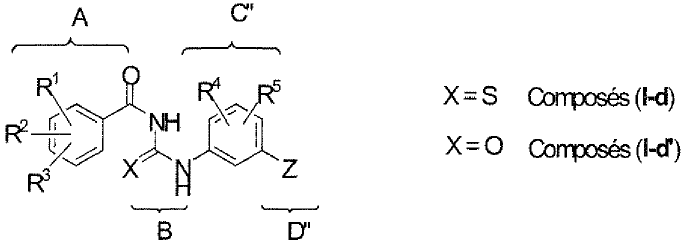 dans lesquelles radicaux R₁ à R₆ ont les mêmes significations que celles indiquées ci-dessus, et le groupement Z représentant un groupement hétéroaryle mono- ou polycyclique, substitué ou non substitué.

Dans ces formules, la sous-unité A correspond à une partie acyl-aryle, la sous-unité B à une partie thiourée (X = S) ou urée (X = O), la sous-unité C à un groupement 1,3-diaminoaryle, la sous-unité C' à un groupement 1,3-aminobenzoyle, la sous-unité C" à un groupement aminophényle, la sous-unité D à un résidu alkyloyle, aroyle ou hétéroaroyle, la sous-unité D' à un résidu alkylamino, arylamino ou hétéroarylamino, et la sous-unité D" à un résidu hétéroaryle mono- ou polycyclique, substitué ou non substitué, directement attaché au radical C".

Au sens de la présente description, on entend par :
- Alkyle : un groupement aliphatique hydrocarboné saturé, linéaire ou ramifié, ayant de 1 à 5 atomes de carbone, de préférence de 1 à 2 atomes de carbone. Le terme "ramifié" signifie qu'au moins un groupe alkyle inférieur tel qu'un méthyle ou un éthyle est porté par une chaîne alkyle linéaire. Le terme alkyle "inférieur" désigne un alkyle ayant 1 ou 2 atomes de carbone ; le terme "alkyle supérieur" désigne un groupe alkyle linéaire ou ramifié ayant de 3 à 5 atomes de carbone. A titre de groupement alkyle, on peut mentionner par exemple les groupements méthyle, éthyle, n-propyle, i-propyle, n-butyle, t-butyle et n-pentyle.
- Atome d'halogéne : désigne un atome de brome, de chlore, d'iode ou de fluor ; les désignations brome, chlore et fluor étant préférées ;
- Perfluoroalkyle : désigne un groupement alkyle tel que défini ci-dessus dans lequel tous les atomes d'hydrogène ont été remplacés par des atomes de fluor. Parmi les groupements perfluoroalkyle, les groupements trifluorométhyle et perfluoroéthyle sont préférés ;
- Alcoxy : désigne un groupement O-alkyle dans lequel le groupe alkyle peut prendre la même signification que celle indiquée ci-dessus. A titre d'exemple de groupements alcoxy, on peut notamment citer les groupements méthoxy, éthoxy, n-propoxy, iso-propoxy, n-butoxy et pentoxy ;
- Alkylthio : désigne un groupement alkyl-S dans lequel le groupe alkyle peut prendre la même signification que celle indiquée ci-dessus. A titre d'exemples de groupement alkylthio, on peut notamment citer les groupements méthylthio, éthylthio, iso-propylthio, butylthio et pentylthio ;
- Groupement aryle : désigne tout groupement fonctionnel ou substituant dérivé d'au moins un cycle aromatique ; un cycle aromatique correspond à tout groupement mono- ou polycyclique plan comportant un système *π* délocalisé dans lequel chaque atome du cycle comporte une orbitale p, lesdites orbitales p se recouvrant les unes les autres ; parmi de tels groupements aryle, on peut mentionner les groupements phényle, benzylcyclobutène, pentalène, naphthalène, benzylphényle, et anthracène ;
- Groupement hétéroaryle: désigne tout groupement fonctionnel ou substituant dérivé d'au moins un cycle aromatique tel que défini ci-dessus et contenant au moins un hétéroatome choisi parmi P, S, O et N ; parmi les groupements hétéroaryle, on peut mentionner les groupements furane, pyridine, pyrrole, thiophène, imidazole, pyrazole, oxazole, isoxazole, thiazole, pyridine, imidazopyridine, imidazothiazole, pyrazine, pyrimidine, pyridazine, benzofurane, isobenzofurane, indole, isoindole, benzothiophène, benzo[c]thiophène, benzimidazole, indazole, benzoxazole, benzisoxazole, benzothiazole, quinoline, isoquinoline, quinoxaline, quinazoline, cinnoline, purine, et acridine ;
- Groupement hydrocarboné mono- ou polycyclique saturé ou insaturé : désigne tout groupement fonctionnel ou substituant dérivé d'un cycle non aromatique comportant au moins 3 atomes de carbone comportant éventuellement, de façon optionnelle, un ou plusieurs hétéroatomes choisis parmi P, S, O et N. Parmi de tels groupements, on peut notamment citer le cyclopropyle, le cyclobutyle, le cyclopentyle, le cyclohexyle ; le groupement cyclohexyle étant préféré.

Selon une forme de réalisation préférée de la description, les composés de formule **(I)** sont choisis parmi ceux dans lesquels :
- R₁, R₂, et R₃, identiques ou différents, représentent un atome d'hydrogène ou un radical méthyloxy ou éthyloxy,
- X représente un atome de soufre ou d'oxygène,
- R₄ et R₅, identiques ou différents, sont choisis parmi hydrogène, chlore, brome, fluor, méthyle et méthoxy ; et
- Y représente un groupement hétéroaryle mono- ou polycyclique, substitué ou non substitué, un groupement -NH-(C=O)-R₆, -(C=O)-NH-R₆ ou -NH-(C=O)-NH-R₆ dans lequel R₆ représente un groupement choisi parmi les groupements phényle ; phényle substitué par un radical méthoxy ou diméthylamino, par un atome de chlore, par un groupement phényle, par un groupement benzyle ; cyclohexyle, isopropyle, pyridinyle, naphtyle, furfuryle et thiophène.

A titre de composés de formule **(I),** on peut en particulier citer, à titre non limitatif :
- le N-[[[3-(4-méthoxy-benzoylamino)phényl]amino]thioxométhyl]-3,4,5-triméthoxy-benzamide **(I-a)** de formule suivante : 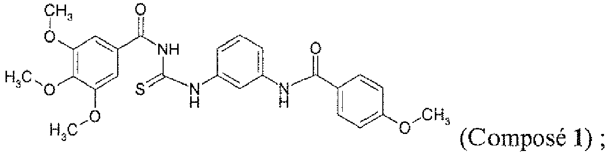
- le N-[[[3-(2-chloro-benzoylamino)phényl]amino]thioxométhyl]-3,4,5-triméthoxy-benzamide **(I-a)** de formule suivante : 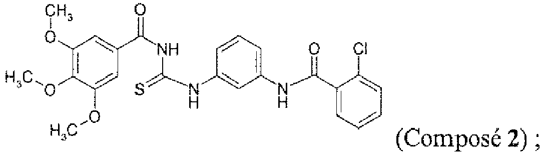
- le N-[[[3-(3-méthoxy-benzoylamino)phényl]amino]thioxométhyl]-3,4,5-triméthoxy-benzamide **(I-a)** de formule suivante : 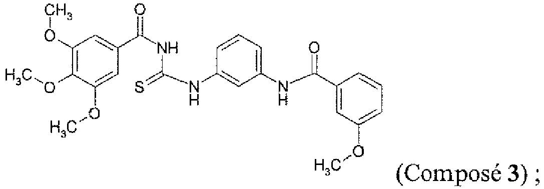
- le N-[[[3-(2-méthoxy-benzoylamino)phényl]amino]thioxométhyl]-3,4,5-triméthoxy-benzamide **(I-a)** de formule suivante : 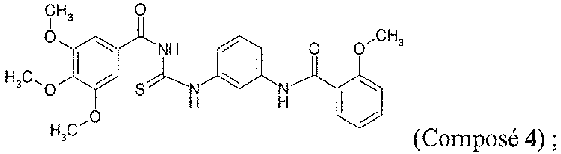
- le N-[[[3-(4-phényl-benzoylamino)phényl]amino]thioxométhyl]-3,4,5-triméthoxy-benzamide **(I-a)** de formule suivante : 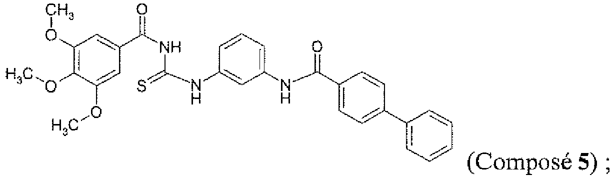
- le N-[[[3-(4-méthoxy-benzoylamino)phényl]amino]oxométhyl]-3,4,5-triméthoxy-benzamide **(I-a')** de formule suivante : 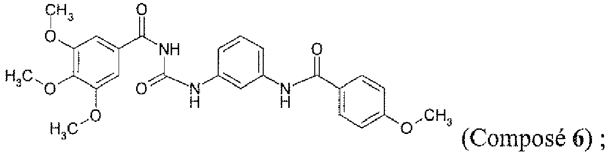
- le N-[[[3-(4-phényl-benzoylamino)phényl]amino]oxométhyl]-3,4,5-triméthoxy-benzamide **(I-a')** de formule suivante : 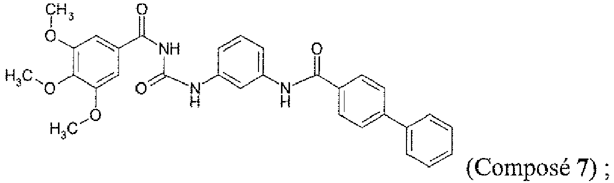
- le N-[[[3-(-cyclohexylcarboxylamino)phényl]amino]thioxométhyl]-3,4,5-triméthoxy-benzamide **(I-a)** de formule suivante : 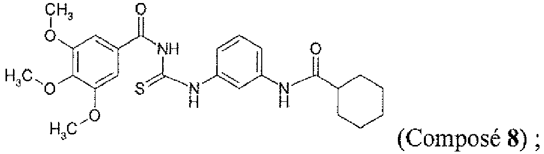
- le N-[[[3-(2-naphtoylamino)phényl]amino]thioxométhyl]-3,4,5-triméthoxy-benzamide **(I-a)** de formule suivante : 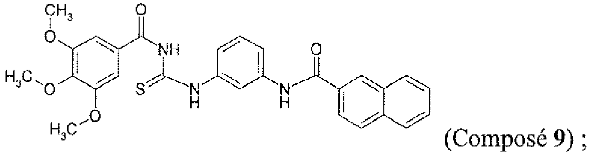
- le N-[[[3-(isopropoylcarboxylamino)phényl]amino]thioxométhyl]-3,4,5-triméthoxy-benzamide **(I-a)** de formule suivante : 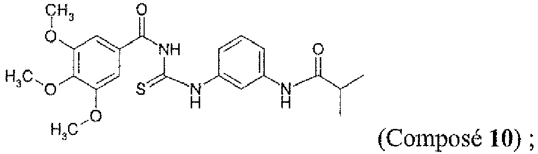
- le N-[[[3-(4-pyridocarboxylamino)phényl]amino]thioxométhyl]-3,4,5-triméthoxy-benzamide **(I-a)** de formule suivante : 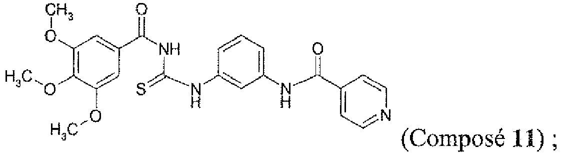
- le N-[[[3-(1-naphtoylamino)phényl]amino]thioxométhyl]-3,4,5-triméthoxy-benzamide **(I-a)** de formule suivante : 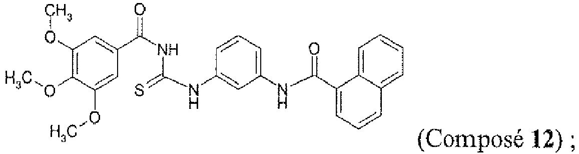
- le N-[[[3-(4-chloro-benzoylamino)phényl]amino]thioxométhyl]-3,4,5-triméthoxy-benzamide **(I-a)** de formule suivante : 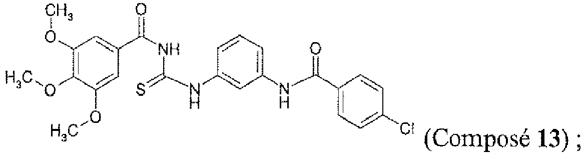
- le N-[[[3-(2-furoylamino)phényl]amino]thioxométhyl]-3,4,5-triméthoxy-benzamide **(I-a)** de formule suivante : 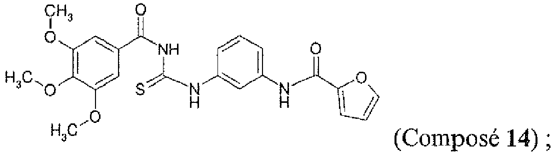
- le N-[[[3-(2-thiophénoylamino)phényl]amino]thioxométhyl]-3,4,5-triméthoxy-benzamide **(I**-**a)** de formule suivante : 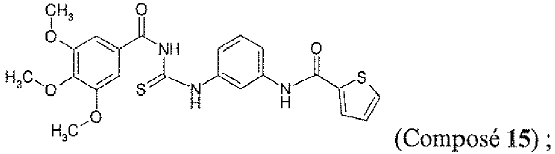
- le N-[[[3-(2-naphtoylamino)phényl]amino]oxométhyl]-3,4,5-triméthoxy-benzamide **(I-a')** de formule suivante : 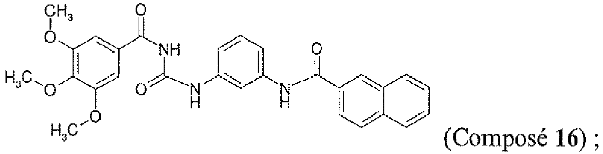
- le N-[[[3-(isopropoylcarboxylamino))phényl]amino]oxométhyl]-3,4,5-triméthoxy-benzamide **(I-a')** de formule suivante : 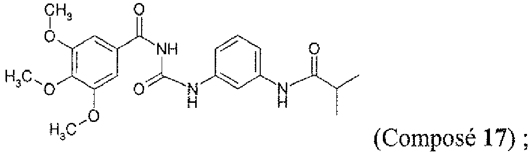
- le N-[[[3-(benzoylamino)phényl]amino]thioxométhyl]-3,5-diméthoxy-benzamide **(I-a)** de formule suivante : 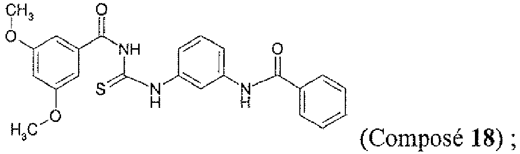
- le N-[[[3-(benzoylamino)phényl]amino]thioxométhyl]-3,5-diméthoxy-4-éthoxy-benzamide **(I-a)** de formule suivante : 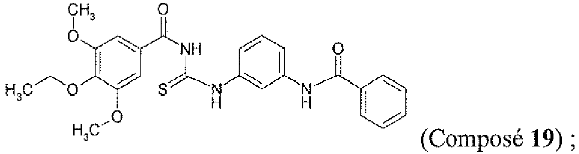
- le N-[[[3-(benzoylamino)phényl]amino]thioxométhyl]-3,4-diéthoxy-5-méthoxy-benzamide **(I-a)** de formule suivante : 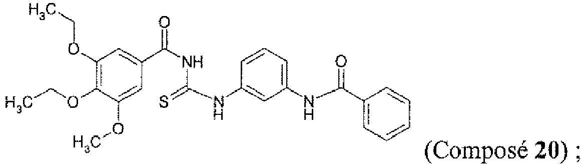
- le N-[[[3-(benzoylamino)phényl]amino]thioxométhyl]-3,4-méthylènedioxy-5-méthoxy-benzamide **(I-a)** de formule suivante : 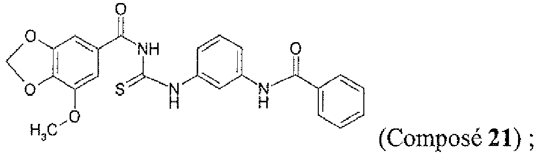
- le N-[[[3-(benzoylamino)phényl]amino]thioxométhyl]-2,4-diméthoxy-benzamide **(I-a)** de formule suivante : 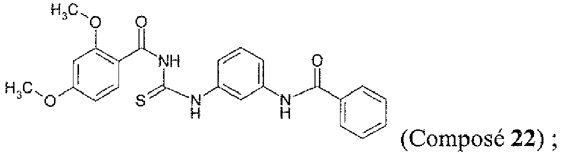
- le N-[[[3-(benzoylamino)phényl]amino]thioxométhyl]-3,4,5-triéthoxy-benzamide **(I-a)** de formule suivante : 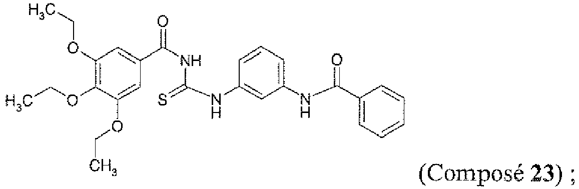
- le N-[[[4-fluoro-3-(benzoylamino)phényl]amino]thioxométhyl]-3,4,5-triméthoxy-benzamide **(I-a)** de formule suivante : 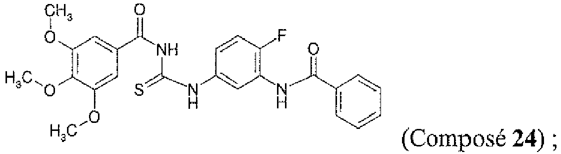
- le N-[[[4-chloro-3-(phénylamino)carbonyl]amino]thioxométhyl]-3,4,5-triméthoxy-benzamide **(I-b)** de formule suivante : 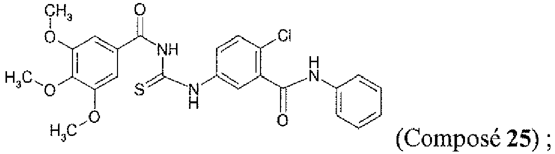
- le N-[[[4-méthoxy-3-(benzoylamino)phényl]amino]thioxométhyl]-3,4,5-triméthoxy-benzamide **(I-a)** de formule suivante : 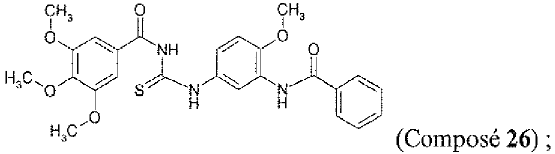
- le N-[[[4-méthyl-3-(benzoylamino)phényl]amino]thioxométhyl]-3,4,5-triméthoxy-benzamide **(I-a)** de formule suivante : 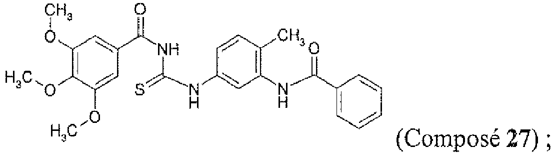
- le N-[[[3-(benzoylamino)phényl]amino]thioxométhyl]-3,4-éthylènedioxo-benzamide **(I-a)** de formule suivante : 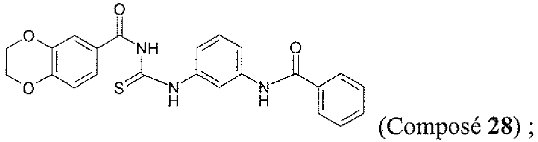
- le N-[[[3-(benzoylamino)phényl]amino]oxométhyl)-3,4,5-triéthoxy-benzamide **(I-a')** de formule suivante : 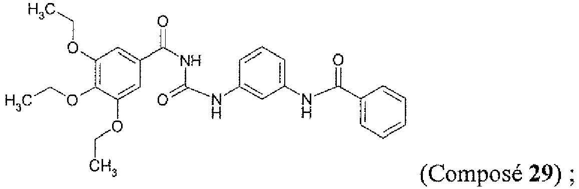
- le N-[[[3-(benzoylamino)phényl]amino]oxométhyl]-3,4-méthylènedioxy-5-méthoxy-benzamide **(I-a')** de formule suivante : 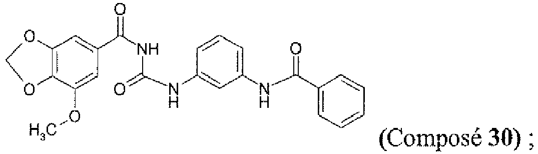
- le N-[[[3-(Benzoylamino)phényl]amino]oxométhyl]-3,4,5-triméthoxy-benzamide **(I-a')** de formule suivante : 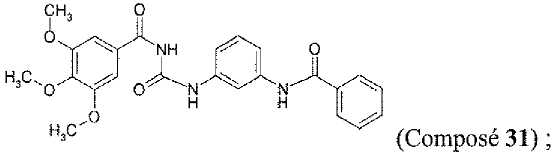
- le N-[[[3-méthyl-3-(benzoylamino)phényl]amino]thioxométhyl]-3,4,5-triméthoxy-benzamide **(I**-**a)** de formule suivante : 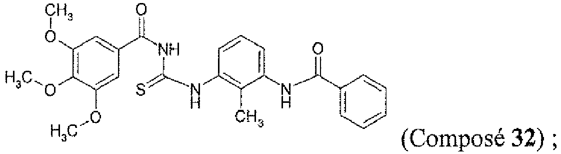
- le N-[[[4-chloro-3-(benzoylamino)phényl]amino]oxométhyl]-3,4,5-triméthoxy-benzamide **(I-a')** de formule suivante : 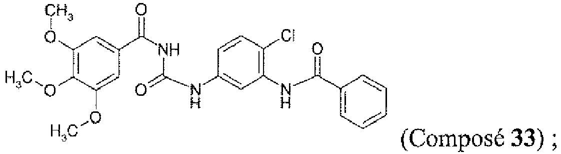
- le N-[[[4-chloro-3-(benzoylamino)phényl]amino]thioxométhyl]-3,4,5-triméthoxy-benzamide **(I-a)** de formule suivante : 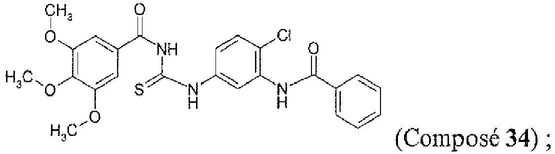
- le N-[[[5-chloro-3-(benzoylamino)phényl]amino]thioxométhyl]-3,4,5-triméthoxy-benzamide **(I-a)** de formule suivante : 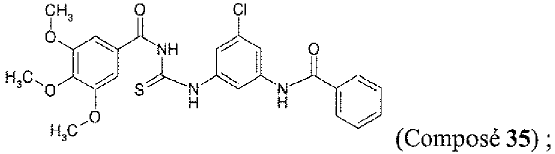
- le N-[[[5-fluoro-3-(benzoylamino)phényl]amino]thioxométhyl]-3,4,5-triméthoxy-benzamide **(I-a)** de formule suivante : 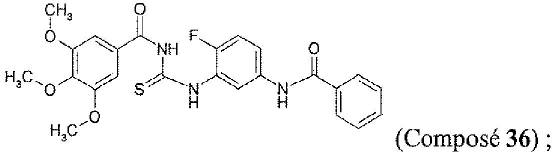
- le N-[[[4-chloro-3-(4-Phényl-benzoylamino)phényl]amino]oxométhyl]-3,4,5-triméthoxy-benzamide **(I-a')** de formule suivante : 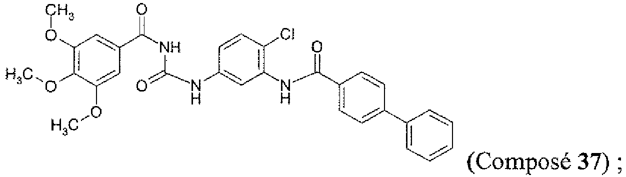
- le N-[[[3-(benzoylamino)phényl]amino]thioxométhyl]-3,4,5-triméthoxy-benzamide **(I-a)** de formule suivante : 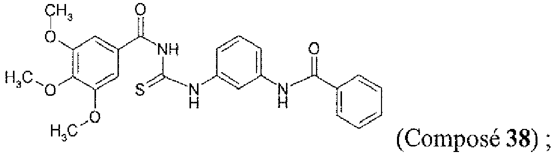
- le N-[[[4-chloro-3-(4-diméthylamino-phénylamino)carbonyl]thioxométhyl]-3,4,5-triméthoxy-benzamide **(I-b)** de formule suivante : 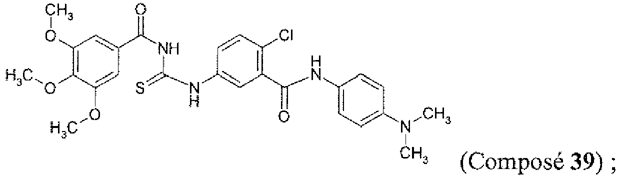
- le N-[[[4-chloro-3-(4-méthoxy-phénylamino)carbonyl]thioxométhyl]-3,4,5-triméthoxy-benzamide **(I-b)** de formule suivante : 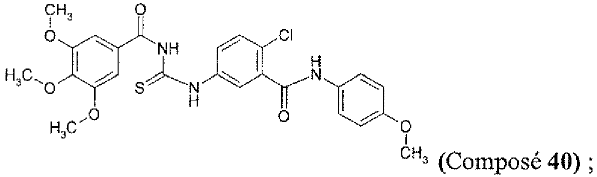
- le N-[[[5-bromo-3-(phénylamino)carbonyl]thioxométhyl]-3,4,5-triméthoxy-benzamide **(I-b)** de formule suivante : 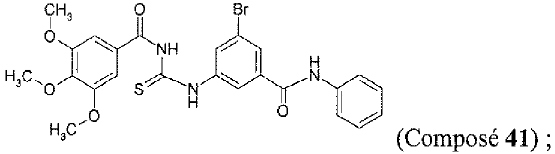
- le N-[[[4-chloro-3-(4-méthoxy-phénylamino)carbonyl]oxométhyl]-3,4,5-triméthoxy-benzamide **(I-b')** de formule suivante : 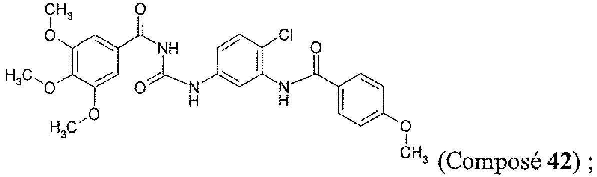
- le N-[[[4-chloro-3-(4-chloro-phénylamino)]carbonyl]phénylamino]thioxométhyl]-3,4,5-triméthoxy-benzamide **(I-b)** de formule suivante : 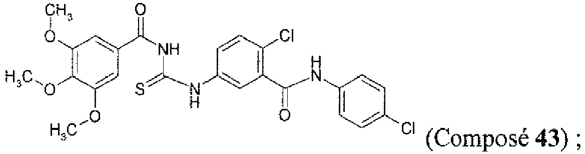
- le N-[[[4-chloro-3-(phénylamino)carbonyl]oxométhyl]-3,4,5-triméthoxy-benzamide **(I-b')** de formule suivante : 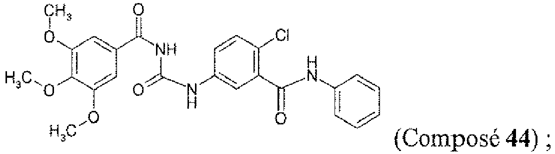
- le N-[[[2-méthyl-3-(benzoylamino)phényl]amino]oxométhyl]-3,4,5-triméthoxy-benzamide **(I-a')** de formule suivante : 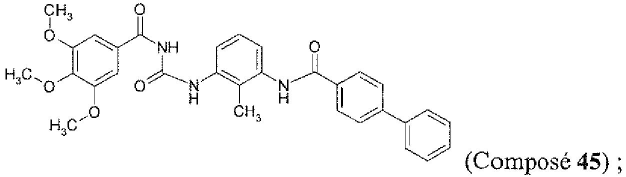
- le N-[[[2-méthyl-3-(benzoylamino)phényl]amino]thioxométhyl]-3,4,5-triméthoxy-benzamide **(I-a)** de formule suivante : 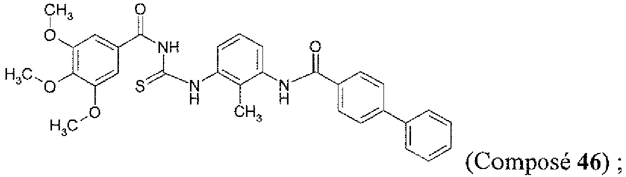
- le N-[[[-3-(4-chloro-benzoylamino)phényl]amino]oxométhyl]-3,4,5-triméthoxy-benzamide **(I-a')** de formule suivante : 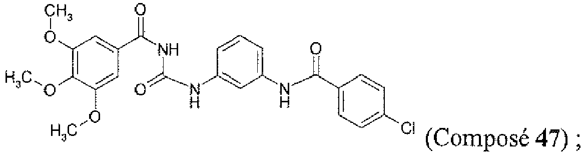
- le N-[[[-3-(benzoylamino)phényl]amino]oxométhyl]-2,4,5-triméthoxy-benzamide **(I-a')** de formule suivante : 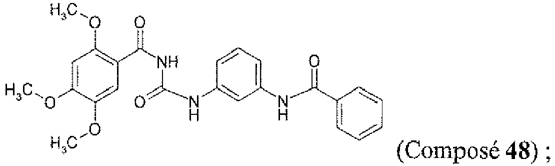
- le N-[[[-3-(benzoylamino)phényl]amino]oxométhyl]-4-méthoxy-benzamide **(I-a')** de formule suivante : 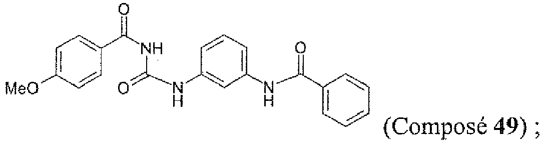
- le N-[[[-3-(benzoylamino)phényl]amino]oxométhyl]-benzamide **(I-a')** de formule suivante : 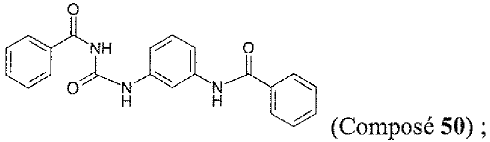
- le 3,4,5-triméthoxy-*N*-(3-(3-phényluréido)phénylcarbamothioyl)benzamide **(I-b)** de formule suivante : 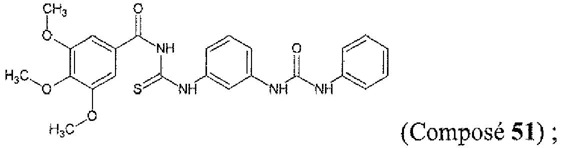
- le *N*-(3-(3-biphényl-4-yluréido)phénylcarbamothioyl)-3,4,5-triméthoxybenzamide **(I-b)** de formule : 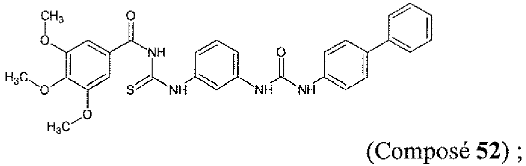
- le *N*-{3-(1*H*-indol-2-yl)phénylcarbamoyl)-3,4,5-triméthoxybenzamide (**I**-**d**') de formule : 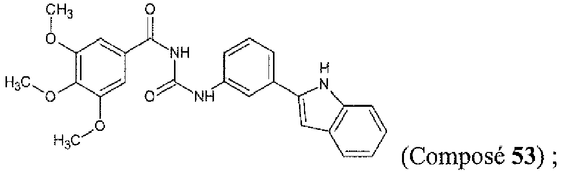
- le *N*-(3-(1*H*-indol-2-yl)phénylcarbamothioyl)-3,4,5-triméthoxybenzamide (**I**-**d**) de formule : 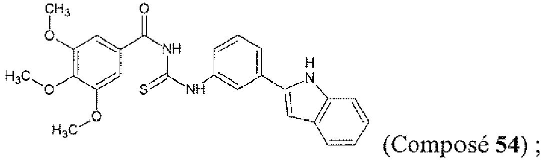
- le *N*-(3-(imidazo[1,2-*a*]pyridin-2-yl)phénylcarbamothioyl)-3,4,5-triméthoxybenzamide (**I**-**d**) de formule : 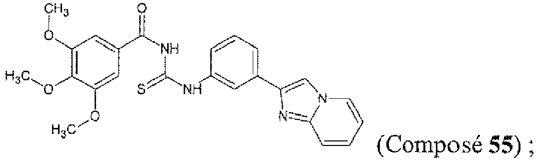
- le *N*-(3-(1*H*-benzo[*d*]imidazol-2-yl)-4-méthylphénylcarbamothioyl)-3,4,5-triméthoxybenzamide (**I-d**) de formule : 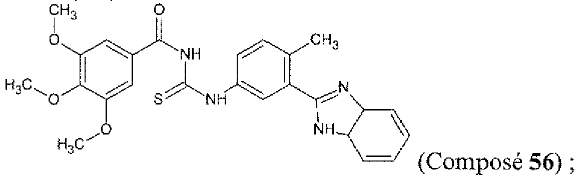
- le *N*-(3-(1*H*-indol-1-yl)phénylcarbamothioyl)-3,4,5-triméthoxybenzamide (**I-d**) de formule : 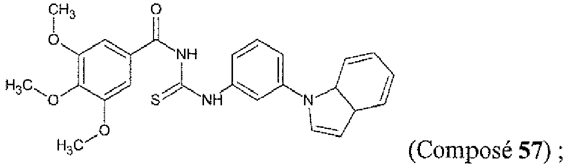
- le *N*-(3-(imidazo[2,1-*b*]thiazol-6-yl)phénylcarbamothioyl)-3,4,5-triméthoxybenzamide (**I**-**d**), de formule : 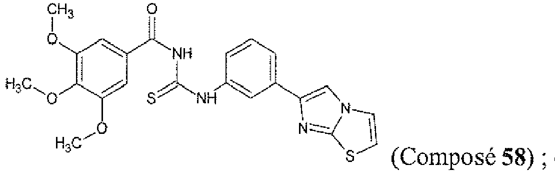 et
- le *N-*(3-(3-biphényl-4-yluréido)phénylcarbamoyl)-3,4,5-triméthoxybenzamide (**I-b'**) de formule :

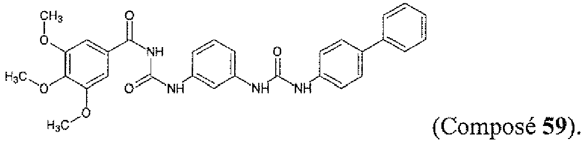

Parmi ces composés, les composés suivants sont particulièrement préférés dans la mesure où ils présentent une activité d'inhibition de la voie de signalisation des protéines Hedgehog qui est supérieure ou égale à 80 % d'inhibition, ladite inhibition étant mesurée après activation de la voie de signalisation des protéines Hedgehog avec un composé activateur synthétique de type chlorobenzothiophène dénommé le SAG (CAS N° : 364590-63-6) selon la méthode décrite par Chen *et al.,* (Proc. Natl. Acad. Sci. USA, 2002, 99, 14071) :
- le N-[[[3-(4-méthoxy-benzoylamino)phényl]amino]thioxométhyl]-3,4,5-triméthoxy-benzamide **(I-a)** (Composé **1**) ;
- le N-[[[3-(2-chloro-benzoylamino)phényl]amino]thioxométhyl]-3,4,5-triméthoxy-benzamide (**I-a**) (Composé **2**) ;
- le N-[[[3-(3-méthoxy-benzoylamino)phényl]amino]thioxométhyl]-3,4,5-triméthoxy-benzamide (**I-a**) (Composé **3**) ;
- le N-[[[3-(2-méthoxy-benzoylamino)phényl]amino]thioxométhyl]-3,4,5-triméthoxy-benzamide (**I-a**) (Composé **4**) ;
- le N-[[[3-(4-méthoxy-benzoylamino)phényl]amino]oxométhyl]-3,4,5-triméthoxy-benzamide (**I-a'**) (Composé **6**) ;
- le N-[[[3-(4-pyridocarboxylamino)phényl]amino]thioxométhyl]-3,4,5-triméthoxy-benzamide (**I-a**) (Composé **11**) ;
- le N-[[[3-(4-chloro-benzoylamino)phényl]amino]thioxométhyl]-3,4,5-triméthoxy-benzamide (**I-a**) (Composé **13**) ;
- le N-[[[3-(isopropoylcarboxylamino)phényl]amino]oxométhyl]-3,4,5-triméthoxy-benzamide (**I-a'**) (Composé **17**) ;
- le N-[[[3-(benzoylamino)phényl]amino]thioxométhyl]-3,5-diméthoxy-benzamide **(I-a)** (Composé **18**) ;
- le N-[[[3-(benzoylamino)phényl]amino]thioxométhyl]-3,5-diméthoxy-4-éthoxy-benzamide (**I-a**) (Composé **19**) ;
- le N-[[[3-(benzoylamino)phényl]amino]thioxométhyl]-3,4-diéthoxy-5-méthoxy-benzamide (**I-a**) (Composé **20**) ;
- le N-[[[3-(benzoylamino)phényl]amino]thioxométhyl]-3,4-méthylènedioxy-5-méthoxy-benzamide **(I-a)** (Composé **21**) ;
- le N-[[[3-(benzoylamino)phényl]amino]thioxométhyl]-3,4,5-triméthoxy-benzamide (**I-a**) (Composé **23**) ;
- le N-[[[4-fluoro-3-(benzoylamino)phényl]amino]thioxométhyl]-3,4,5-triméthoxy-benzamide (**I-a**) (Composé **24**) ;
- le N-[[[4-chloro-3-(phénylamino)carbonyl]amino]thiaxométhyl]-3,4,5-triméthoxy-benzamide (**I-b**) (Composé **25**) ;
- le N-[[[3-(benzoylamino)phényl]amino]oxométhyl]-3,4,5-triméthoxy-benzamide (**I-a'**) (Composé **31**) ;
- le N-[[[4-chloro-3-(benzoylamino)phényl]amino]thioxométhyl]-3,4,5-triméthoxy-benzamide (**I-a**) (Composé **34**) ;
- le N-[[[3-(benzoylamino)phényl]amino]thioxométhyl]-3,4,5-triméthoxy-benzamide (**I-a**) (Composé **38**) ;
- le N-[[[4-chloro-3-(4-diméthylamino-phénylamino)carbonyl] thioxométhyl]-3,4,5-triméthoxy-benzamide (**I-b**) (Composé **39**) ;
- le N-[[[4-chloro-3-(4-chloro-phénylamino)]carbonyl]phénylamino]thioxométhyl]-3,4,5-triméthoxy-benzamide (**I-b**) (Composé **43**) ;
- le N-[[[2-methyl-3-(benzoylamino)phényl]amino]oxométhyl]-3,4,5-triméthoxy-benzamide (**I-a'**) (Composé **45**) ;
- le N-[[[-3-(4-chloro-benzoylamino)phényl]amino]oxométhyl]-3,4,5-triméthoxy-benzamide (**I-a'**) (Composé **47**) ;
- le 3,4,5-triméthoxy-*N*-(3-(3-phényluréido)phénylcarbamothioyl)benzamide **(I-b)** (Composé **51**),
- le *N*-(3-(3-biphényl-4-yluréido)phénylcarbamothioyl)-3,4,5-triméthoxybenzamide (**I-b**) (Composé **52**) ;
- le *N*-(3-(1*H*-indol-2-yl)phénylcarbamoyl)-3,4,5-triméthoxybenzamide **(I-d')** (Composé **53**) ;
- le *N*-(3-(1*H*-indol-2-yl)phénylcarbamothioyl)-3,4,5-triméthoxybenzamide **(I-d)** (Composé **54**) ;
- le *N*-(3-(imidazo[1,2-*a*]pyridin-2-yl)phénylcarbamothioyl)-3,4,5-triméthoxybenzamide (**I-d**) (Composé **55**) ;
- le *N*-(3-(1*H*-indol-1-yl)phénylcarbamothioyl)-3,4,5-triméthoxybenzamide (Composé **57**) ;
- le *N*-(3-(imidazo[2,1-*b*]thiazol-6-yl)phénylcarbamothioyl)-3,4,5-triméthoxybenzamide **(I-d)** (Composé **58**) ; et
- le *N*-(3-(3-biphényl-4-yluréido)phénylcarbamoyl)-3,4,5-triméthoxybenzamide **(I-b')** (Composé **59**).

Les composés de formule (**I**) conformes à la description peuvent être facilement préparés, généralement en trois ou quatre étapes, selon des procédés de synthèse analogues aux procédés classiques connus de l'homme du métier.

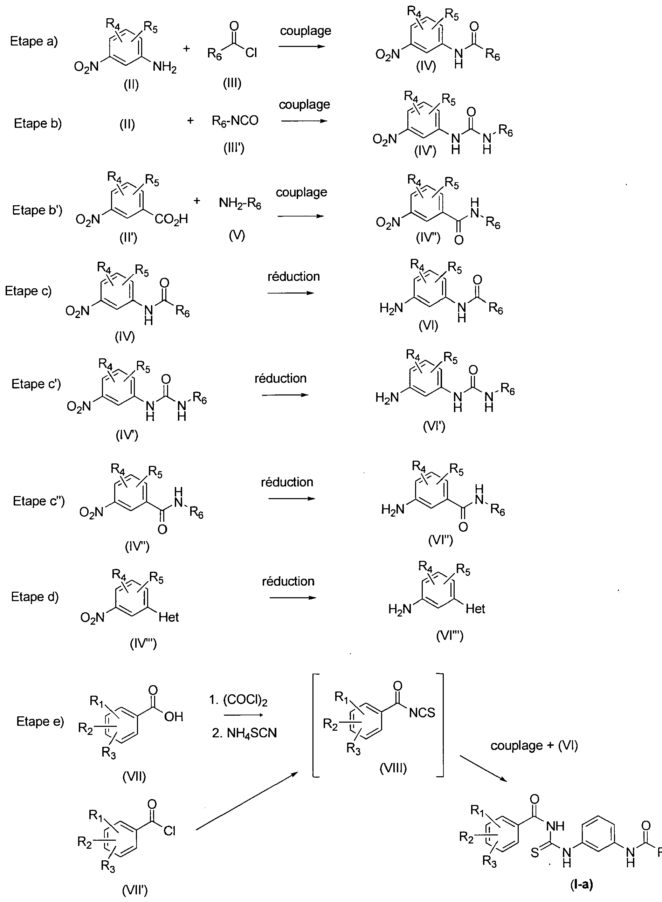

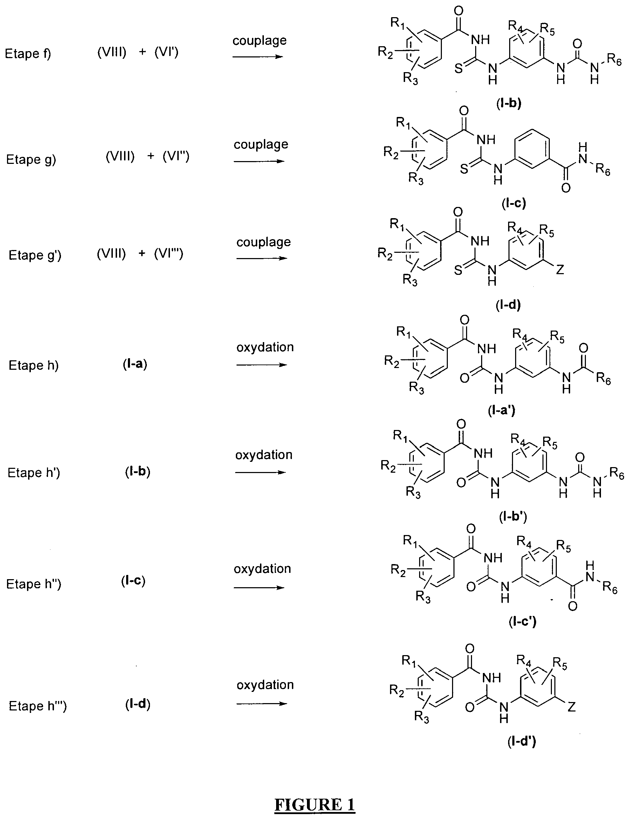

Le schéma de synthèse général des composés de formule (**I**) conforme à la description, dans leurs quatre variantes **(I-a)** ou **(I-a')**, **(I-b)** ou **(I-b')**, **(I-c)** ou **(I-c')**, **(I-d)** ou **(I-d')**, peut être représenté selon la figure 1 annexée.

Conformément au schéma de synthèse représenté sur la figure 1 annexée, dans une **étape a**), utile pour obtenir des composés de formule **(I-a)** ou **(I-a')**, on condense une 3-nitroaniline commerciale de formule (**II**) dans laquelle les radicaux R₄ et R₅ ont les mêmes significations que celles indiquées ci-dessus, avec un chlorure d'acide de formule **(III)** dans laquelle R₆ a la même signification que celle indiquée ci-dessus, par exemple selon la méthode de Schotten-Baumann, pour obtenir le composé amide de formule **(IV)** correspondant. L'**étape b)** permet un couplage entre une 3-nitroaniline commerciale de formule (**II**) dans laquelle les radicaux R₄ et R₅ ont les mêmes significations que celles indiquées ci-dessus, avec un isocyanate commercial **(III')**, pour obtenir une nitro-urée de formule **(IV')** pouvant conduire aux composés (**I-b)** et **(I-b')**. L'**étape b')** consiste à condenser un acide 3-nitrobenzoïque commercial de formule **(II')** avec une amine de formule **(V),** dans lesquelles R₄, R₅ et R₆ ont les mêmes significations que celles indiquées ci-dessus, pour obtenir le composé nitro-amide de formule **(IV").**

D'autres méthodes conventionnelles bien connues de l'homme du métier pour former une liaison amide peuvent être également utilisées pour réaliser ces **étapes a), b)** et **b')** de condensation.

Dans les **étapes c), c'), c")** et **d),** le groupement nitro des composés de formule **(IV)**, **(IV')** ou **(IV")** est réduit en amine pour obtenir respectivement les anilines de formules **(VI)**, **(VI')** ou **(VI")**. Le composé 3-nitroaromatique (**IV'''**) obtenu lors de **l'étape d)** possédant un groupement hétéroaryle Z mono- ou polycyclique, substitué ou non substitué, et des radicaux R₄ et R₅ ayant les mêmes significations que celles indiquées ci-dessus, sont obtenus selon des procédés connus de l'homme de l'art (Yang et al., Angew. Chem. Int. Ed. 2008, 47, 1473 ; Burkholder et al., Tetrahedron Lett. 2001, 42, 3077 ; Zhang et al., J. Org. Chem., 2005, 70, 5164 ; Aggarwal et al., Synth. Comm. 2006, 36, 875 ; Rubin *et al.,* Demande Internationale PCT WO 2006/050506 au nom de CURIS), avant de subir une réduction pour obtenir les anilines correspondantes **(VI''')**. Cette étape de réduction peut être réalisée en milieu réducteur, par exemple par action d'un agent réducteur tel que le dichlorure de plomb ou le dichlorure d'étain, ou bien encore par hydrogénation, en utilisant par exemple une activation par les micro-ondes. D'autres méthodes d'hydrogénation peuvent être également utilisées en fonction de la nature des substituants R₄ et R₅ éventuellement présents sur le cycle phényle. A cet égard, lorsque R₄ et/ou R₅ représentent un atome d'halogène tel que le chlore, le brome, ou l'iode, l'étape de réduction est de préférence réalisée par action du dichlorure d'étain. Dans tous les autres cas, on préfère réaliser une hydrogénation catalytique en présence de Pd/C ou de Nickel de Raney.

Au cours des **étapes e)** et **f)**, on prépare un acylisothiocyanate de formule **(VIII)** dans laquelle les radicaux R₁ à R₃ ont la même signification que celle indiquée ci-dessus pour les composés de formule **(I)**, à partir d'un acide benzoïque de formule **(VII)** ou d'un chlorure d'acide benzoïque de formule **(VII')** correspondant, par exemple dans un milieu solvant au reflux (acétonitrile ou acétone) en présence par exemple de phosgène et de thiocyanate d'ammonium. Le composé de formule **(VIII)**, un benzoylisothiocyanate, ainsi obtenu est ensuite couplé à un composé de formule **(VI)** ou de formule **(VI')** pour conduire aux composés de formule **(I)** correspondants dans lesquels X = S (acylthiourés), i.e. aux composés de formules **(I-a)** et **(I-b)**. Le même benzoylisocyanate **(VIII)** est condensé au reflux dans un solvant, au cours des **étapes g)** et **g')**, avec les anilines **(VI")** et **(VI''')**, pour conduire aux composés de formule **(I-c)** et **(I-d)**. Généralement, les composés de formules **(I-a)** et **(I-b)** sont obtenus sous la forme de solides qui sont ensuite purifiés de façon classique par recristallisation dans un alcool (Rasmussen, C. R. et al.. Synthesis, 1988, 456-459).

Lorsque l'on souhaite obtenir un composé de formule **(I)** dans lequel X = O, on procède alors, dans une **étape h)**, **h')**, **h")** ou **h'''**) respectivement à l'oxydation d'un composé de formule **(I-a)**, **(I-b)**, **(I-c)** ou **(I-d)**, pour obtenir un composé de formule de formule **(I-a')**, **(I-b')**, **(I-c')** ou **(I-d')**. Cette étape d'oxydation peut être réalisée selon des méthodes bien connues de l'homme du métier, en utilisant par exemple, à titre d'agent oxydant du cuivre (Cu**(I)**) en milieu alcalin (Narasimhamurthy, K. A et al., Tetrahedron Lett., 1982, 27, 3911) ou bien d'autres agents oxydant tels que l'acide iodique (HIO₃) supporté sur résine (Yang, G et al., Green Chemistry, 2003, 5, 441-442). Lors de l'étape d'oxydation, une activation par irradiation aux microondes peut être réalisée pour améliorer l'oxydation.

Les composés de formules **(I-a')**, **(I-b')**, **(I-c')** ou **(I-d')** sont également obtenus sous la forme de solides qui peuvent ensuite être purifiés par recristallisation dans un alcool.

Un autre objet de la description concerne des dérivés d'acylthio-urées ou d'acyl-urées, nouveaux en tant que tels, couverts par le formule **(I)** définie ci-dessus, répondant à la formule **(I')** suivante (les composés de formule **(I')** atant une sous-famille des composés de formule (**I**)) : 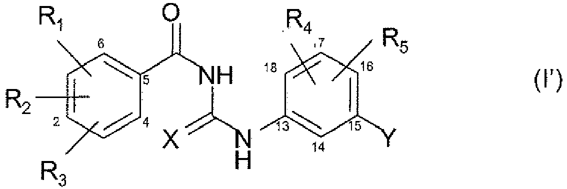 dans laquelle :
- R₁, R₂ et R₃, identiques ou différents et indépendamment les uns des autres, représentent un atome d'hydrogène ou d'halogène, un radical hydroxyle, un groupement alkyle, perfluoroalkyle, alcoxy, alkylthio, nitrile, un alcoxy substitué, ou un hérérocycle fusionné obtenue à partir de deux des radicaux R₁, R₂ et R₃, adjacents, pouvant former, conjointement avec les atomes de carbone du cycle phényle auxquels ils sont liés, un hétérocycle fusionné ;
- X représente un atome de soufre ou d'oxygène,
- Y représente un groupement hétéroaryle mono- ou polycyclique, substitué ou non substitué, un groupement -NH-(C=O)-R6 ou -NH-(C=O)-NH-R₆ dans lequel R₆ représente un groupement aryle non substitué ; un groupement aryle comportant un ou plusieurs substituants choisis parmi un atome d'halogène et un radical alkyle, alcoxy ou mono- ou dialkylamino ; un groupement hétéroaryle mono- ou polycyclique ; un radical alkyle linéaire ou ramifié ; un groupement hydrocarboné mono- ou polycyclique saturé ou insaturé ;
- R₄ et R₅, identiques ou différents et indépendamment l'un de l'autre, représentent un atome d'hydrogène ou d'halogène, un groupe alcoxy, alkylthio, alkyle, perfluoroalkyle, nitrile ou nitro ;
étant entendu que lorsque Y représente un groupement -NH-(C=O)-R₆ ou un groupement -NH-(C=O)-NH-R₆, X représente un atome de soufre.

Les composés de formule **(I')** conformes à la description présentent la propriété d'inhiber la voie de signalisation des protéines Hedgehog et peuvent donc être utilisés, à titre de principe actif, pour la préparation d'une composition pharmaceutique destinée aux traitements des pathologies associées à une hyperactivation de la voie de signalisation des protéines Hedgehog ou bien dans lesquelles une inhibition de cette voie présente un intérêt thérapeutique.

Par conséquent, la présente description a également pour objet les composés de formule **(I')** tels que définis précédemment, pour une utilisation à titre de médicament, en particulier :
i) à titre de médicament destiné au traitement des tumeurs associées à une hyperactivation de la voie de signalisation des protéines Hedgehog ; de telles tumeurs sont notamment les tumeurs du tissu nerveux (médulloblastomes, tumeurs primitives neuroectodermiques, glioblastomes, méningiomes et oligodendrogliomes), les tumeurs cutanées (carcinomes basocellulaires, trichoépithéliomes), les tumeurs des tissus musculaires et osseux (rhabdomyosarcomes, ostéosarcomes) et les tumeurs d'autres tissus (rein, vessie).
ii) à titre de médicament destiné au traitement des pathologies nécessitant un blocage de la voie Hedgehog, notamment des pathologies de type neuro-dégénératif comme la maladie de Parkinson, la Chorée de Huntington, la maladie d'Alzheimer, la sclérose en plaques et la maladie du motoneurone ou bien d'autres pathologies dans lesquelles le blocage de la voie de signalisation Hedgehog pourrait être bénéfique, comme le diabète.

La posologie utile variera en fonction de l'affection à traiter, de la voie et du rythme d'administration, ainsi que de la nature et du poids de l'espèce à traiter (humaine ou animale) ; elle peut varier par exemple de 1 mg à 2 g par jour chez l'adulte par voie orale.

Un autre objet de la présente description est une composition pharmaceutique, caractérisée par le fait qu'elle comprend, à titre de principe actif, au moins un composé de formule **(I')** tel que défini précédemment, et au moins un excipient pharmaceutiquement acceptable.

Au sein des compositions pharmaceutiques conformes à la description, le ou les composés de formule **(I')** sont de préférence utilisés en une quantité permettant d'administrer des doses unitaires comprises entre 1 mg et 2 g environ.

L'homme du métier choisira un ou plusieurs excipients pharmaceutiquement acceptables en fonction de la voie d'administration de la composition pharmaceutique. Bien entendu, l'homme de l'art veillera, à cette occasion, à ce que le ou les excipients utilisés soient compatibles avec les propriétés intrinsèques attachées à la composition conforme à la présente description.

En outre, la forme du médicament ou de la composition pharmaceutique (par exemple, une solution, une suspension, une émulsion, des comprimés, des gélules, des suppositoires, etc...) dépendra de la voie d'administration choisie.

Ainsi, au sens de la présente description, le médicament ou la composition pharmaceutique peut être administré par n'importe quelle voie appropriée, par exemple par la voie orale, anale, locale, systémique, intraveineuse, intramusculaire ou mucosale, ou bien en utilisant un patch, ou encore sous forme encapsulée dans, ou immobilisée sur, des liposomes, des microparticules, des microcapsules, et analogues.

On peut notamment citer, à titre d'exemples non limitatifs d'excipients appropriés pour une administration par voie orale, le talc, le lactose, l'amidon et ses dérivés, la cellulose et ses dérivés, les polyéthylèneglycols, les polymères d'acide acrylique, la gélatine, le stéarate de magnésium, des matières grasses animales, végétales ou synthétiques, les dérivés de la paraffine, les glycols, les stabilisants, les conservateurs, les anti-oxydants, les agents mouillants, les anti-agglomérants, les dispersants, les émulsionnants, les agents modifiants du goût, les agents de pénétrations, de solubilisation, etc...

Les techniques de formulation et d'administration des médicaments et compositions pharmaceutiques sont bien connues dans la technique ici considérée, l'homme du métier pouvant notamment se référer à l'ouvrage Remington's Pharmaceutical Sciences, dernière édition.

Outre les dispositions qui précèdent, la description comprend encore d'autres dispositions qui ressortiront des éléments qui vont suivre, qui se réfèrent à des exemples de synthèse des composés de la description, à un exemple de mise en oeuvre des composés de la description, ainsi qu'aux dessins annexés dans lesquels :
- la figure 1 illustre le schéma de synthèse général des composés de la description,
- la figure 2 illustre par des photographies prises au microscope à fluorescence (DMRXA2, Leica ; logiciel openlab3.1.2, improvision), l'inhibition de la liason de la Bodipycyclopamine par les composés 31 et 38 de la description, ainsi que par la Cyclopamine (Cy), un antagoniste de référence du récepteur Smoothened, et
- la figure 3 représente les courbes dose réponse obtenues pour les composés 31 et 38 de la description, et pour la Cyclopamine (Cy), sur la liaison de la Bodipycyclopamine.

### EXEMPLE 1 : SYNTHÈSES DE DIFFÉRENTS COMPOSÉS DE FORMULA (I)

Dans ces exemples, les réactions ont été conduites sous atmosphère de gaz inerte (azote) en utilisant des techniques de Schlenk (standard). Les solvants ont été séchés selon des méthodes standards et distillés sous azote avant utilisation. Tous les réactifs ont été obtenus dans le commerce et utilisés tels quels sans purification préalable.

Les spectrométries de masse (ESI+) ont été enregistrées sur un spectromètre LC/MSD vendu sous la référence Agilent^{®} 1100. Les spectres de résonance magnétique nucléaire (RMN) ont été enregistré sur un appareil Bruker^{®} AC200 à 200 MHz (¹H) ou sur un appareil Bruker^{®} AC400 à 400 MHz (¹H) ou à 100 MH_{z} (¹³C).

### 1) Synthèse des intermédiaires de formule (IV)

Des intermédiaires de formule **(IV)** telle que définie ci-dessus ont tous été synthétisés selon le principe qui est détaillé en particulier ci-après pour le 4-méthoxy-*N*-(3-nitrophényl)benzamide de formule **(IV-1)** suivante :

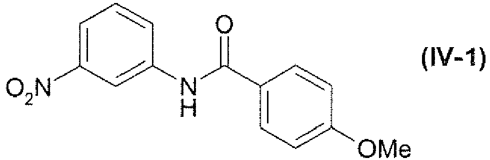

0,88 g (1,21 mL, 8,69 mmol) de triéthylamine ont été ajoutés au goutte à goutte dans une solution de 3-nitroaniline (1 g, 7,24 mmol) dans 10 ml de dichlorométhane (DCM) anhydre. On a ensuite ajouté à la solution résultante, au goutte à goutte et en 5 minutes sur bain de glace, 5 ml d'une solution de chlorure de 4-méthoxybenzoyle (1,48 g, 1,19 mL, 8,69 mmol) dans le DCM anhydre. Le milieu réactionnel a été mélangé à température ambiante pendant 150 minutes ; la réaction a été suivie par chromatographie sur couche mince (CCM) en utilisant comme éluant un mélange éther de pétrole/acétate d'éthyle (AcOEt) 1/2 (v/v). Après disparition de la 3-nitroaniline, la solution a été versée dans 5 ml d'acide chlorhydrique (HCl) 1N. Le précipité résultant a été lavé avec de l'eau et cristallisé dans le méthanol. On a ainsi obtenu 1,45 g du composé de formule **(IV-1)** attendu sous la forme d'un solide brun (rendement 74 %).
**[ES/MS]** m/z 273 [M+1]⁺, 295 [M+Na]⁺
**¹H-RMN 400 MHz** (CDCl₃) δ (ppm) : 8,45 (s, 1H) ; 8,07 (d, = 8,4 Hz, 2H) ; 7,96 (dd, *J1* = 8 Hz, *J2* = 1,2 Hz, 1H) ; 7,85 (d, *J* = 8,8 Hz, 2H) ; 7,51 (t, *J* = 8 Hz, 1H) ; 6,97 (d, *J* = 8,4 Hz, 2H) ; 3,86 (s, 3H).

Les analyses obtenues pour les autres composés de formule **(IV)**, synthétisés par analogie selon le procédé détaillé ci-dessus pour le composé (**IV-1**), sont données ci-aprés :
**Composé (IV-3) : 3-méthoxy-*N*-(3-nitrophényl)benzamide :**
   Solide brun, rendement 71 %.
   **[ES/MS]** m/z 273 [M+1]⁺, 295 [M+Na]⁺
   **¹H-RMN 400 MHz** (CDCl₃) δ (ppm) : 8,48 (s, 1H) ; 8,28 (s, 1H) ; 8,05 (d, *J* = 8 Hz, 1H) ; 7,95 (d, *J* = 7,6 Hz, 1H) ; 7,49 (t, *J* = 8 Hz, 1H) ; 7,41-7,35 (m, 4H) ; 3,83 (s, 3H).
**Composé (IV-4) : 2-Méthoxy-*N*-(3-nitrophényl)benzamide :**
   Solide jaune pale, rendement 75 %
   **[ES/MS]** m/z 273 [M+1]⁺, 295 [M+Na]⁺
   **¹H-RMN 400 MHz** (CDCl₃) δ (ppm) : 8,46 (s, 1H) ; 8,27 (dd, *J1* = 8 Hz, *J2* = 1,6 Hz, 1H) ; 8,11 (dd, *J1* = 8 Hz, *J2* = 1,6 Hz, 1H) ; 7,94 (dd, *J1* = 8,8 Hz, *J2* = 1,6 Hz, 1H) ; 7,54-7,47 (m, 3H) ; 7,14 (t, *J* = 7,6 Hz, 1H) ; 7,04 (d, *J* = 8,4 Hz, 1H) ; 4,08 (s, 3H).
**Composé (IV-13): 4-chloro-*N*-(3-nitrophényl)benzamide :**
   Solide jaune, rendement 94 %.
   **[ES/MS]** m/z 299 [M+Na]⁺
   **¹H-RMN 400 MHz** (CDCl₃) δ (ppm) : 8,48 (s, 1H) ; 8,07 (d, *J* = 8,4 Hz, 1H) ; 7,99 (d, *J* = 8 Hz, 1H) ; 7,83 (d, *J* = 8 Hz, 2H) ; 7,55-7,41 (m, 4H).
**Composé (IV-32) : *N*-(2-méthyl-3-nitrophényl)benzamide :**
   Rendement 70 %, point de fusion = 132°C.
   **¹H-RMN 200 MHz** (DMSO, d6) δ (ppm) : 10,49 (s, 1 H) ; 8,20 (d, *J* = 8 Hz, 2H) ; 8,03-7,67 (m, 6H) ; 2,50 (s, 3H).

### 2) Synthèse des intermédiaires de formule (VI)

Des intermédiaires de formule **(VI)** telle que décrite précédemment ont tous été synthétisés, à partir des composés de formule **(IV)** correspondants, selon le principe qui est détaillé en particulier ci-après pour le 4-méthoxy-*N*-(3-aminophényl)benzamide de formule **(VI-1)** suivante :

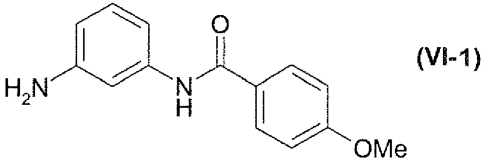

Dans un tube à micro-ondes, on a dissous 200 mg (0,74 mmol) de 4-méthoxy-*N*-(3-nitrophényl)benzamide (200 mg, dans 4 ml de méthanol anhydre. On a ensuite ajouté à la solution ainsi obtenue, 185 mg (2,94 mmol) de formate d'ammonium et 18,5 mg de Pd/C à 10 % à titre de catalyseur. Le milieu réactionnel a été ensuite irradié dans un four à micro-ondes pendant 4 minutes à 80°C. On a ainsi réalisé deux cycles à une puissance de 50 W, tout en suivant la réaction par CCM en utilisant un mélange éther de pétrole /AcOEt (1/2 : v/v) comme éluant.

Après élimination du catalyseur par filtration, et un lavage prolongé à l'eau puis au méthanol, la solution a été évaporée sous vide. Le résidu a été repris dans un mélange composé de carbonate de sodium (Na₂CO₃) saturé et d'acétate d'éthyle. La phase organique a été lavée avec une solution de chlorure de sodium saturé et séchée sur sulfate de sodium Na₂SO₄.

Après élimination du solvant sous vide, on a obtenu 376 mg de 4-méthoxy-*N*-(3-nitrophényl)benzamide de formule **(VI-1)** attendu sous la forme d'un solide (rendement 75 %). Le produit a ensuite été engagé dans les réactions suivantes sans purification supplémentaire.
**[ES/MS]** m/z 243 [M+1]⁺, 265 [M+Na]⁺
**¹H-RMN 400 MHz** (CDCl₃) δ (ppm) : 7,79 (d, *J* = 8,4 Hz, 2H) ; 7,68 (s, 1H) ; 7,28 (s, 1H) ; 7,08 (t, *J* = 8 Hz, 1H) ; 6,93 (d, *J* = 8,4 Hz, 2H) ; 6,75 (d, *J* = 8 Hz, 1H) ; 6,43 (d, *J* = 8 Hz, 1H) ; 3,84 (s, 3H).

Les analyses obtenues pour les autres composés de formule **(VI)**, synthétisés par analogie selon le procédé détaillé ci-dessus pour le composé **(VI-1)**, sont données ci-après :
**Composé (VI-3) : 3-méthoxy-*N*-(3-aminophényl)benzamide :**
   Solide brun, rendement 86 %.
   **[ES/MS]** m/z 243 [M+1]⁺, 265 [M+Na]⁺, 507 [2M+Na]⁺
   **¹H-RMN 400 MHz** (CDCl₃) δ (ppm) : 7,68 (s, 1H) ; 7,40-7,35 (m, 3H) ; 7,29 (s, 1H) ; 7,12-7.04 (m, 2H) ; 6,76 (d, *J* = 7,6 Hz, 1H) ; 6,46 (dd, *J1* = 8,4 Hz, *J2* = 2 Hz, 1H) ; 3,85 (s, 3H).
**Composé (VI-4) : 2-méthoxy-*N*-(3-aminophényl)benzamide :**
   Solide brun, rendement 70 %.
   **[ES/MS]** m/z 243 [M+1]⁺, 265 [M+Na]⁺, 507 [2M+Na]⁺
**Composé (VI-5) : *N*-(3-aminophényl)biphényl-4-carboxamide :**
   Solide brun, rendement 98 %.
   **[ES/MS]** m/z 289 [M+1]⁺, 312 [M+Na]⁺, 599 [2M+Na]⁺
   **¹H-RMN 400 MHz** (CDCl₃) δ (ppm) : 7,92 (d, *J* = 8 Hz, 2H) ; 7,70-7,60 (m, 5H) ; 7,48-7,33 (m, 4H) ; 7,12 (t, *J* = 8 Hz, 1H) ; 6,78 (d, *J* = 8 Hz, 1H) ; 6,47 (dd, *J1* = 8 Hz, *J2* = 1,6 Hz, 1H).

### 3) Synthèse d'intermédiaires chlorés de formule (VI)

Des intermédiaires chlorés de formule **(VI)** telle que décrite précédemment ont tous été synthétisés, à partir des composés chlorés de formule **(IV)** correspondants, selon le principe qui est détaillé en particulier ci-après pour le 4-chloro-*N*-(3-aminophényl)benzamide de formule **(VI-2)** suivante :

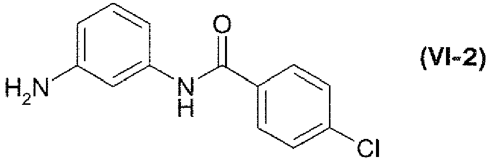

500 mg (1,81 mmol) de 4-chloro-*N*-(3-nitrophényl)benzamide ont été dissous dans 50 ml d'éthanol anhydre. Lorsque la dissolution a été complète, on a ajouté 0,3 ml (3,62 mmol) d'HCl concentré et 1 g (5,43 mmol) de SnCl₂. Le mélange réactionnel a ensuite été chauffé à 60 °C pendant 5 heures, en suivant la formation du produit attendu par CCM en utilisant comme éluant un mélange éther de pétrole/AcOEt (1/2, v/v). Lorsque la réaction a été terminée, on a ajouté au milieu réactionnel une solution saturée de NaHCO₃. Après élimination de l'éthanol sous vide, le milieu réactionnel a été extrait à l'acétate d'éthyle. Après filtration, les phases organiques ont été séchées sur Na₂SO₄, On a obtenu 440 mg de composé de formule **(VI-2)** (rendement 94%), sous la forme d'un solide brun. Ce composé a ensuite été engagé dans les étapes suivantes de synthèse sans purification supplémentaire.
**[ES/MS]** m/z 247 [M+1]⁺, 269 [M+Na]⁺

L'analyse obtenue pour un autre composé de formule **(VI),** synthétisé par analogie selon le procédé détaillé ci-dessus pour le composé **(VI-2),** est donnée ci-après :
**Composé (VI-13) : 4-chloro-*N*-(3-aminophényl)benzamide :**
   Solide brun, rendement 94 %.
   **[ES/MS]** m/z 247 [M+1]⁺, 269 [M+Na]⁺

### 4) Synthèse de composés de formule (I-a)

Des composés de formule **(I**-**a)** telle que décrite précédemment ont tous été synthétisés, à partir des composés de formule **(VII')** et **(VI)** correspondants, selon le principe qui est détaillé en particulier ci-après pour un composé de formule **(I-a)** particulier, le N-[[[3-(4-méthoxy-benzoylamino)phényl]amino]thioxométhyl]-3,4,5-triméthoxy-benzamide de formule suivante :

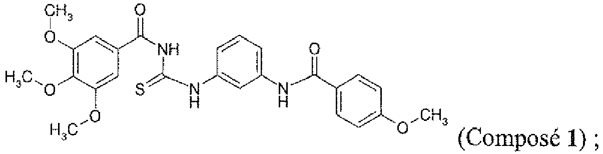

125 mg (1,63 mmol) de thiocyanate d'ammonium thiocyanate ont été dissous dans 4 ml d'acétone anhydre. Après mélange, on a ensuite ajouté à cette solution 330 mg (1,36 mmol) de chlorure 3,4,5-triméthoxybenzoyle (composé de formule **(VII')**, puis le mélange réactionnel a été porté au reflux pendant 20 minutes. Après que la solution soit devenue claire, on a ajouté 314 mg (1,35 mmol) de 4-méthoxy-*N*-(3-aminophényl)benzamide (composé de formule **(VI-1)** tel que préparé ci-dessus), puis le milieu réactionnel a été porté au reflux pendant 60 minutes. La réaction a été suivie par CCM en utilisant comme éluant un mélange éther de pétrole/AcOEt (1/4 : v/v). Après disparition complète du composé de formule **(VI-1)** de départ, le milieu réactionnel a été versé dans un bécher contenant de la glace. Un précipité s'est formé. Le précipité a ensuite été filtré, puis recristallisé du méthanol pour conduire à 357 mg du composé **(1)** attendu sous la forme d'un solide jaune brillant (rendement de 53%).
**[ES/MS]** m/z 496 [M+1]⁺, 518 [M+Na]⁺, 1013 [2M+Na]⁺
**¹H-RMN 400 MHz** (CDCl₃) δ (ppm) : 8,97 (s, 1H) ; 8,19 (s, 1H) ; 7,83 (d, *J* = 8 Hz, 2H) ; 7,77 (s, 1H) ; 7,65-7.55 (m, 1H) ; 7,40-7.39 (m, 2H) ; 7,07 (s, 2H) ; 6,97 (d, *J* = 8,8 Hz, 2H) ; 3,93 (s, 9H); 3,86 (s, 3H).

D'autres composés de formule **(I-a)** ont ainsi été synthétisés à partir des intermédiaires correspondants :
**Composé (2) : N-[[[3-(2-chloro-benzoylamino)phényl]amino] thioxométhyl]-3,4,5-triméthoxy-benzamide** :
   Solide jaune brillant, rendement 70%.
   **[ES/MS]** m/z 501 [M+1]⁺, 521 [M+Na]⁺, 1022 [2M+Na]⁺
   **¹H-RMN 400 MHz** (CDCl₃) δ (ppm) : 8,96 (s, 1H) ; 8,17 (s, 1H) ; 7,92 (s, 1H) ; 7,68 (d, *J* = 8 Hz, 1H) ; 7,60 (d, *J* = 8 Hz, 1H) ; 7,47-7.40 (m, 5H) ; 7,06 (s, 2H) ; 3,93 (s, 9H).
**Composé (3) N-[[[3-(3-méthoxy-benzoylamino)phényl]amino] thioxométhyl]**-**3,4,5**-**triméthoxy**-**benzamide :**
   Solide jaune pale, rendement 70 %.
   **[ES/MS]** m/z 496 [M+1]⁺, 518 [M+Na]⁺, 1013 [2M+Na]⁺
   **¹H-RMN 400 MHz** (CDCl₃) δ (ppm) : 9,00 (s, 1H) ; 8,20 (s, 1H) ; 7,88 (s, 1H) ; 7,59 (bd, 1H) ; 7,43-7,37 (m, 5H) ; 7,07 (s, 3H) ; 3,93 (s, 9H) ; 3,86 (s, 3H).
**Composé (4) : N-[[[3-(2-méthoxy-benzoylamino)phényl]amino] thioxométhyl]-3,4,5-triméthoxy-benzamide** :
   Solide brun, rendement 71 %.
   **[ES/MS]** m/z 496 [M+1]⁺, 518 [M+Na]⁺, 1013 [2M+Na]⁺
   **¹H-RMN 400 MHz** (CDCl₃) δ (ppm) : 9,88 (s, 1H) ; 9,02 (s, 1H) ; 8,27 (d, *J* = 7,6 Hz, 1H) ; 8,16 (s, 1H) ; 7,60 (d, *J* = 8 Hz, 1H) ; 7,49-7,40 (m, 2H) ; 7,39 (t, *J* = 8,4 Hz, 1H) ; 7,13-7,02 (m, 4H) ; 4,06 (s, 3H) ; 3,93 (s, 9H).
**Composé (13) : N-[[[3-(4-chloro-benzoylamino)phényl]amino] thioxométhyl]-3**,**4**,**5**-**triméthoxy**-**benzamide :**
   Solide blanc, rendement 70 %.
   **[ES/MS]** m/z 521 [M+Na]⁺
   **¹H-RMN 400 MHz** (CDCl₃) δ (ppm) : 8,97 (s, 1H) ; 8,21 (s, 1H) ; 7,84-7,80 (m, 3H) ; 7,58 (bd, 1H) ; 7,47-7,41 (m, 4H) ; 7,06 (s, 2H) ; 3,93 (s, 9H).

Les autres composés de type **(I-b)**, **(I-c)** et **(I-d)** sont obtenus de manière analogue.

### 5) Synthèse de composés de formule (I-a')

Les différents composés de formule **(I-a')** ont été obtenus par oxydation des composés de formule **(I-a)** correspondants.

On détaille ci-après la préparation du N-[[[3-(4-méthoxy-benzoylamino)phényl]amino]oxométhyl]-3,4,5-triméthoxy-benzamide (composé **(6)**) à partir du composé **(1).**

100 mg (0,20 mmol) de composé **(1),** tel que préparé ci-dessus, ont été mis en suspension dans 2 ml d'acétonitrile (MeCN). Cette suspension a été ajoutée au goutte à goutte à 2 ml d'une solution de chlorure de cuivre (20 mg, 0.2 mmol) dans l'acétonitrile préalablement préparée dans un tube à micro-ondes. On a ensuite ajouté au mélange ainsi obtenu 80 µl (0,2 mmol) d'une solution de soude 2,5 M.

Le tube a ensuite été placé dans un four à micro-ondes et soumis à deux cycles successifs d'irradiations selon le régime suivant :
- Pression : 350 K Pa,
- Puissance : 200 W,
- Température 120 °C,
- Temps d'ajustement de l'ensemble des paramètres : 1 min.,
- Temps de maintien des paramètres : 6 min.

A la fin de la réaction, le produit brut obtenu a été dilué avec du dichlorométhane, la phase organique (de couleur bleue) a été lavée avec une solution aqueuse d'ammoniaque à 30 % (v/v) jusqu'à disparition de la couleur bleue dans la phase aqueuse. La phase organique a ensuite été séchée, filtrée, puis évaporée sous vide pour conduire à un produit brut qui a été purifié par "flash" chromatographie en utilisant comme éluant un mélange éther de pétrole/acétate d'éthyle dans une proportion allant progressivement de 95/5 à 1/1. On a obtenu 80 mg du composé (6) attendu sous la forme d'un solide brun, avec un rendement de 72 %.
**[ES/MS]** m/z 521 [M+Na]⁺
**¹H-RMN 400 MHz** (CDCl₃) δ (ppm) : 8,77 (s, 1H) ; 8,18 (s, 1H) ; 7,80 (d, *J* = 8 Hz, 2H) ; 7,71 (s, 1H) ; 7,65-7,50 (m, 1H) ; 7,40-7,30 (m, 2H) ; 7,00 (s, 2H) ; 6,90 (d, *J* = 8 Hz, 2H) ; 3,90 (s, 9H) ; 3,85 (s, 3H).

Les autres composés de type **(I**-**b**'**)**, **(I-c')** et **(I-d')** sont obtenus de manière analogue.

### Paramètres physiques de composés de formule (I) conformes à la description :

- **Composé (1) :** N-[[[3-(4-méthoxy-benzoylamino)phényl]amino]thioxométhyl]-3,4,5-triméthoxy-benzamide (C₂₅H₂₅N₃O₆S) :
   Poids Moléculaire (PM) = 495 ; **[ES/MS]** m/z 518 [M+Na]⁺ ; Point de Fusion (pf)=147°C.
- **Composé (2) :** N-[[[3-(2-chloro-benzoylamino)phényl]amino]thioxométhyl]-3,4,5-triméthoxy-benzamide (C₂₄H₂₂ClN₃O₅) :
   PM = 499 ; **[ES/MS]** m/z 522 [M+Na]⁺ ; pf = 178°C.
- **Composé (3) :** N-[[[3-(3-méthoxy-benzoylamino)phényl]amino]thioxométhyl]-3,4,5-triméthoxy-benzamide (C₂₅H₂₅N₃O₆S) :
   PM = 495 ; **[ES/MS]** m/z 518 [M+Na]⁺ ; pf = 121°C.
- **Composé (4) :** N-[[[3-(2-méthoxy-benzoylamino)phényl]amino]thioxométhyl]-3,4,5-triméthoxy-benzamide (C₂₅H₂₅N₃O₆S) :
   PM = 495 ; **[ES/MS]** m/z 518 [M+Na]⁺ ; pf = 137°C.
- **Composé (5) :** N-[[[3-(4-phényl-benzoylamino)phényl]amino]thioxométhyl]-3,4,5-triméthoxy-benzamide (C₃₀H₂₇N₃O₅S) :
   PM = 541 ; **[ES/MS]** m/z 542 [M+H]⁺ 564 [M+Na]⁺ ; pf = 144°C.
- **Composé (6):** N-[[[3-(4-méthoxy-benzoylamino)phényl]amino]oxométhyl]-3,4,5-triméthoxy-benzamide (C₂₅H₂₅N₃O₆S) :
   PM = 479 ; **[ES/MS]** m/z 502 [M+Na]⁺; pf = 167°C.
- **Composé (7)** : N-[[[3-(4-phényl-benzoylamino)phényl]amino]oxométhyl]-3,4,5-triméthoxy-benzamide (C₃₀H₂₇N₃O₆) :
   PM = 525 ; **[ES/MS]** m/z 548 [M+Na]⁺ ; pf = 180°C (avec décomposition).
- **Composé (8) :** N-[[[3-(-cyclohexylcarboxylamino)phényl]amino]thioxométhyl]-3,4,5-triméthoxy-benzamide (C₂₄H₂₉N₃O₅S) :
   PM = 471 ; **[ES/MS]** m/z 494 [M+Na]⁺ ; pf = 67°C.
- **Composé (9)** : N-[[[3-(2-naphtoylamino)phényl]amino]thioxométhyl]-3,4,5-triméthoxy-benzamide (C₂₈H₂₅N₃O₅S) :
   PM = 515 ; **[ES/MS]** m/z 538 [M+Na]⁺ ; pf = 140°C
- **Composé (10) :** N-[[[3-(isopropoylcarboxylamino)phényl]amino]thioxométhyl]-3,4,5-triméthoxy-benzamide (C₂₁H₂₅N₃O₅S) :
   PM = 431 ; **[ES/MS]** m/z 454 [M+Na]⁺ ; pf = 102°C (avec décomposition).
- **Composé (11)** : N-[[[3-(4-pyridocarboxylamino)phényl]amino]thioxométhyl]-3,4,5-triméthoxy-benzamide (C₂₃H₂₂N₄O₅S) :
   PM = 466 ; **[ES/MS]** 467[M+H]⁺ m/z 489 [M+Na]⁺ ; pf= 187°C (avec décomposition).
- **Composé (12)** : N-[[[3-(1-naphtoylamino)phényl]amino]thioxométhyl]-3,4,5-triméthoxy-benzamide (C₂₈H₂₅N₃O₅S) :
   PM = 515 ; **[ES/MS]** m/z 538 [M+Na]⁺ ; pf= 139°C.
- **Composé (13) :** N-[[[3-(4-chloro-benzoylamino)phényl]amino]thioxométhyl]- 3,4,5-triméthoxy-benzamide (C₂₄H₂₂ClN₃OS) :
   PM = 499 ; **[ES/MS]** m/z 522 [M+Na]⁺ ; pf = 151°C.
- **Composé (14) :** N-[[[3-(2-furoylamino)phényl]amino]thioxométhyl]-3,4,5-triméthoxy-benzamide (C₂₂H₂₁N₃O₆S) :
   PM = 455 ; **[ES/MS]** m/z 456 [M+H]⁺ 478 [M+Na]⁺ ; pf= 94°C.
- **Composé (15)** : N-[[[3-(2-thiophénoylamino)phényl]amino]thioxométhyl]-3,4,5-triméthoxy-benzamide (C₂₂H₂₁N₃O₅S₂) :
   PM = 471 ; **[ES/MS]** m/z 472[M+H]⁺ 494 [M+Na]⁺ ; pf = >150°C (avec décomposition).
- **Composé (16) :** N-[[[3-(2-naphtoylamino)phényl]amino]oxométhyl]-3,4,5-triméthoxy-benzamide (C₂₈H₂₅N₃O₆) :
   PM = 499 ; **[ES/MS]** m/z 522 [M+Na]⁺ ; pf= 188°C.
- **Composé (17) :** N-[[[3-(isopropoylcarboxylamino))phényl]amino]oxométhyl]-3,4,5-triméthoxy-benzamide (C₂₁H₂₅N₃O₆) :
   PM = 415 ; **[ES/MS]** m/z 438 [M+Na]⁺.
- **Composé (18) :** N-[[[3-(benzoylamino)phényl]amino]thioxométhyl]-3,5-diméthoxy-benzamide (C₂₃H₂₁N₃O₄S) :
   PM = 435 ; **[ES/MS]** m/z 436 [M+1]⁺, pf = 194°C.
- **Composé (19) :** N-[[[3-(benzoylamino)phényl]amino]thioxométhyl]-3,5-diméthoxy-4-éthoxy-benzamide (C₂₅H₂₅N₃O₅S) :
   PM = 479 ; **[ES/MS]** m/z 480 [M+1]⁺; pf= 192°C.
- **Composé (20) :** N-[[[3-(benzoylamino)phényl]amino]thioxométhyl]-3,4-diéthoxy-5-méthoxy-benzamide (C₂₆H₂₇N₃O₅S) :
   PM = 493 ; **[ES/MS]** m/z 494 [M+1]⁺; pf = 172°C.
- **Composé (21)** : N-[[[3-(benzoylamino)phényl]amino]thioxométhyl]-3,4-méthylènedioxy-5-méthoxy-benzamide (C₂₃H₁₉N₃O₅S) :
   PM = 449 ; **[ES/MS]** m/z 450 [M+1]⁺; pf = 206°C.
- **Composé (22) :** N-[[[3-(benzoylamino)phényl]amino]thioxométhyl]-2,4-diméthoxy-benzamide (C₂₃H₂₁N₃O₄S) :
   PM = 435 ; **[ES/MS]** m/z 436 [M+1]⁺; pf 210°C.
- **Composé (23) :** N-[[[3-(benzoylamino)phényl]amino]thioxométhyl]-3,4,5-triéthoxy-benzamide (C₂₇H₂₉N₃O₅S) :
   PM = 507 ; **[ES/MS]** m/z 508 [M+1]⁺; pf = 176°C.
- **Composé (24) :** N-[[[4-fluoro-3-(benzoylamino)phényl]amino]thioxométhyl]-3,4,5-triméthoxy-benzamide (C₂₄H₂₂FN₃O₅S) :
   PM = 483 ; **[ES/MS]** m/z 484 [M+1]⁺; pf = 178°C.
- **Composé (25) :** N-[[[4-chloro-3-(phénylamino)carbonyl]amino]thioxométhyl]-3,4,5-triméthoxy-benzamide (C₂₄H₂₂N₃O₅S) :
   PM = 499 ; **[ES/MS]** m/z 500 [M+1]⁺; pf 174°C.
- **Composé (26) :** N-[[[4-méthoxy-3-(benzoylamino)phényl]amino]thioxométhyl]-3,4,5-triméthoxy-benzamide (C₂₅H₂₅N₃O₆S) :
   PM = 495 ; **[ES/MS]** m/z 496 [M+1]⁺; pf = 186°C.
- **Composé (27) :** N-[[[4-méthyl-3-(benzoylamino)phényl]amino]thioxométhyl]-3,4,5-triméthoxy-benzamide (C₂₅H₂₅N₃O₅S) :
   PM = 479 ; **[ES/MS]** m/z 480 [M+1]⁺; pf = 190°C.
- **Composé (28)** : N-[[[3-(benzoylamino)phényl]amino]thioxométhyl]-3,4-éthylènedioxo-benzamide (C₂₃H₁₉N₃O₄S) :
   PM = 433 ; **[ES/MS]** m/z 434 [M+1]⁺; pf = 206°C.
- **Composé (29)** : N-[[[3-(benzoylamino)phényl]amino]oxométhyl]-3,4,5-triéthoxy-benzamide (C₂₇H₂₉N₃O₆) :
   PM = 435 ; **[ES/MS]** m/z 436 [M+1]⁺; pf= 220°C.
- **Composé (30)** : N-[[[3-(benzoylamino)phényl]amino]oxométhyl]-3,4-méthylènedioxy-5-méthoxy-benzamide (C₂₃H₁₉N₃O₆) :
   PM = 433 ; **[ES/MS]** m/z 434 [M+1]⁺; pf = 252°C.
- **Composé (31) :** N-[[[3-(benzoylamino)phényl]amino]oxométhyl]-3,4,5-triméthoxy-benzamide (C₂₄H₂₃N₃O₆) :
   PM - 449 ; **[ES/MS]** m/z 450 [M+1]⁺; pf = 216°C.
- **Composé (32) :** N-[[[3-méthyl-3-(benzoylamino)phényl]amino]thioxométhyl]-3,4,5-triméthoxy-benzamide (C₂₅H₂₅N₃O₅S) :
   PM = 479 ; **[ES/MS]** m/z 480 [M+1]⁺; pf = 207°C.
- **Composé (33)** : N-[[[4-chloro-3-(benzoylamino)phényl]amino]oxométhyl]-3,4,5-triméthoxy-benzamide (C₂₄H₂₂ClN₃O₆) :
   PM = 483 ; **[ES/MS]** m/z 484 [M+1]⁺, pf = 244°C.
- **Composé (34) :** N-[[[4-chloro-3-(benzoylamino)phényl]amino]thioxométhyl]-3,4,5-triméthoxy-benzamide (C₂₄H₂₂ClN₃O₆S) :
   PM = 499 ; **[ES/MS]** m/z 500 [M+1]⁺; pf = 174°C.
- **Composé (35) :** N-[[[5-chloro-3-(benzoylamino)phényl]amino]thioxométhyl]-3,4,5-triméthoxy-benzamide (C₂₄H₂₂ClN₃O₆S) :
   PM = 499 ; **[ES/MS]** m/z 500 [M+1]⁺; pf = 164°C.
- **Composé (36) :** N-[[[5-fluoro-3-(benzoylamino)phényl]amino]thioxométhyl]-3,4,5-triméthoxy-benzamide (C₂₄H₂₂FN₃O₅S) :
   PM = 483 ; **[ES/MS]** m/z 484 [M+1]⁺; pf = 153°C.
- **Composé (37)** : N-[[[4-Chloro-3-(4-phényl-benzoylamino)phenyl]amino] oxométhyl]-3,4,5-triméthoxy-benzamide (C₃₀H₂₆ClN₃O₆) :
   PM = 560 ; **[ES/MS]** m/z 561 [M+H]⁺ ; pf = 144°C.
- **Composé (38) :** N-[[[3-(benzoylamino)phényl]amino]thioxométhyl]-3,4,5-triméthoxy-benzamide (C₂₄H₂₃N₃O₅S) :
   PM = 465, **[ES/MS]** m/z 466 [M+H]⁺ ; pf = 164°C.
- **Composé (39)** : N-[[[4-chloro-3-(4-diméthylamino-phénylamino)carbonyl] thioxométhyl]-3,4,5-triméthoxy-benzamide (C₂₆H₂₇N₄ClO₅S) :
   PM = 543 ; **[ES/MS]** m/z 544 [M+H]⁺ ; pf = 201°C.
- **Composé (40) :** N-[[[4-chloro-3-(4-méthoxy-phénylamino)carbonyl]thioxométhyl]-3,4,5-triméthoxy-benzamide (C₂₅H₂₄N₃ClO₆S) :
   PM = 530 ; **[ES/MS]** m/z 531 [M+H]⁺ ; pf= 165°C.
- **Composé (41)** : N-[[[5-bromo-3-(phénylamino)carbonyl]thioxométhyl]-3,4,5-triméthoxy-benzamide (C₂₄H₂₂BrN₃O₅S) :
   **PM** = 544 ; **[ES/MS]** m/z 545 [M+H]⁺ ; pf = 196°C.
- **Composé (42) :** N-[[[4-chloro-3-(4-méthoxy-phénylamino)carbonyl]oxométhyl]-3,4,5-triméthoxy-benzamide (C₂₅H₂₄N₃ClO₇) :
   PM = 513 ; **[ES/MS]** m/z 514 [M+H]⁺ ; pf= 135°C.
- **Composé** (**43**) : N-[[[4-chloro-3-(4-chloro-phénylamino)]carbonyl]phénylamino] thioxométhyl]-3,4,5-triméthoxy-benzamide (C₂₄H₂₁Cl₂N₃O₅S) :
   PM = 534 ; **[ES/MS]** m/z 535 [M+H]⁺ ; pf = 188°C.
- **Composé (44)** : N-[[[4-chloro-3-(phénylamino)carbonyl]oxométhyl]-3,4,5-triméthoxy-benzamide (C₂₄H₂₂ClN₃O₆) :
   PM = 483 ; **[ES/MS]** m/z 484 [M+H]⁺ ; pf = 160°C.
- **Composé (45)** : N-[[[2-méthyl-3-(benzoylamino)phényl]amino]oxométhyl]-3,4,5-triméthoxy-benzamide (C₃₁H₂₉N₃O₅) :
   PM = 539, **[ES/MS]** m/z 540 [M+H]⁺ ; pf = 181°C.
- **Composé (46) :** N-[[[2-méthyl-3-(benzoylamino)phényl]amino]thioxométhyl]-3,4,5-triméthoxy-benzamide (C₃₁H₂₉N₃O₅S) :
   PM = 555 ; **[ES/MS]** m/z 556 [M+H]⁺ ; pf = 181°C.
- **Composé (47)** : N-[[[-3-(4-chloro-benzoylamino)phényl]amino]oxométhyl]-3,4,5-triméthoxy-benzamide (C₂₄H₂₂ClN₃O₅S) :
   PM = 539 ; **[ES/MS]** m/z 540 [M+H]⁺ ; pf = 156°C.
- **Composé (48) :** N-[[[-3-(benzoylamino)phényl]amino]oxométhyl]-2,4,5-triméthoxy-benzamide (C₂₄H₂₃N₃O₆) :
   PM = 449 ; **[ES/MS]** m/z 550 [M+H]⁺ ; pf = 192°C.
- **Composé (49) :** N-[[[-3-(benzoylamino)phényl]amino]oxométhyl]-4-méthoxy-benzamide (C₂₂H₁₉N₃O₄) :
   PM = 389 ; **[ES/MS]** m/z 390 [M+H]⁺ ; pf = 264°C.
- **Composé (50)** : N-[[[-3-(benzoylamino)phényl]amino]oxométhyl]-benzamide (C₂₁H₁₇N₃O₃) :
   PM = 359 ; **[ES/MS]** m/z 360 [M+H]⁺ ; pf = 244°C.
- **Composé** (**51**) : 3,4,5-triméthoxy-*N*-(3-(3-phényluréido)phénylcarbamothioyl)-benzamide (C₃₀H₂₀N₄O₄S₂) :
   PM 556 ; **[ES/MS]** m/z 557 [M+H]⁺ ; pf = 220°C.
- **Composé (52)** : *N*-(3-(3-biphényl-4-yluréido)phénylcarbamothioyl)-3,4,5-triméthoxybenzamide (C₂₄H₂₄N₄O₅S) :
   PM 480 ; **[ES/MS]** m/z 481 [M+H]⁺; pf = 250°C.
- **Composé (53) :** *N*-(3-(1*H*-indol-2-yl)phénylcarbamoyl)3,4,5-triméthoxybenzamide (C₂₅H₂₃N₃O₅) :
   PM 445 ; **[ES/MS]** m/z 446 [M+H]⁺ ; pf = 169°C.
- **Composé (54) :** *N*-(3-(1*H*-indol-2-yl)phenylcarbamothioyl)-3,4,5-triméthoxybenzamide (C₂₅H₂₃N₃O₄S) :
   PM 461 ; **[ES/MS]** m/z 462 [M+H]⁺ ; pf= 131°C.
- **Composé (55) :** *N*-(3-(imidazo[1,2-*a*]pyridin-2-yl)phénylcarbamothioyl)-3,4,5-triméthoxybenzamide (C₂₄H₂₂N₄O₄) :
   PM 462 ; **[ES/MS]** m/z 463 [M+H]⁺; pf = 136°C.
- **Composé** (**56**) : *N*-(3-(1*H*-benzo[*d*]imidazol-2-yl)-4-méthylphénylcarbamothioyl)-3,4,5-triméthoxybenzamide (C₂₅H₂₄N₄O₄S) :
   PM 476 ; **[ES/MS]** m/z 477 [M+H]⁺ ; pf = 187°C.
- **Composé (57)** : *N*-(3-(1*H*-indol-1-yl)phénylcarbamothioyl)-3,4,5-triméthoxybenzamide (C₂₅H₂₃N₃O₄S) :
   PM 461 ; **[ES/MS]** m/z 462 [M+H]⁺ ; pf = 131°C.
- **Composé (58) :** *N*-(3-(imidazo[2,1-*b*]thiazol-6-yl)phénylcarbamothioyl)-3,4,5-triméthoxybenzamide (C₂₂H₂₀N₄O₄S₂) :
   PM 468 ; **[ES/MS]** m/z 469 [M+H]⁺ ; pf = 133°C.
- **Composé (59)** : *N*-(3-(3-biphényl-4-yluréido)phénylcarbamoyl)-3,4,5-triméthoxybenzamide
   PM 525 ; **[ES/MS]** m/z 526 [M+H]⁺ ; pf= 183°C.

### EXEMPLE 2 MISE EN ÉVIDENCE DE L'EFFET INHIBITEUR DES COMPOSÉS DE FORMULE (I) SUR LA VOIE DE SIGNALISATION DES PROTÉINES HEDGEHOG

L'effet des composés de formule **(I)** conformes à la description sur l'inhibition de la voie de signalisation des protéines Hedgehog a été déterminé par analyse de la différenciation de la lignée de cellules fibroblastiques pluripotentes C3H10T1/2 après activation de cette voie dans ces cellules par un activateur synthétique : le SAG.

### 1) Matériels et méthodes

Les composés de formule **(I)** à tester ont été dissous dans le diméthylsulfoxyde jusqu'à une concentration de 10 mM, puis stockés à une température de -20°C jusqu'à utilisation.

La lignée de cellules fibroblastiques pluripotentes C3H10T1/2 (ATCC) a été cultivée dans les conditions recommandées par l'ATCC. L'activation de ces cellules a été réalisée en utilisant 0,1 µM de SAG selon les méthodes décrites par Chen et al., Proc. Natl. Acad. Sci. USA, 2002, 99, 14071 et Frank-Kamenetsky et al., J. Biol., 2002, 1, 10.

L'activation par le SAG provoque la différenciation de la lignée cellulaire et leur permet d'exprimer la phosphatase alcaline. On a ainsi pu mesurer l'activité de la voie de signalisation des protéines Hedgehog via la mesure de l'activité phosphatase alcaline.

Les cellules C3H10T1/2 ont été ensemencées sur des plaques de 96 puits à une densité de 5.10⁵ cellules par puits, 24 heures avant l'addition des composés à tester à une concentration variant de 1 nM à 30 µM et en présence de 0,1 µM de SAG en utilisant comme milieu de culture du DMEM supplémenté avec 10% de sérum de veau foetal. Les essais ont été réalisés en quadruplate. Les plaques ont ensuite été incubées pendant 5-6 jours à une température de 37°C sous atmosphère à 5% de CO₂. Les cellules ont ensuite été lavées dans du tampon phosphate froid ("Phosphate Buffer Sérum" : PBS) puis lysées par sonication à 4°C dans 50 µl d'une solution contenant 0,9% de NaCl et 0,2% de Triton X-100.

A titre comparatif, l'activité d'un inhibiteur connu de la voie de signalisation des protéines Hedgehog, le CURIS 61 414 tel que décrit par exemple par Frank-Kamenetsky M. et al. (J. Biol., 2002, 1, 10) a été testée dans les mêmes conditions que celles utilisées pour tester les différents composés de formule **(I)** conformes à la description.

La mesure de l'activité phosphatase alcaline dans les lysats ainsi obtenus a été ensuite réalisée selon la méthode décrite par Pepinsky et al. (J. Biol, Chem., 1998, 273, 14037). Après addition de 100 µl de tampon réactionnel (200 mM Tris-HCl ; pH 10,5 ; 0,4 M de 2-amino-2-méthylpropanol et 8 mM de MgCl₂) et de 50 µl de substrat (4 mM de p-nitrophényl phosphate disodium), les lysats ont été incubés à 37°C pendant 30-60 min, puis la densité optique a été lue à une longueur d'onde de 415 nm.

### 2) Résultats

Les résultats obtenus sont reportés dans les tableaux 1 et 2 ci-après dans lesquels :
- le Tableau 1 présente l'inhibition de l'activité phosphatase alcaline induite par 10 µM de chacun des composés testés. Ces résultats sont exprimés en pourcentage de l'activité phosphatase alcaline induite par action du SAG. Dans ce tableau, la lettre **A** correspond à une inhibition supérieure à 80 %, la lettre **B** correspond à une inhibition comprise entre 20 et 80 % et la lettre **C** correspond à une inhibition comprise entre 5 et 20%,
- le Tableau 2 présente la concentration testée pour chacun des composés qui permet d'inhiber 50% de l'activité phosphatase alcaline (IC₅₀) après induction par le SAG à 0,1 µM. Dans ce tableau, la lettre **A** correspond à une IC₅₀ comprise entre 0,01 et 1 µM et la lettre **B** correspond à une IC₅₀ comprise entre 1 et 3 µM.

**TABLEAU 1**

| **COMPOSÉS** | **% D'INHIBITION** |
|---|---|
| **1** | **A** |
| **2** | **A** |
| **3** | **A** |
| **4** | **A** |
| **5** | **B** |
| **6** | **A** |
| **7** | **B** |
| **8** | **B** |
| **9** | **B** |
| **10** | **C** |
| **11** | **A** |
| **12** | **B** |
| **13** | **A** |
| **14** | **B** |
| **15** | **B** |
| **16** | **C** |
| **17** | **A** |
| **18** | **A** |
| **19** | **A** |
| **20** | **A** |
| **21** | **A** |
| **22** | **B** |
| **23** | **A** |
| **24** | **A** |
| **25** | **A** |
| **26** | **B** |
| **27** | **B** |
| **28** | **C** |
| **29** | **B** |
| **30** | **C** |
| **31** | **A** |
| **32** | **B** |
| **33** | **B** |
| **34** | **A** |
| **35** | **B** |
| **36** | **B** |
| **37** | **B** |
| **38** | **A** |
| **39** | **A** |
| **40** | **B** |
| **41** | **B** |
| **42** | **B** |
| **43** | **A** |
| **44** | **B** |
| **45** | **A** |
| **46** | **C** |
| **47** | **A** |
| **48** | **B** |
| **49** | **B** |
| **50** | **A** |
| **51** | **A** |
| **52** | **A** |
| **53** | **A** |
| **54** | **A** |
| **55** | **A** |
| **56** | **B** |
| **57** | **A** |
| **58** | **A** |
| **59** | **A** |

**TABLEAU 2**

| **COMPOSÉS** | **IC₅₀ (µM)** |
|---|---|
| **Curis 61 414 (*)** | **A** |
| **1** | **B** |
| **2** | **B** |
| **3** | **B** |
| **4** | **B** |
| **5** | **A** |
| **13** | **A** |
| **18** | **B** |
| **19** | **A** |
| **20** | **A** |
| **21** | **B** |
| **22** | **B** |
| **23** | **A** |
| **31** | **A** |
| **34** | **A** |
| **38** | **A** |
| **39** | **B** |
| **45** | **A** |
| **48** | **B** |
| **51** | **B** |
| **52** | **A** |
| **53** | **A** |
| **54** | **A** |
| **55** | **A** |
| **57** | **B** |
| **58** | **B** |

| | |
|---|---|
| (*) : Composé de référence ne faisant pas partie de l'invention | |

Ces résultats démontrent que les composés de formule (I) conformes à la description sont des antagonistes de la voie de signalisation des protéines Hedgehog et qu'ils sont par conséquent utiles pour le traitement des pathologies nécessitant un blocage de la voie Hedgehog comme le cancer, les maladies neurodégénératives et le diabète.

### EXEMPLE 3 : COMPETITION DE LIAISON AVEC LA BODIPYCYCLOPAMINE

### 1) Matériels et méthodes

Des cellules HEK293 sont ensemencées à 70 000 cellules par puits sur des lamelles de verre traitées à la poly-D-lysine en plaque 24 puits, et transfectées le lendemain par 0,25 µg de plasmide encodant la protéine Smoothened de souris (Masdeu C., Faure H., Coulombe J., Schoenfelder A., Mann A., Brabet I., Pin J-P., Traiffort E. et Ruat M., Identification and characterization of Hedgehog modulator properties after functional coupling of Smoothened to G15, Biochem Biophys Res. Commun., 2006, 349:471-479), en utilisant 0,7 µl de fugene6 (Roche biochemicals). Après 48 heures, le milieu de culture est éliminé, les cellules rincées une fois avec 1 mL d'une solution tampon phosphate PBS (Phosphate Buffered Saline), puis fixées 20 minutes en présence d'une solution glacée de paraformaldéhyde (PFA) à 4%, de glucose à 0,12 M dans une solution tampon phosphate PBS. Les cellules sont ensuite rincées une fois et lavées 2 fois pendant 5 minutes avec 1 mL d'une solution tampon phosphate PBS, 0,5% de sérum de veau foetal (PBS-SVF). Ensuite, 1 ml de Bodipycyclopamine (BC) (Chen, J. K., Taipale, J., Cooper, M. K., and Beachy, P. A., Genes Dev., 2002, 16(21), 2743-2748), diluée à 5 nM dans du PBS-SVF, en présence des composés 31 et 38 de la description, est appliqué sur les cellules pendant 2 heures à 37°C. Les cellules sont ensuite lavées 2 fois pendant 5 minutes avec 1 mL de PBS-SVF, puis mises en présence de 1 mL d'une solution tampon phosphate PBS 1X. Enfin, les lamelles sont montées sur une lame de verre en présence de Vectashield contenant du DAPI (4',6'-DiAmidino-2-Phényle Indole) pour marquer les noyaux cellulaires (Vector).

### 2) Résultats

L'activité d'un inhibiteur connu de la voie de signalisation des protéines Hedgehog, la Cyclopamine (Cy), a également été testée dans les mêmes conditions que celles utilisées pour les composés 31 et 38 de la description.

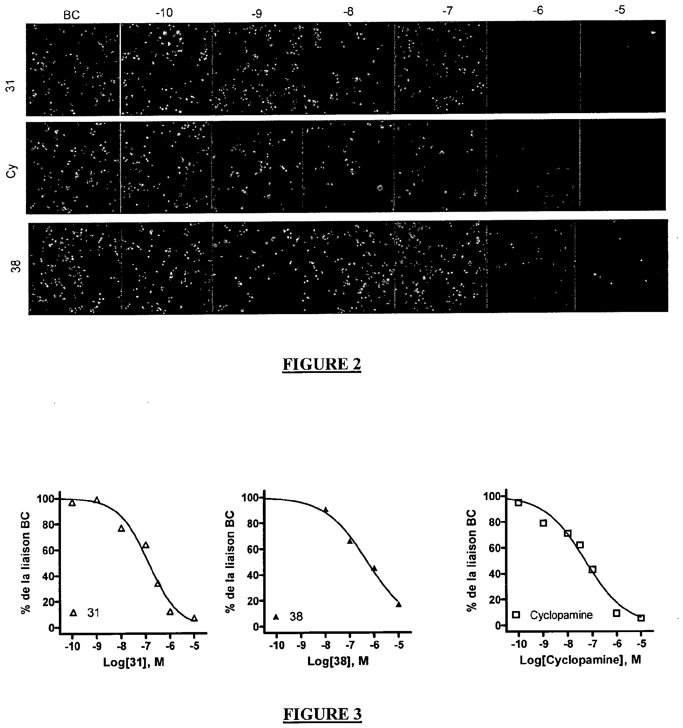

Les résultats obtenus sont représentés sur les Figures 2 et 3 annexées.

Sur la Figure 2, l'intensité de la fluorescence a été analysée à l'aide du logiciel Simple PCI 6.2 (Hamamatsu corporation), puis rapportée à la surface des noyaux présents sur la photographie. Cette intensité dépend de l'inhibition de la Bodipycyclopamine par les composés analysés.

On observe une compétition de la liaison de la Bodipycyclopamine en présence des composés 31 et 38 de la description, ainsi qu'avec la Cyclopamine (Cy). Les Figures 2 et 3 indiquent que les composés 31 et 38 de la description intéragissent avec le récepteur Smoothened au niveau du site de liaison de la Bodipycyclopamine.

Les concentrations inhibitrices IC₅₀ moyennes obtenues pour les composés 31 et 38 de la description, et pour la Cyclopamine (Cy), sont les uivantes :
- pour le composé 31 de la description : IC₅₀ = 0,12 µM,
- pour le composé 38 de la description : IC₅₀ = 0,5 µM, et
- pour la Cyclopamine (Cy) : IC₅₀ = 0,05 µM.

Ces résultats démontrent que les composés de la description sont des antagonistes de la voie de signalisation des protéines Hedgehog, et qu'ils agissent sur le récepteur Smoothened.

## Revendications

1. Utilisation de composés de formule **(I)** suivante : 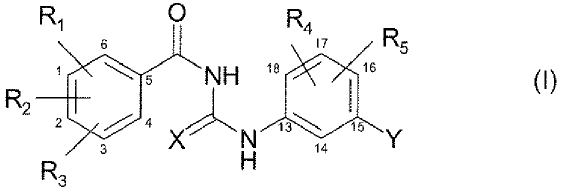 dans laquelle :
- R₁, R₂ et R₃, identiques ou différents et indépendamment les uns des autres, représentent un atome d'hydrogène ou d'halogène, un radical hydroxyle, un groupement alkyle, perfluoroalkyle, alcoxy, alkylthio, nitrile, un alcoxy substitué, ou un hétérocycle fusionné obtenu à partir de deux des radicaux R₁, R₂ et R₃, adjacents, pouvant former, conjointement avec les atomes de carbone du cycle phényle auxquels ils sont liés, un hétérocycle fusionné,
- X représente un atome de soufre ou d'oxygène,
- Y représente un groupement hétéroaryle mono- ou polycyclique, substitué ou non substitué, un groupement -NH-(C=O)-R₆, -(C=O)-NH-R₆ ou -NH-(C=O)-NH-R₆ dans lequel R₆ représente un groupement aryle non substitué ; un groupement aryle comportant un ou plusieurs substituants choisis parmi un atome d'halogène et un radical alkyle, alcoxy ou mono- ou dialkylamino ; un groupement hétéroaryle mono- ou polycyclique ; un radical alkyle linéaire ou ramifié ; un groupement hydrocarboné mono- ou polycyclique saturé ou insaturé ;
- R₄ et R₅, identiques ou différents et indépendamment l'un de l'autre, représentent un atome d'hydrogène ou d'halogène, un groupe alcoxy, alkylthio, alkyle, perfluoroalkyle, nitrile ou nitro,
pour la fabrication d'un médicament pour le traitement des tumeurs associées à une hyperactivation de la voie de signalisation des protéines Hedgehog, lesdites tumeurs étant choisies parmi les tumeurs du tissu nerveux (médulloblastomes, tumeurs primitives neuroectodermiques, glioblastomes, méningiomes et oligodendrogliomes), des tumeurs cutanées (carcinomes basocellulaires, trichoépithéliomes), des tumeurs des tissus musculaires et osseux (rhabdomyosarcomes, ostéosarcomes) ou des tumeurs d'autres tissus (rein, vessie) ou des pathologies de type neuro-dégénératif choisies parmi la sclérose en plaques ou la maladie du motoneurone.

2. Utilisation selon la revendication 1, **caractérisée en ce que** les composés sont choisis parmi ceux dans lesquels, dans la formule **(I)** :
- R₁, R₂, et R₃, identiques ou différents, représentent un atome d'hydrogène ou un radical méthyloxy ou éthyloxy,
- X représente un atome de soufre ou d'oxygène,
- Y représente un groupement hétéroaryle mono- ou polycyclique, substitué ou non substitué, un groupement -NH-(C=O)-R₆, -(C=O)-NH-R₆ ou -NH-(C=O)-NH-R₆ dans lequel R₆ représente un groupement choisi parmi les groupements phényle ; phényle substitué par un radical méthoxy ou diméthylamino, par un atome de chlore, par un groupement phényle, par un groupement benzyle ; cyclohexyle, isopropyle, pyridinyle, naphtyle, furfuryle et thiophène ; et
- R₄ et R₅, identiques ou différents, sont choisis parmi hydrogène, chlore, brome, fluor, méthyle et méthoxy.

3. Utilisation selon l'une des revendications 1 à 2, **caractérisée en ce que** les composés de formule **(I)** sont choisis parmi :
- le N-[[[3-(4-méthoxy-benzoylamino)phényl]amino]thioxométhyl]-3,4,5-triméthoxy-benzamide (**I**-**a**) de formule suivante : 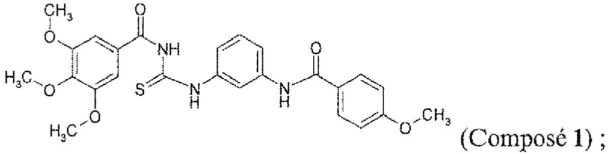
- le N-[[[3-(2-chloro-benzoylamino)phényl]amino]thioxométhyl]-3,4,5-triméthoxy-benzamide **(I-a)** de formule suivante : 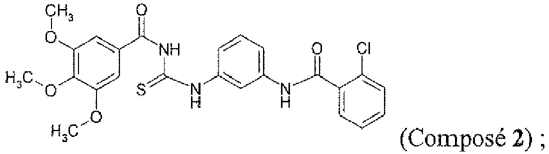
- le N-[[[3-(3-méthoxy-benzoylamino)phényl]amino]thioxométhyl]-3,4,5-triméthoxy-benzamide **(I-a)** de formule suivante : 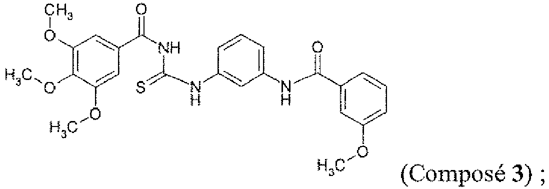
- le N-[[[3-(2-méthoxy-benzoylamino)phényl]amino]thioxométhyl]-3,4,5-triméthoxy-benzamide **(I-a)** de formule suivante : 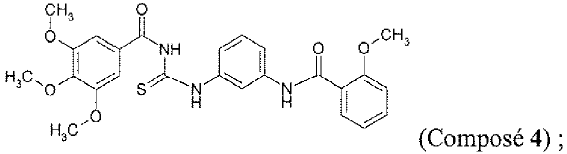
- le N-[[[3-(4-phényl-benzoylamino)phényl]amino]thioxométhyl]-3,4,5-triméthoxy-benzamide **(I-a)** de formule suivante : 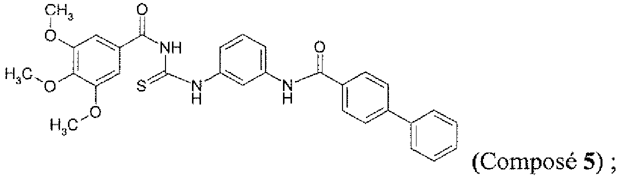
- le N-[[[3-(4-méthoxy-benzoylamino)phényl]amino]oxométhyl]-3,4,5-triméthoxy-benzamide **(I-a')** de formule suivante : 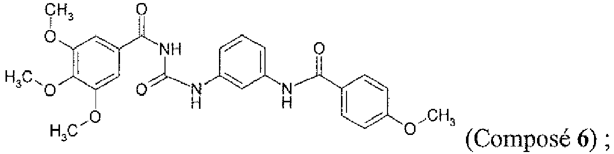
- le N-[[[3-(4-phényl-benzoylamino)phényl]amino]oxométhyl]-3,4,5-triméthoxy-benzamide **(I-a')** de formule suivante : 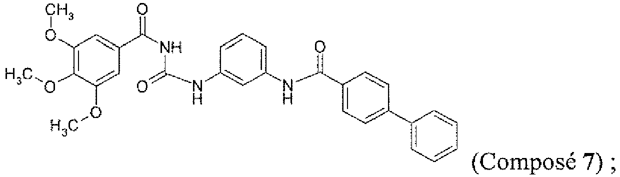
- le N-[[[3-(-cyclohexylcarboxylamino)phényl]amino]thioxométhyl]-3,4,5-triméthoxy-benzamide **(I-a)** de formule suivante : 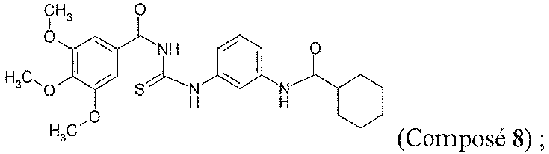
- le N-[[[3-(2-naphtoylamino)phényl]amino]thioxométhyl]-3,4,5-triméthoxy-benzamide **(I-a)** de formule suivante : 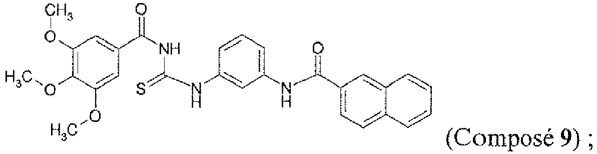
- le N-[[[3-(isopropoylcarboxylamino)phényl]amino]thioxométhyl]-3,4,5-triméthoxy-benzamide **(I-a)** de formule suivante : 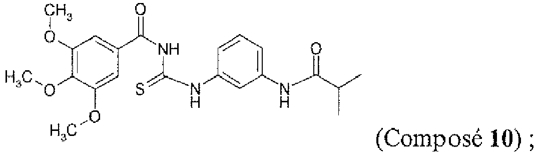
- le N-[[[3-(4-pyridocarboxylamino)phényl]amino]thioxométhyl]-3,4,5-triméthoxy-benzamide **(I-a)** de formule suivante : 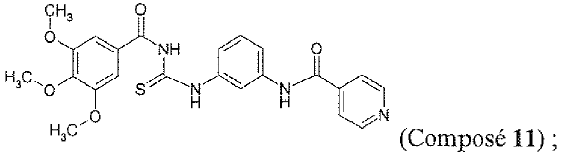
- le N-[[[3-(1-naphtoylamino)phényl]amino]thioxométhyl]-3,4,5-triméthoxy-benzamide **(I-a)** de formule suivante : 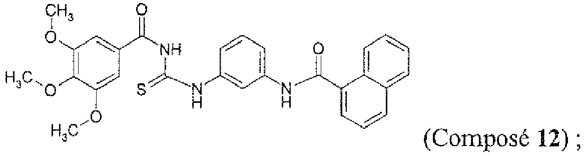
- le N-[[[3-(4-chloro-benzoylamino)phényl]amino]thioxométhyl]-3,4,5-triméthoxy-benzamide **(I-a)** de formule suivante : 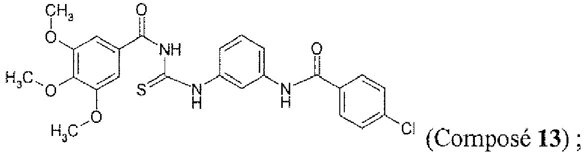
- le N-[[[3-(2-furoylamino)phényl]amino]thioxométhyl]-3,4,5-triméthoxy-benzamide **(I-a)** de formule suivante : 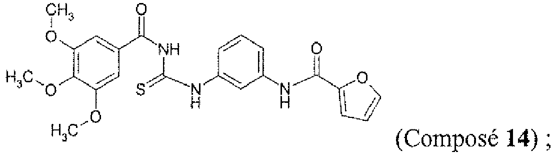
- le N-[[[3-(2-thiophénoylamino)phényl]amino]thioxométhyl]-3,4,5-triméthoxy-benzamide **(I-a)** de formule suivante : 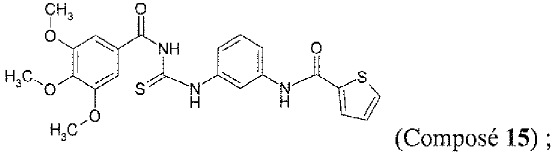
- le N-[[[3-(2-naphtoylamino)phényl]amino]oxométhyl]-3,4,5-triméthoxy-benzamide **(I-a')** de formule suivante : 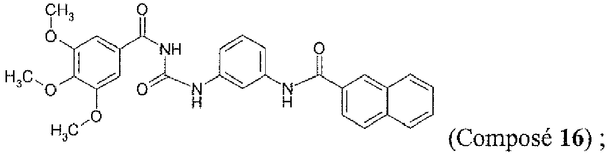
- le N-[[[3-(isopropoylcarboxylamino))phényl]amino]oxométhyl]-3,4,5-triméthoxy-benzamide **(I-a')** de formule suivante : 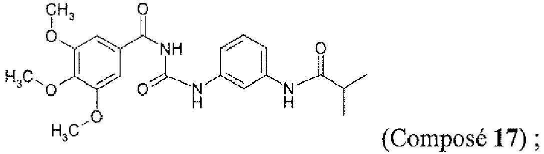
- le N-[[[3-(benzoylamino)phényl]amino]thioxométhyl]-3,5-diméthoxy-benzamide **(I-a)** de formule suivante : 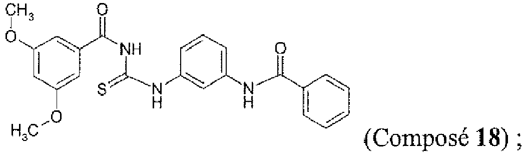
- le N-[[[3-(benzoylamino)phényl]amino]thioxométhyl]-3,5-diméthoxy-4-éthoxy-benzamide **(I-a)** de formule suivante : 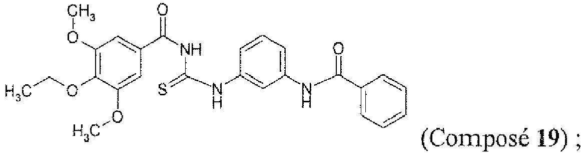
- le N-[[[3-(benzoylamino)phényl]amino]thioxométhyl]-3,4-diéthoxy-5-méthoxy-benzamide **(I-a)** de formule suivante : 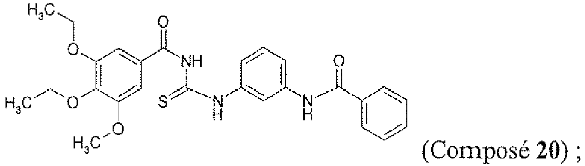
- le N-[[[3-(benzoylamino)phényl]amino]thioxométhyl]-3,4-méthylènedioxy-5-méthoxy-benzamide **(I-a)** de formule suivante : 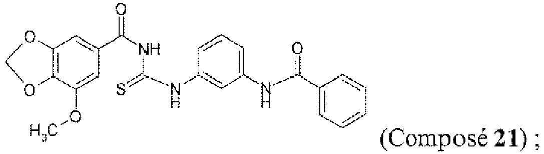
- le N-[[[3-(benzoylamino)phényl]amino]thioxométhyl]-2,4-diméthoxy-benzamide **(I-a)** de formule suivante : 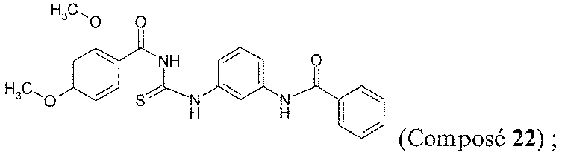
- le N-[[[3-(benzoylamino)phényl]amino]thioxométhyl]-3,4,5-triéthoxy-benzamide **(I-a)** de formule suivante : 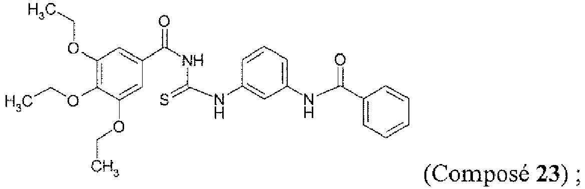
- le N-[[[4-fluoro-3-(benzoylamino)phenyl]amino]thioxométhyl]-3,4,5-triméthoxy-benzamide **(I-a)** de formule suivante : 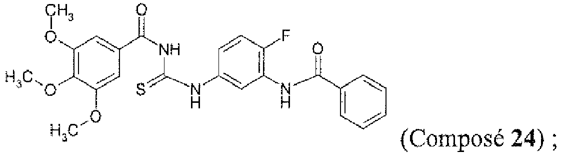
- le N-[[[4-chloro-3-(phénylamino)carbonyl]amino]thioxométhyl]-3,4,5-triméthoxy-benzamide **(I-b)** de formule suivante : 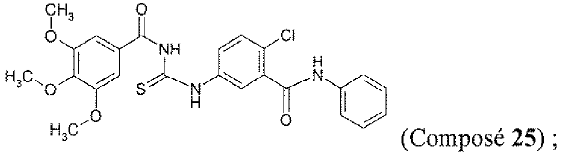
- le N-[[[4-méthoxy-3-(benzoylamino)phényl]amino]thioxométhyl]-3,4,5-triméthoxy-benzamide **(I-a)** de formule suivante : 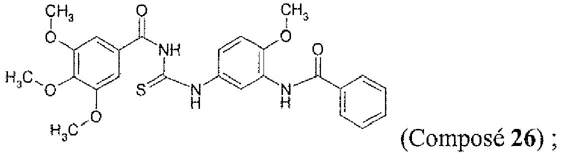
- le N-[[[4-méthyl-3-(benzoylamino)phényl]amino]thioxométhyl]-3,4,5-triméthoxy-benzamide **(I-a)** de formule suivante : 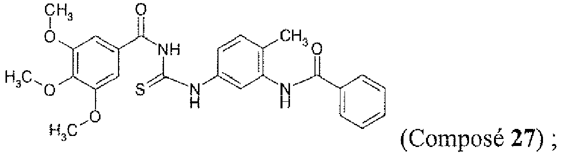
- le N-[[[3-(benzoylamino)phényl]amino]thioxométhyl]-3,4-éthylènedioxo-benzamide **(I-a)** de formule suivante : 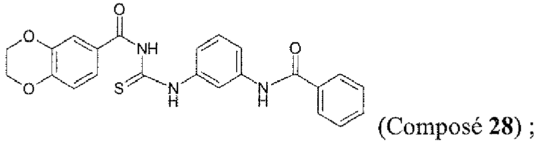
- le N-[[[3-(benzoylamino)phényl]amino]oxométhyl]-3,4,5-triéthoxy-benzamide **(I-a')** de formule suivante : 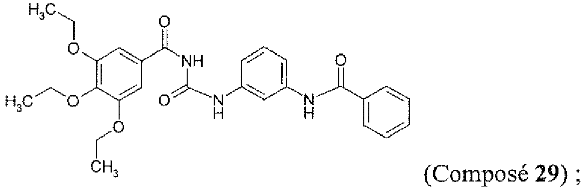
- le N-[[[3-(benzoylamino)phényl]amino]oxométhyl]-3,4-méthylènedioxy-5-méthoxy-benzamide **(I-a')** de formule suivante : 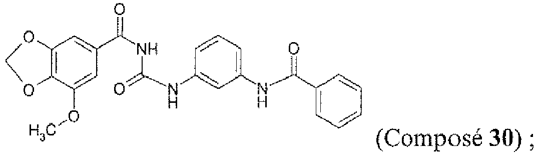
- le N-[[[3-(benzoylamino)phényl]amino]oxométhyl]-3,4,5-triméthoxy-benzamide **(I-a')** de formule suivante : 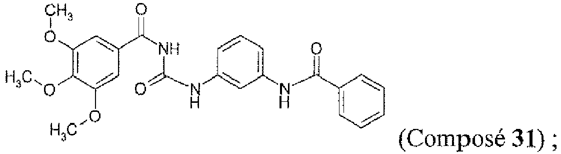
- le N-[[[3-méthyl-3-(benzoylamino)phényl]amino]thioxomethyl]-3,4,5-triméthoxy-benzamide **(I-a)** de formule suivante : 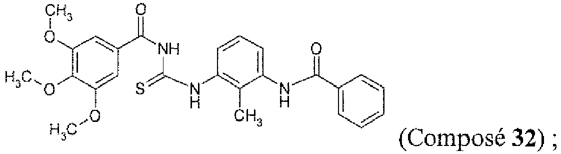
- le N-[[[4-chloro-3-(benzoylamino)phényl]amino]oxométhyl]-3,4,5-triméthoxy-benzamide **(I-a')** de formule suivante : 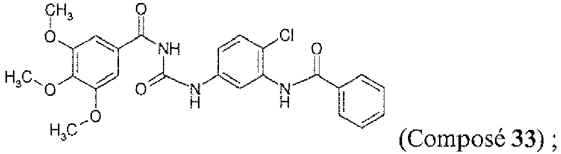
- le N-[[[4-chloro-3-(benzoylamino)phényl]amino]thioxométhyl]-3,4,5-triméthoxy-benzamide (**I**-**a**) de formule suivante : 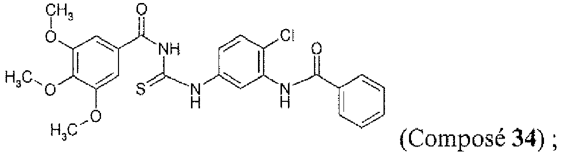
- le N-[[[5-chloro-3-(benzoylamino)phényl]amino]thioxométhyl]-3,4,5-triméthoxy-benzamide (I-a) de formule suivante : 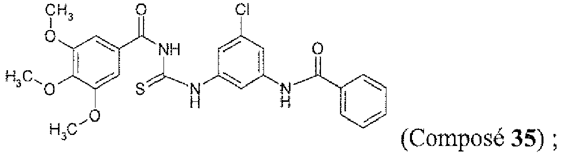
- le N-[[[5-fluoro-3-(benzoylamino)phényl]amino]thioxométhyl]-3,4,5-triméthoxy-benzamide **(I-a)** de formule suivante : 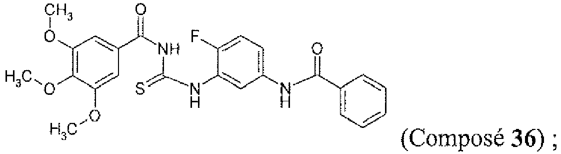
- le N-[[[4-chloro-3-(4-Phényl-benzoylamino)phényl]amino]oxométhyl]-3,4,5-triméthoxy-benzamide **(I-a')** de formule suivante : 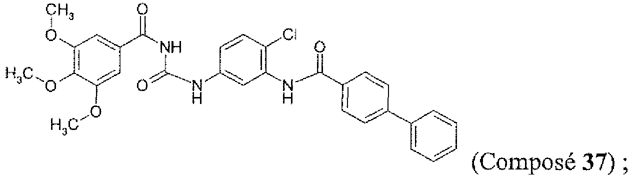
- le N-[[[3-(benzoylamino)phényl]amino]thioxométhyl]-3,4,5-triméthoxy-benzamide **(I-**a) de formule suivante : 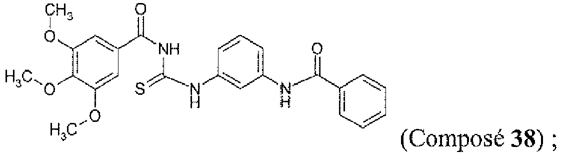
- le N-[[[4-chloro-3-(4-diméthylamino-phénylamino)carbonyl] thioxométhyl]-3,4,5-triméthoxy-benzamide **(I-b)** de formule suivante : 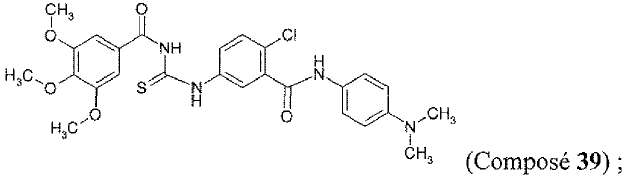
- le N-[[[4-chloro-3-(4-méthoxy-phénylamino)carbonyl]thioxométhyl]-3,4,5-triméthoxy-benzamide **(I-b)** de formule suivante : 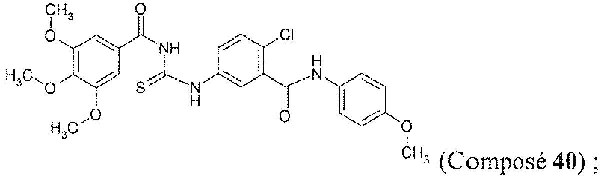
- le N-[[[5-bromo-3-(phénylamino)carbonyl]thioxométhyl]-3,4,5-triméthoxy-benzamide **(I-b)** de formule suivante : 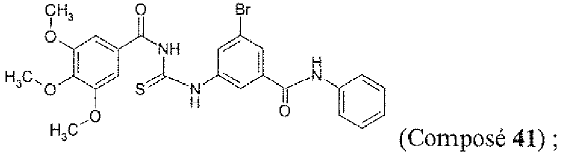
- le N-[[[4-chloro-3-(4-méthoxy-phénylamino)carbonyl]oxométhyl]-3,4,5-triméthoxy-benzamide **(I-b')** de formule suivante : 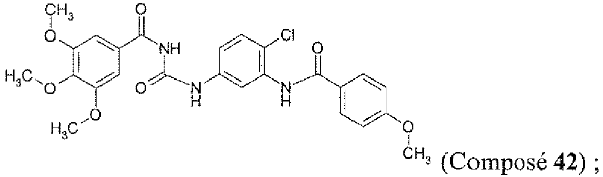
- le N-[[[4-chloro-3-(4-chloro-phénylamino)]carbonyl]phénylamino]thioxométhyl]-3,4,5-triméthoxy-benzamide **(I-b)** de formule suivante : 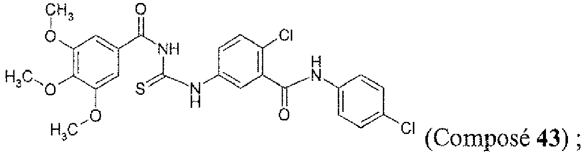
- le N-[[[4-chloro-3-(phénylamino)carbonyl]oxométhyl]-3,4,5-triméthoxy-benzamide **(I-b')** de formule suivante : 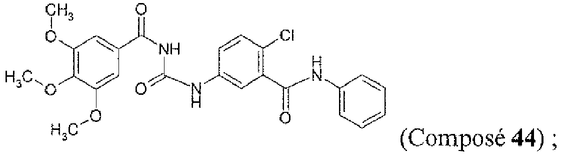
- le N-[[[2-méthyl-3-(benzoylamino)phényl]amino]oxométhyl]-3,4,5-triméthoxy-benzamide **(I-a')** de formule suivante : 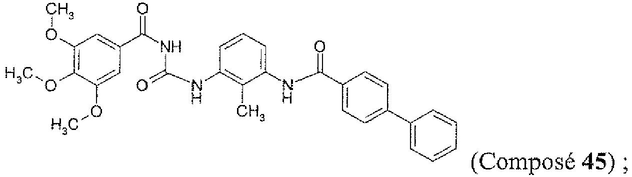
- le N-[[[2-méthyl-3-(benzoylamino)phényl]amino]thioxométhyl]-3,4,5-triméthoxy-benzamide **(I-a)** de formule suivante : 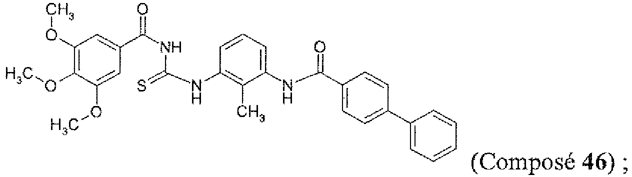
- le N-[[[-3-(4-chloro-benzoylamino)phényl]amino]oxométhyl]-3,4,5-triméthoxy-benzamide **(I-a')** de formule suivante : 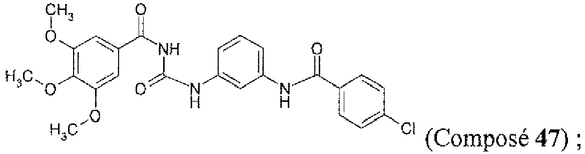
- le N-[[[-3-(benzoylamino)phényl]amino]oxométhyl]-2,4,5-triméthoxy-benzamide (I-a') de formule suivante : 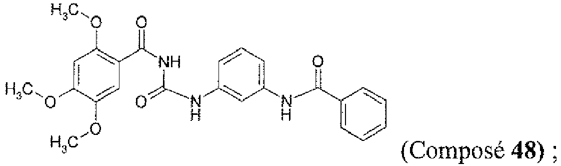
- le N-[[[-3-(benzoylamino)phényl]amino]oxométhyl]-4-méthoxy-benzamide **(I-a')** de formule suivante : 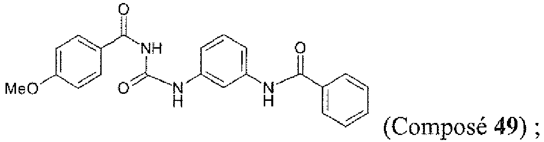
- le N-[[[-3-(benzoylamino)phényl]amino]oxométhyl]-benzamide **(I-a')** de formule suivante : 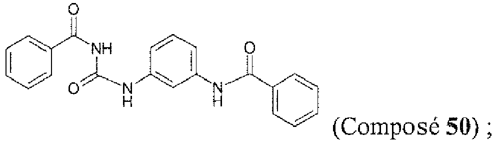
- le 3,4,5-triméthoxy-*N*-(3-(3-phényluréido)phénylcarbamothioyl)benzamide **(I-b)** de formule suivante : 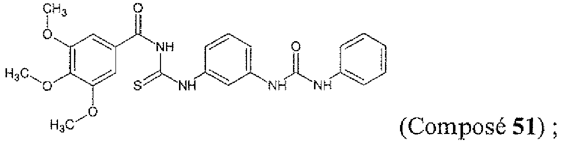
- le *N*-(3-(3-biphényl-4-yluréido)phénylcarbamothioyl)-3.4,5-triméthoxybenzamide **(I-b)** de formule : 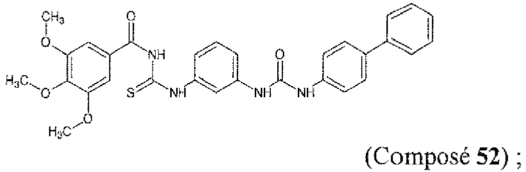
- le *N*-(3-(1*H*-indol-2-yl)phénylcarbamoyl)-3,4,5-triméthoxybenzamide **(I-d')** de formule : 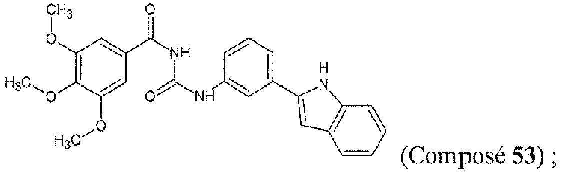
- le *N*-(3-(1*H*-indol-2-yl)phénylcarbamothioyl)-3,4,5-triméthoxybenzamide **(I-d)** de formule : 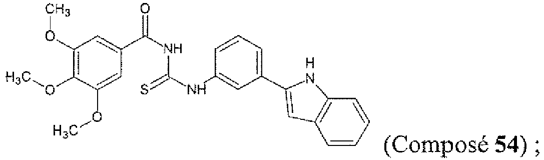
- le *N*-(3-(imidazo[1,2-*a*]pyridin-2-yl)phénylcarbamothioyl)-3,4,5-triméthoxybenzamide **(I-d)** de formule : 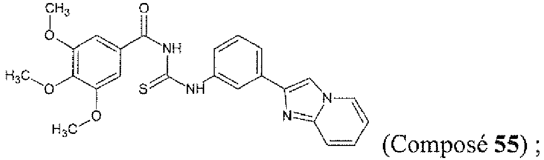
- le *N*-(3-(1*H*-benzo[*d*]imidazol-2-yl)-4-méthylphénylcarbamothioyl)-3,4,5-triméthoxybenzamide **(I-d)** de formule : 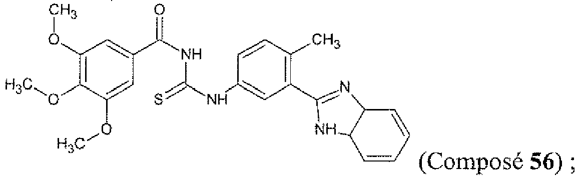
- le *N*-(3-(1*H*-indol-1-yl)phénylcarbamothioyl)-3,4,5-triméthoxybenzamide **(I-d)** de formule : 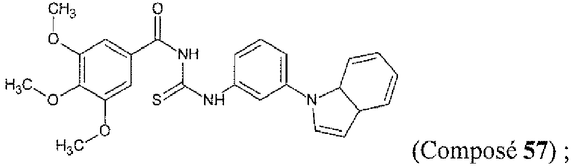 et
- le *N*-(3-(imidazo[2,1-*b*]thiazol-6-yl)phénylcarbamothioyl)-3,4,5-triméthoxybenzamide (**I-d**), de formule : 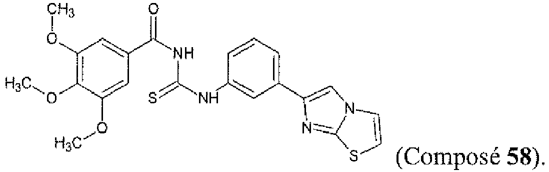
- le *N*-(3-(3-biphényl-4-yluréido)phénylcarbamoyl)-3,4,5-triméthoxybenzamide (**I-b'**) de formule :

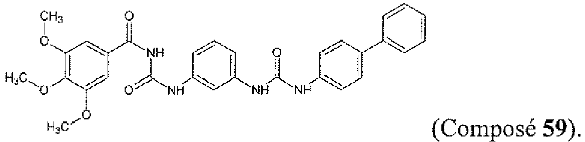

4. Utilisation selon l'une des revendications 1 à 3, **caractérisée en ce que** les composés de formule **(I)** sont choisis parmi :
- le N-[[[3-(4-méthoxy-benzoylamino)phényl]amino]thioxométhyl]-3,4,5-triméthoxy-benzamide (Composé **1**) ;
- le N-[[[3-(2-chloro-benzoylamino)phényl]amino]thioxométhyl]-3,4,5-triméthoxy-benzamide (Composé **2**) ;
- le N-[[[3-(3-méthoxy-benzoylamino)phényl]amino]thioxométhyl]-3,4,5-triméthoxy-benzamide (Composé **3**) ;
- le N-[[[3-(2-méthoxy-benzoylamino)phényl]amino]thioxométhyl]-3,4,5-triméthoxy-benzamide (Composé **4**) ;
- le N-[[[3-(4-méthoxy-benzoylamino)phényl]amino]oxométhyl]-3,4,5-triméthoxy-benzamide (Composé **6**) ;
- le N-[[[3-(4-pyridocarboxylamino)phényl]amino]thioxométhyl]-3,4,5-triméthoxy-benzamide (Composé **11**) ;
- le N-[[[3-(4-chloro-benzoylamino)phényl]amino]thioxométhyl]- 3,4,5-triméthoxy-benzamide (Composé **13**) ;
- le N-[[[3-(isopropoylcarboxylamino))phényl]amino]oxométhyl]-3,4,5-triméthoxy-benzamide (Composé **17**) ;
- le N-[[[3-(benzoylamino)phényl]amino]thioxométhyl]-3,5-diméthoxy-benzamide (Composé **18**) ;
- le N-[[[3-(benzoylamino)phényl]amino]thioxométhyl]-3,5-diméthoxy-4-éthoxy-benzamide (Composé **19**) ;
- le N-[[[3-(benzoylamino)phényl]amino]thioxométhyl]-3,4-diéthoxy-5-méthoxy-benzamide (Composé **20**) ;
- le N-[[[3-(benzoylamino)phényl]amino]thioxométhyl]-3,4-méthylènedioxy-5-méthoxy-benzamide (Composé **21**) ;
- le N-[[[3-(benzoylamino)phényl]amino]thioxométhyl]-3,4,5-triéthoxy-benzamide (Composé **23**) ;
- le N-[[[4-fluoro-3-(benzoylamino)phényl]amino]thioxométhyl]-3,4,5-triméthoxy-benzamide (Composé **24**) **;**
- le N-[[[4-chloro-3-(phénylamino)carbonyl]amino]thioxométhyl]-3,4,5-triméthoxy-benzamide (Composé **25**) ;
- le N-[[[3-(benzoylamino)phényl]amino]oxométhyl]-3,4,5-triméthoxy-benzamide (Composé **31**) ;
- le N-[[[4-chloro-3-(benzoylamino)phényl]amino]thioxométhyl]-3,4,5-triméthoxy-benzamide (Composé **34**) ;
- le N-[[[3-(benzoylamino)phényl]amino]thioxométhyl]-3,4,5-triméthoxy-benzamide (Composé **38**) ;
- le N-[[[4-chloro-3-(4-diméthylamino-phénylamino)carbonyl] thioxométhyl]-3,4,5-triméthoxy-benzamide (Composé **39**) ;
- le N-[[[4-chloro-3-(4-chloro-phénylamino)]carbonyl]phénylamino]thioxométhyl]-3,4,5-triméthoxy-benzamide (Composé **43**) ;
- le N-[[[2-méthyl-3-(benzoylamino)phényl]amino]oxométhyl]-3,4,5-triméthoxy-benzamide (Composé **45**) ;
- le N-[[[-3-(4-chloro-benzoylamino)phényl]amino]oxométhyl]-3,4,5-triméthoxy-benzamide (Composé **47**) ;
- le 3,4,5-triméthoxy-*N*-(3-(3-phényluréido)phénylcarbamothioyl)benzamide (**I-b**) (Composé **51**),
- le *N*-(3-(3-biphényl-4-yluréido)phénylcarbamothioyl)-3,4,5-triméthoxybenzamide (**I-b**) (Composé **52**) ;
- le *N*-(3-(1*H*-indol-2-yl)phénylcarbamoyl)-3,4,5-triméthoxybenzamide (**I-d'**) (Composé **53**) ;
- le *N*-(3-(1*H*-indol-2-yl)phénylcarbamothioyl)-3,4,5-triméthoxybenzamide (**I**-**d**) (Composé **54**) ;
- le *N*-(3-(imidazo[1,2-*a*]pyridin-2-yl)phénylcarbamothioyl)-3,4,5-triméthoxybenzamide (**I-d**) (Composé **55**) ;
- le *N*-(3-(1*H*-indol-1-yl)phénylcarbamothioyl)-3,4,5-triméthoxybenzamide (Composé **57**) ;
- le *N*-(3-(imidazo[2,1-*b*]thiazol-6-yl)phénylcarbamothioyl)-3,4,5-triméthoxybenzamide (**I-d**) (Composé **58**) ; et
- le *N*-(3-(3-biphényl-4-yluréido)phénylcarbamoyl)-3,4,5-triméthoxybenzamide (**I-b'**) (Composé **59**).

5. Utilisation de composés de formule **(I)** suivante : 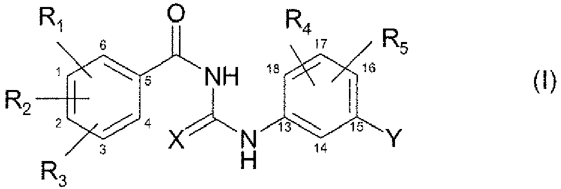 dans laquelle :
- R₁, R₂ et R₃, identiques ou différents et indépendamment les uns des autres, représentent un atome d'hydrogène ou d'halogène, un radical hydroxyle, un groupement alkyle, perfluoroalkyle, alcoxy, alkylthio, nitrile, un alcoxy substitué, ou un hétérocycle fusionné obtenu à partir de deux des radicaux R₁, R₂ et R₃, adjacents, pouvant former, conjointement avec les atomes de carbone du cycle phényle auxquels ils sont liés, un hétérocycle fusionné,
- X représente un atome de soufre ou d'oxygène,
- Y représente un groupement -NH-(C=O)-R₆, -(C=O)-NH-R₆ ou - NH-(C=O)-NH-R₆ dans lequel R₆ représente un groupement aryle non substitué ; un groupement aryle comportant un ou plusieurs substituants choisis parmi un atome d'halogène et un radical alkyle, alcoxy ou mono- ou dialkylamino ; un groupement hétéroaryle mono- ou polycyclique ; un radical alkyle linéaire ou ramifié ; un groupement hydrocarboné mono- ou polycyclique saturé ou insaturé ;
- R₄ et R₅, identiques ou différents et indépendamment l'un de l'autre, représentent un atome d'hydrogène ou d'halogène, un groupe alcoxy, alkylthio, alkyle, perfluoroalkyle, nitrile ou nitro,
pour la fabrication d'un médicament pour le traitement des tumeurs associées à une hyperactivation de la voie de signalisation des protéines Hedgehog ou des pathologies de type neuro-dégénératif.

6. Utilisation de composés de formule **(I)** suivante : 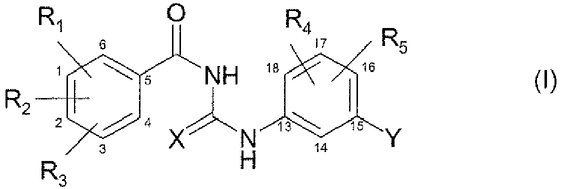 dans laquelle :
- R₁, R₂ et R₃, identiques ou différents et indépendamment les uns des autres, représentent un atome d'hydrogène ou d'halogène, un radical hydroxyle, un groupement alkyle, perfluoroalkyle, alcoxy, alkylthio, nitrile, ou un alcoxy substitué ;
- X représente un atome de soufre ou d'oxygène,
- Y représente un groupement -NH-(C=O)-R₆, -(C=O)-NH-R₆ ou - NH-(C=O)-NH-R₆ dans lequel R₆ représente un groupement aryle non substitué ; un groupement aryle comportant un ou plusieurs substituants choisis parmi un atome d'halogène et un radical alkyle, alcoxy ou mono- ou dialkylamino ; un groupement hétéroaryle mono- ou polycyclique ; un radical alkyle linéaire ou ramifié ; un groupement hydrocarboné mono- ou polycyclique saturé ou insaturé ;
- R₄ et R₅, identiques ou différents et indépendamment l'un de l'autre, représentent un atome d'hydrogène ou d'halogène, un groupe alcoxy, alkylthio, alkyle, perfluoroalkyle ou nitrile ;
étant entendu que lorsque Y représente un groupement -NH-(C=O)-R₆ :
* R₄ et/ou R₅ peuvent également représenter un groupement nitro, et
* deux des R₁, R₂ et R₃, adjacents, peuvent aussi former, conjointement avec les atomes de carbone du cycle phényle auxquels ils sont liés, un hétérocycle fusionné,
pour la fabrication d'un médicament pour le traitement des tumeurs associées à une hyperactivation de la voie de signalisation des protéines Hedgehog ou des pathologies de type neuro-dégénératif.

7. Utilisation selon la revendication 5 ou 6, **caractérisée en ce que** les composés sont choisis parmi ceux dans lesquels, dans la formule **(I)** :
- R₁, R₂, et R₃, identiques ou différents, représentent un atome d'hydrogène ou un radical méthyloxy ou éthyloxy,
- X représente un atome de soufre ou d'oxygène,
- Y représente un groupement -NH-(C=O)-R₆, -(C=O)-NH-R₆ ou - NH-(C=O)-NH-R₆ dans lequel R₆ représente un groupement choisi parmi les groupements phényle ; phényle substitué par un radical methoxy ou diméthylamino, par un atome de chlore, par un groupement phényle, par un groupement benzyle ; cyclohexyle, isopropyle, pyridinyle, naphtyle, furfuryle et thiophène ; et
- R₄ et R₅, identiques ou différents, sont choisis parmi hydrogène, chlore, brome, fluor, méthyle et méthoxy.

8. Utilisation selon l'une des revendications 5 à 7, **caractérisée en ce que** les composés de formule **(I)** sont choisis parmi :
- le N-[[[3-(4-méthoxy-benzoylamino)phényl]amino]thioxométhyl]-3,4,5-triméthoxy-benzamide **(I-a)** de formule suivante : 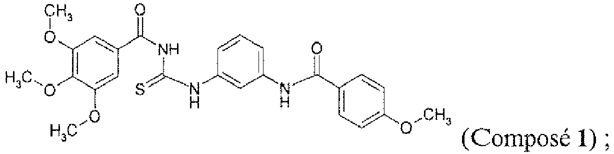
- le N-[[[3-(2-chloro-benzoylamino)phényl]amino]thioxométhyl]-3,4,5-triméthoxy-benzamide **(I-a)** de formule suivante : 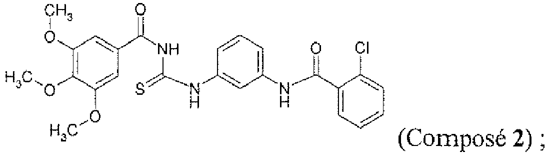
- le N-[[[3-(3-méthoxy-benzoylamino)phényl]amino]thioxométhyl]-3,4,5-triméthoxy-benzamide (**I-a**) de formule suivante : 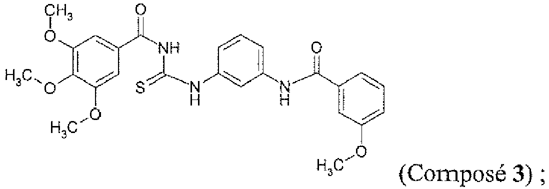
- le N-[[[3-(2-méthoxy-benzoylamino)phényl]amino]thioxomethyl]-3,4,5-trimethoxy-benzamide (I-a) de formule suivante : 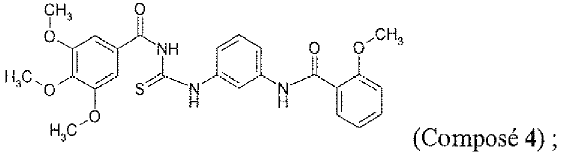
- le N-[[[3-(4-phényl-benzoylamino)phényl]amino]thioxométhyl]-3,4,5-triméthoxy-benzamide **(I-a)** de formule suivante : 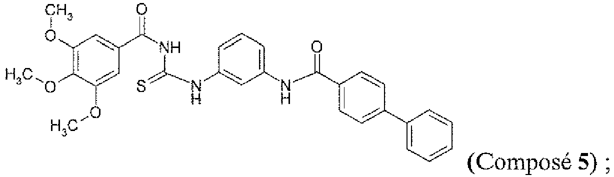
- le N-[[[3-(4-méthoxy-benzoylamino)phényl]amino]oxométhyl]-3,4,5-triméthoxy-benzamide (**I-a'**) de formule suivante : 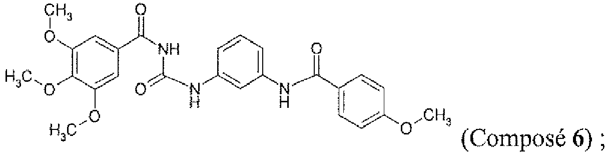
- le N-[[[3-(4-phényl-benzoylamino)phényl]amino]oxométhyl]-3,4,5-triméthoxy-benzamide (**I-a'**) de formule suivante : 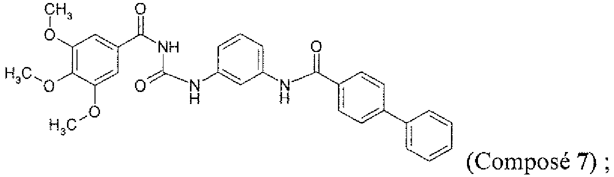
- le N-[[[3-(-cyclohexylcarboxylamino)phényl]amino]thioxométhyl]-3,4,5-triméthoxy-benzamide (**I-a**) de formule suivante : 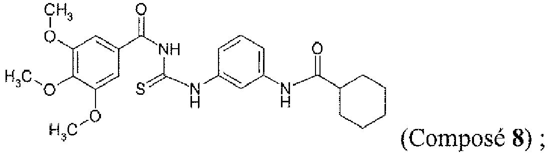
- le N-[[[3-(2-naphtoylamino)phényl]amino]thioxométhyl]-3,4,5-triméthoxy-benzamide (**I-a**) de formule suivante : 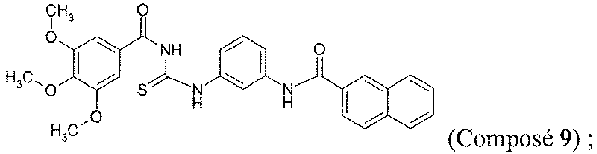
- le N-[[[3-(isopropoylcarboxylamino)phényl]amino]thioxométhyl]-3,4,5-triméthoxy-benzamide **(I-a)** de formule suivante : 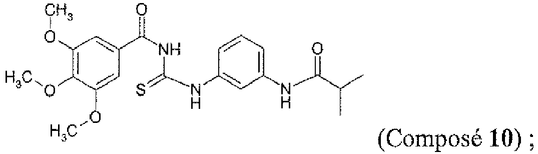
- le N-[[[3-(4-pyridocarboxylamino)phenyl]amino]thioxométhyl]-3,4,5-triméthoxy-benzamide **(I-a)** de formule suivante : 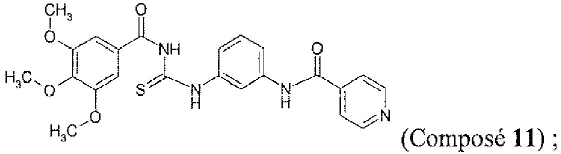
- le N-[[[3-(1-naphtoylamino)phényl]amino]thioxométhyl]-3,4,5-triméthoxy-benzamide **(I-a)** de formule suivante : 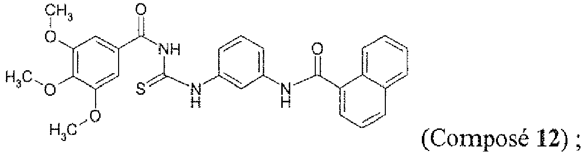
- le N-[[[3-(4-chloro-benzoylamino)phényl]amino]thioxométhyl]-3,4,5-triméthoxy-benzamide **(I-a)** de formule suivante : 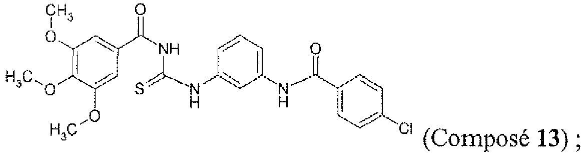
- le N-[[[3-(2-furoylamino)phényl]amino]thioxométhyl]-3,4,5-triméthoxy-benzamide **(I-a)** de formule suivante : 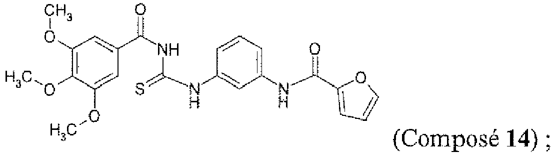
- le N-[[[3-(2-thiophénoylamino)phényl]amino]thioxométhyl]-3,4,5-triméthoxy-benzamide **(I-a)** de formule suivante : 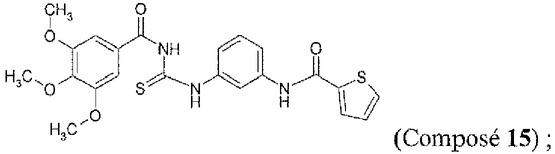
- le N-[[[3-(2-naphtoylamino)phényl]amino]oxométhyl]-3,4,5-triméthoxy-benzamide (**I-a'**) de formule suivante : 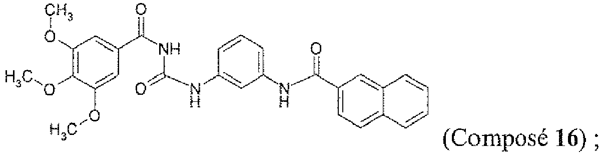
- le N-[[[3-(isopropoylcarboxylamino))phényl]amino]oxométhyl]-3,4,5-triméthoxy-benzamide (**I-a'**) de formule suivante : 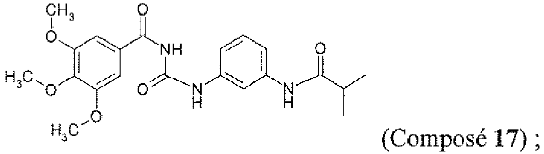
- le N-[[[3-(benzoylamino)phényl]amino]thioxométhyl]-3,5-diméthoxy-benzamide **(I-a)** de formule suivante : 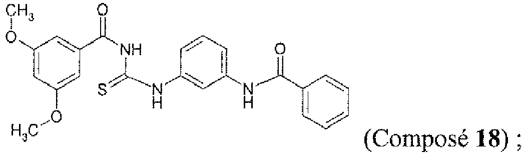
- le N-[[[3-(benzoylamino)phényl]amino]thioxométhyl]-3,5-diméthoxy-4-éthoxy-benzamide **(I-a)** de formule suivante : 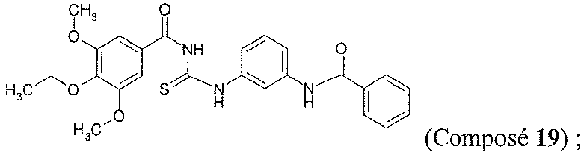
- le N-[[[3-(benzoylamino)phényl]amino]thioxométhyl]-3,4-diéthoxy-5-méthoxy-benzamide **(I-a)** de formule suivante : 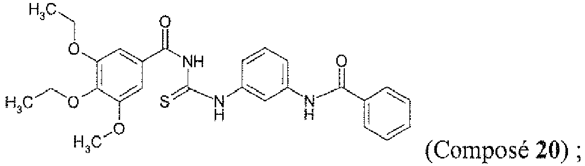
- le N-[[[3-(benzoylamino)phényl]amino]thioxométhyl]-3,4-méthylènedioxy-5-méthoxy-benzamide **(I-a)** de formule suivante : 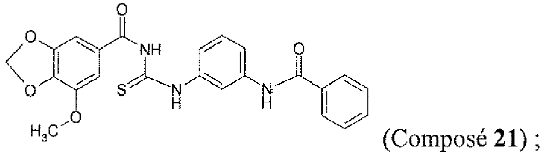
- le N-[[[3-(benzoylamino)phényl]amino]thioxométhyl]-2,4-diméthoxy-benzamide **(I-a)** de formule suivante : 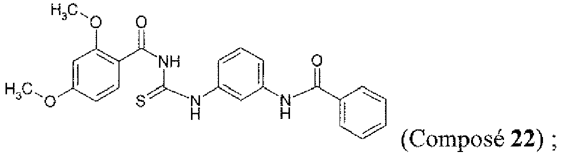
- le N-[[[3-(benzoylamino)phényl]amino]thioxométhyl]-3,4,5-triéthoxy-benzamide **(I-a)** de formule suivante : 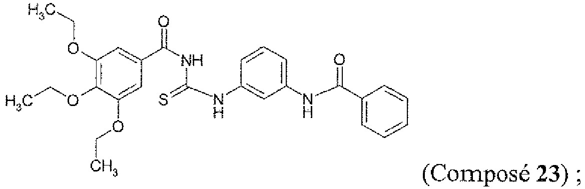
- le N-[[[4-fluoro-3-(benzoylamino)phényl]amino]thioxométhyl]-3,4,5-triméthoxy-benzamide **(I-a)** de formule suivante : 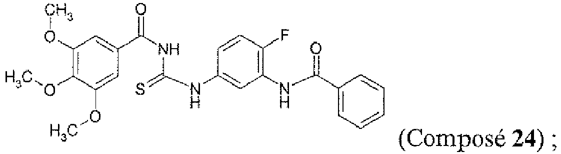
- le N-[[[4-chloro-3-(phénylamino)carbonyl]amino]thioxométhyl]-3,4,5-triméthoxy-benzamide **(I-b)** de formule suivante : 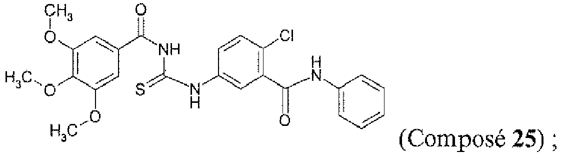
- le N-[[[4-méthoxy-3-(benzoylamino)phényl]amino]thioxométhyl]-3,4,5-triméthoxy-benzamide **(I-a)** de formule suivante : 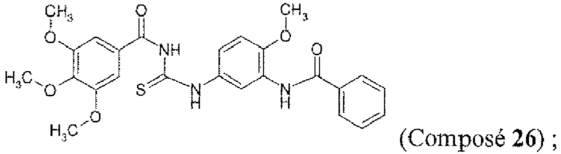
- le N-[[[4-méthyl-3-(benzoylamino)phényl]amino]thioxométhyl]-3,4,5-triméthoxy-benzamide **(I-a)** de formule suivante : 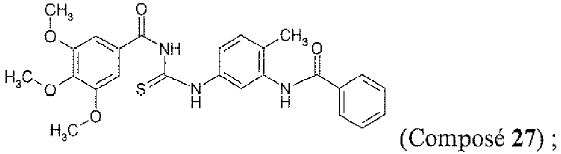
- le N-[[[3-(benzoylamino)phényl]amino]thioxométhyl]-3,4-éthylènedioxo-benzamide **(I-a)** de formule suivante : 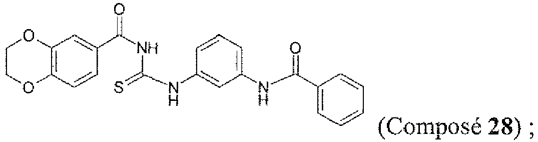
- le N-[[[3-(benzoylamino)phényl]amino]oxométhyl]-3,4,5-triéthoxy-benzamide (**I-a'**) de formule suivante : 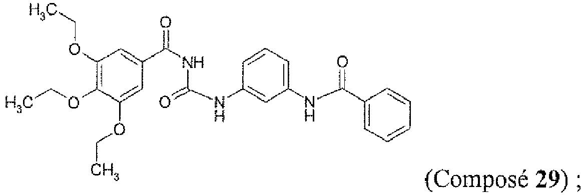
- le N-[[[3-(benzoylamino)phényl]amino]oxométhyl]-3,4-méthylènedioxy-5-méthoxy-benzamide (**I-a'**) de formule suivante : 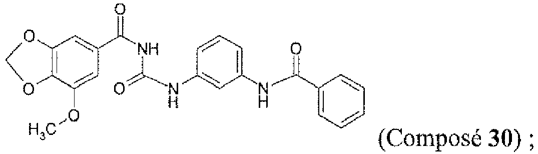
- le N-[[[3-(benzoylamino)phényl]amino]oxométhyl]-3,4,5-triméthoxy-benzamide (**I-a'**) de formule suivante : 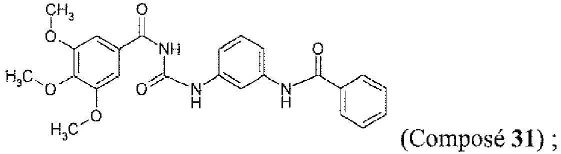
- le N-[[[3-méthyl-3-(benzoylamino)phényl]amino]thioxométhyl]-3,4,5-triméthoxy-benzamide **(I-a)** de formule suivante : 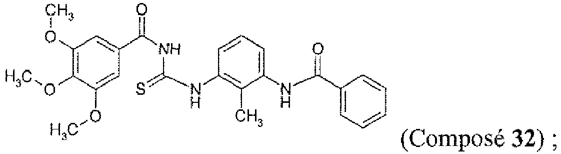
- le N-[[[4-chloro-3-(benzoylamino)phényl]amino]oxométhyl]-3,4,5-triméthoxy-benzamide **(I-a')** de formule suivante : 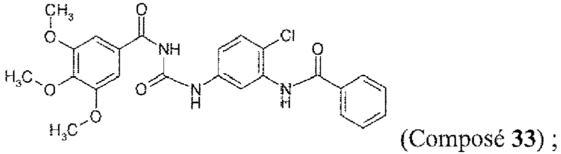
- le N-[[[4-chloro-3-(benzoylamino)phényl]amino]thioxométhyl]-3,4,5-triméthoxy-benzamide **(I-a)** de formule suivante : 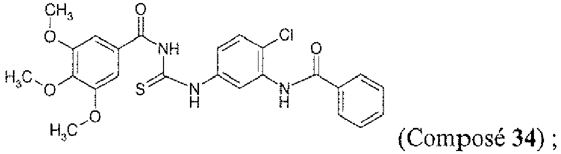
- le N-[[[5-chloro-3-(benzoylamino)phényl]amino]thioxométhyl]-3,4,5-triméthoxy-benzamide **(I-a)** de formule suivante : 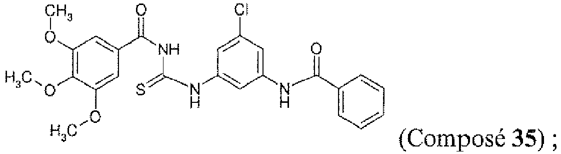
- le N-[[[5-fluoro-3-(benzoylamino)phényl]amino]thioxométhyl]-3,4,5-triméthoxy-benzamide **(I-a)** de formule suivante : 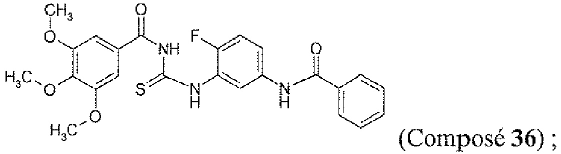
- le N-[[[4-chloro-3-(4-Phényl-benzoylamino)phényl]amino]oxométhyl]-3,4,5-triméthoxy-benzamide **(I-a')** de formule suivante : 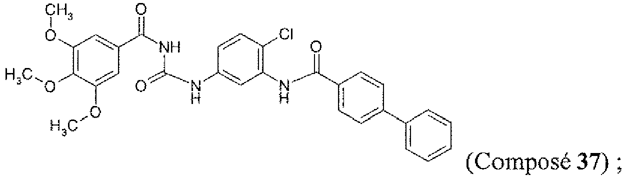
- le N-[[[3-(benzoylamino)phényl]amino]thioxométhyl]-3,4,5-triméthoxy-benzamide **(I-a)** de formule suivante : 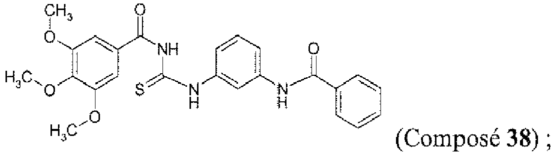
- le N-[[[4-chloro-3-(4-diméthylamino-phénylamino)carbonyl] thioxométhyl]-3,4,5-triméthoxy-benzamide (**I-b**) de formule suivante : 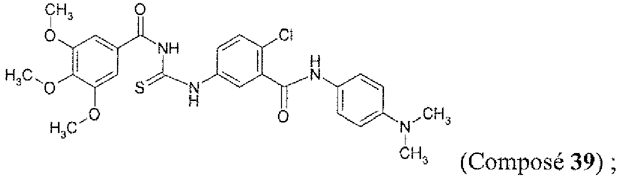
- le N-[[[4-chloro-3-(4-méthoxy-phénylamino)carbonyl]thioxométhyl]-3,4,5-triméthoxy-benzamide (**I-b**) de formule suivante : 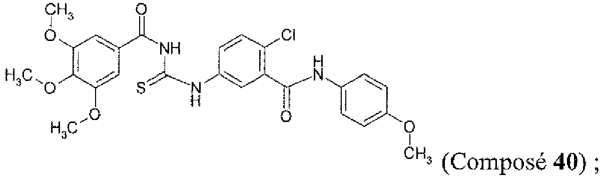
- le N-[[[5-bromo-3-(phénylamino)carbonyl]thioxométhyl]-3,4,5-triméthoxy-benzamide (**I-b**) de formule suivante : 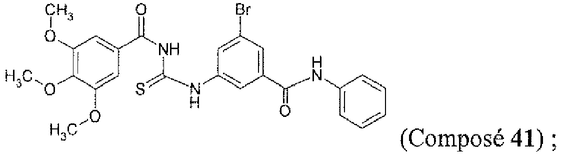
- le N-[[[4-chloro-3-(4-méthoxy-phénylamino)carbonyl]oxométhyl]-3,4,5-triméthoxy-benzamide **(I-b')** de formule suivante : 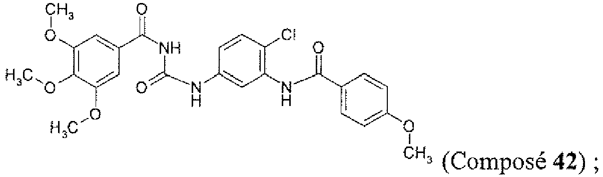
- le N-[[[4-chloro-3-(4-chloro-phénylamino)]carbonyl]phénylamino]thioxométhyl]-3,4,5-triméthoxy-benzamide **(I-b)** de formule suivante : 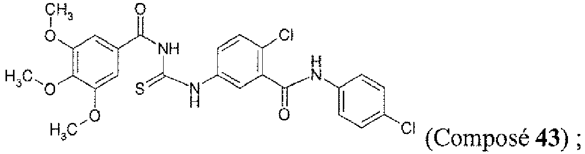
- le N-[[[4-chloro-3-(phénylamino)carbonyl]oxométhyl]-3,4,5-triméthoxy-benzamide (**I-b'**) de formule suivante : 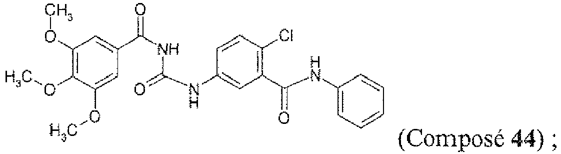
- le N-[[[2-méthyl-3-(benzoylamino)phényl]amino]oxométhyl]-3,4,5-triméthoxy-benzamide **(I-a')** de formule suivante : 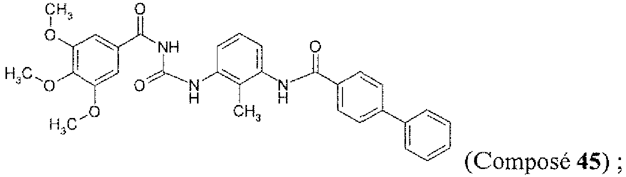
- le N-[[[2-méthyl-3-(benzoylamino)phényl]amino]thioxométhyl]-3,4,5-triméthoxy-benzamide (**I-a**) de formule suivante : 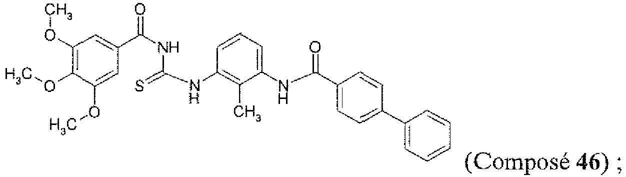
- le N-[[[-3-(4-chloro-benzoylamino)phényl]amino]oxométhyl]-3,4,5-triméthoxy-benzamide **(I-a')** de formule suivante : 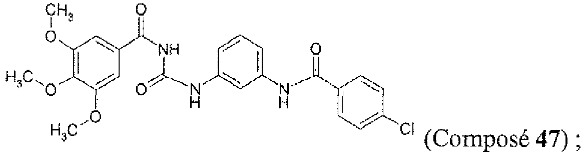
- le N-[[[-3-(benzoylamino)phényl]amino]oxométhyl]-2,4,5-triméthoxy-benzamide **(I-a')** de formule suivante : 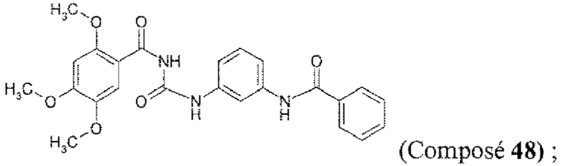
- le N-[[[-3-(benzoylamino)phényl]amino]oxométhyl]-4-méthoxy-benzamide **(I-a')** de formule suivante : 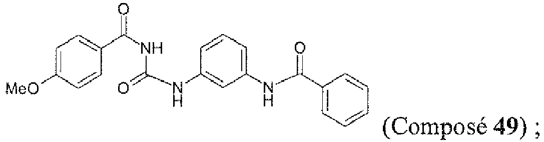
- le N-[[[-3-(benzoylamino)phényl]amino]oxométhyl]-benzamide **(I-a')** de formule suivante : 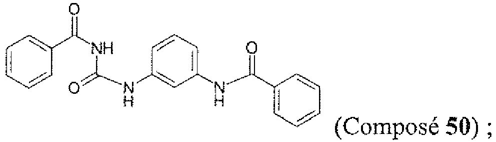
- le 3,4,5-triméthoxy-*N*-(3-(3-phényluréido)phénylcarbamothioyl)benzamide (**I-b**) de formule suivante : 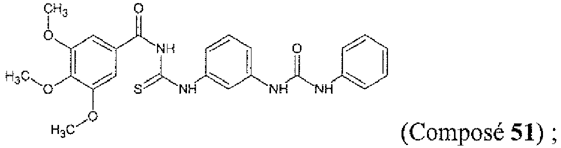
- le *N*-(3-(3-biphényl-4-yluréido)phénylcarbamothioyl)-3,4,5-triméthoxybenzamide (**I-b**) de formule : 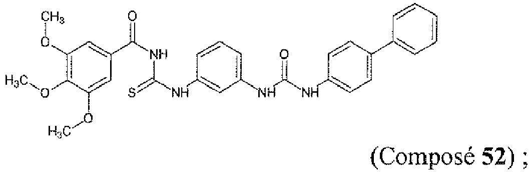
- le *N*-(3-(3-biphényl-4-yluréido)phénylcarbamoyl)-3,4,5-triméthoxybenzamide (**I**-**b'**) de formule :

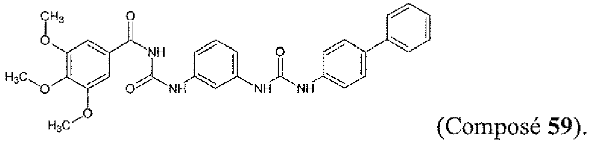

9. Utilisation selon l'une des revendications 5 à 8, **caractérisée en ce que** les composés de formule **(I)** sont choisis parmi :
- le N-[[[3-(4-méthoxy-benzoylamino)phényl]amino]thioxométhyl]-3,4,5-triméthoxy-benzamide (Composé **1**) ;
- le N-[[[3-(2-chloro-benzoylamino)phényl]amino]thioxométhyl]-3,4,5-triméthoxy-benzamide (Composé **2**) ;
- le N-[[[3-(3-méthoxy-benzoylamino)phényl]amino]thioxométhyl]-3,4,5-triméthoxy-benzamide (Composé **3**) ;
- le N-[[[3-(2-méthoxy-benzoylamino)phényl]amino]thioxométhyl]-3,4,5-triméthoxy-benzamide (Composé **4**) ;
- le N-[[[3-(4-méthoxy-benzoylamino)phényl]amino]oxométhyl]-3,4,5-triméthoxy-benzamide (Composé **6**) ;
- le N-[[[3-(4-pyridocarboxylamino)phényl]amino]thioxométhyl]-3,4,5-triméthoxy-benzamide (Composé **11**) ;
- le N-[[[3-(4-chloro-benzoylamino)phényl]amino]thioxométhyl]- 3,4,5-triméthoxy-benzamide (Composé **13**) ;
- le N-[[[3-(isopropoylcarboxylamino))phényl]amino]oxométhyl]-3,4,5-triméthoxy-benzamide (Composé **17**) ;
- le N-[[[3-(benzoylamino)phényl]amino]thioxométhyl]-3,5-diméthoxy-benzamide (Composé **18**) ;
- le N-[[[3-(benzoylamino)phényl]amino]thioxométhyl]-3,5-diméthoxy-4-éthoxy-benzamide (Composé **19**) ;
- le N-[[[3-(benzoylamino)phényl]amino]thioxométhyl]-3,4-diéthoxy-5-méthoxy-benzamide (Composé **20**) ;
- le N-[[[3-(benzoylamino)phényl]amino]thioxométhyl]-3,4-méthylénedioxy-5-méthoxy-benzamide (Composé **21**) ;
- le N-[[[3-(benzoylamino)phényl]amino]thioxométhyl]-3,4,5-triéthoxy-benzamide (Composé **23**) ;
- le N-[[[4-fluoro-3-(benzoylamino)phényl]amino]thioxométhyl]-3,4,5-triméthoxy-benzamide (Composé **24**) ;
- le N-[[[4-chloro-3-(phénylamino)carbonyl]amino]thioxométhyl]-3,4,5-triméthoxy-benzamide (Composé **25**) ;
- le N-[[[3-(benzoylamino)phényl]amino]oxométhyl]-3,4,5-triméthoxy-benzamide (Composé **31**) ;
- le N-[[[4-chloro-3-(benzoylamino)phényl]amino]thioxométhyl]-3,4,5-triméthoxy-benzamide (Composé **34**) ;
- le N-[[[3-(benzoylamino)phényl]amino]thioxométhyl]-3,4,5-triméthoxy-benzamide (Composé **38**) ;
- le N-[[[4-chloro-3-(4-diméthylamino-phénylamino)carbonyl] thioxométhyl]-3,4,5-triméthoxy-benzamide (Composé **39**) ;
- le N-[[[4-chloro-3-(4-chloro-phénylamino)]carbonyl]phénylamino]thioxométhyl]-3,4,5-triméthoxy-benzamide (Composé **43**) ;
- le N-[[[2-méthyl-3-(benzoylamino)phényl]amino]oxométhyl]-3,4,5-triméthoxy-benzamide (Composé **45**) ;
- le N-[[[-3-(4-chloro-benzoylamino)phényl]amino]oxométhyl],-3,4,5-triméthoxy-benzamide (Composé **47**) ;
- le 3,4,5-triméthoxy-*N*-(3-(3-phényluréido)phénylcarbamothioyl)benzamide (**I-b**) (Composé **51**),
- le *N*-(3-(3-biphényl-4-yluréido)phénylcarbamothioyl)-3,4,5-triméthoxybenzamide **(I-b)** (Composé **52**) ; et
- le *N*-(3-(3-biphényl-4-yluréido)phénylcarbamoyl)-3,4,5-triméthoxybenzamide (**I**-**b**') (Composé **59**).

10. Utilisation selon l'une des revendications 5 à 9, dans laquelle la pathologie de type neuro-dégénératif est la maladie de Parkinson, la Chorée de Huntington, la maladie d'Alzheimer, la sclérose en plaques ou la maladie du motoneurone.

11. Composés de formule **(I')** suivante : 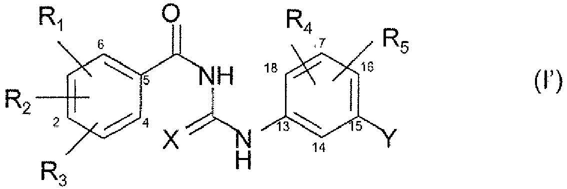 dans laquelle :
- R₁, R₂ et R₃, identiques ou différents et indépendamment les uns des autres, représentent un atome d'hydrogène ou d'halogène, un radical hydroxyle, un groupement alkyle, perfluoroalkyle, alcoxy, alkylthio, nitrile, un alcoxy substitué, ou un hétérocycle fusionné obtenu à partir de deux des radicaux R₁, R₂ et R₃, adjacents, pouvant former, conjointement avec les atomes de carbone du cycle phényle auxquels ils sont liés, un hétérocycle fusionné ;
- X représente un atome de soufre,
- Y représente un groupement -NH-(C=O)-R₆ ou -NH-(C-O)-NH-R₆ dans lequel R₆ représente un groupement aryle non substitué ; un groupement aryle comportant un ou plusieurs substituants choisis parmi un atome d'halogène et un radical alkyle, alcoxy ou mono- ou dialkylamino ; un groupement hétéroaryle mono- ou polycyclique ; un radical alkyle ramifié ; un groupement hydrocarboné mono- ou polycyclique saturé ou insaturé ;
- R₄ et R₅, identiques ou différents et indépendamment l'un de l'autre, représentent un atome d'hydrogène ou d'halogène, un groupe alcoxy, alkylthio, alkyle, perfluoroalkyle, nitrile ou nitro.

12. Composés selon la revendication 11, **caractérisés en ce qu'**ils sont choisis parmi :
- le N-[[[3-(4-méthoxy-benzoylamino)phényl]amino]thioxométhyl]-3,4,5-triméthoxy-benzamide **(I-a)** de formule suivante : 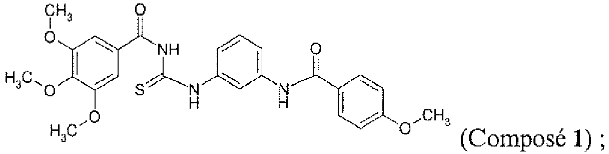
- le N-[[[3-(2-chloro-benzoylamino)phényl]amino]thioxométhyl]-3,4,5-trimethoxy-benzamide **(I-a)** de formule suivante : 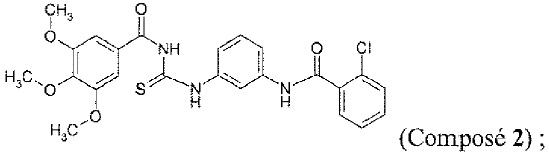
- le N-[[[3-(3-méthoxy-benzoylamino)phényl]amino]thioxométhyl]-3,4,5-triméthoxy-benzamide **(I-a)** de formule suivante : 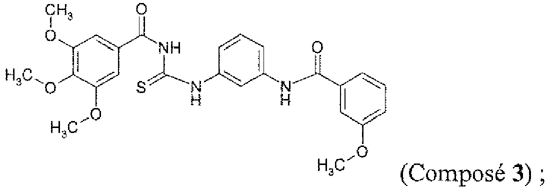
- le N-[[[3-(2-méthoxy-benzoyiamino)phényl]amino]thioxométhyl]-3,4,5-triméthoxy-benzamide **(I-a)** de formule suivante : 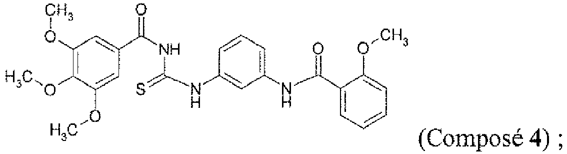
- le N-[[[3-(4-phényi-benzoylamino)phényl]amino]thioxométhyl]-3,4,5-triméthoxy-benzamide **(I-a)** de formule suivante : 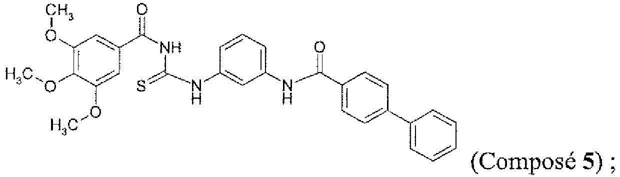
- le N-[[[3-(-cyclohexylcarboxylamino)phényl]amino]thioxométhyl]-3,4,5-triméthoxy-benzamide **(I-a)** de formule suivante : 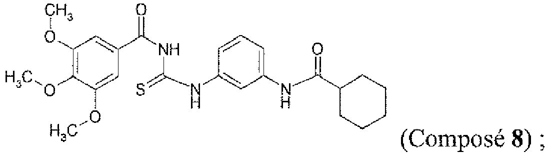
- le N-[[[3-(2-naphtoylamino)phényl]amino]thioxométhyl]-3,4,5-triméthoxy-benzamide **(I-a)** de formule suivante : 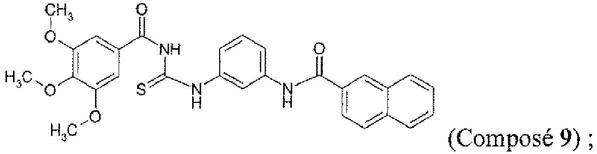
- le N-[[[3-(isopropoylcarboxylamino)phényl]amino]thioxométhyl]-3,4,5-triméthoxy-benzamide **(I-a)** de formule suivants : 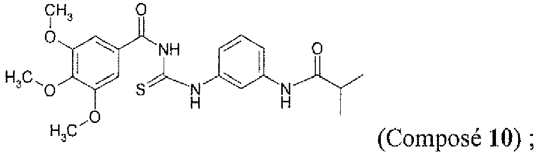
- le N-[[[3-(4-pyridocarboxylamino)phényl]amino]thioxométhyl]-3,4,5-triméthoxy-benzamide (**I**-**a**) de formule suivante : 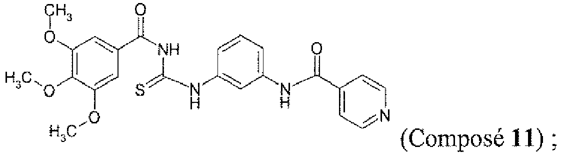
- le N-[[[3-(1-naphtoylamino)phényl]amino]thioxométhyl]-3,4,5-triméthoxy-benzamide **(I-a)** de formule suivante : 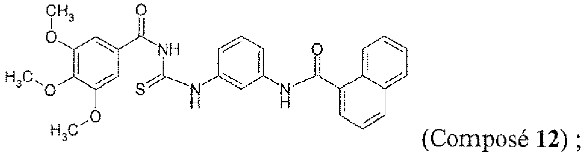
- le N-[[[3-(4-chloro-benzoylamino)phényl]amino]thioxométhyl]-3,4,5-triméthoxy-benzamide **(I-a)** de formule suivante : 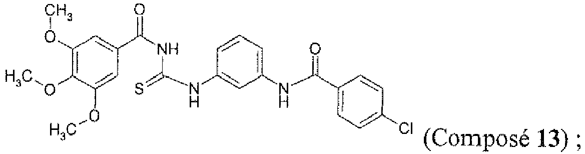
- le N-[[[3-(2-furoylamino)phényl]amino]thioxométhyl]-3,4,5-triméthoxy-benzamide **(I-a)** de formule suivante : 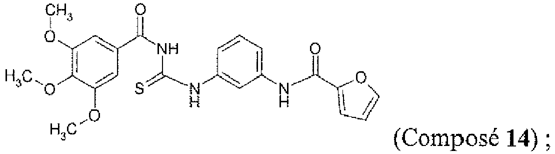
- le N-[[[3-(2-thiophénoylamino)phényl]amino]thioxométhyl]-3,4,5-triméthoxy-benzamide **(I-a)** de formule suivante : 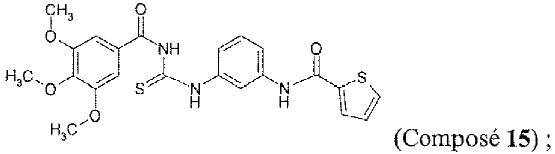
- le N-[[[3-(benzoylamino)phényl]amino]thioxométhyl]-3,5-diméthoxy-benzamide (**I**-**a**) de formule suivante : 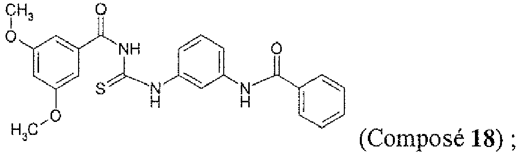
- le N-[[[3-(benzoylamino)phényl]amino]thioxométhyl]-3,5-diméthoxy-4-éthoxy-benzamide **(I-a)** de formule suivante : 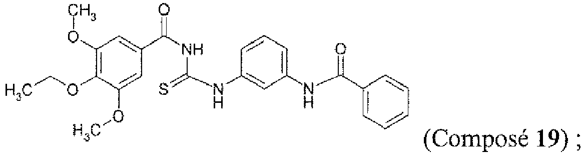
- le N-[[[3-(benzoylamino)phényl]amino]thioxométhyl]-3,4-diéthoxy-5-méthoxy-benzamide **(I-a)** de formule suivante : 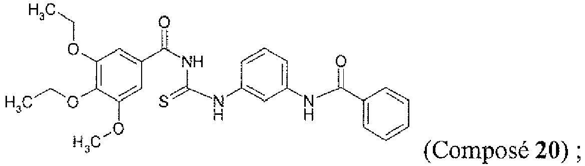
- le N-[[[3-(benzoylamino)phényl]amino]thioxométhyl]-3,4-méthyîènedioxy-5-méthoxy-benzamide (**I**-**a**) de formule suivante : 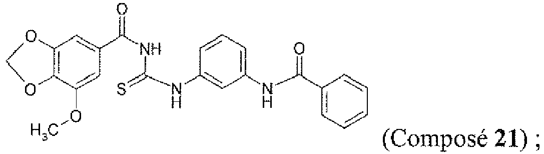
- le N-[[[3-(benzoylammo)phényl]amino]thioxométhyl]-2,4-dimétlioxy-benzamide **(I-a)** de formule suivante : 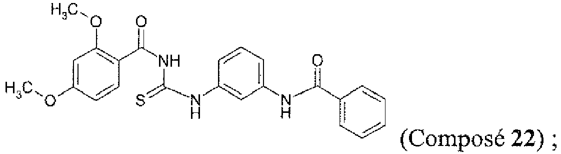
- le N-[[[3-(benzoylamino)phényl]amino]thioxométhyl]-3,4,5-triéthoxy-benzamide **(I-a)** de formule suivante : 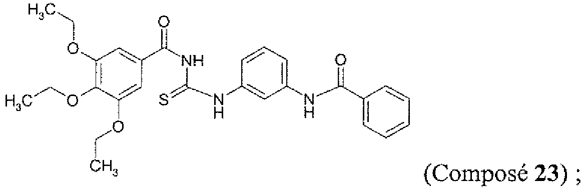
- le N-[[[4-fluoro-3-(benzoylamino)phényl]amino]thioxométhyl]-3,4,5-triméthoxy-benzamide **(I-a)** de formule suivante : 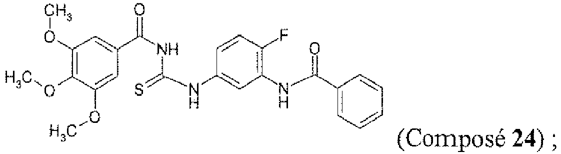
- le N-[[[4-chloro-3-(phénylamino)carbonyl]amino]thioxométhyl]-3,4,5-triméthoxy-benzamide **(I-b)** de formule suivante : 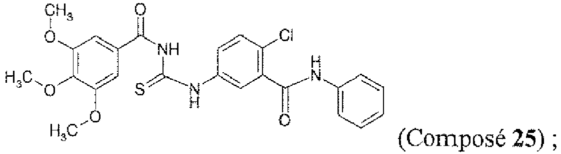
- le N-[[[4-méthoxy-3-(benzoylamino)phényl]amino]thioxométhyl]-3,4,5-triméthoxy-benzamide (**I**-**a**) de formule suivante : 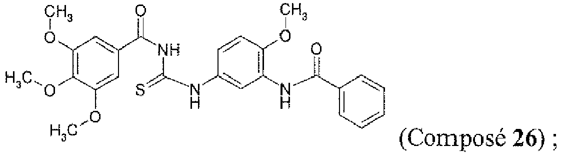
- le N-[[[4-méthyl-3-(benzoylamino)phényl]amino]thioxométhyl]-3,4,5-triméthoxy-benzamide **(I-a)** de formule suivante : 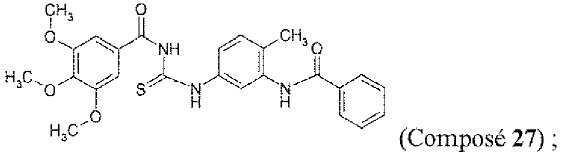
- le N-[[[3-(bonzoylamino)phényl]amino]thioxométhyl]-3,4-éthylènedioxo-benzamide **(I-a)** de formule suivante : 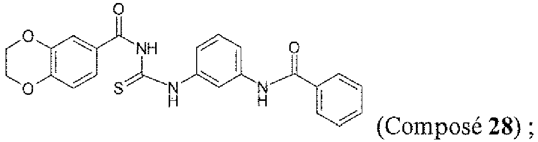
- le N-[[[3-méthyl-3-(benzoylamino)phényl]amino]thioxométhyl]-3,4,5-triméthoxy-benzamide **(I-a)** de formule suivante : 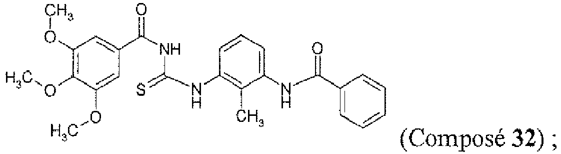
- le N-[[[4-chloro-3-(benzoylamino)phényl]amino]thioxométhyl]-3,4,5-triméthoxy-benzamide **(I-a)** de formule suivante : 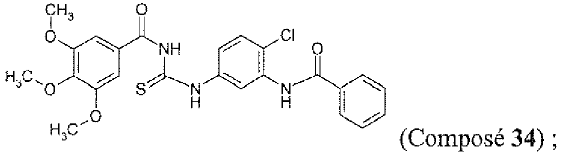
- le N-[[[5-chloro-3-(benzoylamino)phényl]amino]thioxométhyl]-3,4,5-tnméthoxy-benzamide **(I-a)** de formule suivante : 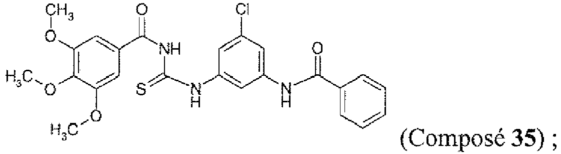
- le N-[[[5-fluoro-3-(benzoylamino)phényl]amino]thioxométhyl]-3,4,5-triméthoxy-benzamide **(I-a)** de formule suivante : 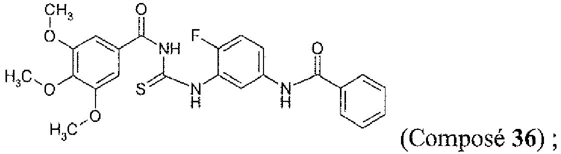
- le N-[[[3-(benzoylamino)phényl]amino]thioxométhyl]-3,4,5-triméthoxy-benzamide **(I-a)** de formule suivante : 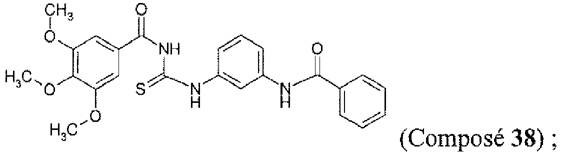
- le N-[[[2-méthyl-3-(benzoylamino)phényl]amino]thioxométhyl]-3,4,5-triméthoxy-benzamide (**I**-**a**) de formule suivante : 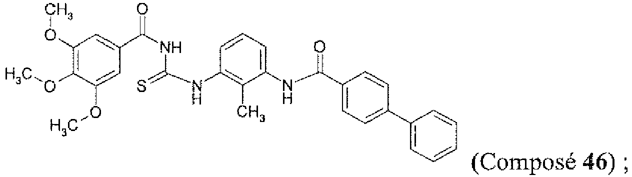
- le 3,4,5-triméthoxy-*N-*(3-(3-phényluréido)phénylcarbamothioyl)benzamide (**I**-**b**) de formule suivante : 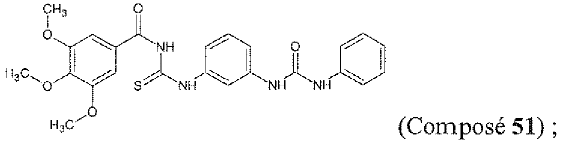
- le *N-*(3-(3-biphényl-4-yluréido)phénylcarbamothioyl)-3,4,5-triméthoxybenzamide **(I-b)** de formule:

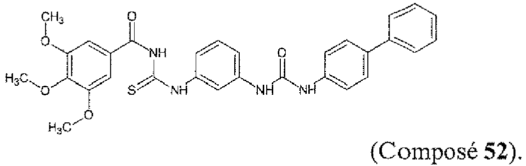

13. Composés selon la revendication 11 ou 12, **caractérisés par le fait qu'**ils sont choisis parmi :
- le N-[[[3-(4-méthoxy-benzaylamino)phényl]amino]thioxométhyl]-3,4,5-triméthoxy-benzamide (Composé **1**) ;
- le N-[[[3-(2-chloro-benzoylamino)phényl]amino]thioxométhyl]-3,4,5-triméthoxy-benzamide (Composé **2**) ;
- le N-[[[3-(3-méthoxy-benzoylamino)phényl]amino]thioxométhyl]-3,4,5-triméthoxy-benzamide (Composé **3**) ;
- le N-[[[3-(2-méthoxy-benzoylamino)phényl]amino]thioxométhyl]-3,4,5-triméthoxy-benzamide (Composé **4**) ;
- le N-[[[3-(4-pyridocarboxylamino)phényl]amino]thioxométhyl]-3,4,5-triméthoxy-benzamide (Composé **11**) ;
- le N-[[[3-(4-chloro-benzoylamino)phényl]amino]thioxométhyl]- 3,4,5-triméthoxy-benzamide (Composé **13**) ;
- le N-[[[3-(benzoylamino)phényl]amino]thioxométhyl]-3,5-diméthoxy-benzamide (Composé **18**) ;
- le N-[[[3-(benzoylamino)phényl]amino]thioxométhyl]-3,5-diméthoxy-4-éthoxy-benzamide (Composé **19**) ;
- le N-[[[3-(benzoylamino)phényl]amino]thioxométhyl]-3,4-diéthoxy-5-méthoxy-benzamide (Composé **20**) ;
- le N-[[[3-(benzoylamino)phényl]amino]thioxométhyl]-3,4-méthylènedioxy-5-méthoxy-benzamide (Composé **21**) ;
- le N-[[[3-(benzoylamino)phényl]amino]thloxométhyl]-3,4,5-triéthoxy-benzamide (Composé **23**) ;
- le N-[[[4-fluoro-3-(benzoylamino)phényl]amino]thioxométhyl]-3,4,5-triméthoxy-benzamide (Composé **24**) ;
- le N-[[[4-chloro-3-(phénylamino)carbonyl]amino]thioxométhyl]-3,4,5-triméthoxy-benzamide (Composé **25**) ;
- le N-[[[4-chloro-3-(benzoylamino)phényl]amino]thioxométhyl]-3,4,5-triméthoxy-benzamide (Composé **34**) ;
- le N-[[[3-(benzoylamino)phényl]amino]thioxométhyl]-3,4,5-triméthoxy-benzamide (Composé **38**) ;
- le 3,4,5-triméthoxy-*N-*(3-(3-phényluréido)phénylcarbamothioyl)benzamide **(I-b)** ) (Composé **51**),
- le *N-*(3-(3-biphényl-4-yluréido)phénylcarbamothïoyl)-3,4,5-triméthoxybenzamide **(I-b)** (Composé **52**).

14. Composition pharmaceutique **caractérisée en ce qu'**elle comprend, à titre de principe actif, au moins un composé tel que défini selon l'une des revendications 11 à 13, et au moins un excipient pharmaceutiquement acceptable.

15. Utilisation des composés tels que définis selon l'une des revendications 11 à 13 pour la fabrication d'un médicament pour le traitement des pathologies de type neuro-dégénératif

16. Utilisation selon la revendication 15 pour le traitement de la maladie de Parkinson, la Chorée de Huntington, la maladie d'Alzheimer, la sclérose en plaques et la maladie du motoneurone.

17. Utilisation des composés tels que définis selon l'une des revendications 11 à 13 pour la fabrication d'un médicament pour le traitement du diabète.

18. Composés tels que définis selon l'une des revendications 11 à 13 pour leur utilisation dans le traitement des pathologies de type neuro-dégénératif.

19. Composés selon la revendication 18 pour leur utilisation dans le traitement de la maladie de Parkinson, la Chorée de Huntington, la maladie d'Alzheimer, la sclérose en plaques et la maladie du motoneurone.

20. Composés tels que définis selon l'une des revendications 11 à 13 pour leur utilisation dans le traitement du diabète.

## Patentansprüche

1. Verwendung von Verbindungen mit der folgenden Formel (I): 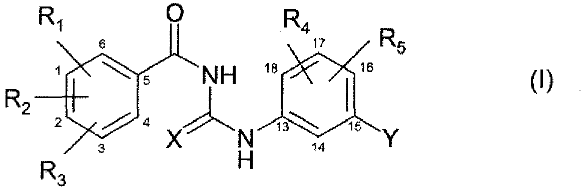 wobei:
- R₁, R₂ und R₃, identisch oder verschieden und unabhängig voneinander, ein Wasserstoff- oder Halogenatom darstellen, ein Hydroxylradiakal, eine Alkyl-, Perfluoroalkyl-, Alcoxy-, Alkylthio-, NitrilGruppierung, ein substituiertes Alcoxy oder einen fusionierten Heterocyclus, erhalten auf der Grundlage von zwei der benachbarten Radikale R₁, R₂ und R₃, die zusammen mit den Kohlenstoffatomen des Phenylcyclus, mit dem sie verbunden sind, einen fusionierten Heterocyclus bilden können,
- X ein Schwefel- oder Sauerstoffatom darstellt,
- Y eine mono- oder polycyclische Heteroaryl-Gruppierung darstellt, substituiert oder nicht substituiert, eine Gruppierung -NH-(C=O)-R₆, -(C=O)-NH-R₆ oder -NH-(C=O)-NH-R₆, wobei R₆ eine nicht substituierte Arylgruppierung darstellt; eine Arylgruppierung, umfassend einen oder mehrere Substituenten, ausgewählt aus einem Halogenatom und einem Alkyl-, Alcoxy oder Mono- oder Dialkylaminoradiakal; eine mono- oder polycyclische Heteroaryl-Gruppierung; ein lineares oder verzweigtes Alkylradikal; eine gesättigte oder ungesättigte mono oder polycyclische Kohlenwasserstoff-Gruppierung;
- R₄ und R₅, identisch oder verschieden und unabhängig voneinander, ein Wasserstoff- oder Halogenatom darstellen, eine Alcoxy-, Alkylthio-, Alkyl-, Perfluoroalkyl-, Nitril- oder Nitro-Gruppe,
zur Herstellung eines Arzneimittels zur Behandlung von Tumoren, die mit einer Hyperaktivierung des Signalwegs der Hedgehog-Proteine assoziiert sind, wobei die Tumore ausgewählt sind aus den Tumoren des Nervengewebes (Medulloblastome, primitive neuroektodermale Tumore, Glioblastome, Meningiome oder Ologodendrogliome) Hauttumoren (Basalzellkarzinom, Trichoepitheliom), Tumoren der Muskel- und Knochengewebe (Rhabdomyosarkome, Osteosarkome) oder Tumoren von anderen Geweben (Niere, Blase) oder Pathologien der neurodegenerativen Art, ausgewählt aus einer multiplen Sklerose oder einer Motoneuronenerkrankung.

2. Verwendung nach Anspruch 1, **dadurch gekennzeichnet, dass** die Verbindungen ausgewählt sind aus denjenigen, in denen, in der Formel (I):
- R₁, R₂ und R₃, identisch oder verschieden, ein Wasserstoffatom oder ein Methyloxy- oder Ethyloxyradikal darstellen,
- X ein Schwefel- oder Sauerstoffatom darstellt,
- Y eine mono- oder polycyclische Heteroaryl-Gruppierung darstellt, substituiert oder nicht substituiert, eine Gruppierung -NH-(C=O)-R₆, -(C=O)-NH-R₆ oder -NH-(C=O)-NH-R₆, wobei R₆ eine Gruppierung darstellt, ausgewählt aus den Phenyl-Gruppierungen; Phenyl, substituiert durch ein Methoxy- oder Dimethylaminoradikal, durch ein Chloratom, durch eine Phenyl-Gruppierung, durch eine Benzyl-, Cyclohexyl-, Isopropyl-, Pyridinyl-, Naphtyl-, Furfinyl- und Thiophen- Gruppierung; und
- R₄ und R₅, identisch oder verschieden, ausgewählt sind aus Wasserstoff, Chlor, Brom, Fluor, Methyl und Methoxy.

3. Verwendung nach einem der Ansprüche 1 bis 2, **dadurch gekennzeichnet, dass** die Verbindungen der Formel (I) ausgewählt sind aus:
- N-[[[3-(4-Methoxy-benzoylamino)phenyl]amino]thioxnmethyl]-3,4,5-trimethoxy-benzamid (I-a) mit der folgenden Formel: 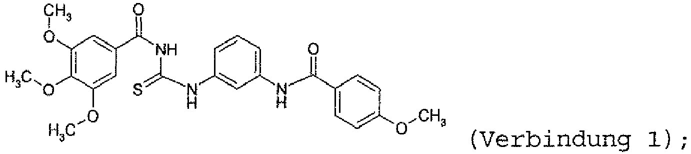
- N-[[[3-(2-Chloro-benzoylamino)phenyl]amino]thioxomethyl]-3,4,5-trimethoxy-benzamid (I-a) mit der folgenden Formel: 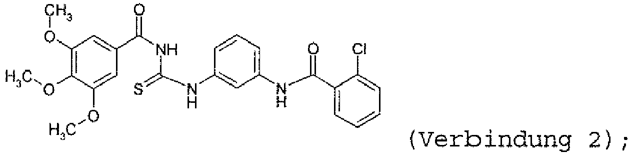
- N-[[[3-(3-Methoxy-benzoylamino)phenyl]amino]thioxomethyl]-3,4,5-trimethoxy-benzamid (I-a) mit der folgenden Formel: 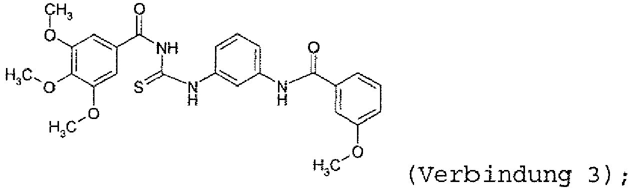
- N-[[[3-(2-Methoxy-benzoylamino)phenyl]amino]thioxomethyl]-3,4,5-trimethoxy-benzamid (I-a) mit der folgenden Formel: 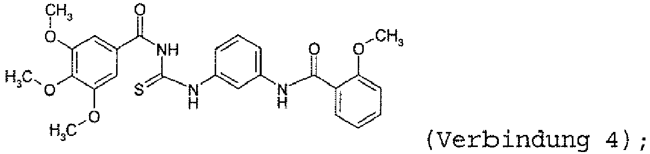
- N-[[[3-(4-Phenyl-benzoylamino)phenyl]amino]thioxomethyl]-3,4,5-trimethoxy-benzamid (I-a) mit der folgenden Formel: 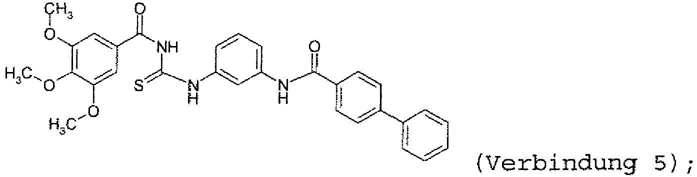
- N-[[[3-(4-Methoxy-benzoylamino)phenyl]amino]oxomethyl]-3,4,5-trimethoxy-benzamid (I-a') mit der folgenden Formel: 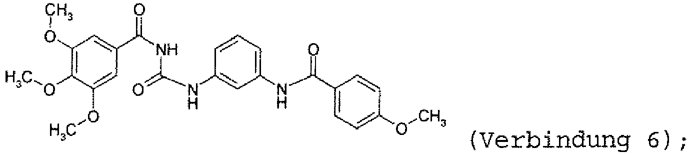
- N-[[[3-(4-Phenyl-benzoylamino)phenyl]amino]oxomethyl]-3,4,5-trimethoxy-benzamid (I-a') mit der folgenden Formel: 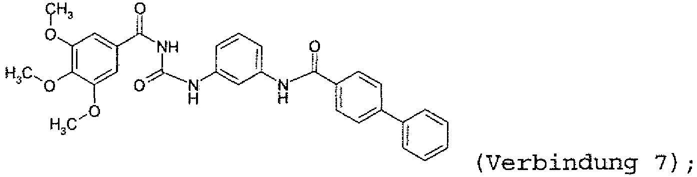
- N-[[[3-(-Cyclohexylcarboxylamino)phenyl]amino]thioxomethyl]-3,4,5-trimethoxy-benzamid (I-a) mit der folgenden Formel: 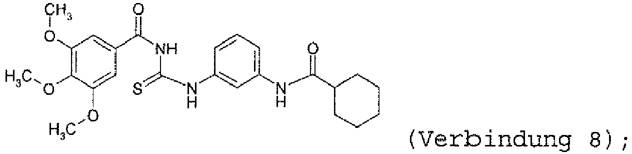
- N-[[[3-(2-Naphtoylamino)phenyl]amino]thioxomethyl]-3,4,5-trimethoxy-benzamid (I-a) mit der folgenden Formel: 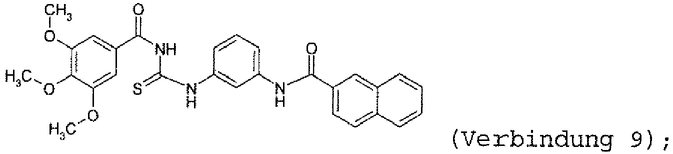
- N-[[[3-(Isopropoylcarboxylamino)phenyl]amino]thioxomethyl]-3,4,5-trimethoxy-benzamid (I-a) mit der folgenden Formel: 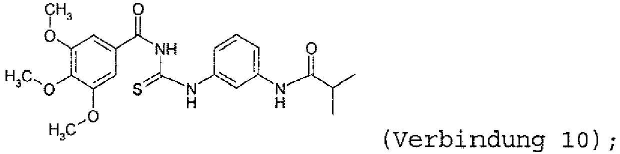
- N-[[[3-(4-Pyridocarboxylamino)phenyl]amino]thioxomethyl]-3,4,5-trimethoxy-benzamid (I-a) mit der folgenden Formel: 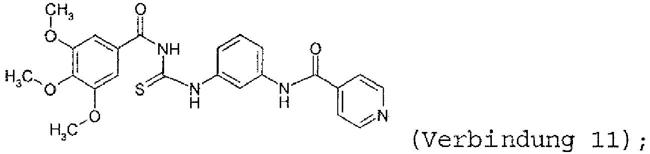
- N-[[[3-(1-Naphtoylamino)phenyl]amino]thioxomethyl]-3,4,5-trimethoxy-benzamid (I-a) mit der folgenden Formel: 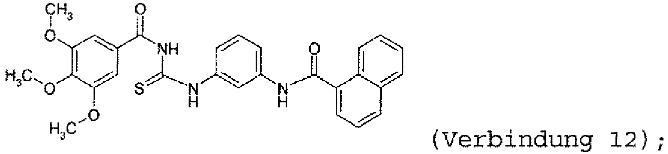
- N-[[[3-(4-Chloro-benzoylamino)phenyl]amino]thioxomethyl]-3,4,5-trimethoxy-benzamid (I-a) mit der Folgenden Formel: 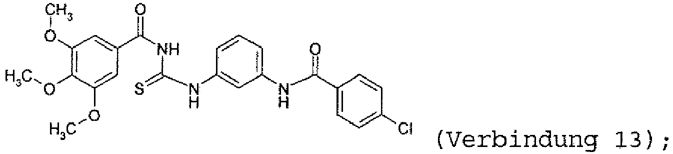
- N-[[[3-(2-Furoylamino)phenyl]amino]thioxomethyl]-3,4,5-trimethoxy-benzamid (I-a) mit der folgenden Formel: 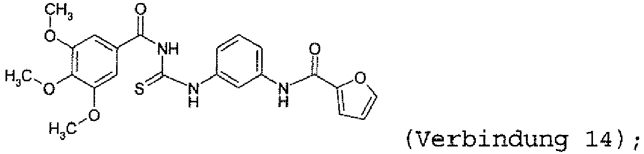
- N-[[[3-(2-Thiophénoylamino)phenyl]amino]thioxomethyl]-3,4,5-trimethoxy-benzamid (I-a) mit der folgenden Formel: 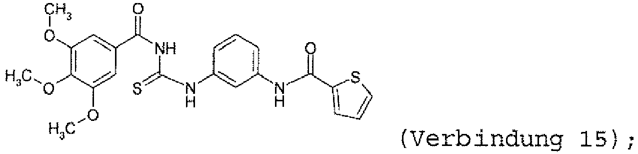
- N-[[[3-(2-Naphtoylamino)phenyl]amino]oxomethyl]-3,4,5-trimethoxy-benzamid (I-a') mit der folgenden Formel: 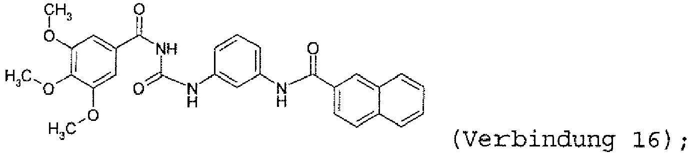
- N-[[[3-(Isopropoylcarboxylamino))phenyl]amino]oxomethyl]-3,4,5-trimethoxy-benzamid (I-a') mit der folgenden Formel: 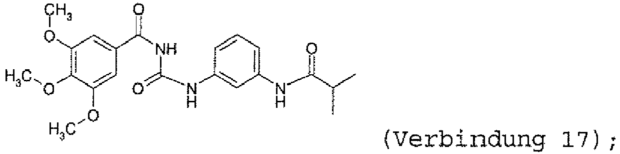
- N-[[[3-(Benzoylamino)phenyl]amino]thioxomethyl]-3,5-dimethoxy-benzamid (I-a) mit der folgenden Formel: 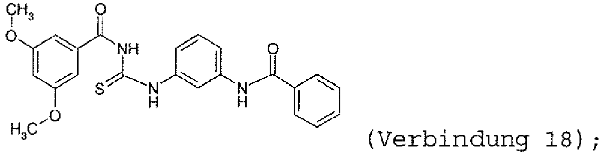
- N-[[[3-(Benzoylamino)phenyl]amino]thioxomethyl]-3,5-dimethoxy-4-ethoxy-benzamid (I-a) mit der folgenden Formel: 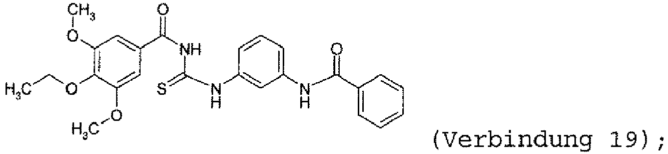
- N-[[[3-(Benzoylamino)phenyl]amino]thioxomethyl]-3,4-diethoxy-5-methoxy-benzamid (I-a) mit der folgenden Formel: 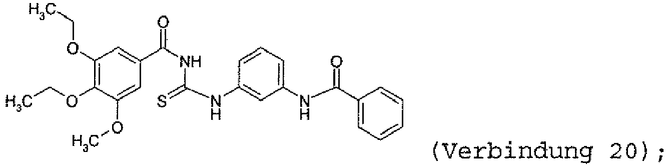
- N-[[[3-(Benzoylamino)phenyl]amino]thioxomethyl]-3,4-methylendioxy-5-methoxy-benzamid (I-a) mit der folgenden Formel: 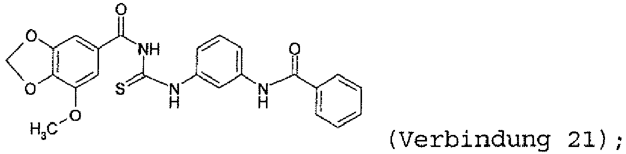
- N-[[[3-(Benzoylamino)phenyl]amino]thioxomethyl]-2,4-dimethoxy-benzamid (I-a) mit der folgenden Formel: 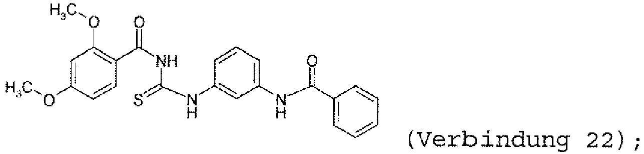
- N-[[[3-(Benzoylamino)phenyl]amino]thioxomethyl]-3,4,5-triethoxy-benzamid (I-a) mit der folgenden Formel: 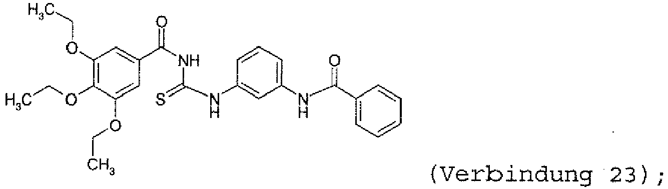
- N-[[[4-Fluoro-3-(benzoylamino)phenyl]amino]thioxomethyl]-3,4,5-trimethoxy-benzamid (I-a) mit der folgenden Formel: 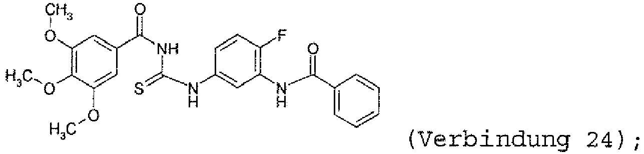
- N-[[[4-Chloro-3-(ph3nylamino)carbonyl]amino]thioxomethyl]-3,4,5-trimethoxy-benzamid (I-b) mit der folgenden Formel: 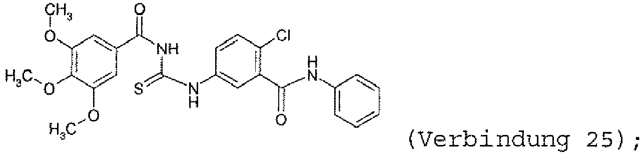
- N-[[[4-Methoxy-3-(benzoylamino)phenyl]amino]thioxomethyl]-3,4,5-trimethoxy-benzamid (I-a) mit der folgenden Formel: 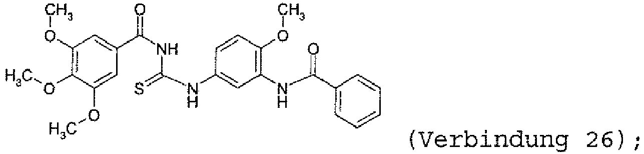
- N-[[[4-Methyl-3-(benzoylamino)phenyl]amino]thioxomethyl]-3,4,5-trimethoxy-benzamid (I-a) mit der folgenden Formel: 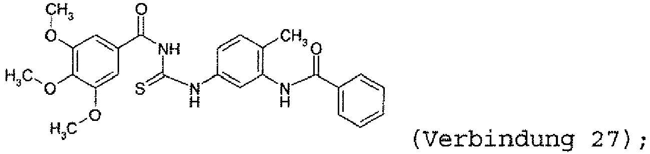
- N-[[[3-(Benzoylamino)phenyl]amino]thioxomethyl]-3,4-ethylendioxo-benzamid (I-a) mit der folgenden Formel: 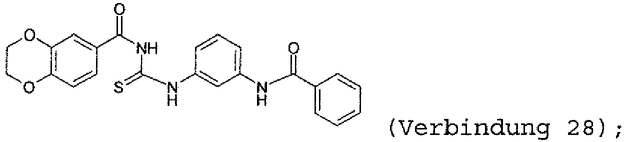
- N-[[[3-(Benzoylamino)phenyl]amino]oxomethyl]-3,4,5-triethoxy-benzamid (I-a') mit der folgenden Formel: 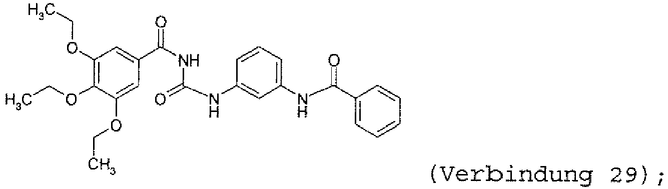
- N-[[[3-(Benzoylamino)phenyl]amino]oxomethyl]-3,4-methylendioxy-5-methoxy-benzamid (I-a') mit der folgenden Formel: 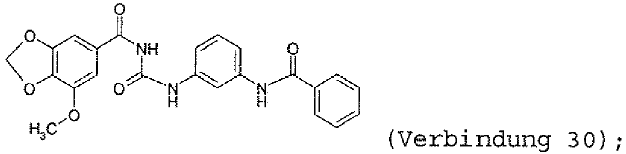
- N-[[[3-(Benzoylamino)phenyl]amino]oxomethyl]-3,4,5-trimethoxy-benzamid (I-a') mit der folgenden Formel: 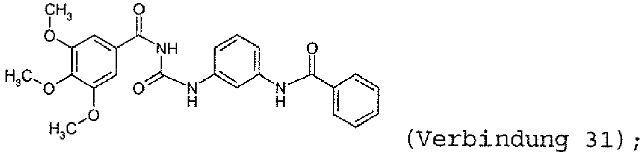
- N-[[[3-Methyl-3-(benzoylamino)phenyl]amino]thioxomethyl]-3,4,5-trimethoxy-benzamid (I-a) mit der folgenden Formel: 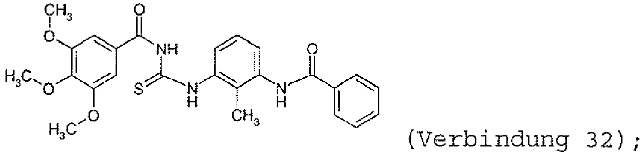
- N-[[[4-Chloro-3-(benzoylamino)phenyl]amino]oxomethyl]-3,4,5-trimethoxy-benzamid (I-a') mit der folgenden Formel: 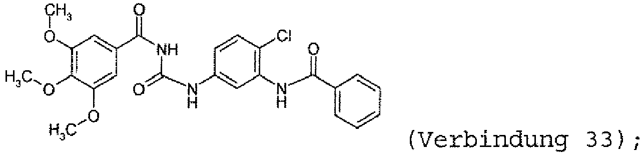
- N-[[[4-Chloro-3-(benzoylamino)phenyl]amino]thioxomethyl]-3,4,5-trimethoxy-benzamid (I-a) mit der folgenden Formel: 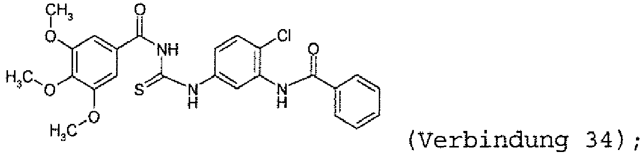
- N-[[[5-Chloro-3-(benzoylamino)phenyl]amino]thioxomethyl]-3,4,5-trimethoxy-benzamid (I-a) mit der folgenden Formel: 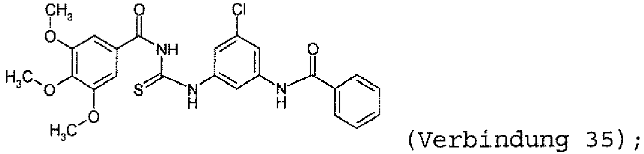
- N-[[[5-Fluoro-3-(benzoylamino)phenyl]amino]thioxomethyl]-3,4,5-trimethoxy-benzamid (I-a) mit der folgenden Formel: 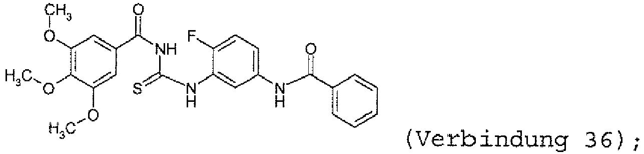
- N-[[[4-Chloro-3-(4-phenyl-benzoylamino)phenyl]amino]oxomethyl]-3,4,5-trimethoxy-benzamid (I-a') mit der folgenden Formel: 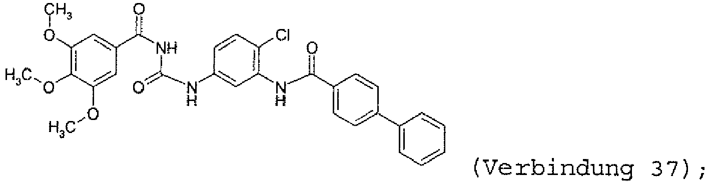
- N-[[[3-(Benzoylamino)phenyl]amino]thioxomethyl]-3,4,5-trimethoxy-benzamid (I-a) mit der folgenden Formel: 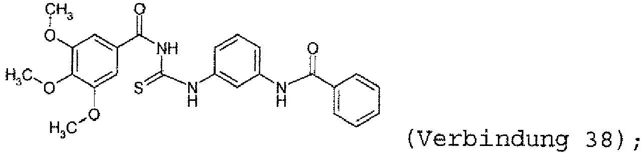
- N-[[[4-Chloro-3-(4-dimethylamino-phenylamino)carbonyl]thioxomethyl]-3,4,5-trimethoxy-benzamid (I-b) mit der folgenden Formel: 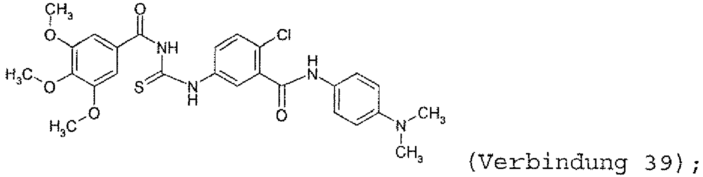
- N-[[[4-Chloro-3-(4-dimethylamino-phenylamino)caxbonyl]thiaxomethyl]-3,4,5-trimethoxy-benzamid (I-b) mit der folgenden Formel: 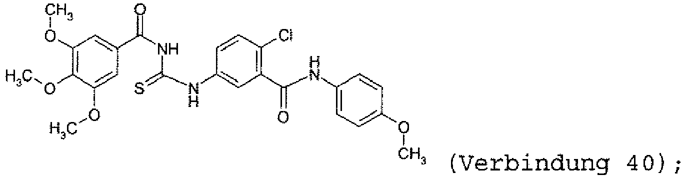
- N-[[[5-Bromo-3-(phenylamino)carbonyl]thioxomethyl]-3,4,5-trimethoxy-benzamid (I-b) mit der folgenden Formel: 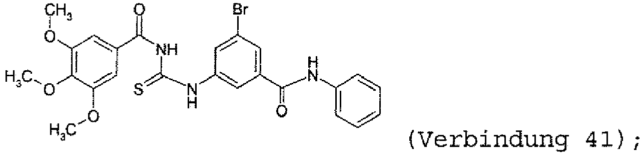
- N-[[[4-Chloro-3-(4-methoxy-phenylamino)carbonyl]oxomethyl]-3,4,5-trimethoxy-benzamid (I-b') mit der folgenden Formel: 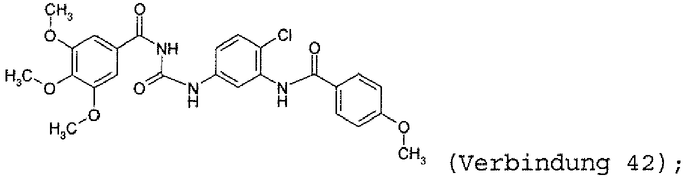
- N-[[[4-Chloro-3-(4-chloro-phenylamino)]carbonyl]phenylamino]thioxomethyl]-3,4,5-trimethoxy-benzamid (I-b) mit der folgenden Formel: 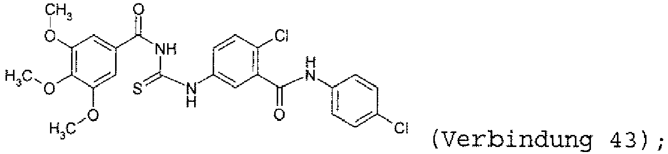
- N-[[[4-Chloro-3-(phenylamino)carbonyl]oxomethyl]-3,4,5-trimethoxy-benzamid (I-b') mit der folgenden Formel: 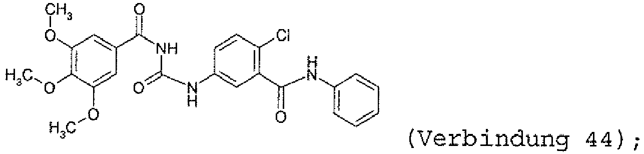
- N-[[[2-Methyl-3-(benzoylamino)phenyl]amino]oxomethyl]-3,4,5-trimethoxy-benzamid (I-a') mit der folgenden Formel: 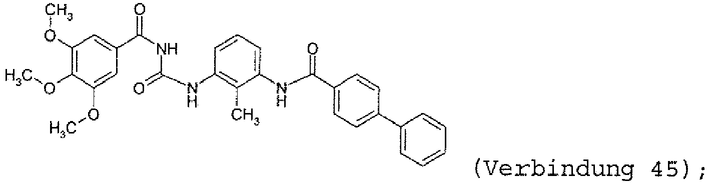
- N-[[[2-Methyl-3-(benzoylamino)phenyl]amino]thioxomethyl]-3,4,5-trimethoxy-benzamid (I-a) mit der folgenden Formel: 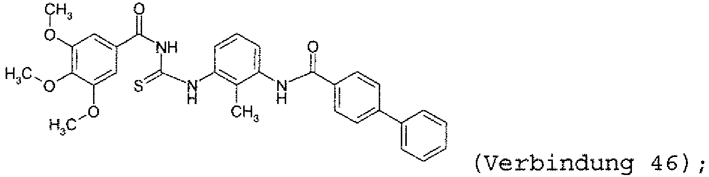
- N-[[[-3-(4-Chloro-benzoylamino)phenyl]amino]oxomethyl]-3,4,5-trimethoxy-benzamid (I-a') mit der folgenden Formel: 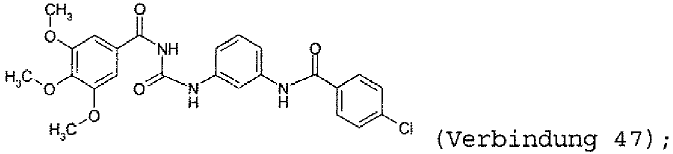
- N-[[[-3-(Benzoylamino)phenyl]amino]oxomethyl]-2,4,5-trimethoxy-benzamid (I-a') mit der folgenden Formel: 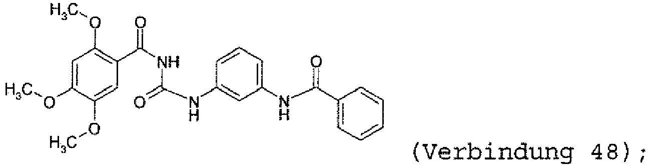
- N-[[[-3-(Benzoylamino)phenyl]amino]oxomethyl]-4-methoxy-benzamid (I-a') mit der folgenden Formel: 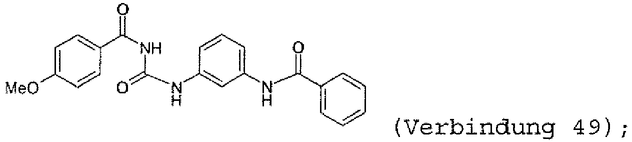
- N-[[[-3-(Benzoylamino)phenyl]amino]oxomethyl]-benzamid (I-a') mit der folgenden Formel: 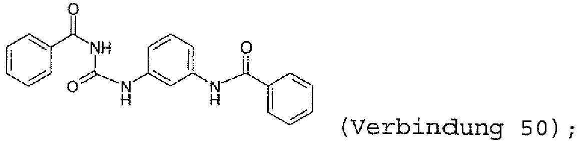
- 3,4,5-Trimethoxy-*N*-(3-(3-phenylureido)phenylcarbamothioyl)benzamid (I-b) mit der folgenden Formel: 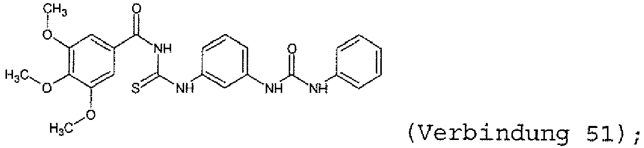
- *N*-(3-(3-Biphenyl-4-ylureido)phenylcarbamothioyl)-3,4,5-trimethoxybenzamid (I-b) mit der folgenden Formel: 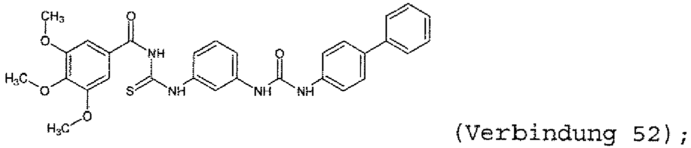
- *N*-(3-(1*H*-Indol-2-yl)phenylcarbamoyl)-3,4,5-trimethoxybenzamid (I-d') mit der folgenden Formel: 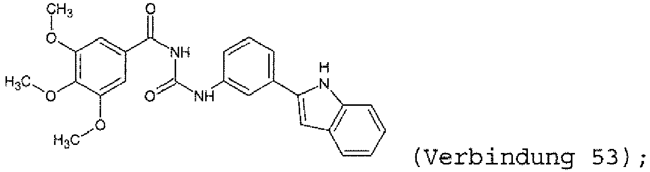
- *N*-(3-(1*H*-Indol-2-yl)phenylcarbamothioyl)-3,4,5-trimethoxybenzamid (I-d) mit der folgenden Formel: 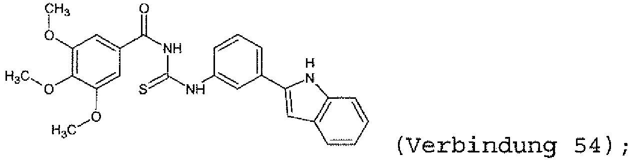
- *N*-(3-(Imidazo[1,2-a]pyridin-2-yl)phenylcarbamothioyl)-3,4,5-trimethoxybenzamid (I-d) mit der Formel: 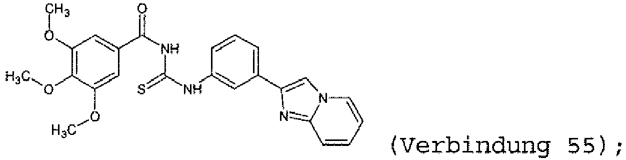
- *N*-(3-(1*H*-Benzo[*d*]imidazol-2-yl)-4-methylphenylcarbamothioyl)-3,4,5-trimethoxybenzamid (I-d) mit der Folgenden Formel: 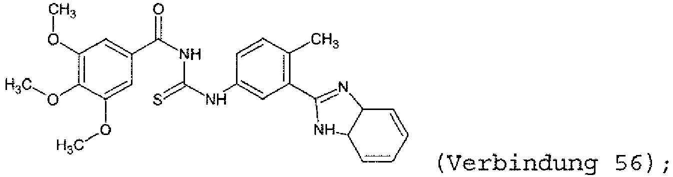
- *N*-(3-(1*H*-Indol-1-yl)phenylcarbamothioyl)-3,4,5-trimethoxybenzamid (I-d) mit der folgenden Formel: 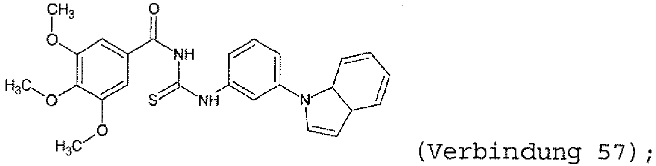 und
- *N*-(3-(Imidazo[2,1-*b*]thiazol-6-yl)phenylcarbamothioyl)-3,4,5-trimethoxybenzamid (I-d) mit der folgenden Formel: 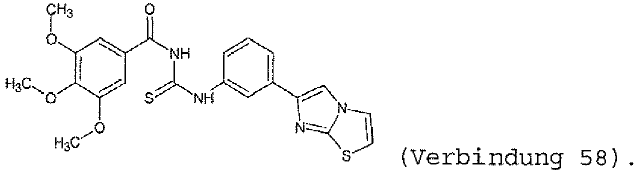
- *N*-(3-(3-Biphenyl-4-ylureido)phenylcarbamoyl)-3,4,5-trimethoxybenzamid (I-b') mit der folgenden Formel:

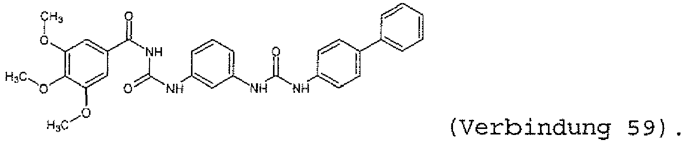

4. Verwendung nach einem der Ansprüche 1 bis 3, **dadurch gekennzeichnet, dass** die Verbindungen mit der Formel (I) ausgewählt sind aus:
- N-[[[3-(4-Methoxy-benzoylamino)phenyl]amino]thioxomeéthyl]-3,4,5-trimethoxy-benzamid (Verbindung 1);
- N-[[[3-(2-Chloro-benzoylamino)phenyl]amino]thioxomethyl]-3,4,5-trimethoxy-benzamid (Verbindung 2);
- N-[[[3-(3-Methoxy-benzoylamino)phenyl]amino]thioxomethyl]-3,4,5-trimethoxy-benzamid (Verbindung 3);
- N-[[[3-(2-Methoxy-benzoylamino)phenyl]amino]thioxomethyl]-3,4,5-trimethoxy-benzamid (Verbindung 4);
- N-[[[3-(4-Methoxy-benzoylamino)phenyl]amino]oxomethyl]-3,4,5-trimethoxy-benzamid (Verbindung 6);
- N-[[[3-(4-Pyridocarboxylamino)phenyl]amina]thioxomethyl]-3,4,5-trimethoxy-benzamid (Verbindung 11);
- N-[[[3-(4-Chloro-benzoylamino)phenyl]amino]thioxomethyl]-3,4,5-trimethoxy-benzamid (Verbindung 13);
- N-[[[3-(Isopropoylcarboxylamino))phenyl]amino]oxomethyl]-3,4,5-trimethoxy-benzamid (Verbindung 17);
- N-[[[3-(Benzoylamino)phenyl]amino]thioxomethyl]-3,5-dimethoxy-benzamid (Verbindung 18);
- N-[[[3-(Benzoylamino)phenyl]amino]thioxomethyl]-3,5-dimethoxy-4-ethoxy-benzamid (Verbindung 19);
- N-[[[3-(Benzoylamino)phenyl]amino]thioxomethyl]-3,4-diethoxy-5-methoxy-benzamid (Verbindung 20);
- N-[[[3-(Benzoylamino)phenyl]amino]thioxomethyl]-3,4-methylendioxy-5-methoxy-benzamid (Verbindung 21);
- N-[[[3-(Benzoylamino)phenyl]amino]thioxomethyl]-3,4,5-triethoxy-benzamid (Verbindung 23);
- N-[[[4-Fluoro-3-(benzoylamino)phenyl]amino]thioxomethyl]-3,4,5-trimethoxy-benzamid (Verbindung 24);
- N-[[[4-Chloro-3-(phenylamino)carbonyl]amino]thioxomethyl]-3,4,5-trimethoxy-benzamid (Verbindung 25);
- N-[[[3-(Benzoylamino)phenyl]amino]oxomethyl]-3,4,5-trimethoxy-benzamid (Verbindung 31);
- N-[[[4-Chloro-3-(Benzoylamino)phenyl]amino]thioxomethyl]-3,4,5-trimethoxy-benzamid (Verbindung 34);
- N-[[[3-(Benzoylamino)phenyl]amino]thioxomethyl]-3,4,5-trimethoxy-benzamid (Verbindung 38);
- N-[[[4-Chloro-3-(4-dimethylamino-phenylamino)carbonyl]thioxomethyl]-3,4,5-trimethoxy-benzamid (Verbindung 39);
- N-[[[4-Chloro-3-(4-chloro-phenylamino)]carbonyl]phenylamino]thioxomethyl]-3,4,5-trimethoxy-benzamide (Verbindung 43);
- N-[[[2-Methyl-3-(benzoylamino)phenyl]amino]oxomethyl]-3,4,5-trimethoxy-benzamid (Verbindung 45);
- N-[[[-3-(4-Chloro-benzoylamino)phenyl]amino]oxomethyl]-3,4,5-trimethoxy-benzamid (Verbindung 47);
- 3,4,5-Trimethoxy-*N*-(3-(3-phenylureido)phenylcarbamothioyl)benzamid (I-b) (Verbindung 51),
- *N*-(3-(3-Biphenyl-4-ylureido)phenylcarbamothioyl)-3,4,5-trimethoxybenzamid (I-b) (Verbindung 52);
- *N*-(3-(1*H*-Indol-2-yl)phenylcarbamoyl)-3,4,5-trimethoxybenzamid (I-d') (Verbindung 53);
- *N*-(3-(1*H*-Indol-2-yl)phenylcarbamothioyl)-3,4,5-trimethoxybenzamid (I-d) (Verbindung 54);
- *N*-(3-(Imidazo[1,2-a]pyridin-2-yl)phenylcarbamothioyl)-3,4,5-trimehoxybenzamid (I-d) (Verbindung 55);
- *N*-(3-(1*H*-Indol-1-yl)phenylcarbamothioyl)-3,4,5-trimethoxybenzamid (Verbindung 57);
- *N*-(3-(imidazo[2,1-*b*]thiazol-6-yl)phenylcarbamothioyl)-3,4,5-trimethoxybenzamid (I-d) (Verbindung 58); und
- *N*-(3-(3-Biphenyl-4-ylureido)phenylcarbamoyl)-3,4,5-trimethoxybenzamid (I-b') (Verbindung 59).

5. Verwendung von Verbindungen mit der folgenden Formel (I): 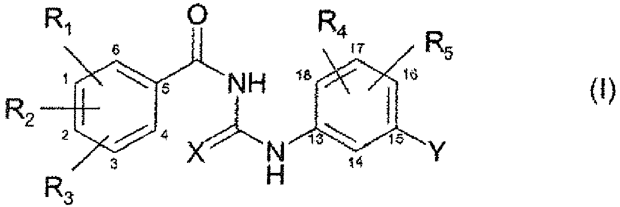 wobei:
- R₁, R₂ und R₃, identisch oder verschieden und unabhängig voneinander, ein Wasserstoff- oder Halogenatom darstellen, ein Hydroxylradiakal, eine Alkyl-, Perfluoroalkyl-, Alcoxy-, Alkylthio-, Nitrilgruppierung, ein substituiertes Alcoxy oder einen fusionierten Heterocyclus, erhalten auf der Grundlage von zwei der benachbarten Radikale R₁, R₂ und R₃, die zusammen mit den Kohlenstoffatomen des Phenylcyclus, mit dem sie verbunden sind, einen fusionierten Heterocyclus bilden können,
- X ein Schwefel- oder Sauerstoffatom darstellt,
- Y eine Gruppierung -NH-(C=O)-R₆, -(C=O)-NH-R₆ oder -NH-(C=O)-NH-R₆ darstellt, wobei R₆ eine nicht substituierte Arylgruppierung darstellt; eine Arylgruppierung, umfassend einen oder mehrere Substituenten, ausgewählt aus einem Halogenatom und einem Alkyl-, Alcoxy oder Mono- oder Dialkylaminoradiakal; eine mono- oder polycyclische Heteroaryl-Gruppierung; ein lineares oder verzweigtes Alkylradikal; eine gesättigte oder ungesättigte mono oder polycyclische Kohlenwasserstoff-Gruppierung;
- R₄ und R₅, identisch oder verschieden und unabhängig voneinander, ein Wasserstoff- oder Halogenatom darstellen, eine Alcoxy-, Alkylthio-, Alkyl-, Perfluoroalkyl-, Nitril- oder Nitro-Gruppe,
zur Herstellung eines Arzneimittels zur Behandlung von Tumoren, die mit einer Hyperaktivierung des Signalwegs der Hedgehog-Proteine assoziiert sind, oder von Pathologien der neurodegenerativen Art.

6. Verwendung von Verbindungen mit der folgenden Formel (I): 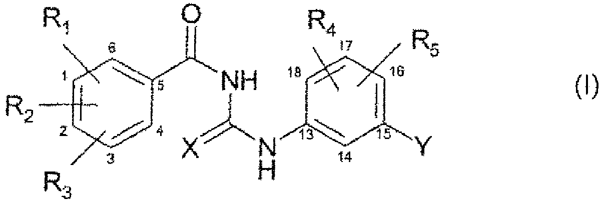 wobei:
- R₁, R₂ und R₃, identisch oder verschieden und unabhängig voneinander, ein Wasserstoff- oder Halogenatom darstellen, ein Hydroxylradiakal, eine Alkyl-, Perfluoroalkyl-, Alcoxy-, Alkylthio-, Nitrilgruppierung, oder ein substituiertes Alcoxy;
- X ein Schwefel- oder Sauerstoffatom darstellt,
- Y eine Gruppierung -NH-(C=O)-R₆, -(C=O)-NH-R₆ oder -NH-(C=O)-NH-R₆ darstellt, wobei R₆ eine nicht substituierte Arylgruppierung darstellt; eine Arylgruppierung, umfassend einen oder mehrere Substituenten, ausgewählt aus einem Halogenatom und einem Alkyl-, Alcoxy oder Mono- oder Dialkylaminoradiakal; eine mono- oder polycyclische Heteroaryl-Gruppierung; ein lineares oder verzweigtes Alkylradikal; eine gesättigte oder ungesättigte mono oder polycyclische Kohlenwasserstoff-Gruppierung;
- R₄ und R₅, identisch oder verschieden und unabhängig voneinander, ein Wasserstoff- oder Halogenatom darstellen, eine Alcoxy-, Alkylthio-, Alkyl-, Perfluoroalkyl- oder Nitril-Gruppe,
wobei davon ausgegangen wird, dass, wenn Y eine Gruppierung -NH-(C=O)-R₆ darstellt:
-- R₄ und/oder R₅ auch eine Nitro-Gruppierung darstellen können, und
-- zwei der benachbarten R₁, R₂ und R₃, zusammen mit den Kohlenstoffatomen des Phenylcyclus, mit dem sie verbunden sind, einen fusionierten Heterocyclus bilden können,
zur Herstellung eines Arzneimittels zur Behandlung von Tumoren, die mit einer Hyperaktivierung des Signalwegs der Hedgehog-Proteine assoziiert sind, oder von Pathologien der neurodegenerativen Art.

7. Verwendung nach Anspruch 5 oder 6, **dadurch gekennzeichnet, dass** die Verbindungen ausgewählt sind aus denjenigen, in denen, in der Formel (I):
- R₁, R₂, und R₃, identisch oder verschieden, ein Wasserstoffatom oder ein Methyloxy- oder ein Ethyloxyradiakal darstellen,
- X ein Schwefel- oder Sauerstoffatom darstellt,
- Y eine Gruppierung -NH-(C=O)-R₆, -(C=O)-NH-R₆ oder -NH-(C=O)-NH-R₆ darstellt, wobei R₆ eine Gruppierung darstellt, ausgewählt aus den Phenylgruppierungen; Phenyl, substituiert durch ein Methoxy- oder Dimethylaminoradikal, durch ein Chloratom, durch eine Phenylgruppierung, durch eine Benzyl-; Cyclohexyl-, Isopropyl-, Pyridinyl-, Naphtyl-, Furfuryl- und Thiophen-Gruppierung; und
- R₄ und R₅, identisch oder verschieden, ausgewählt sind aus Wasserstoff, Chlor, Brom, Fluor, Methyl und Methoxy.

8. Verwendung nach einem der Ansprüche 5 bis 7, **dadurch gekennzeichnet, dass** die Verbindungen der Formel (I) ausgewählt sind aus:
- N-[[[3-(4-Methoxy-benzoylamino)phenyl]amino]thioxomethyl]-3,4,5-trimethoxy-benzamid (I-a) mit der folgenden Formel: 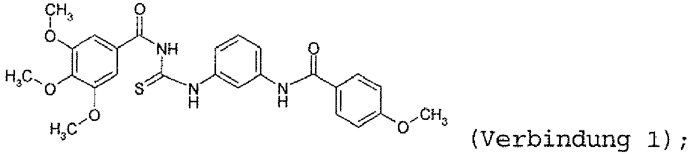
- N-[[[3-(2-Chloro-benzoylamino)phenyl]amino]thioxomethyl]-3,4,5-trimethoxy-benzamid (I-a) mit der folgenden Formel: 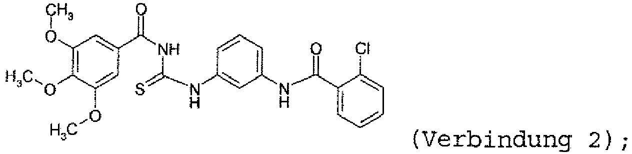
- N-[[[3-(3-Methoxy-benzoylamino)phenyl]amino]thioxomethyl]-3,4,5-trimethoxy-benzamid (I-a) mit der folgenden Formel: 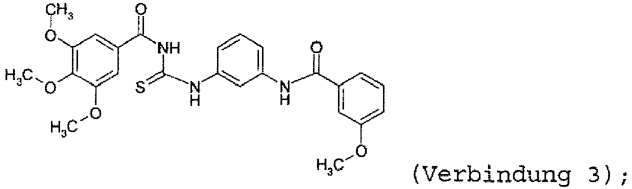
- N-[[[3-(2-Methoxy-benzoylamino)phenyl]amino]thioxomethyl]-3,4,5-trimethoxy-benzamid (I-a) mit der folgenden Formel: 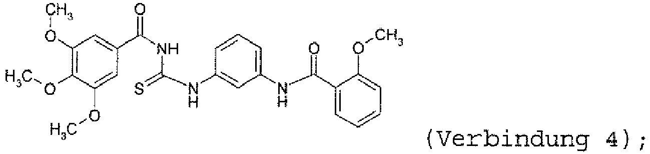
- N-[[[3-(4-Phenyl-benzoylamino)phenyl]amino]thioxomethyl]-3,4,5-trimethoxy-benzamid (I-a) mit der folgenden Formel: 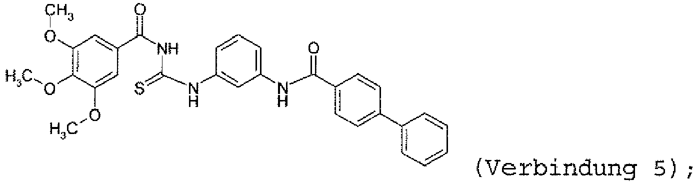
- N-[[[3-(4-Methoxy-benzoylamino)phenyl]amino]oxomethyl]-3,4,5-trimethoxy-benzamid (I-a') mit der folgenden Formel: 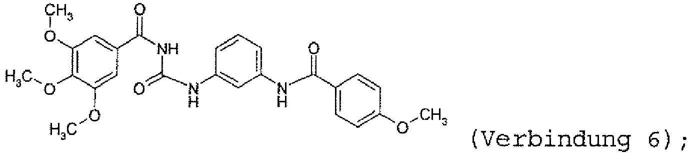
- N-[[[3-(4-Phenyl-benzoylamino)phenyl]amino]oxomethyl]-3,4,5-trimethoxy-benzamid (I-a') mit der folgenden Formel: 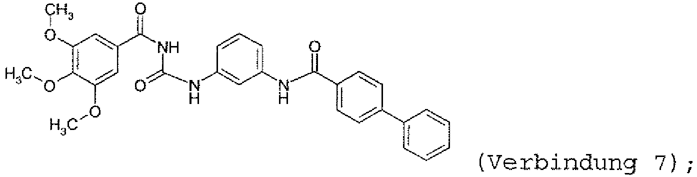
- N-[[[3-(-Cyclohexylcarboxylamino)phenyl]amino]thioxomethyl]-3,4,5-trimethoxy-benzamid (I-a) mit der folgenden Formel: 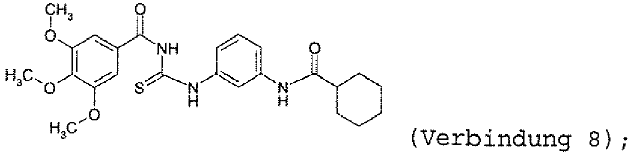
- N-[[[3-(2-Naphtoylamino)phenyl]amino]thioxomethyl]-3,4,5-trimethoxy-benzamid (I-a) mit der folgenden Formel: 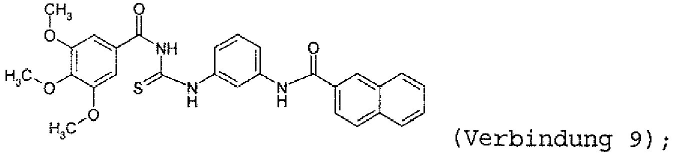
- N-[[[3-(Isopropoylcarboxylamino)phenyl]amino]thioxomethyl]-3,4,5-trimethoxy-benzamid (I-a) mit der folgenden Formel: 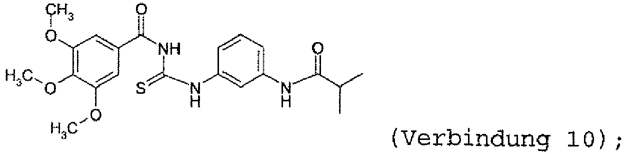
- N-[[3-4-Pyridocarboxylamino)phenyl]amino]thioxomethyl]-3,4,5-trimethoxy-benzamid (I-a) mit der folgenden Formel: 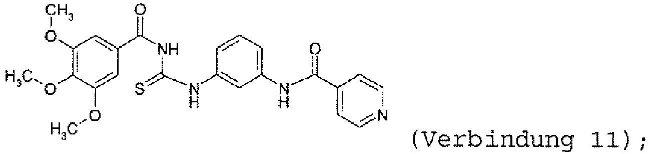
- N-[[[3-(1-naphtoylamino)phenyl]amino]thioxomethyl]-3,4,5-trimethoxy-benzamid (I-a) mit der folgenden Formel: 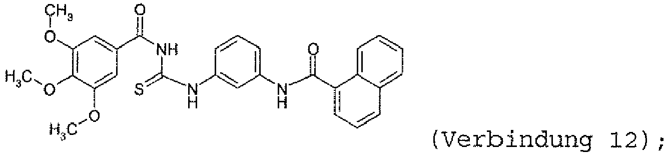
- N-[[[3-(4-Chloro-benzoylamino)phenyl]amino]thioxomethyl]-3,4,5-trimethoxy-benzamid (I-a) mit der folgenden Formel: 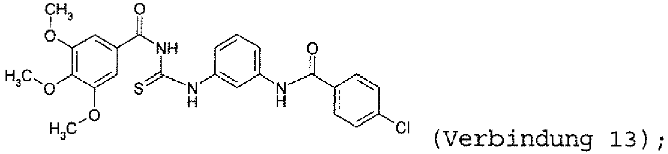
- N-[[[3-(2-Furoylamino)phenyl]amino]thioxomethyl]-3,4,5-trimethoxy-benzamid (I-a) mit der folgenden Formel: 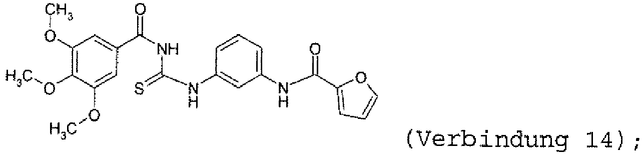
- N-[[[3-(2-Thiophenoylamino)phenyl]amino]thioxomehyl]-3,4,5-trimethoxy-benzamid (I-a) mit der folgenden Formel 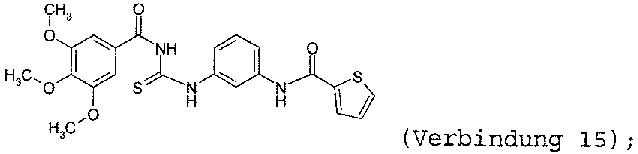
- N-[[[3-(2-Naphtoylamino)phenyl]amino]oxomethyl]-3,4,5-trimethoxy-benzamid (I-a') mit der folgenden Formel: 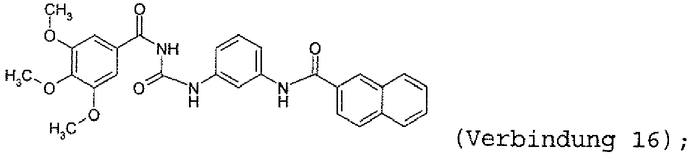
- N-[[[3-(Isopropoylcarboxylamino))phenyl]amino]oxomethyl]-3,4,5-trimethoxy-benzamid (I-a') mit der folgenden Formel: 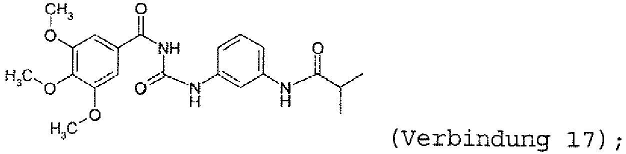
- N-[[[3-(Benzoylamino)phenyl]amino]thioxomehyl]-3,5-dimethoxy-benzamid (I-a) mit der folgenden Formel: 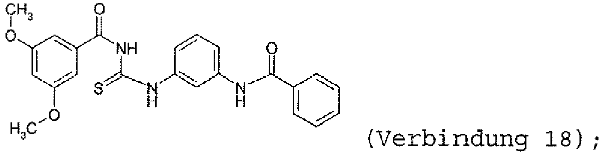
- N-[[[3-(Benzoylamino)phenyl]amino]thioxomethyl]-3,5-dimethoxy-4-ethoxy-benzamid (I-a) mit der folgenden Formel: 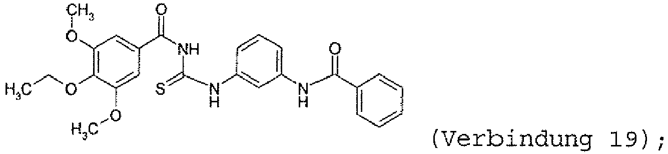
- N-[[[3-(Benzoylamino)phenyl]amino]thioxomethyl]-3,4-diethoxy-5-methoxy-benzamid (I-a) mit der folgenden Formel: 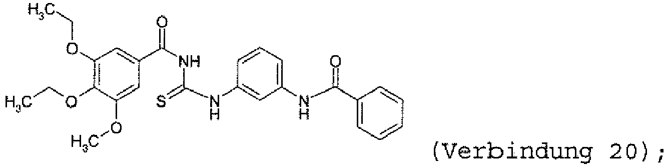
- N-[[[3-(Benzoylamino)phenyl]amino]thioxomethyl]-3,4-methylendioxy-5-methoxy-benzamid (I-a) mit der folgenden Formel: 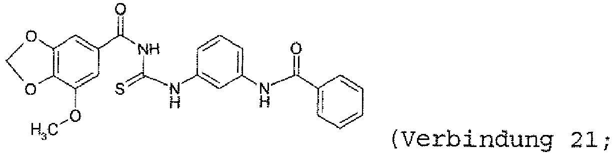
- N-[[[3-(Benzoylamino)phenyl]amino]thioxomethyl] - 2,4-dimethoxy-benzamid (I-a) mit der folgenden Formel: 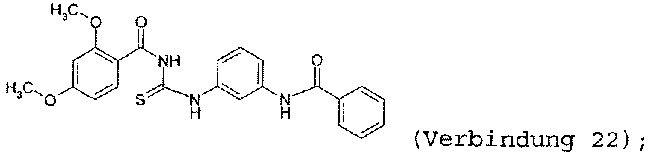
- N-[[[3-(Benzoylamino)phenyl]amino]thioxomethyl]-3,4,5-triethoxy-benzamid (I-a) mit der folgenden Formel: 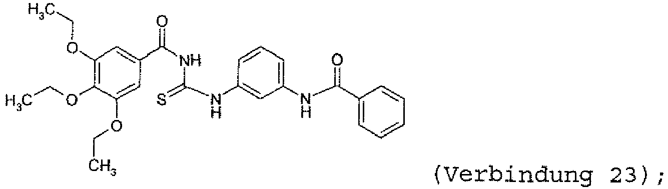
- N-[[[4-Fluoro-3-(Benzoylamino)phenyl]amino]thioxomethyl]-3,4,5-trimethoxy-benzamid (I-a) mit der folgenden Formel: 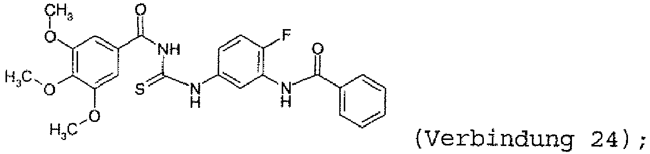
- N-[[[4-Chloro-3-(phenylamino)carbonyl]amino]thioxomethyl]-3,4,5-trimethoxy-benzamid (I-b) mit der folgenden Formel: 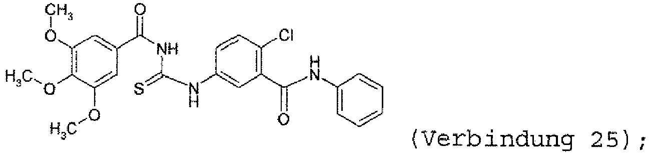
- N-[[[4-Methoxy-3-(benzoylamino)phenyl]amino]thioxomethyl]-3,4,5-trimethoxy-benzamid (I-a) mit der folgenden Formel: 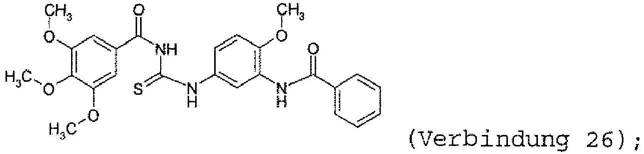
- N-[[[4-Methyl-3-(benzoylamino)phenyl]amino]thioxomethyl]-3,4,5-trimethoxy-benzamid (I-a) mit der folgenden Formel: 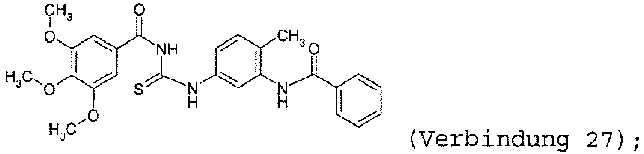
- N-[[[3-(Benzoylamino)phenyl]amino]thioxomethyl]-3,4-ethylendioxo-benzamid (I-a) mit der folgenden Formel: 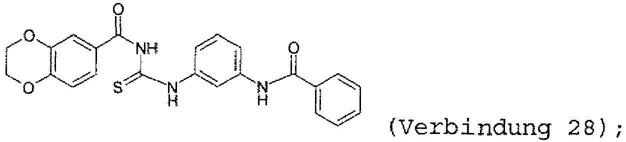
- N-[[[3-(Benzoylamino)phenyl]amino]oxomethyl]-3,4,5-triethoxy-benzamid (I-a') mit der folgenden Formel: 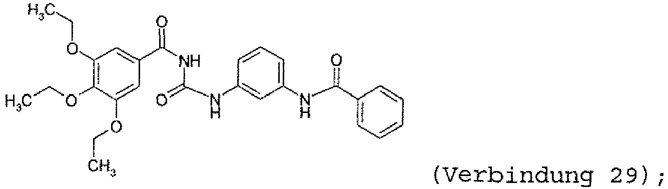
- N-[[[3-(Benzoylamino)phenyl]amino]oxomethyl]-3,4-methylendioxy-5-methoxy-benzamid (I-a') mit der folgenden Formel: 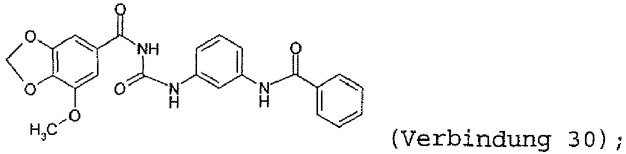
- N-[[[3-(Benzoylamino)phenyl]amino]oxomehyl]-3,4,5-trimethoxy-benzamid (I-a') mit der folgenden Formel: 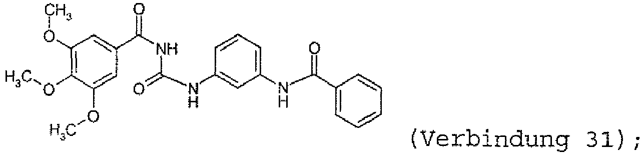
- N-[[[3-Methyl-3-(benzoylamino)phenyl]amino]thioxomethyl]-3,4,5-trimethoxy-benzamid (I-a) mit der folgenden Formel: 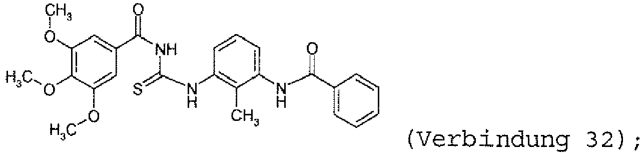
- N-[[[4-Chloro-3-(Benzoylamino)phenyl]amino]oxomethyl]-3,4,5-trimethoxy-benzamid (I-a') mit der folgenden Formel: 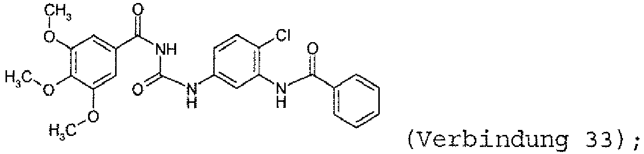
- N-[[[4-Chloro-3-(Benzoylamino)phenyl]amino]thioxomethyl]-3,4,5-trimethoxy-benzamid (I-a) mit der folgenden Formel: 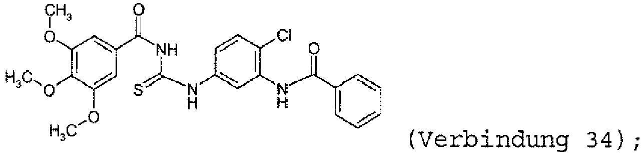
- N-[[[5-Chloro-3-(benzoylamino)phenyl]amino]thioxomethyl]-3,4,5-trimethoxy-benzamid (I-a) mit der folgenden Formel: 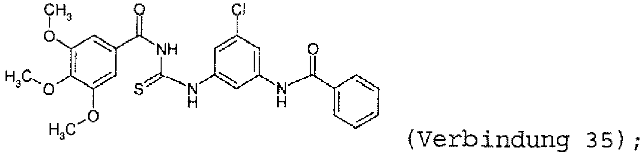
- N-[[[5-Fluoro-3-(benzoylamino)phenyl]amino]thioxomethyl]-3,4,5-trimethoxy-benzamid (I-a) mit der folgenden Formel: 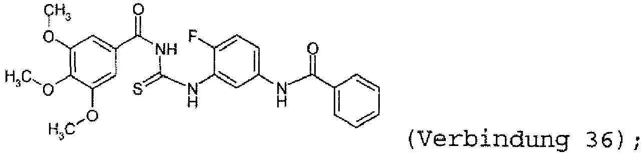
- N-[[[4-Chloro-3-(4-phenyl-benzoylamino)phenyl]amino]oxomethyl]-3,4,5-trimethoxy-benzamid (I-a') mit der folgenden Formel: 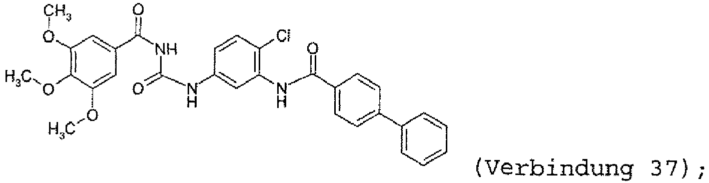
- N-[[[3-(Benzoylamino)phenyl]amino]thioxomethyl]-3,4,5-trimethoxy-benzamid (I-a) mit der folgenden Formel: 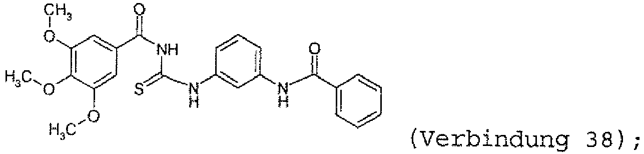
- N-[[[4-Chloro-3-(4-dimethylamino-phenylamino)carbonyl]thioxomethyl]-3,4,5-trimethoxy-benzamid (I-b) mit der folgenden Formel: 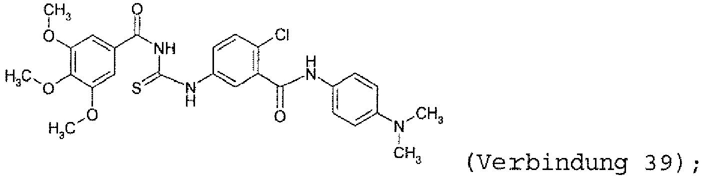
- N-[[[4-Chloro-3-(4-methoxy-phenylamino)carbonyl]thioxomethyl]-3,4,5-trimethoxy-benzamid (I-b) mit der folgenden Formel: 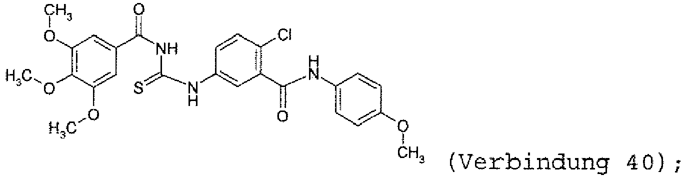
- N-[[[5-Bromo-3-(phenylamino)carbonyl]thioxomethyl]-3,4,5-trimethoxy-benzamid (I-b) mit der folgenden Formel: 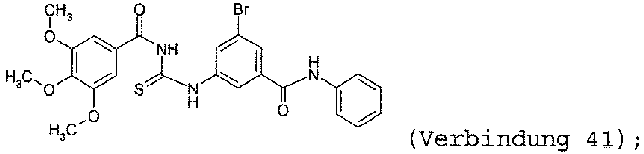
- N-[[[4-Chloro-3-(4-methoxy-phenylamino)carbonyl]oxomethyl]-3,4,5-trimethoxy-benzamide (I-b') mit der folgenden Formel: 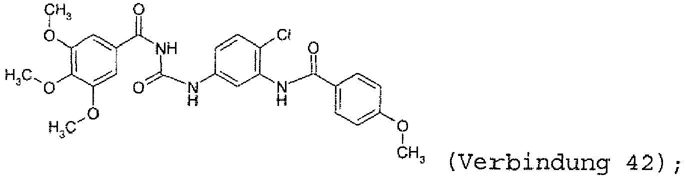
- N-[[[4-Chloro-3-(4-chloro-phenylamino)]carbonyl]phenylamino]thioxomethyl]-3,4,5-trimethoxy-benzamid (I-b) mit der folgenden Formel: 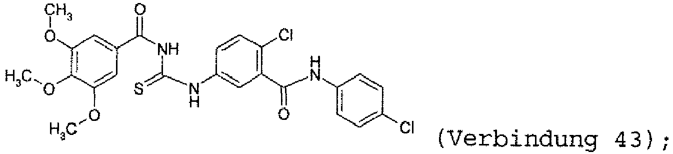
- N-[[[4-Chloro-3-(phenylamino)carbonyl]oxomethyl]-3,4,5-trimethoxy-benzamid (I-b') mit der folgenden Formel: 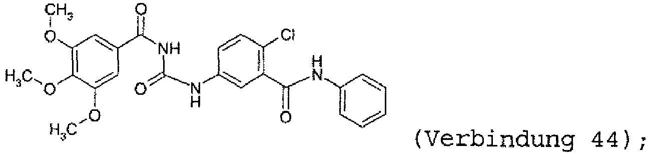
- N-[[[2-Methyl-3-(benzoylamino)phenyl]aminojoxomrthyl]-3,4,5-trimethoxy-benzamid (I-a') mit der folgenden Formel: 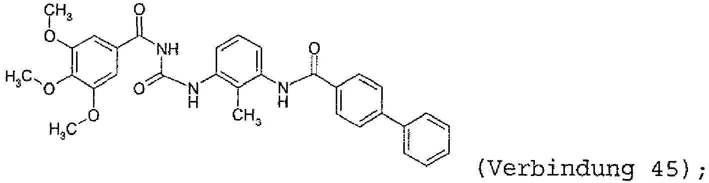
- N-[[[2-Methyl-3-(benzoylamino)phenyl]amino]thioxomethyl]-3,4,5-trimethoxy-benzamid (I-a) mit der folgenden Formel: 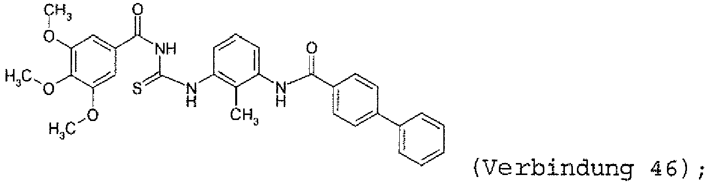
- N-[[[-3-(4-Chloro-benzoylamino)phenyl]amino]oxomethyl]-3,4,5-trimethoxy-benzamid (I-a') mit der folgenden Formel: 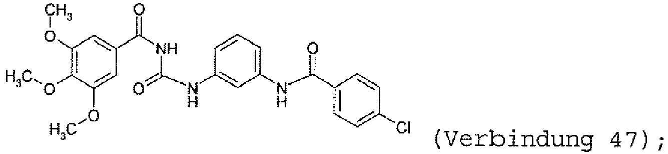
- N-[[[-3-(Benzoylamino)phenyl]amino]oxomethyl]-2,4,5-trimethoxy-benzamid (I-a') mit der folgenden Formel: 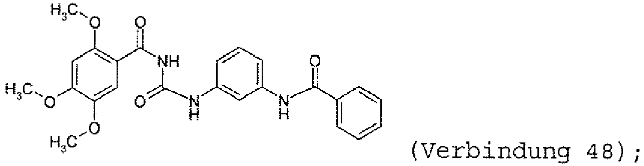
- N-[[[-3-(Benzoylamino)phenyl]amino]oxomethyl]-4-methoxy-benzamid (I-a') mit der folgenden Formel: 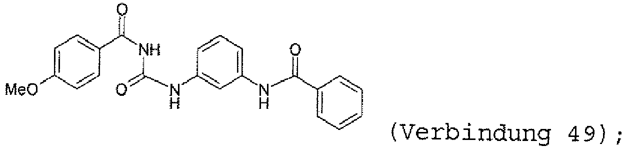
- N-[[[-3-(Benzoylamino)phenyl]amino]oxomethyl]-benzamid (I-a') mit der folgenden Formel: 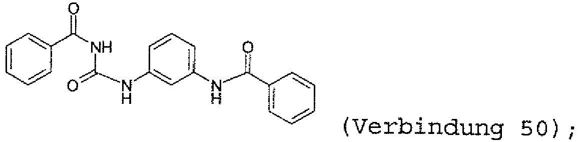
- 3,4,5-Trimethoxy-*N*-(3-(3-phenylureido)phenylcarbamothioyl)benzamid (I-b) mit der folgenden Formel: 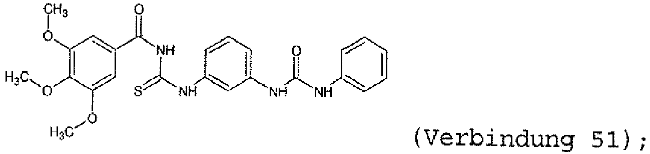
- *N*-(3-(3-Biphenyl-4-ylureido)phenylcarbamothioyl)-3,4,5-trimethoxybenzamid (I-b) mit der folgenden Formel: 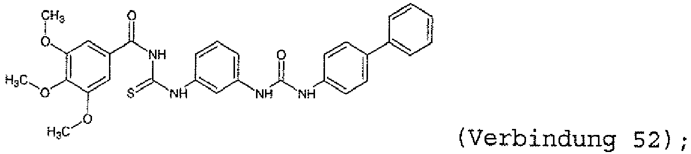
- *N*-(3-(3-Biphenyl-4-ylureido)phenylcarbamoyl)-3,4,5-trimethoxybenzamid (i-b') mit der folgenden Formel:

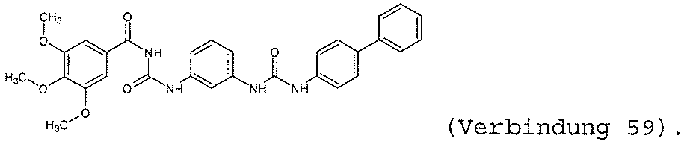

9. Verwendung nach einem der Ansprüche 5 bis 8, **dadurch gekennzeichnet, dass** die Verbindungen der Formel (I) ausgewählt sind aus:
- N-[[[3-(4-Methoxy-benzoylamino)phenyl]amino]thioxomethyl]-3,4,5-trimethoxy-benzamid (Verbindung 1);
- N-[[[3-(2-Chloro-benzoylamino)phenyl]amino]thioxomethyl]-3,4,5-trimethoxy-benzamid (Verbindung 2);
- N-[[[3-(3-Methoxy-benzoylamino)phenyl]amino]thioxomethyl]-3,4,5-trimethoxy-benzamid (Verbindung 3);
- N-[[[3-(2-Methoxy-benzoylamino)phenyl]amino]thioxomethyl]-3,4,5-trimethoxy-benzamid (Verbindung 4);
- N-[[[3-(4-Methoxy-benzoylamino)phenyl]amino]oxomethyl]-3,4,5-trimethoxy-benzamid (Verbindung 6);
- N-[[[3-(4-Pyridocarboxylamino)phenyl]amino]thioxomethyl]-3,4,5-trimethoxy-benzamid (Verbindung 11);
- N-[[[3-(4-Chloro-benzoylamino)phenyl]amino]thioxomethyl]-3,4,5-trimethoxy-benzamid (Verbindung 13);
- N-[[[3-(Isopropoylcarboxylamino))phenyl]amino]oxomethyl]-3,4,5-trimethoxy-benzamid (Verbindung 17);
- N-[[[3-(Benzoylamino)phenyl]amino]thioxomethyl]-3,5-dimethoxy-benzamid (Verbindung 18);
- N-[[[3-(Benzoylamino)phenyl]amino]thioxomethyl]-3,5-dimethoxy-4-ethoxy-benzamid (Verbindung 19);
- N-[[[3-(benzoylamino)phenyl]amino]thioxomethyl]-3,4-diethoxy-5-methoxy-benzamid (Verbindung 20);
- N-[[[3-(Benzoylamino)phenyl]amino]thioxomethyl]-3,4-methylendioxy-5-methoxy-benzamid (Verbindung 21);
- N-[[[3-(Benzoylamino)phenyl]amino]thioxomethyl]-3,4,5-triethoxy-benzamid (Verbindung 23);
- N-[[[4-Fluoro-3-(benzoylamino)phenyl]amino]thioxomethyl]-3,4,5-trimethoxy-benzamid (Verbindung 24);
- N-[[[4-Chloro-3-(phenylamino)carbonyl]amino]thioxomethyl]-3,4,5-trimethoxy-benzamid (Verbindung 25);
- N-[[[3-(Benzoylamino)phenyl]amino]oxomethyl]-3,4,5-trimethoxy-benzamid (Verbindung 31);
- N-[[[4-Chloro-3-(benzoylamino)phenyl]amino]thioxomethyl]-3,4,5-trimethoxy-benzamid (Verbindung 34);
- N-[[[3-(Benzoylamino)phenyl]amino]thioxomehyl]-3,4,5-trimethoxy-benzamid (Verbindung 38);
- N-[[[4-Chloro-3-(4-dimethylamino-phenylamino)carbonyl]thioxomethyl]-3,4,5-trimethoxy-benzamid (Verbindung 39);
- N-[[[4-Chloro-3-(4-chloro-phenylamino)]carbonyl]phenylamino]thioxamethyl]-3,4,5-trimethoxy-benzamid (Verbindung 43);
- N-[[[2-Methyl-3-(benzoylamino)phenyl]amino]oxomethyl]-3,4,5-trimethoxy-benzamid (Verbindung 45);
- N-[[[-3-(4-Chloro-benzoylamino)phenyl]amino]oxomethyl]-3,4,5-trimethoxy-benzamid (Verbindung 47);
- 3,4,5-Trimethoxy-N-(3-(3-phenylureido)phenylcarbamothioyl)benzamid (I-b) (Verbindung 51),
- *N*-(3-(3-Biphenyl-4-ylureido)phenylcarbamothioyl)-3,4,5-trimethoxybenzamid (I-b) (Verbindung 52); und
- *N*-(3-(3-Biphenyl-4-ylureido)phenylcarbamoyl)-3,4,5-trimethoxybenzamid (I-b') (Verbindung 59).

10. Verwendung nach einem der Ansprüche 5 bis 9, wobei die Pathologie der neurodegenerativen Art die Parkinson'sche Erkrankung, die Huntington'sche Erkrankung, die Alzheimer'sche Erkrankung, die multiple Sklerose oder die Motoneuronenerkrankung ist.

11. Verbindungen mit der folgenden Formel (I'): 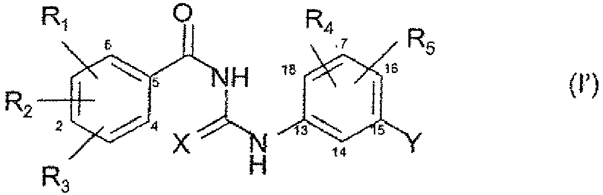 wobei:
- R₁, R₂ und R₃, identisch oder verschieden und unabhängig voneinander, ein Wasserstoff- oder Halogenatom darstellen, ein Hydroxylradiakal, eine Alkyl-, Perfluoroalkyl-, Alcoxy-, Alkylthio-, Nitrilgruppierung, ein substituiertes Alcoxy, oder einen fusioniertes Heterocyclus, erhalten auf der Grundlage von zwei der benachbarten Radikale R₁, R₂ und R₃, die zusammen mit den Kohlenstoffatomen des Phenylcyclus, mit dem sie verbunden sind, einen fusionierten Heterocyclus bilden können;
- X ein Schwefelatom darstellt,
- Y eine Gruppierung -NH-(C=O)-R₆ oder -NH-(C=O)-NH-R₆ darstellt, wobei R₆ eine nicht substituierte Arylgruppierung darstellt; eine Arylgruppierung, umfassend einen oder mehrere Substituenten, ausgewählt aus einem Halogenatom und einem Alkyl-, Alcoxy oder Mono- oder Dialkylaminoradiakal; eine mono- oder polycyclische Heteroaryl-Gruppierung; ein verzweigtes Alkylradikal; eine gesättigte oder ungesättigte mono oder polycyclische Kohlenwasserstoff-Gruppierung;
- R₄ und R₅, identisch oder verschieden und unabhängig voneinander, ein Wasserstoff- oder Halogenatom darstellen, eine Alcoxy-, Alkylthio-, Alkyl-, Perfluoroalkyl- oder Nitril- oder Nitro-Gruppe.

12. Verbindungen nach Anspruch 11, **dadurch gekennzeichnet, dass** sie ausgewählt sind aus:
- N-[[[3-(4-Methoxy-benzoylamino)phenyl]amino]thioxomethyl]-3,4,5-trimethoxy-benzamid (I-a) mit der folgenden Formel: 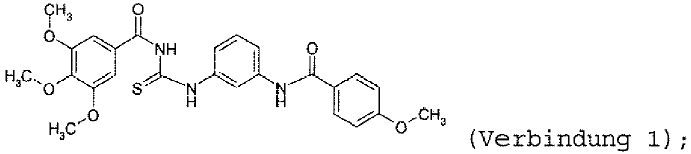
- N-[[[3-(2-Chloro-benzoylamino)phenyl]amino]thioxomethyl]-3,4,5-trimethoxy-benzamid (I-a) mit der folgenden Formel: 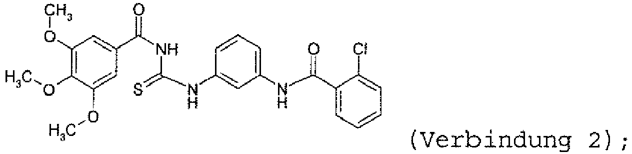
- N-[[[3-(3-Methoxy-benzoylamino)phenyl]amino]thioxomethyl]-3,4,5-trimethoxy-benzamid (I-a) mit der folgenden Formel: 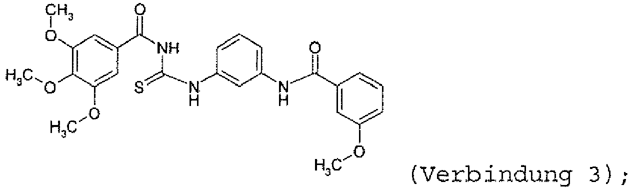
- N-[[[3-(2-Methoxy-benzoylamino)phenyl]amino]thioxomethyl]-3,4,5-trimethoxy-benzamid (I-a) mit der folgenden Formel: 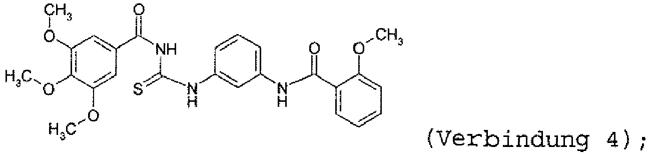
- N-[[[3-(4-Phenyl-benzoylamino)phenyl]amino]thioxomethyl]-3,4,5-trimethoxy-benzamid (I-a) mit der folgenden Formel: 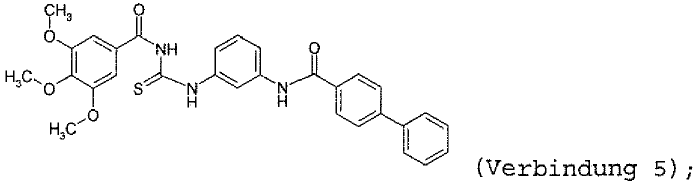
- N-[[[3-(-Cyclohexylcarboxylamino)phenyl]amino]thioxomethyl]-3,4,5-trimethoxy-benzamid (I-a) mit der folgenden Formel: 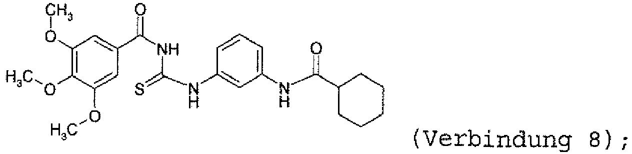
- N-[[[3-2-Naphtoylamino)phenyl]amino]thioxomethyl]-3,4,5-trimethoxy-benzamid (I-a) mit der folgenden Formel: 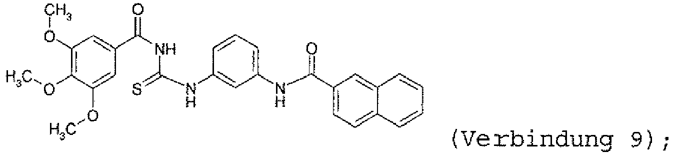
- N-[[[3-(Isopropoylcarboxylamino)phenyl]amino]thioxomethyl]-3,4,5-trimethoxy-benzamid (I-a) mit der folgenden Formel: 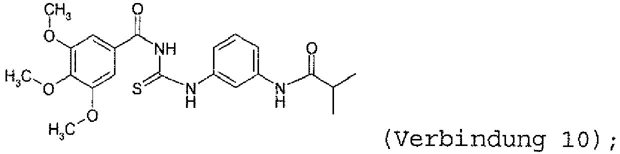
- N-[[[3-(4-Pyridocarboxylamino)phenyl]amino]thioxomethyl]-3,4,5-trimethoxy-benzamid (I-a) mit der folgenden Formel: 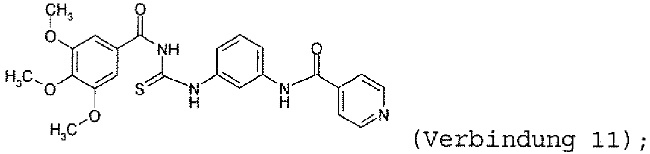
- N-[[[3-(1-Naphtoylamino)phenyl]amino]thioxomethyl]-3,4,5-trimethoxy-benzamid (I-a) mit der folgenden Formel: 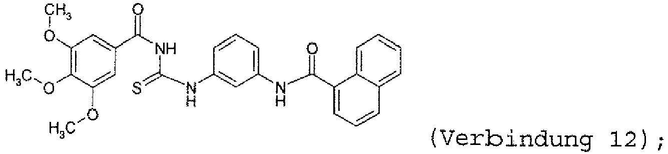
- N-[[[3-(4-Chloro-benzoylamino)phenyl]aminolthioxomethyl]-3,4,5-trimethoxy-benzamid (I-a) mit der folgenden Formel: 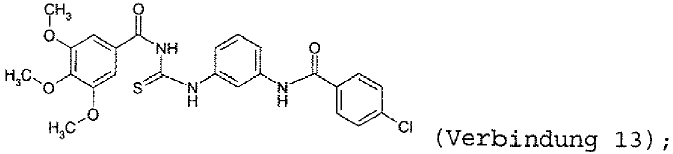
- N-[[[3-(2-Furoylamino)phenyl]amino]thioxomethyl]-3,4,5-trimethoxy-benzamid (I-a) mit der folgenden Formel: 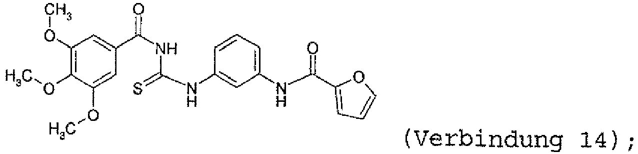
- N-[[[3-(2-Thiophenoylamino)phenyl]aminolthioxomethyl]-3,4,5-trimethoxy-benzamid (I-a) mit der folgenden Formel: 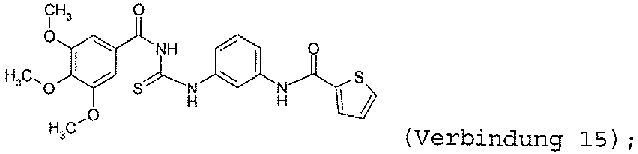
- N-[[[3-(Benzoylamino)phenyl]amino]thioxomethyl]-3,5-dimethoxy-benzamid (I-a) mit der folgenden Formel: 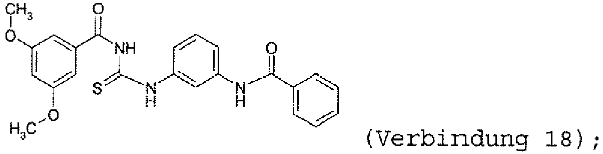
- N-[[[3-(Benzoylamino)phenyl]amino]thioxomethyl]-3,5-dimethoxy-4-ethoxy-benzamid (I-a) mit der folgenden Formel: 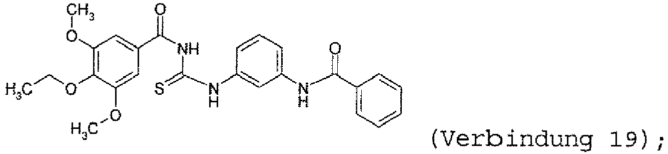
- N-[[[3-(Benzoylamino)phenyl]amino]thioxomethyl]-3,4-diethoxy-5-methoxy-benzamid (I-a) mit der folgenden Formel: 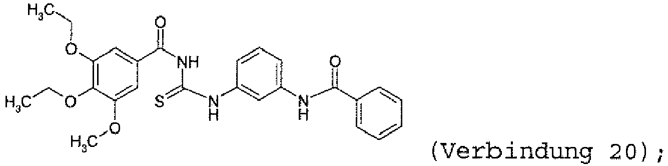
- N-[[[3-(Benzoylamino)phenyl]amino]thioxomethyl]-3,4-methylendioxy-5-methoxy-benzamid (I-a) mit der folgenden Formel: 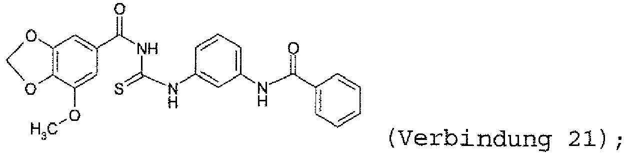
- N-[[[3-(Benzoylamino)phenyl]amino]thioxomethyl]-2,4-dimethoxy-benzamid (I-a) mit der folgenden Formel: 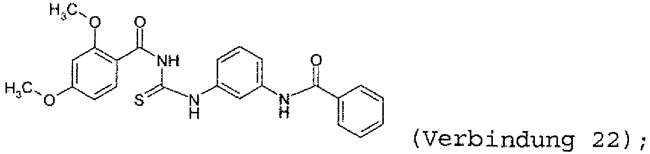
- N-[[[3-(Benzoylamino)phenyl]amino]thioxomethyl]-3,4,5-triethoxy-benzamid (I-a) mit der folgenden Formel: 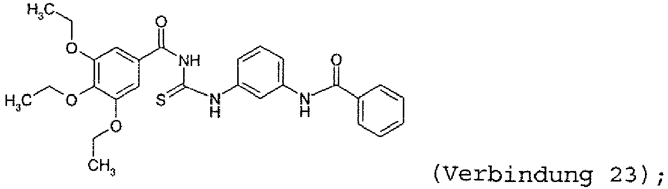
- N-[[[4-Fluoro-3-(benzoylamino)phenyl]amino]thioxomethyl]-3,4,5-trimethoxy-benzamid (I-a) mit der folgenden Formel: 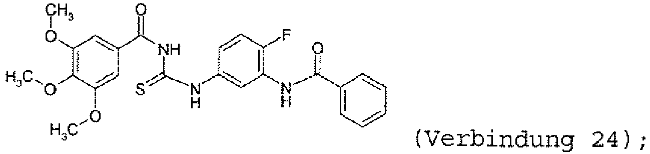
- N-[[[4-Chloro-3-(phenylamino)carbonyl]amino]thioxomethyl]-3,4,5-trimethoxy-benzamid (I-b) mit der folgenden Formel: 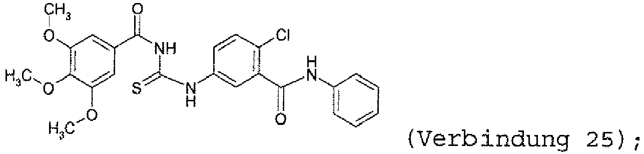
- N-[[[4-Methoxy-3-(benzoylamino)phenyl]amino]thioxomethyl]-3,4,5-trimethoxy-benzamid (I-a) mit der folgenden Formel: 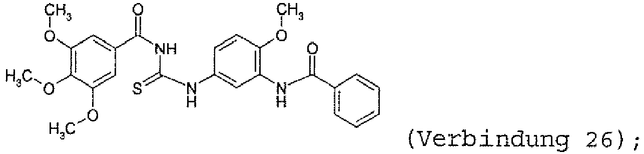
- N-[[[4-Methyl-3-(benzoylamino)phenyl]amino]thioxomethyl]-3,4,5-trimethoxy-benzamid (I-a) mit der folgenden Formel: 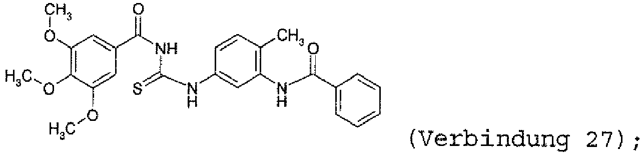
- N-[[[3-(Benzoylamino)phenyl]amino]thioxomethyl]-3,4-ethylendioxo-benzamid (I-a) mit der folgenden Formel: 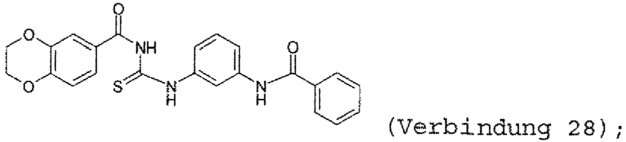
- N-[[[3-methyl-3-(benzoylamino)phenyl]amino]thioxomethyl]-3,4,5-trimethoxy-benzamid (I-a) mit der folgenden Formel: 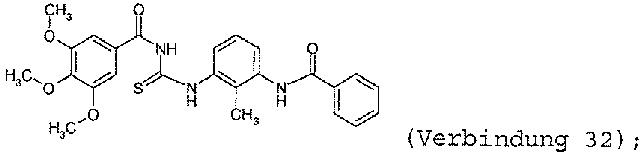
- N-[[[4-Chloro-3-(benzaylamino)phenyl]amino]thioxomethy3]-3,4,5-trimethoxy-benzamid (I-a) mit der folgenden Formel: 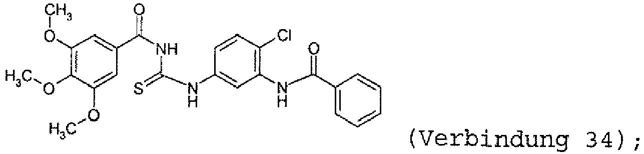
- N-[[[5-Chloro-3-(benzoylamino)phenyl]amino]thioxomethyl]-3,4,5-trimethoxy-benzamid (I-a) mit der folgenden Formel: 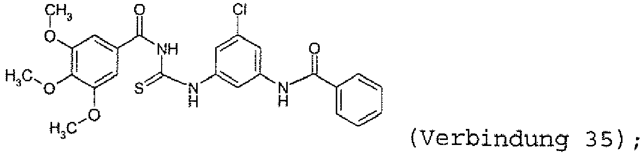
- N-[[[5-Fluoro-3-(benzoylamino)phenyl]amino]thioxomethyl]-3,4,5-trimethoxy-benzamid (I-a) mit der folgenden Formel: 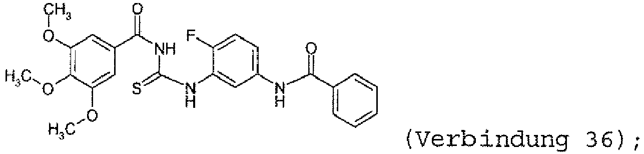
- N-[[[3-(Benzoylamino)phenyl]amino]thioxomethyl]-3,4,5-trimethoxy-benzamid (I-a) mit der folgenden Formel: 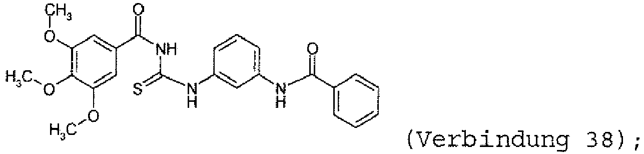
- N-[[[2-Methyl-3-(benzoylamino)phenyl]amino]thioxomethyl]-3,4,5-trimethoxy-benzamid (I-a) mit der folgenden Formel: 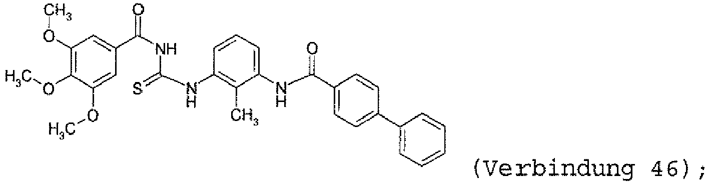
- 3,4,5-Trimethoxy-N-(3-(3-phenylureido)phenylcarbamothioyl)benzamid (I-b) mit der folgenden Formel: 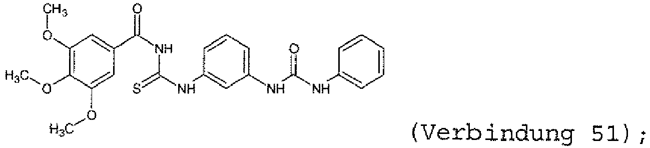
- *N*-(3-(3-Biphenyl-4-ylureido)phenylcarbamothioyl)-3,4,5-trimethoxybenzamid (I-b) mit der folgenden Formel:

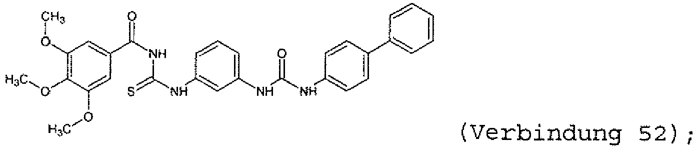

13. Verbindungen nach Anspruch 11 oder 12, **dadurch gekennzeichnet, dass** sie ausgewählt sind aus:
- N-[[[3-(4-Methoxy-henzoylamino)phenyl]amino]thioxomethyl]-3,4,5-trimethoxy-benzamid (Verbindung 1);
- N-[[[3-(2-Chloro-benzoylamino)phenyl]amino]thioxomethyl]-3,4,5-trimethoxy-benzamid (Verbindung 2);
- N-[[[3-(3-Methoxy-benzoylamino)phenyl]amino]thioxomethyl]-3,4,5-trimethoxy-benzamid (Verbindung 3);
- N-[[[3-(2-Methoxy-benzoylamino)phenyl]amino]thioxomethyl]-3,4,5-trimethoxy-benzamid (Verbindung 4);
- N-[[[3-(4-Pyridocarboxylamino)phenyl]amino]thioxomethyl]-3,4,5-trimethoxy-benzamid (Verbindung 11);
- N-[[[3-(4-Chloro-benzoylamino)phenyl]amino]thioxomethyl]-3,4,5-trimethoxy-benzamid (Verbindung 13);
- N-[[[3-(Benzoylamino)phenyl]amino]thioxomethyl]-3,5-dimethoxy-benzamid (Verbindung 18);
- N-[[[3-(Benzoylamino)phenyl]amino]thioxomethyl]-3,5-dimethoxy-4-ethoxy-benzamid (Verbindung 19);
- N-[[[3-(benzoylamino)phenyl]amino]thioxomethyl]-3,4-diethoxy-5-methoxy-benzamid (Verbindung 20);
- N-[[[3-(Benzoylamino)phenyl]amino]thioxomethyl]-3,4-methylendioxy-5-methoxy-benzamid (Verbindung 21);
- N-[[[3-(Benzoylamino)phenyl]amino]thioxomethyl]-3,4,5-triethoxy-benzamid (Verbindung 23);
- N-[[[4-Fluoro-3-(benzoylamino)phenyl]amino]thioxomethyl]-3,4,5-trimethoxy-benzamid (Verbindung 24);
- N-[[[4-Chloro-3-(phenylamino)carbonyl]amino]thioxomethyl]-3,4,5-trimethoxy-benzamid (Verbindung 25);
- N-[[[4-Chloro-3-(benzoylamino)phenyl]amino]thioxomethyl]-3,4,5-trimethoxy-benzamid (Verbindung 34);
- N-[[[3-(Benzoylamino)phenyl]amino]thioxomehyl]-3,4,5-trimethoxy-benzamid (Verbindung 38);
- 3,4,5-Trimethoxy-*N*-(3-(3-phenylureido)phenylcarbamothioyl)benzamid (I-b) (Verbindung 51),
- *N*-(3-(3-Biphenyl-4-ylureido)phenylcarbamathioyl)-3,4,5-trimethoxybenzamid (I-b) (Verbindung 52).

14. Pharmazeutische Zusammensetzung, **dadurch gekennzeichnet, dass** sie, als Wirkstoff, mindestens eine Verbindung umfasst, wie in einem der Ansprüche 11 bis 13 definiert, und mindestens einen pharmazeutisch annehmbaren Trägerstoff.

15. Verwendung der Verbindungen, wie nach einem der Ansprüche 11 bis 13 definiert, zur Herstellung eines Arzneimittels zur Behandlung der Pathologien der neurodegenerativen Art.

16. Verwendung nach Anspruch 15 zur Behandlung der Parkinson'schen Erkrankung, der Huntington'schen Erkrankung, der Alzheimer'sehen Erkrankung, der multiplen Sklerose oder der Motoneuronenerkrankung.

17. Verwendung der Verbindungen, wie nach einem der Ansprüche 11 bis 13 definiert, zur Herstellung eines Arzneimittels zur Behandlung von Diabetes.

18. Verbindungen, wie nach einem der Ansprüche 11 bis 13 definiert, für ihre Verwendung bei der Behandlung von Pathologien der neurodegenerativen Art.

19. Verbindungen nach Anspruch 18 für ihre Verwendung bei der Behandlung der Parkinson'schen Erkrankung, der Huntington'schen Erkrankung, der Alzheimer'schen Erkrankung, der multiplen Sklerose oder der Motoneuronenerkrankung.

20. Verbindungen, wie nach einem der Ansprüche 11 bis 13 definiert, für ihre Verwendung bei der Behandlung von Diabetes.

## Claims

1. Use of compounds of the following formula (I): 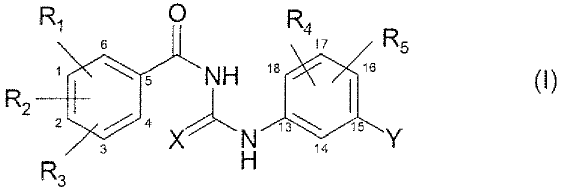 wherein:
- R₁, R₂ and R₃, identical or different and independently of one another, represent a hydrogen or halogen atom, a hydroxyl radical, an alkyl, perfluoroalkyl, alkoxy, alkylthio, nitrile group, a substituted alkoxy, or a fused heterocycle obtained from two of the adjacent R₁, R₂ and R₃ radicals, that can form, jointly with the carbon atoms of the phenyl cycle to which they are bonded, a fused heterocycle,
- X represents a sulphur or oxygen atom,
- Y represents a substituted or unsubstituted mono- or polycyclic heteroaryl group, a -NH-(C=O)-R₆, - (C=O)-NH-R₆ or -NH-(C=O)-NH-R₆ group wherein R₆ represents an unsubstituted aryl group; an aryl group comprising one or several substituents chosen from a halogen atom and an alkyl, alkoxy or mono- or dialkylamino radical; a mono- or polycyclic heteroaryl group; a linear or branched alkyl radical; a saturated or unsaturated mono- or polycyclic hydrocarbon group;
- R₄ and R₅, identical or different and independently of one another, represent a hydrogen or halogen atom, an alkoxy, alkylthio, alkyl, perfluoroalkyl, nitrile or nitro group,
for the manufacture of a drug for the treatment of tumours associated with a hyperactivation of the Hedgehog protein signalling pathway, said tumours being chosen from nerve tissue tumours (medulloblastomas, primitive neuroectodermal tumours, glioblastomas, meningeomas and oligodendrogliomas), skin tumours (basal cell carcinomas, trichoepitheliomas), bone and muscle tissue tumours (rhabdomyosarcomas, osteosarcomas) or tumours of other tissues (kidney, bladder) or pathologies of the neurodegenerative type chosen from multiple sclerosis or motor neuron disease.

2. Use according to claim 1, **characterised in that** the compounds are chosen from those wherein, in the formula (I):
- R₁, R₂, and R₃, identical or different, represent an atom of hydrogen or a methyloxy or ethyloxy radical,
- X represents a sulphur or oxygen atom,
- Y represents a substituted or unsubstituted mono- or polycyclic heteroaryl group, a -NH-(C=O)-R₆,-(C=O)-NH-R₆ or -NH-(C=O)-NH-R₆ group wherein R₆ represents a group chosen from the phenyl groups; phenyl substituted with a methoxy or dimethylamino radical, by a chlorine atom, with a phenyl group, with a benzyl group; cyclohexyl, isopropyl, pyridinyl, naphtyl, furfuryl and thiophene; and
- R₄ and R₅, identical or different, are chosen from hydrogen, chlorine, bromine, fluorine, methyl and methoxy.

3. Use according to one of claims 1 to 2, **characterised in that** the compounds of formula (I) are chosen from:
- N-[[[3-(4-methoxy-benzoylamino)phenyl]amino]thioxomethyl]-3,4,5-trimethoxy-benzamide (I-a) of the following formula: 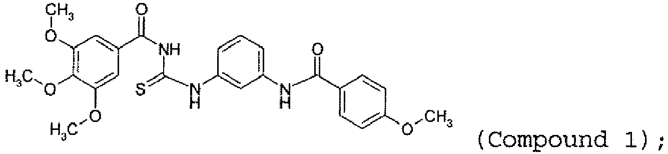
- N-[[[3-(2-chloro-benzoylamino)phenyl]amino]thioxomethyl]-3,4,5-trimethoxy-benzamide (I-a) of the following formula: 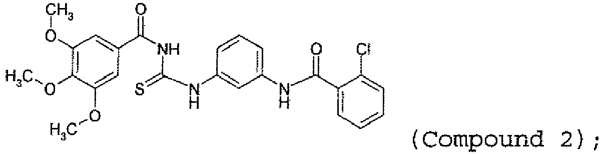
- N-[[[3-(3-methoxy-benzoylamino)phenyl]amino]thioxomethyl]-3,4,5-trimethoxy-benzamide (I-a) of the following formula: 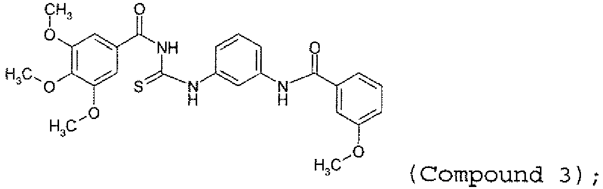
- N-[[[3-(2-methoxy-benzoylamino)phenyl]amino]thioxomethyl]-3,4,5-trimethoxy-benzamide (I-a) of the following formula: 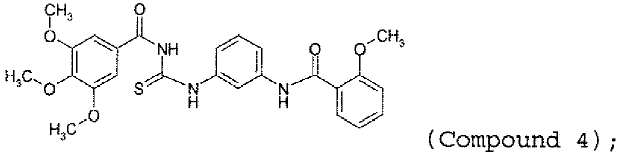
- N-[[[3-(4-phenyl-benzoylamino)phenyl]amino]thioxomethyl]-3,4,5-trimethoxy-benzamide (I-a) of the following formula: 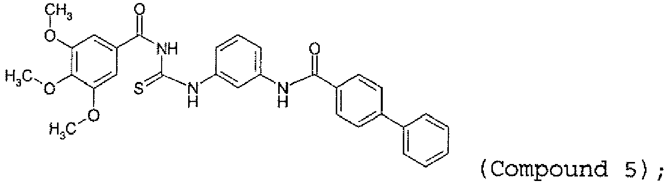
- N-[[[3-(4-methoxy-benzoylamino)phenyl]amino]oxomethyl]-3,4,5-trimethoxy-benzamide (I-a') of the following formula: 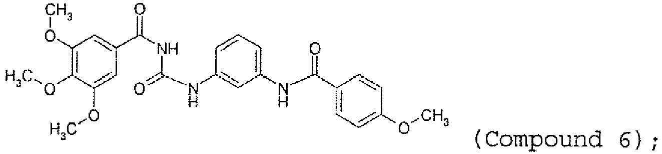
- N-[[[3-(4-phenyl-benzoylamino)phenyl]amino]oxomethyl]-3,4,5-trimethoxy-benzamide (I-a') of the following formula: 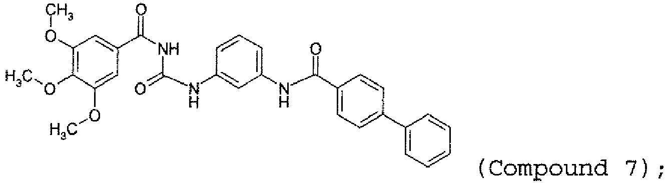
- N-[[[3-(-cyclohexylcarboxylamino)phenyl]amino]thioxomethyl]-3,4,5-trimethoxy-benzamide (I-a) of the following formula: 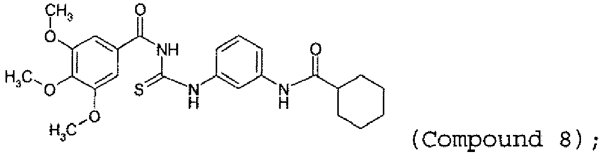
- N-[[[3-(2-naphtoylamino)phenyl]amino]thioxomethyl]-3,4,5-trimethoxy-benzamide (I-a) of the following formula: 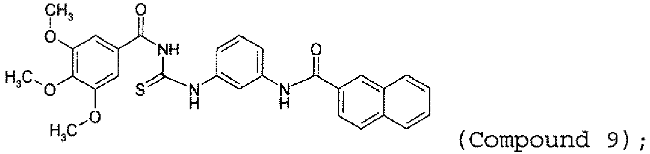
- N-[[[3-(isopropoylcarboxylamino)phenyl]amino]thioxomethyl]-3,4,5-trimethoxy-benzamide (I-a) of the following formula: 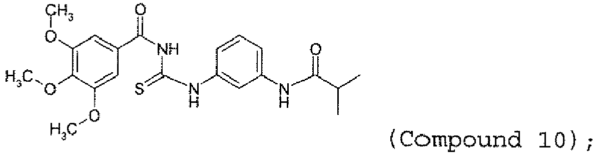
- N-[[[3-(4-pyridocarboxylamino)phenyl]amino]thioxomethyl]-3,4,5-trimethoxy-benzamide (I-a) of the following formula: 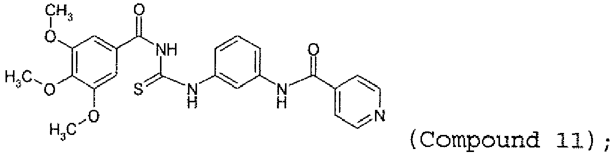
- N-[[[3-(1-naphtoylamino)phenyl]amino]thioxomethyl]-3,4,5-trimethoxy-benzamide (I-a) of the following formula: 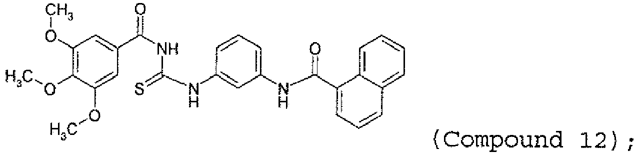
- N-[[[3-(4-chloro-benzoylamino)phenyl]amino]thioxomethyl]-3,4,5-trimethoxy-benzamide (I-a) of the following formula: 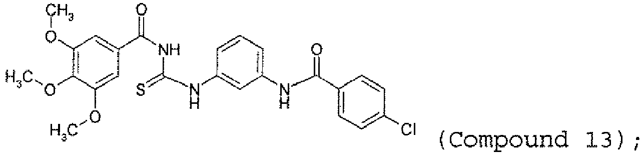
- N-[[[3-(2-furoylamino)phenyl]amino]thioxomethyl]-3,4,5-trimethoxy-benzamide (Ia) of the following formula: 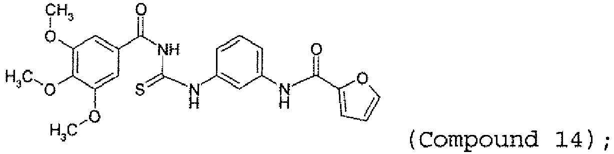
- N-[[[3-(2-thiophenoylamino)phenyl]amino]thioxomethyl]-3,4,5-trimethoxy-benzamide (I-a) of the following formula: 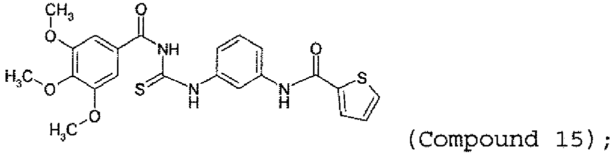
- N-[[[3-(2-naphtoylamino)phenyl]amino]oxomethyl]-3,4,5-trimethoxy-benzamide (I-a') of the following formula: 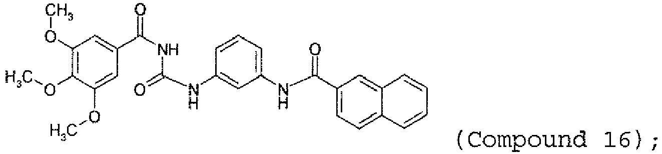
- N-[[[3-(isopropoylcarboxylamino)phenyl]amino]oxomethyl]-3,4,5-trimethoxy-benzamide (I-a') of the following formula: 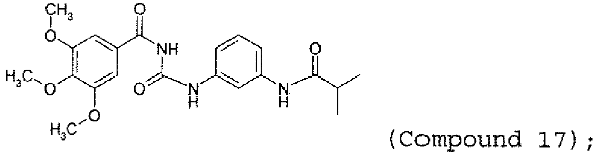
- N-[[[3-(benzoylamino)phenyl]amino]thioxomethyl]-3,5-dimethoxy-benzamide (I-a) of the following formula: 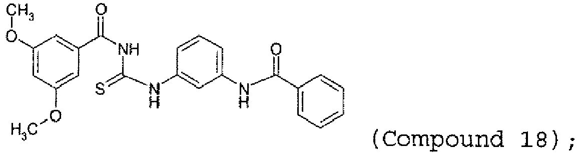
- N-[[[3-(benzoylamino)phenyl]amino]thioxomethyl]-3,5-dimethoxy-4-ethoxy-benzamide (I-a) of the following formula: 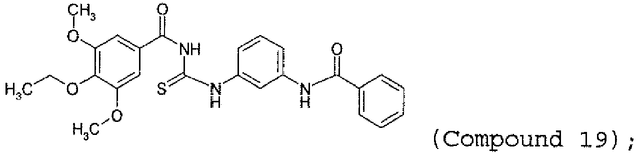
- N-[[[3-(benzoylamino)phenyl]amino]thioxomethyl]-3,4-diethoxy-5-methoxy-benzamide (I-a) of the following formula: 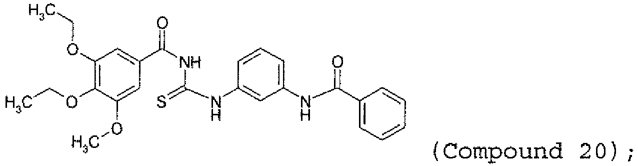
- N-[[[3-(benzoylamino)phenyl]amino]thioxomethyl]-3,4-methylenedioxy-5-methoxy-benzamide (I-a) of the following formula: 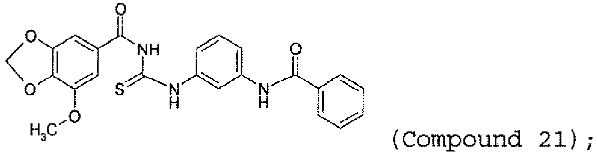
- N-[[[3-(benzoylamino)phenyl]amino]thioxomethyl]-2,4-dimethoxy-benzamide (I-a) of the following formula: 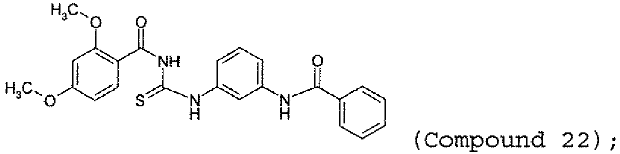
- N-[[[3-(benzoylamino)phenyl]amino]thioxomethyl]-3,4,5-triethoxy-benzamide (I-a) of the following formula: 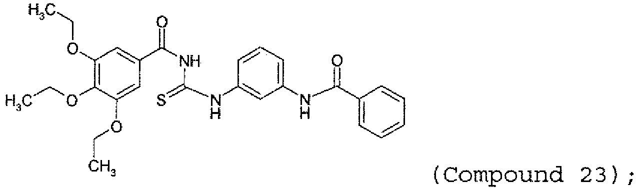
- N-[[[4-fluoro-3-(benzoylamino)phenyl]amino]thioxomethyl]-3,4,5-trimethoxy-benzamide (I-a) of the following formula: 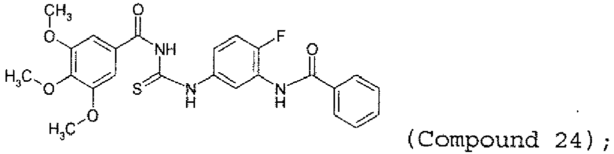
- N-[[[4-chloro-3-(phenylamino)carbonyl]amino]thioxomethyl]-3,4,5-trimethoxy-benzamide (I-b) of the following formula: 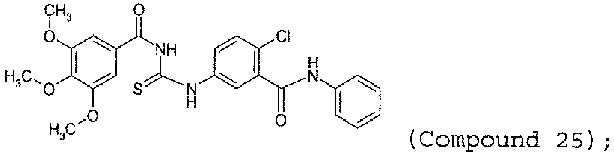
- N-[[[4-methoxy-3-(benzoylamino)phenyl]amino]thioxomethyl]-3,4,5-trimethoxy-benzamide (I-a) of the following formula: 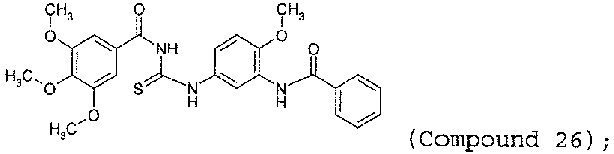
- N-[[[4-methyl-3-(benzoylamino)phenyl]amino]thioxomethyl]-3,4,5-trimethoxy-benzamide (I-a) of the following formula: 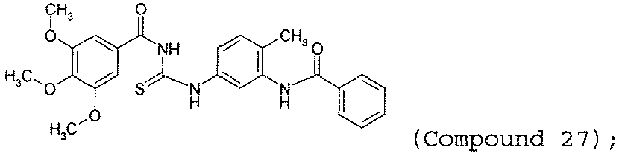
- N-[[[3-(benzoylamino)phenyl]amino]thioxomethyl]-3,4-ethylenedioxo-benzamide (I-a) of the following formula: 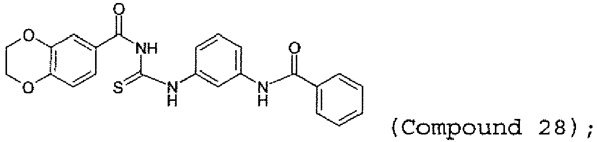
- N-[[[3-(benzoylamino)phenyl]amino]oxomethyl]-3,4,5-triethoxy-benzamide (I-a') of the following formula: 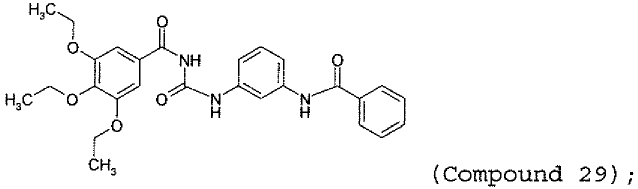
- N-[[[3-(benzoylamino)phenyl]amino]oxomethyl]-3,4-methylenedioxy-5-methoxy-benzamide (I-a') of the following formula: 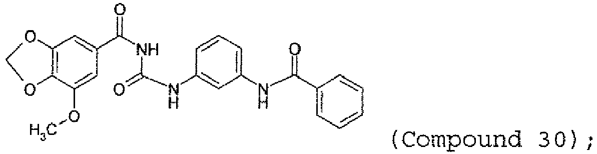
- N-[[[3-(benzoylamino)phenyl]amino]oxomethyl]-3,4,5-trimethoxy-benzamide (I-a') of the following formula: 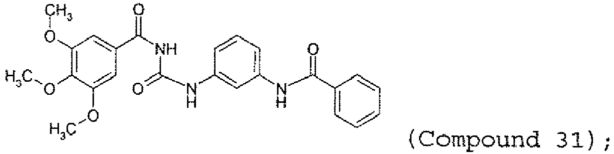
- N-[[[3-methyl-3-(benzoylamino)phenyl]amino]thioxomethyl]-3,4,5-trimethoxy-benzamide (I-a) of the following formula: 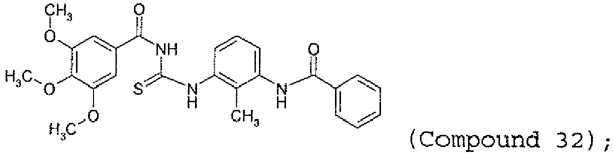
- N-[[[4-chloro-3-(benzoylamino)phenyl]amino]oxomethyl]3-3,4,5-trimethoxy-benzamide (I-a') of the following formula: 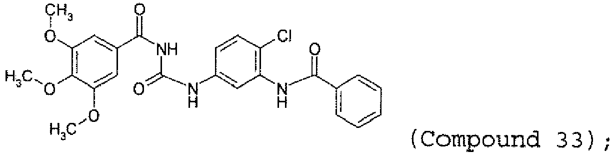
- N-[[[4-chloro-3-(benzoylamino)phenyl]amino]thioxomethyl]-3,4,5-trimethoxy-benzamide (I-a) of the following formula: 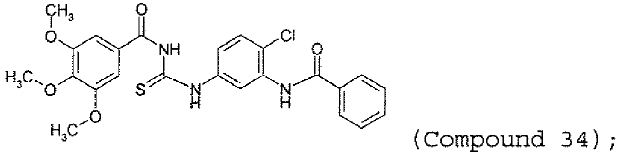
- N-[[[5-chloro-3-(benzoylamino)phenyl]amino]thioxomethyl]-3,4,5-trimethoxy-benzamide (I-a) of the following formula: 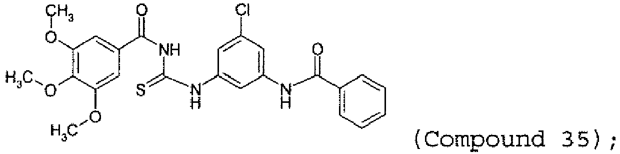
- N-[[[5-fluoro-3-(benzoylamino)phenyl]amino]thioxomethyl]-3,4,5-trimethoxy-benzamide (I-a) of the following formula: 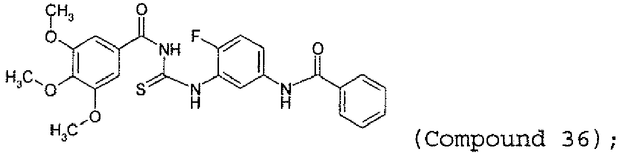
- N-[[[4-chloro-3-(4-Phenyl-benzoylamino)phenyl]amino]oxomethyl]-3,4,5-methoxy-benzamide (I-a') of the following formula: 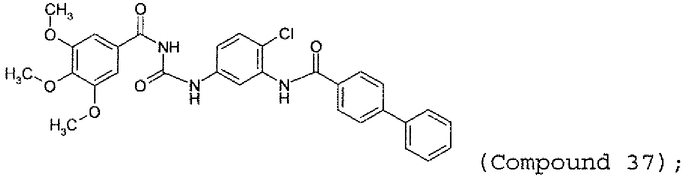
- N-[[[3-(benzoylamino)phenyl]amino]thioxomethyl]-3,4,5-trimethoxy-benzamide (I-a) of the following formula: 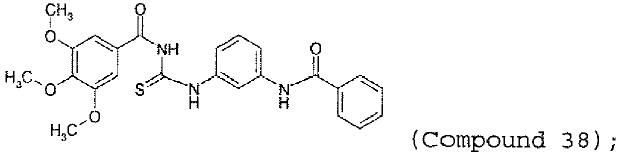
- N-[[[4-chloro-3-(4-dimethylamino-phenylamino)carbonyl]thioxomethyl]-3,4,5-trimethoxy-benzamide (I-b) of the following formula: 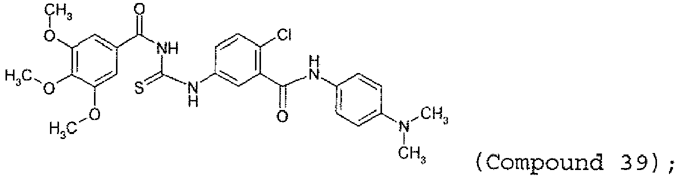
- N-[[[4-chloro-3-(4-methoxy-phenylamino)carbonyl] thioxomethyl]-3,4,5-trimethoxy-benzamide (I-b) of the following formula: 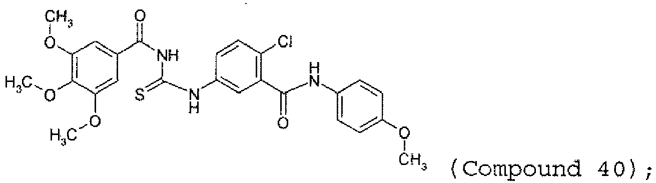
- N-[[[5-bromo-3-(phenylamino)carbonyl]thioxomethyl]-3,4,5-trimethoxy-benzamide (I-b) of the following formula: 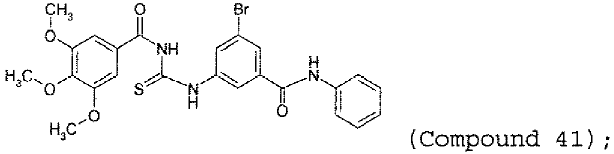
- N-[[[4-chloro-3-(4-methoxy-phenylamino)carbonyl] oxomethyl]-3,4,5-trimethoxy-benzamide (I-b') of the following formula: 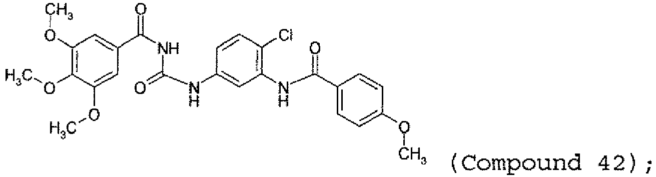
- N-[[[4-chloro-3-(4-chloro-phenylamino)]caxbonyl]phenylamino]thioxomethyl]-3,4,5-trimethoxy-benzamide (I-b) of the following formula: 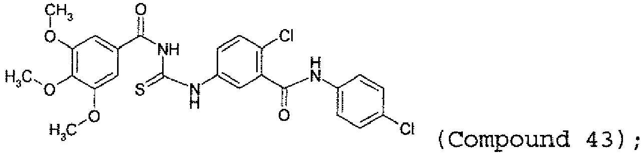
- N-[[[4-chloro-3-(phenylamino)carbonyl]oxomethyl]-3,4,5-trimethoxy-benzamide (I-b') of the following formula: 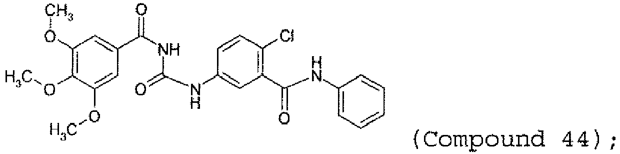
- N-[[[2-methyl-3-(benzoylamino)phenyl]amino]oxomethyl]-3,4,5-trimethoxy-benzamide (I-a') of the following formula: 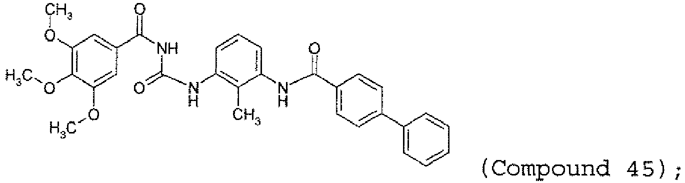
- N-[[[2-methyl-3-(benzoylamino)phenyl]amino]thioxomethyl]-3,4,5-trimethoxy-benzamide (I-a) of the following formula: 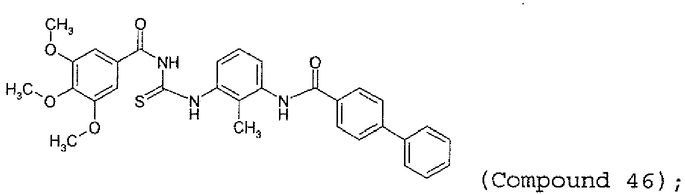
- N-[[[-3-(4-chloro-benzoylamino)phenyl]amino]oxomethyl]-3,4,5-trimethoxy-benzamide (I-a') of the following formula: 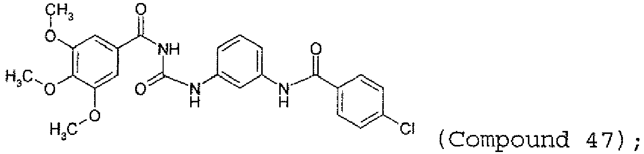
- N-[[[-3-(benzoylamino)phenyl]amino]oxomethyl]-2,4,5-trimethoxy-benzamide (I-a') of the following formula: 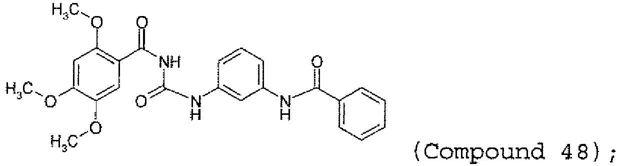
- N-[[[-3-(benzoylamino)phenyl]amino]oxomethyl]-4-methoxy-benzamide (I-a') of the following formula: 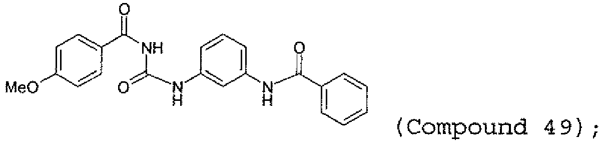
- N-[[[-3-(benzoylamino)phenyl]amino]oxomethyl]-benzamide (I-a') of the following formula: 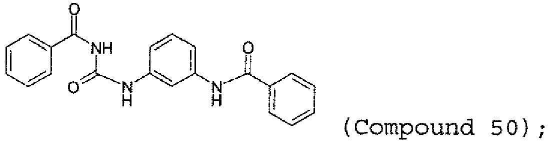
- 3,4,5-trimethoxy-*N*-(3-(3-phenylureido)phenylcarbamothioyl)benzamide (I-b) of the following formula: 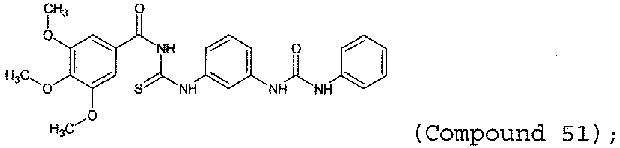
- N-(3-(3-biphenyl-4-ylureido)phenylcarbamothioyl)-3,4,5-trimethoxybenzamide (I-b) of formula: 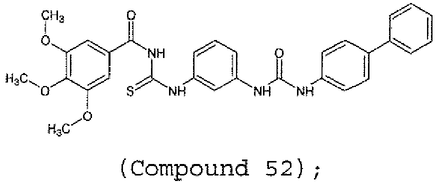
- *N*-(3-(1*H*-indol-2-yl)phenylcarbamoyl)-3,4,5-trimethoxybenzamide (I-d') of formula: 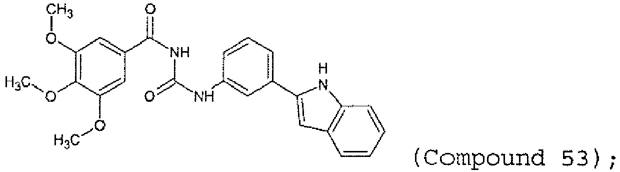
- *N*-(3-(1*H*-3ndo1-2-yl)phenylcarbamothioyl)-3,4,5-trimethoxybenzamide (I-d) of formula: 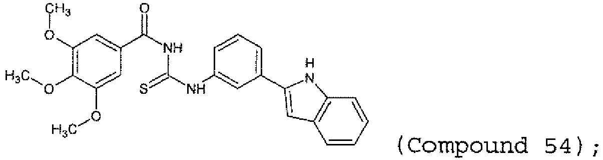
- *N*-(3-(imidazo[1,2-*a*]pyridin-2-yl)phenylcarbamothioyl)-3,4,5-trimethoxybenzamide (I-d) of formula: 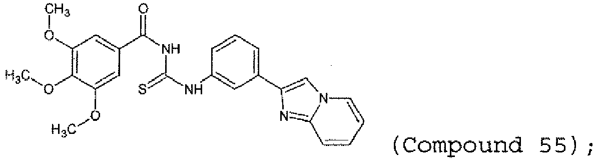
- *N*-(3-(1*H*-benzo[*d*]imidazol-2-yl)-4-methylphenylcarbamothioyl)-3,4,5-trimethoxybenzamide (I-d) of formula: 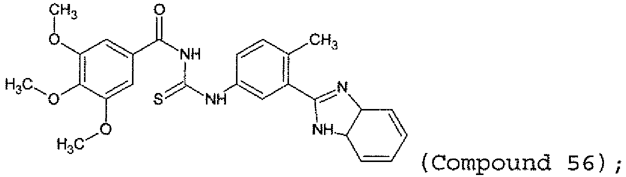
- *N*-(3-(1*H*-indol-1-yl)phenylcarbamothioyl)-3,4,5-trimethoxybenzamide (I-d) of formula: 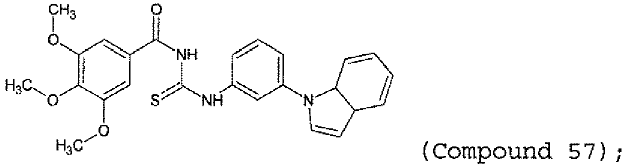 and
- *N*-(3-(imidazo[2,1-*b*]thiazol-6-yl)phenylcarbamothioyl)-3,4,5-trimethoxybenzamide (I-d), of formula: 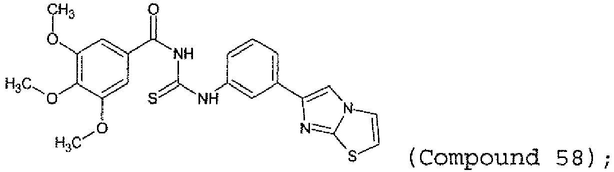
- *N*-(3-(3-biphenyl-4-ylureido)phenylcarbamoyl)-3,4,5-trimethoxybenzamide (I-b') of formula:

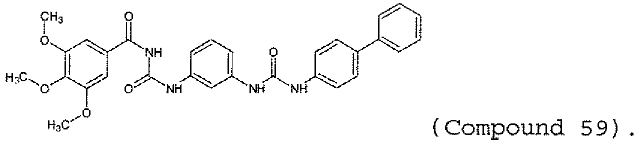

4. Use according to one of claims 1 to 3, **characterised in that** the compounds of formula (I) are chosen from:
- N-[[[3-(4-methoxy-benzoylamino)phenyl]amino]thioxomethyl]-3,4,5-trimethoxy-benzamide (Compound 1);
- N-[[[3-(2-chloro-benzoylamino)phenyl]amino]thioxomethyl]-3,4,5-trimethoxy-benzamide (Compound 2);
- N-[[[3-(3-methoxy-benzoylamino)phenyl]amino]thioxomethyl]-3,4,5-trimethoxy-benzamide (Compound 3);
- N-[[[3-(2-methoxy-benzoylamino)phenyl]amino]thioxomethyl]-3,4,5-trimethoxy-benzamide (Compound 4);
- N-[[[3-(4-methoxy-benzoylamino)phenyl]amino]oxomethyl]-3,4,5-trimethoxy-benzamide (Compound 6);
- N-[[[3-(4-pyridocarboxylamino)phenyl]amino]thioxomethyl]-3,4,5-trimethoxy-benzamide (Compound 11);
- N-[[[3-(4-chloro-benzoylamino)phenyl]amino]thioxomethyl]- 3,4,5-trimethoxy-benzamide (Compound 13);
- N-[[[3-(isopropoylcarboxylamino))phenyl]amino]oxomethyl]-3,4,5-trimethoxy-benzamide (Compound 17);
- N-[[[3-(benzoylamino)phenyl]amino]thioxomethyl]-3,5-dimethoxy-benzamide (Compound 18);
- N-[[[3-(benzoylamino)phenyl]amino]thioxomethyl]-3,5-dimethoxy-4-ethoxy-benzamide (Compound 19);
- N-[[[3-(benzoylamino)phenyl]amino]thioxomethyl]-3,4-diethoxy-5-methoxy-benzamide (Compound 20);
- N-[[[3-(benzoylamino)phenyl]amino]thioxomethyl]-3,4-methylenedioxy-5methoxy-benzamide (Compound 21);
- N-[[[3-(benzoylamino)phenyl]amino]thioxomethyl]-3,4,5-triethoxy-benzamide (Compound 23);
- N-[[[4-fluoro-3-(benzoylamino)phenyl]amino]thioxomethyl]-3,4,5-trimethoxy-benzamide (Compound 24);
- N-[[[4-chloro-3-(phenylamino)carbonyl]amino]thioxomethyl]-3,4,5-trimethoxy-benzamide (Compound 25);
- N-[[[3-(benzoylamino)phenyl]amino]oxomethyl]-3,4,5-trimethoxy-benzamide (Compound 31);
- N-[[[4-chloro-3-(benzoylamino)phenyl]amino]thioxomethyl]-3,4,5-trimethoxy-benzamide (Compound 34);
- N-[[[3-(benzoylamino)phenyl]amino]thioxomethyl]-3,4,5-trimethoxy-benzamide (Compound 38);
- N-[[[4-chloro-3-(4-dimethylamino-phenylamino)carbonyl] thioxomethyl]-3,4,5-trimethoxy-benzamide (Compound 39);
- N-[[[4-chloro-3-(4-chloro-phenylamino)]carbonyl]phenylaminol]thioxomethyl]-3,4,5-trimethoxy-benzamide (Compound 43);
- N-[[[2-methyl-3-(benzoylamino)phenyl]amino]oxomethyl]-3,4,5-trimethoxy-benzamide (Compound 45);
- N-[[[-3-(4-chloro-benzoylamino)phenyl]amino]oxomethyl]-3,4,5-trimethoxy-benzamide (Compound 47);
- 3,4,5-trimethoxy-*N*-(3-(3-phenylureido)phenylcarbamothioyl)benzamide (I-b) (Compound 51),
- *N-* (3- (3-biphenyl-4-ylureido)phenylcarbamothioyl)-3,4,5-trimethoxybenzamide (I-b) (Compound 52);
- *N*-(3-(1*H*-indol-2-yl)phenylcarbamoyl)-3,4,5-trimethoxybenzamide (I-d') (Compound 53);
- *N*-(3-(1*H*-indol-2-yl)phenylcarbamothioyl)-3,4,5-trimethoxybenzamide (I-d) (Compound 54);
- *N*-(3-(imidazo[1,2-*a*]pyridin-2-yl)phenylcarbamothioyl)-3,4,5-trimethoxybenzamide (I-d) (Compound 55);
- *N*-(3-(1*H*-indol-1-yl)phenylcarbamothioyl)-3,4,5-trimethoxybenzamide (Compound 57);
- *N*-(3-(imidazo[2,1-*b*]thiazol-6-yl)phenylcarbamothioyl)-3,4,5-trimethoxybenzamide (I-d) (Compound 58); and
- *N*-(3-(3-biphenyl-4-ylureido)phenylcarbamoyl)-3,4,5-trimethoxybenzamide (I-b') (Compound 59).

5. Use of compounds of the following formula (I): 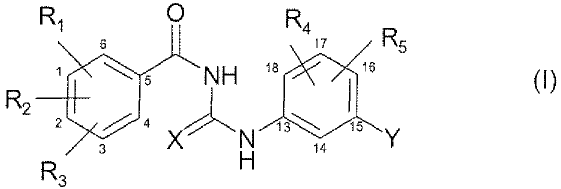 wherein:
- R₁, R₂ and R₃, identical or different and independently of one another, represent a hydrogen or halogen atom, a hydroxyl radical, an alkyl, perfluoroalkyl, alkoxy, alkylthio, nitrile group or a substituted alkoxy, or a fused heterocycle obtained from two of the adjacent R₁, R₂ and R₃ radicals, that can form, jointly with the carbon atoms of the phenyl cycle to which they are bonded, a fused heterocycle,
- X represents a sulphur or oxygen atom,
- Y represents a -NH-(C=O)-R₆, -(C=O)-NH-R₆ or -NH-(C=O)-NH-R₆ group wherein R₆ represents an unsubstituted aryl group; an aryl group comprising one or several substituents chosen from a halogen atom and an alkyl, alkoxy or mono- or dialkylamino radical; a mono- or polycyclic heteroaryl group; a linear or branched alkyl radical; a saturated or unsaturated mono- or polycyclic hydrocarbon group;
- R₄ and R₅, identical or different and independently of one another, represent a hydrogen or halogen atom, an alkoxy, alkylthio, alkyl, perfluoroalkyl or nitrile or nitro group,
for the manufacture of a drug for the treatment of tumours associated with a hyperactivation of the Hedgehog protein signalling pathway or pathologies of the neurodegenerative type.

6. Use of compounds of the following formula (I): 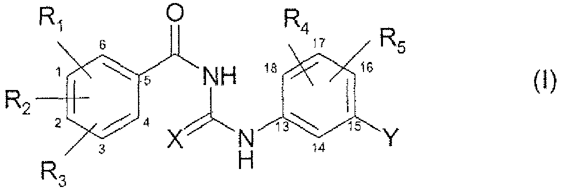 wherein:
- R₁, R₂ and R₃, identical or different and independently of one another, represent a hydrogen or halogen atom, a hydroxyl radical, an alkyl, perfluoroalkyl, alkoxy, alkylthio, nitrile, or substituted alkoxy group;
- X represents a sulphur or oxygen atom,
- Y represents a -NH-(C=O)-R₆, - (C=O)-NH-R₆ or -NH-(C=O)-NH-R₆ group wherein R₆ represents an unsubstituted aryl group; an aryl group comprising one or several substituents chosen from a halogen atom and an alkyl, alkoxy or mono- or dialkylamino radical; a mono- or polycyclic heteroaryl group; a linear or branched alkyl radical; a saturated or unsaturated mono- or polycyclic hydrocarbon group;
- R₄ and R₅, identical or different and independently of one another, represent a hydrogen or halogen atom, an alkoxy, alkylthio, alkyl, perfluoroalkyl or nitrile group;
with the understanding that when Y represents a NH(C=O)R₆ group:
-- R₄ and/or R₅ can also represent a nitro group, and
-- two of the adjacent R₁, R₂ and R₃ can also form, jointly with the carbon atoms of the phenyl cycle to which they are bonded, a fused heterocycle,
for the manufacture of a drug for the treatment of tumours associated with a hyperactivation of the Hedgehog protein signalling pathway or pathologies of the neurodegenerative type.

7. Use according to claim 5 or 6, **characterised in that** the compounds are chosen from those wherein, in the formula (I):
- R₁, R₂, and R₃, identical or different, represent an atom of hydrogen or a methyloxy or ethyloxy radical,
- X represents a sulphur or oxygen atom,
- Y represents a -NH-(C=O)-R₆, -(C=O)-NH-R₆ or -NH-(C=O)-NH-R₆ group wherein R₆ represents a group chosen from the phenyl groups; phenyl substituted with a methoxy or dimethylamino radical, by a chlorine atom, with a phenyl group, with a benzyl group; cyclohexyl, isopropyl, pyridinyl, naphtyl, furfuryl and thiophene; and
- R₄ and R₅, identical or different, are chosen from hydrogen, chlorine, bromine, fluorine, methyl and methoxy.

8. Compounds according to one of claims 5 to 7, **characterised in that** the compounds of formula (I) are chosen from:
- N-[[[3-(4-methoxy-benzoylamino)phenyl]amino]thioxomethyl]-3,4,5-trimethoxy-benzamide (I-a) of the following formula: 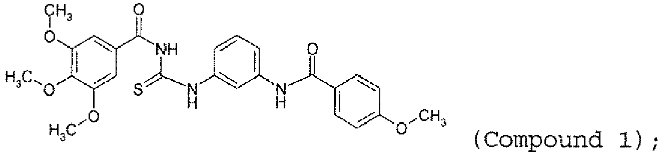
- N-[[[3-(2-chloro-benzoylamino)phenyl]amino]thioxomethyl]-3,4,5-trimethoxy-benzamide (I-a) of the following formula: 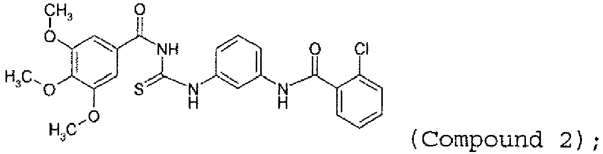
- N-[[[3-(3-methoxy-benzoylamino)phenyl]amino]thioxomethyl]-3,4,5-trimethoxy-benzamide (I-a) of the following formula: 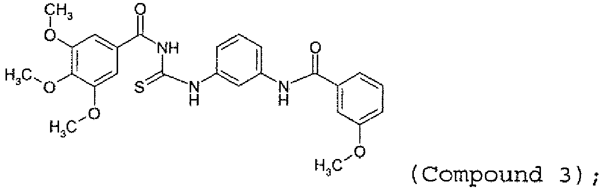
- N-[[[3-(2-methoxy-benzoylamino)phenyl]amino]thioxomethyl]-3,4,5-trimethoxy-benzamide (I-a) of the following formula: 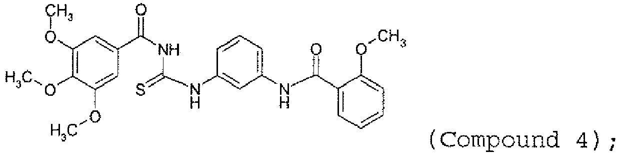
- N-[[[3-(4-phenyl-benzoylamino)phenyl]amino]thioxomethyl]-3,4,5-trimethoxy-benzamide (I-a) of the following formula: 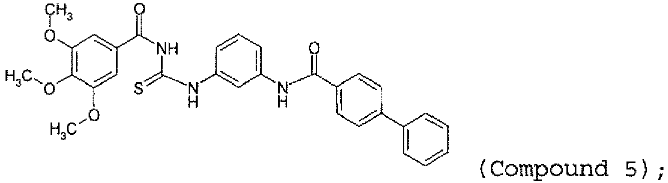
- N-[[[3-(4-methoxy-benzoylamino)phenyl]amino]oxomethyl]-3,4,5-trimethoxy-benzamide (I-a') of the following formula: 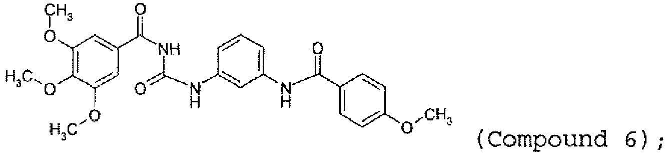
- N-[[[3-(4-phenyl-benzoylamino)phenyl]amino]oxomethyl]-3,4,5-trimethoxy-benzamide (I-a') of the following formula: 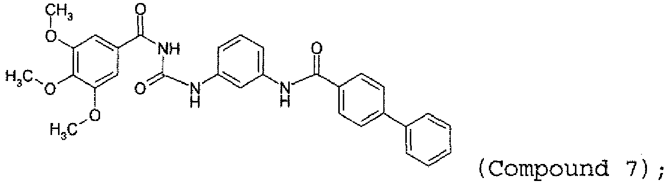
- N-[[[3-(-cyclohexylcarboxylamino)phenyl]amino]thioxomethyl]-3,4,5-trimethoxy-benzamide (I-a) of the following formula: 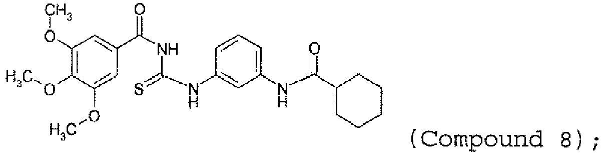
- N-[[[3-(2-naphtoylamino)phenyl]amino]thioxomethyl]-3,4,5-trimethoxy-benzamide (I-a) of the following formula: 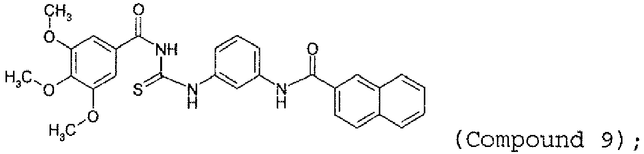
- N-[[[3-(isopropoylcarboxylamino)phenyl]amino]thioxomethyl]-3,4,5-trimethoxy-benzamide (I-a) of the following formula: 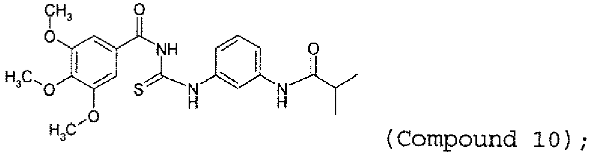
- N-[[[3-(4-pyridocarboxylamino)phenyl]amino]thioxomethyl]-3,4,5-trimethoxy-benzamide (I-a) of the following formula: 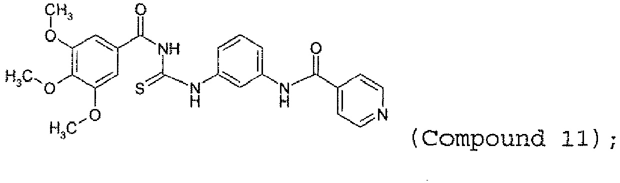
- N-[[[3-(1-naphtoylamino)phenyl]amino]thioxomethyl]-3,4,5-trimethoxy-benzamide (I-a) of the following formula: 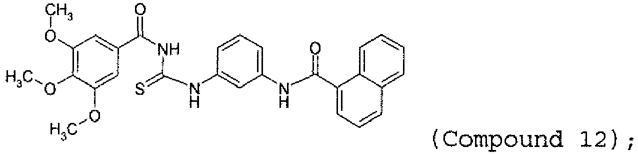
- N-[[[3-(4-chloro-benzoylamino)phenyl]amino]thioxomethyl]-3,4,5-trimethoxy-benzamide (I-a) of the following formula: 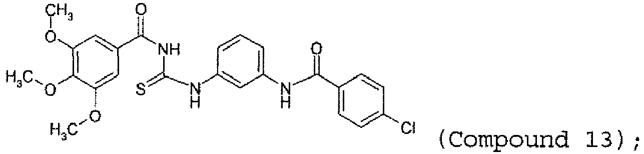
- N-[[[3-(2-furoylamino)phenyl]amino]thioxomethyl]-3,4,5-trimethoxy-benzamide (I-a) of the following formula: 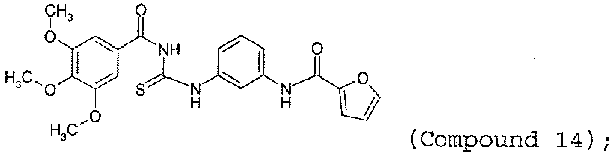
- N-[[[3-(2-thiophenoylamino)phenyl]amino]thioxomethyl]-3,4,5-trimethoxy-benzamide (I-a) of the following formula: 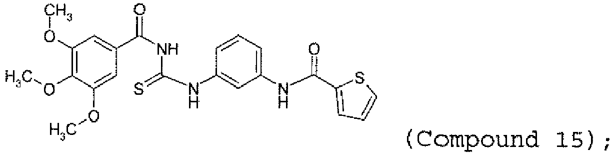
- N-[[[3-(2-naphtoylamino)phenyl]amino]oxomethyl]-3,4,5-trimethoxy-benzamide (I-a') of the following formula: 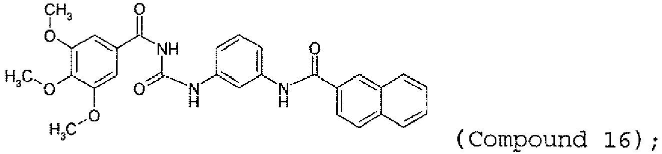
- N-[[[3-(isopropoylcarboxylamino)phenyl]amino]oxomethyl]-3,4,5-trimethoxy-benzamide (I-a') of the following formula: 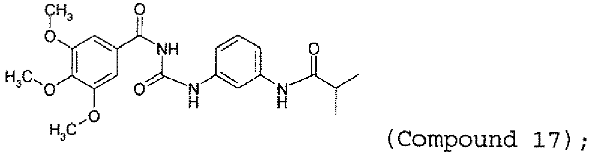
- N-[[[3-(benzoylamino)phenyl]amino]thioxomethyl]-3,5-dimethoxy-benzamide (I-a) of the following formula: 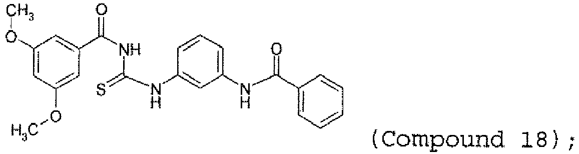
- N-[[[3-(benzoylamino)phenyl]amino]thioxomethyl]-3,5-dimethoxy-4-ethoxy-benzamide (I-a) of the following formula: 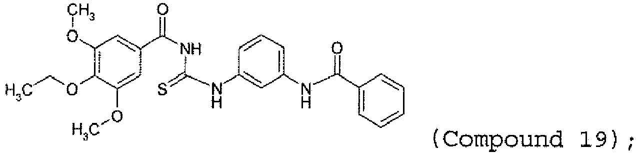
- N-[[[3-(benzoylamino)phenyl]amino]thioxomethyl]-3,4-diethoxy-5-methoxy-benzamide (I-a) of the following formula: 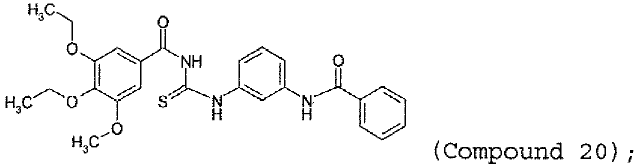
- N-[[[3-(benzoylamino)phenyl]amino]thioxomethyl]-3,4-methylenedioxy-5-methoxy-benzamide (I-a) of the following formula: 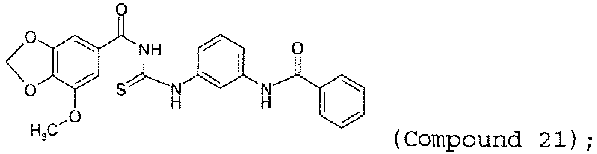
- N-[[[3-(benzoylamino)phenyl]amino]thioxomethyl]-2,4-dimethoxy-benzamide (I-a) of the following formula: 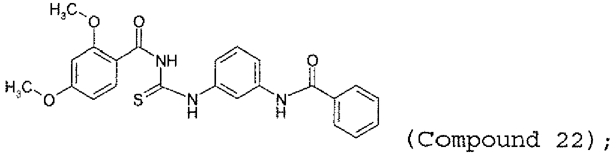
- N-[[[3-(benzoylamino)phenyl]amino]thioxomethyl]-3,4,5-triethoxy-benzamide (I-a) of the following formula: 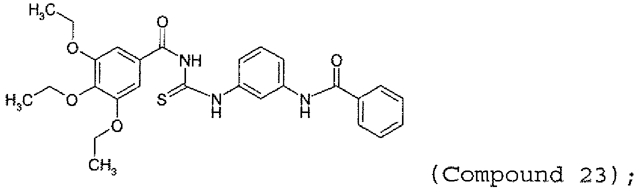
- N-[[[4-fluoro-3-(benzoylamino)phenyl]amino]thioxomethyl]-3,4,5-trimethoxy-benzamide (I-a) of the following formula: 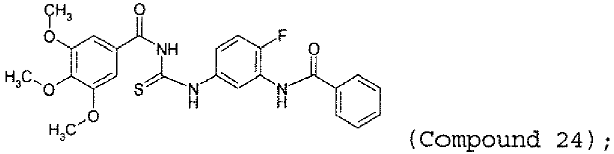
- N-[[[4-chloro-3-(phenylamino)carbonyl]amino]thioxomethyl-3,4,5-trimethoxy-benzamide (I-b) of the following formula: 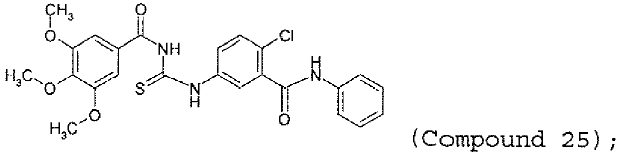
- N-[[[4-methoxy-3-(benzoylamino)phenyl]amino]thioxomethyl]-3,4,5-trimethoxy-benzamide (I-a) of the following formula: 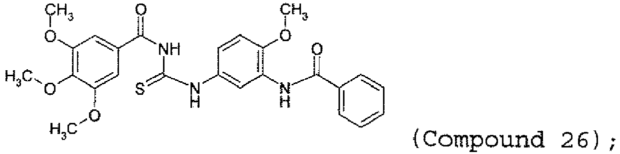
- N-[[[4-methyl-3-(benzoylamino)phenyl]amino]thioxomethyl]-3,4,5-trimethoxy-benzamide (I-a) of the following formula: 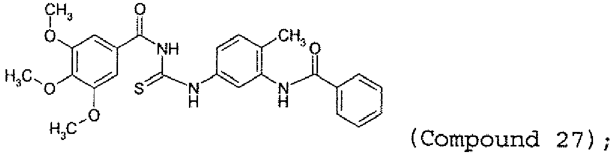
- N-[[[3-(benzoylamino)phenyl]amino]thioxomethyl]-3,4-ethylenedioxo-benzamide (I-a) of the following formula: 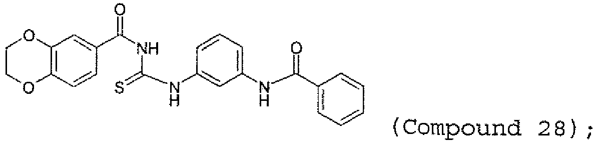
- N-[[[3-(benzoylamino)phenyl]amino]oxomethyl]-3,4,5-triethoxy-benzamide (I-a') of the following formula: 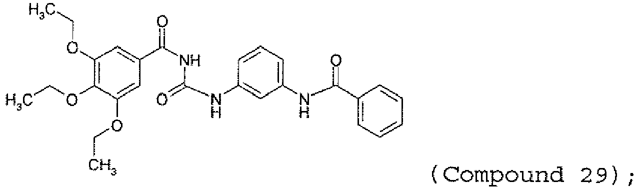
- N-[[[3-(benzoylamino)phenyl]amino]oxomethyl]-3,4-methylenedioxy-5-methoxy-benzamide (I-a') of the following formula: 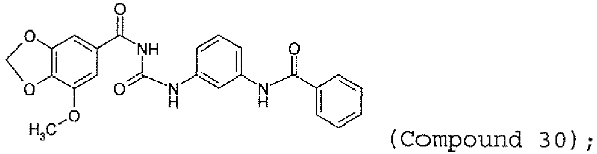
- N-[[[3-(benzoylamino)phenyl]amino]oxomethyl]-3,4,5-trimethoxy-benzamide (I-a') of the following formula: 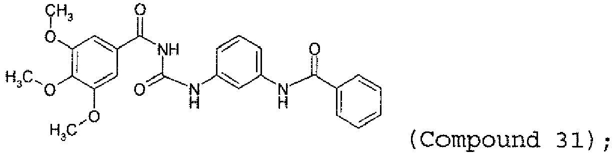
- N-[[[3-methyl-3-(benzoylamino)phenyl]amino]thioxomethyl]-3,4,5-trimethoxy-benzamide (I-a) of the following formula: 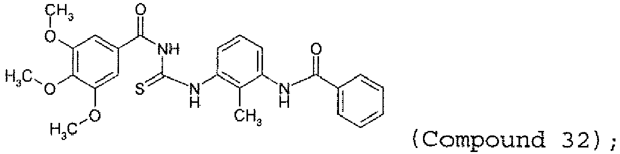
- N-[[[4-chloro-3-(benzoylamino)phenyl]amino]thioxomethyl]-3,4,5-trimethoxy-benzamide (I-a) of the following formula: 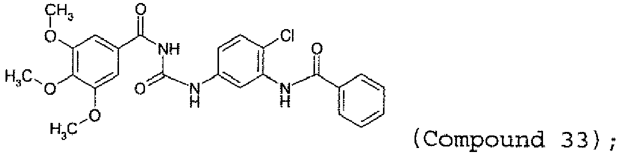
- N-[[[4-chloro-3-(benzoylamino)phenyl]amino]thioxomethyl]-3,4,5-trimethoxy-benzamide (I-a) of the following formula: 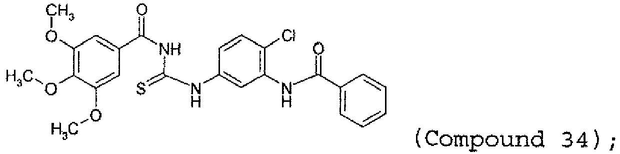
- N-[[[5-chloro-3-(benzoylamino)phenyl]amino]thioxomethyl]-3,4,5-trimethoxy-benzamide (I-a) of the following formula: 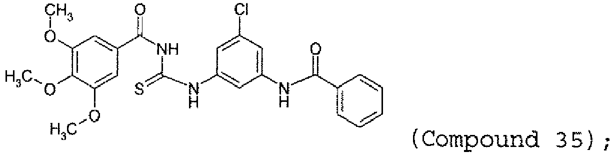
- N-[[[5-fluoro-3-(benzoylamino)phenyl]amino]thioxomethyl]-3,4,5-trimethoxy-benzamide (I-a) of the following formula: 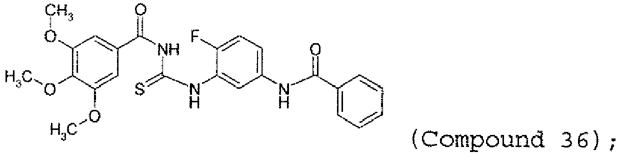
- N-[[[4-chloro-3-(4-Phenyl-benzoylamino)phenyl]amino]oxomethyl]-3,4,5-trimethoxy-benzamide (I-a') of the following formula: 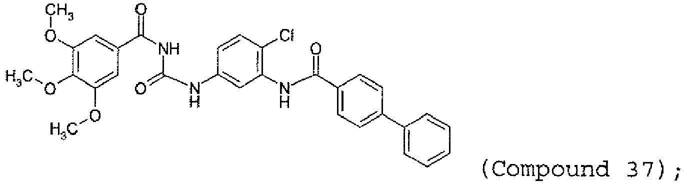
- N-[[[3-(benzoylamino)phenyl]amino]thioxomethyl]-3,4,5-trimethoxy-benzamide (I-a) of the following formula: 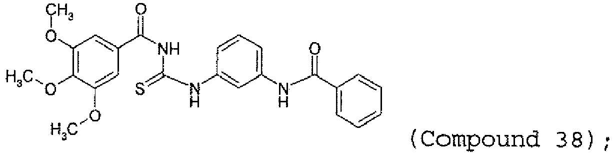
- N-[[[4-chloro-3-(4-dimethylamino-phenylamino)carbonyl] thioxomethyl]-3,4,5-trimethoxy-benzamide (I-b) of the following formula: 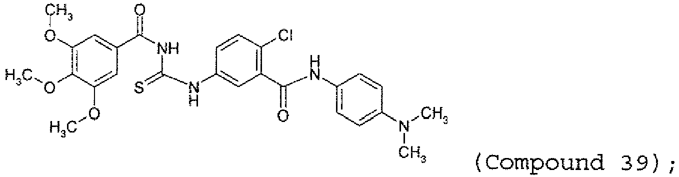
- N-[[[4-chloro-3-(4-methoxy-phenylamino)carbonyl] thioxomethyl]-3,4,5-trimethoxy-benzamide (I-b) of the following formula: 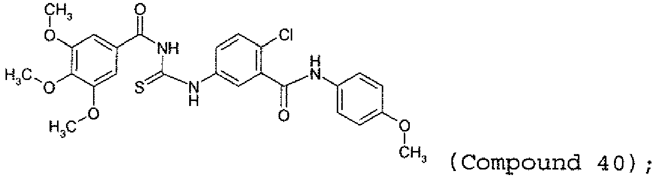
- N-[[[5-bromo-3-(phenylamino)carbonyl]thioxomethyl]-3,4,5-trimethoxy-benzamide (I-b) of the following formula: 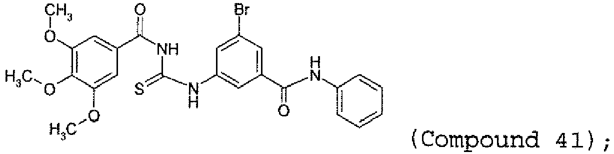
- N-[[[4-chloro-3-(4-methoxy-phenylamino)carbonyl] oxomethyl]-3,4,5-trimethoxy-benzamide (I-b') of the following formula: 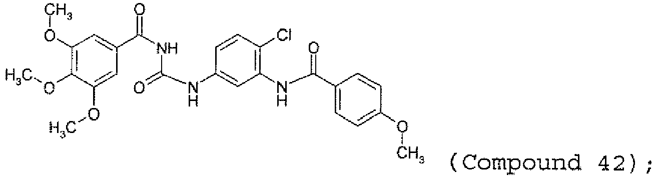
- N-[[[4-chloro-3-(4-chloro-phenylamino)]carbonyl]phenylamino]thioxomethyl]-3,4,5-trimethoxy-benzamide (I-b) of the following formula: 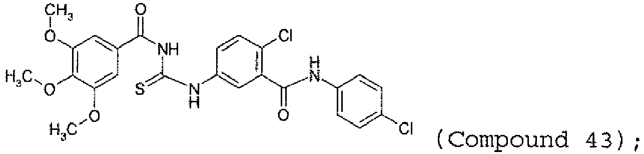
- N-[[[4-chloro-3-(phenylamino)carbonyl]oxomethyl]-3,4,5-trimethoxy-benzamide (I-b') of the following formula: 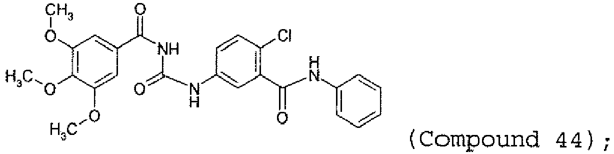
- N-[[[2-methyl-3-(benzoylamino)phenyl]amino]oxomethyl]-3,4,5-trimethoxy-benzamide (I-a') of the following formula: 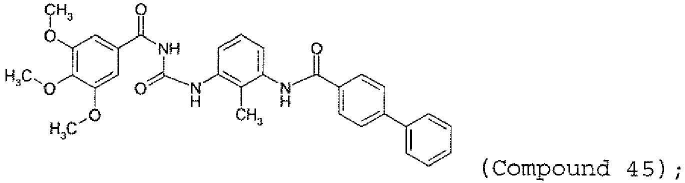
- N-[[[2-methyl-3-(benzoylamino)phenyl]amino]thioxomethyl]-3,4,5-trimethoxy-benzamide (I-a) of the following formula: 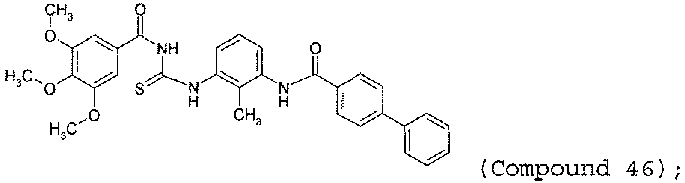
- N-[[[-3-(4-chloro-benzoylamino)phenyl]amino]oxomethyl]-3,4,5-trimethoxy-benzamide (I-a') of the following formula: 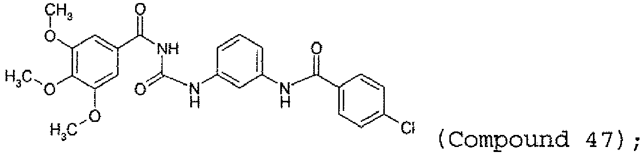
- N-[[[-3-(benzoylamino)phenyl]amino]oxomethyl]-2,4,5-trimethoxy-benzamide (I-a') of the following formula: 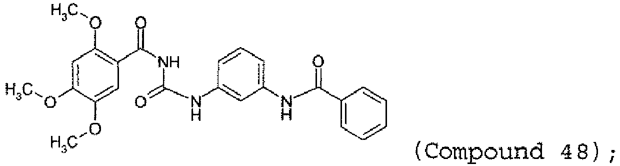
- N-[[[-3-(benzoylamino)phenyl]amino]oxomethyl]-4-methoxy-benzamide (I-a') of the following formula: 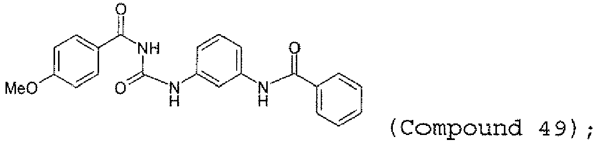
- N-[[[-3-(benzoylamino)phenyl]amino]oxomethyl]-benzamide (I-a') of the following formula: 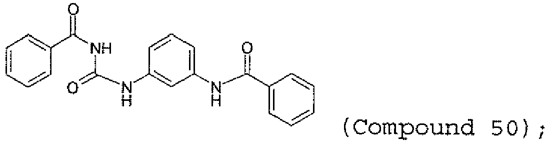
- 3,4,5-trimethoxy-*N*-(3-(3-phenylureido)phenylcarbamothioyl)benzamide (I-b) of the following formula: 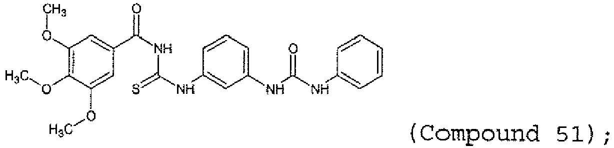
- *N*-(3-(3-biphenyl-4-ylureido)phenylcarbamothioyl)-3,4,5-trimethoxybenzamide (I-b) of formula: 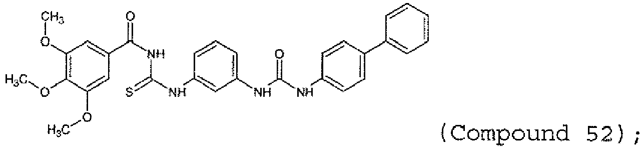
- *N*-(3-(3-biphenyl-4-ylureido)phenylcarbamoyl)-3,4,5-trimethoxybenzamide (I-b') of formula:

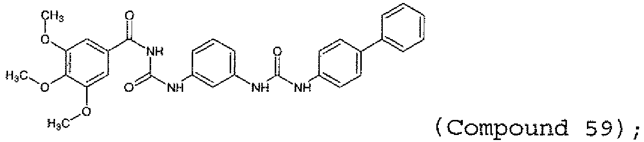

9. Compounds according to one of claims 5 to 8, **characterised in that** the compounds of formula (I) are chosen from:
- N-[[[3-(4-methoxy-benzoylamino)phenyl]amino]thioxomethyl]-3,4,5-trimethoxy-benzamide (Compound 1);
- N-[[[3-(2-chloro-benzoylamino)phenyl]amino]thioxomethyl]-3,4,5-trimethoxy-benzamide (Compound 2);
- N-[[[3-(3-methoxy-benzoylamino)phenyl]amino]thioxomethyl-3,4,5-trimethoxy-benzamide (Compound 3);
- N-[[[3-(2-methoxy-benzoylamino)phenyl]amino]thioxomethyl]-3,4,5-trimethoxy-benzamide (Compound 4);
- N-[[[3-(4-methoxy-benzoylamino)phenyl]amino]oxomethyl]-3,4,5-trimethoxy-benzamide (Compound 6);
- N-[[[3-(4-pyridocarboxylamino)phenyl]amino]thioxomethyl]-3,4,5-trimethoxy-benzamide (Compound 11);
- N-[[[3-(4-chloro-benzoylamino)phenyl]amino]thioxomethyl]- 3,4,5-trimethoxy-benzamide (Compound 13);
- N-[[[3-(isopropoylcarboxylamino))phenyl]amino]oxomethyl]-3,4,5-trimethoxy-benzamide (Compound 17);
- N-[[[3-(benzoylamino)phenyl]amino]thioxomethyl]-3,5-dimethoxy-benzamide (Compound 18);
- N-[[[3-(benzoylamino)phenyl]amino]thioxomethyl]-3,5-dimethoxy-4-ethoxy--benzamide (Compound 19);
- N-[[[3-(benzoylamino)phenyl]amino]thioxomethyl]-3,4-diethoxy-5-methoxy-benzamide (Compound 20);
- N-[[[3-(benzoylamino)phenyl]amino]thioxomethyl]-3,4-methylenedioxy-5-methoxy-benzamide (Compound 21);
- N-[[[3-(benzoylamino)phenyl]amino]thioxomethyl]-3.4,5-triethoxy-benzamide (Compound 23);
- N-[[[4-fluoro-3-(benzoylamino)phenyl]amino]thioxomethyl]-3,4,5-trimethoxy-benzamide (Compound 24);
- N-[[[4-chloro-3-(phenylamino)carbonyl]amino]thioxomethyl]-3,4,5-trimethoxy-benzamide (Compound 25);
- N-[[[3-(benzoylamino)phenyl]amino]oxomethyl]-3,4,5-trimethoxy-benzamide (Compound 31);
- N-[[[4-chloro-3-(benzoylamino)phenyl]amino]thioxomethyl]-3,4,5-trimethoxy-benzamide (Compound 34);
- N-[[[3-(benzoylamino)phenyl]amino]thioxomethyl]-3,4,5-trimethoxy-benzamide (Compound 38);
- N-[[[4-chloro-3-(4-dimethylamino-phenylamino)carbonyl] thioxomethyl]-3,4,5-trimethoxy-benzamide (Compound 39);
- N-[[[4-chloro-3-(4-Chloro-phenylamino)]carbonyl]phenylamino]thioxomethyl]-3,4,5-trimethoxy-benzamide (Compound 43);
- N-[[[2-methyl-3-(benzoylamino)phenyl]amino]oxomethyl]-3,4,5-trimethoxy-benzamide (Compound 45);
- N-[[[-3-(4-chloro-benzoylamino)phenyl]amino]oxomethyl]-3,4,5-trimethoxy-benzamide (Compound 47);
- 3,4,5-trimethoxy-*N*-(3-(3-phenylureido)phenylcarbamothioyl)benzamide (I-b) (Compound 51),
- *N*-(3-(3-biphenyl-4-ylureido)phenylcarbamothioyl)-3,4,5-trimethoxybenzamide (I-b) (Compound 52); and
- *N*-(3-(3-biphenyl-4-ylureido)phenylcarbamoyl)-3,4,5-trimethoxybenzamide (I-b') (Compound 59).

10. Use according to claims 5 to 9, wherein the pathology of the neurodegenerative type is Parkinson's disease, Huntington's disease, Alzheimer's disease, multiple sclerosis or motor neuron disease.

11. Compounds of the following formula (I'): 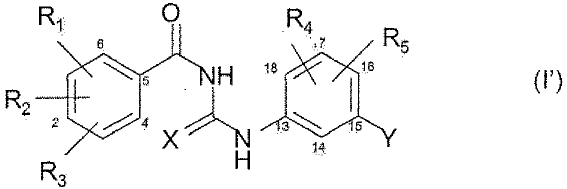 wherein:
- R₁, R₂ and R₃, identical or different and independently of one another, represent a hydrogen or halogen atom, a hydroxyl radical, an alkyl, perfluoroalkyl, alkoxy, alkylthio, nitrile group, a substituted alkoxy, or a fused heterocycle obtained from two of the adjacent R₁, R₂ and R₃ radicals, that can form, jointly with the carbon atoms of the phenyl cycle to which they are bonded, a fused heterocycle;
- X represents a sulphur atom,
- Y represents a -NH-(C=O)-R₆ or -NH-(C=O)-NH-R₆ group wherein R₆ represents an unsubstituted aryl group; an aryl group comprising one or several substituents chosen from a halogen atom and an alkyl, alkoxy or mono- or dialkylamino radical; a mono- or polycyclic heteroaryl group; a branched alkyl radical; a saturated or unsaturated mono- or polycyclic hydrocarbon group;
- R₄ and R₅, identical or different and independently of one another, represent a hydrogen or halogen atom, an alkoxy, alkylthio, alkyl, perfluoroalkyl, nitrile or nitro group.

12. Compounds according to claim 11, **characterised in that** they are chosen from:
- N-[[[3-(4-methoxy-benzoylamino)phenyl]amino]thioxomethyl]-3,4,5-trimethoxy-benzamide (I-a) of the following formula: 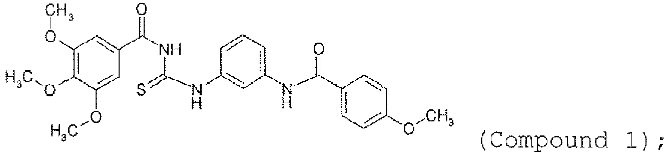
- N-[[[3-(2-chloro-benzoylamino)phenyl]amino]thioxomethyl]-3,4,5-trimethoxy-benzamide (I-a) of the following formula: 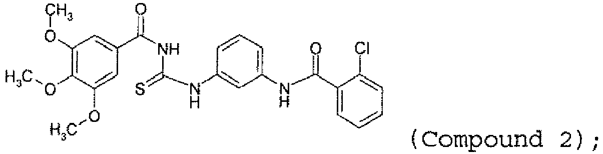
- N-[[[3-(3-methoxy-benzoylamino)phenyl]amino]thioxomethyl]-3,4,5-trimethoxy-benzamide (I-a) of the following formula: 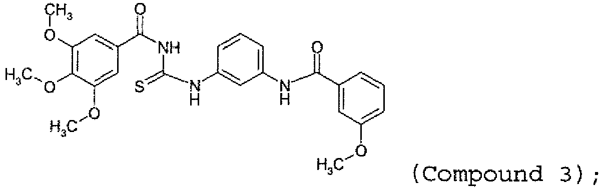
- N-[[[3-(2-methoxy-benzoylamino)phenyl]amino]thioxomethyl]-3,4,5-trimethoxy-benzamide (I-a) of the following formula: 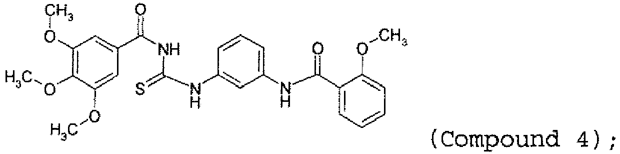
- N-[[[3-(4-phenyl-benzoylamino)phenyl]amino]thioxomethyl]-3,4,5-trimethoxy-benzamide (I-a) of the following formula: 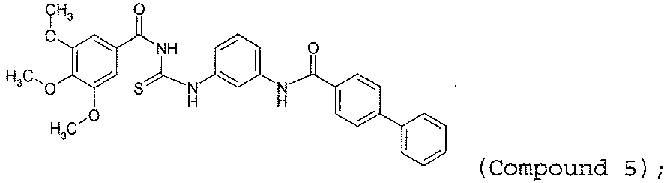
- N-[[[3-(-cyclohexylcarboxylamino)phenyl]amino]thioxomethyl]-3,4,5-trimethoxy-benzamide (I-a) of the following formula: 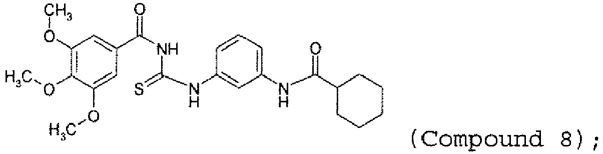
- N-[[[3-(2-naphtoylamino)phenyl]amino]thioxomethyl]-3,4,5-trimethoxy-benzamide (I-a) of the following formula: 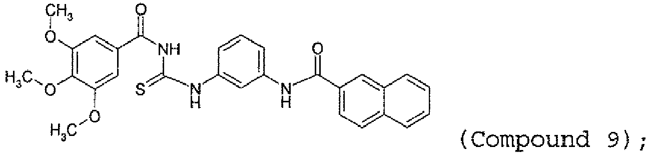
- N-[[[3-(isopropoylcarboxylamino)phenyl]amino]thioxomethyl]-3,4,5-trimethoxy-benzamide (I-a) of the following formula: 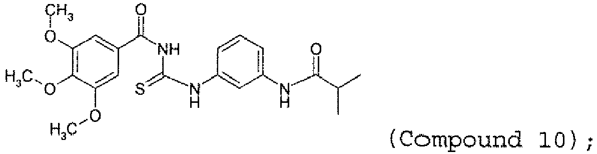
- N-[[[3-(4-pyridocarboxylamino)phenyl]amino]thioxomethyl]-3,4,5-trimethoxy-benzamide (I-a) of the following formula: 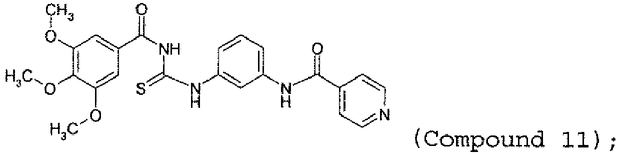
- N-[[[3-(1-naphtoylamino)phenyl]amino]thioxomethyl]-3,4,5-trimethoxy-benzamide (I-a) of the following formula: 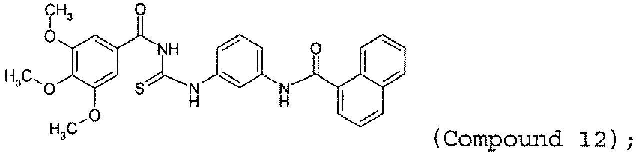
- N-[[[3-(4-chloro-benzoylamino)phenyl]amino]thioxomethyl]-3,4,5-trimethoxy-benzamide (I-a) of the following formula: 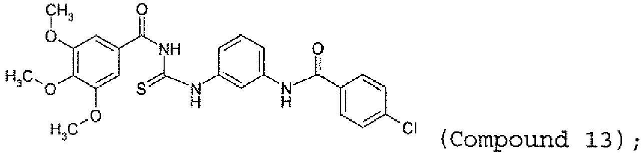
- N-[[[3-(2-furoylamino)phenyl]amino]thioxomethyl]-3,4,5-trimethoxy-benzamide (I-a) of the following formula: 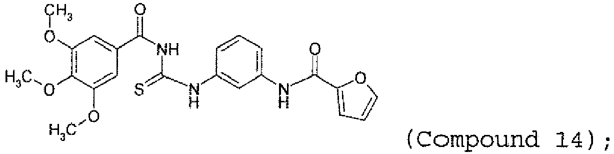
- N-[[[3-(2-thiophenoylamino)phenyl]amino]thioxomethyl]-3,4,5-trimethoxy-benzamide (I-a) of the following formula: 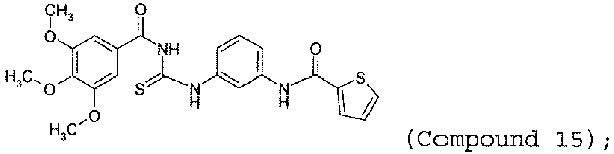
- N-[[[3-(benzoylamino)phenyl]amino]thioxomethyl]-3,5-dimethoxy-benzamide (I-a) of the following formula: 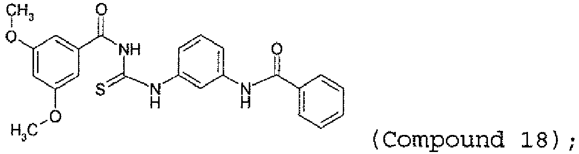
- N-[[[3-(benzoylamino)phenyl]amino]thioxomethyl]-3,5-dimethoxy-4-ethoxy-benzamide (I-a) of the following formula: 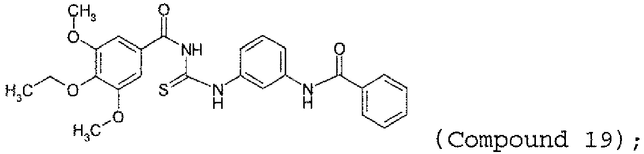
- N-[[[3-(benzoylamino)phenyl]amino]thioxomethyl]-3,4-diethoxy-5-methoxy-benzamide (I-a) of the following formula: 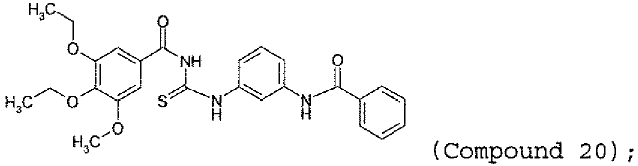
- N-[[[3-(benzoylamino)phenyl]amino]thioxomethyl]-3,4-methylenedioxy-5-methoxy-benzamide (I-a) of the following formula: 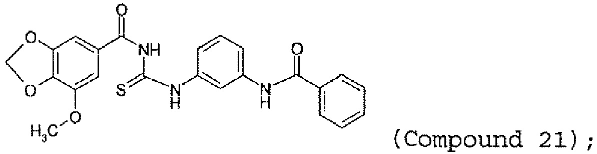
- N-[[[3-(benzoylamino)phenyl]amino]thioxomethyl]-2,4-dimethoxy-benzamide (I-a) of the following formula: 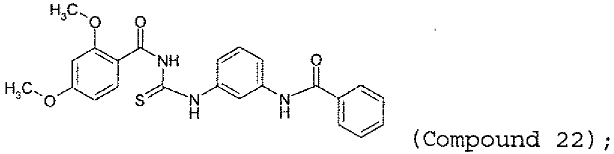
- N-[[[3-(benzoylamino)phenyl]amino]thioxomethyl]-3,4,5-triethoxy-benzamide (I-a) of the following formula: 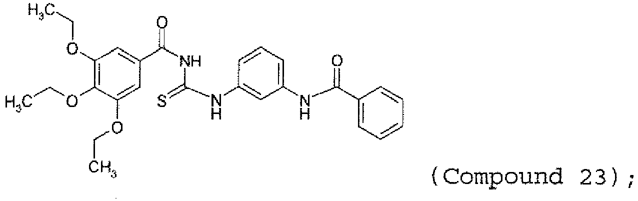
- N-[[[4-fluoro-3-(benzoylamino)phenyllamino]thioxomethyl]-3,4,5-trimethoxy-benzamide (I-a) of the following formula: 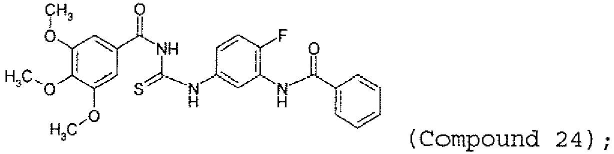
- N-[[[4-chloro-3-(phenylamino)carbonyl]amino]thioxomethyl]-3,4,5-trimethoxy-benzamide (I-b) of the following formula: 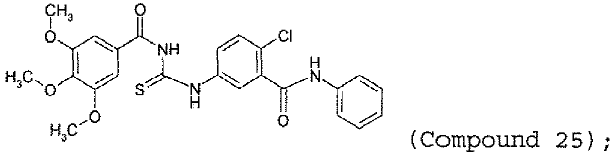
- N-[[[4-methoxy-3-(benzoylamino)phenyl]amino]thioxomethyl]-3,4,5-trimethoxy-benzamide (I-a) of the following formula: 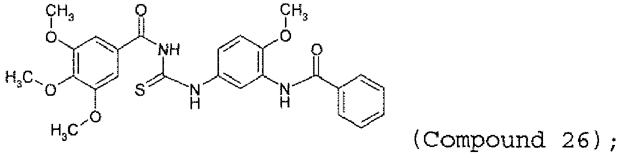
- N-[[[4-methyl-3-(benzoylamino)phenyl]amino]thioxomethyl]-3,4,5-trimethoxy-benzamide (I-a) of the following formula: 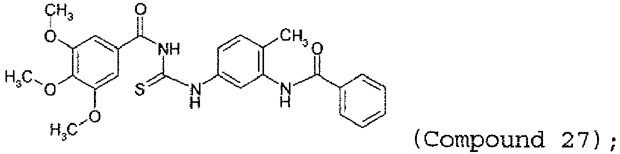
- N-[[[3-(benzoylamino)phenyl]amino]thioxomethyl]-3,4-ethylenedioxo-benzamide (I-a) of the following formula: 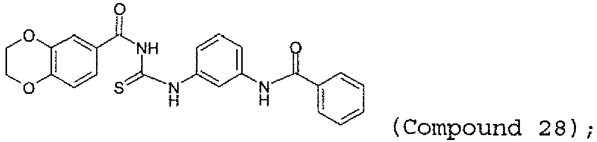
- N-[[[3-methyl-3-(benzoylamino)phenyl]amino]thioxomethyl]-3,4,5-trimethoxy-benzamide (I-a) of the following formula: 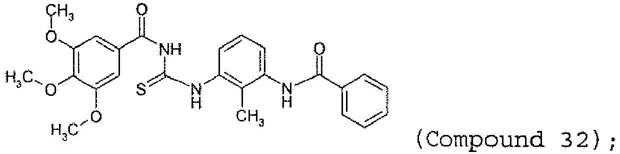
- N-[[[4-chloro-3-(benzoylamino)phenyl]amino]thioxomethyl]-3,4,5-trimethoxy-benzamide (I-a) of the following formula: 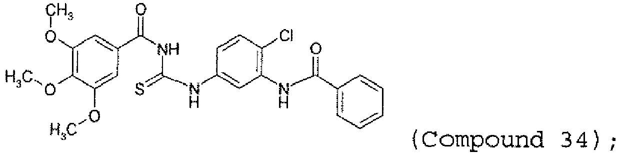
- N-[[[5-chloro-3-(benzoylamino)phenyl]amino]thioxomethyl]-3,4,5-trimethoxy-benzamide (I-a) of the following formula: 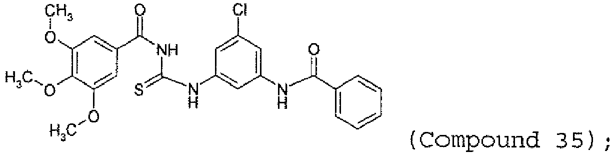
- N-[[[5-fluoro-3-(benzoylamino)phenyl]amino]thioxomethyl]-3,4,5-trimethoxy-benzamide (I-a) of the following formula: 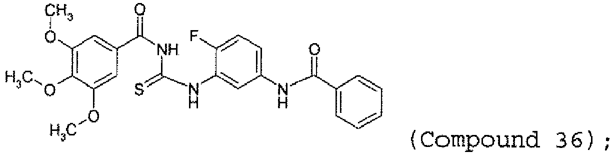
- N-[[[3-(benzoylamino)phenyl]amino]thioxomethyl]-3,4,5-trimethoxy-benzamide (I-a) of the following formula: 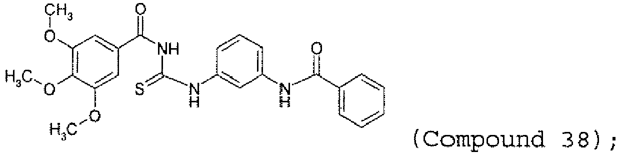
- N-[[[2-methyl-3-(benzoylamino)phenyl]amino]thioxomethyl]-3,4,5-trimethoxy-benzamide (I-a) of the following formula: 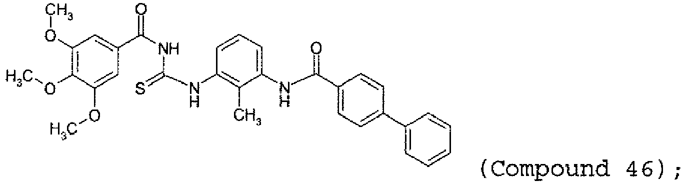
- 3,4,5-trimethoxy-*N*-(3-(3-phenylureido)phenylcarbamothioyl)benzamide (I-b) of the following formula: 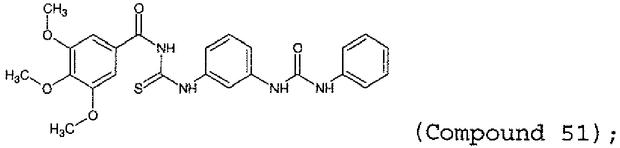
- *N*-(3-(3-biphenyl-4-ylureido)phenylcarbamothioyl)-3,4,5-trimethoxybenzamide (I-b) of formula:

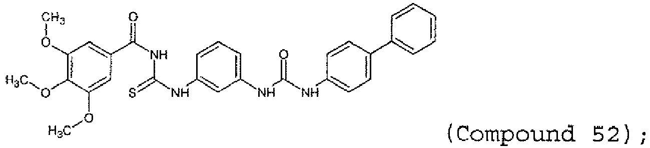

13. Compounds according to claim 11 or 12, **characterised by** the fact that they are chosen from:
- N-[[[3-(4-methoxy-benzoylamino)phenyl]amino]thioxomethyl]-3,4,5-trimethoxy-benzamide (Compound 1) ;
- N-[[[3-(2-chloro-benzoylamino)phenyl]amino]thioxomethyl]-3,4,5-trimethoxy-benzamide (Compound 2) ;
- N-[[[3-(3-methoxy-benzoylamino)phenyl]amino]thioxomethyl]-3,4,5-trimethoxy-benzamide (Compound 3);
- N-[[[3-(2-methoxy-benzoylamino)phenyl]amino]thioxomethyl]-3,4,5-trimethoxy-benzamide (Compound 4);
- N-[[[3-(4-pyridocarboxylamino)phenyl]amino]thioxomethyl]-3,4,5-trimethoxy-benzamide (Compound 11);
- N-[[[3-(4-chloro-benzoylamino)phenyl]amino]thioxomethyl]-3,4,5-trimethoxy-benzamide (Compound 13);
- N-[[[3-(benzoylamino)phenyl]amino]thioxomethyl]-3,5-dimethoxy-benzamide (Compound 18);
- N-[[[3-(benzoylamino)phenyl]amino]thioxomethyl]-3,5-dimethoxy-4-ethoxy-benzamide (Compound 19);
- N-[[[3-(benzoylamino)phenyl]amino]thioxomethyl]-3,4-diethoxy-5-methoxy-benzamide (Compound 20);
- N-[[[3-(benzoylamino)phenyl]amino]thioxomethyl]-3,4-methylenedioxy-5methoxy-benzamide (Compound 21);
- N-[[[3-(benzoylamino)phenyl]amino]thioxomethyl]-3,4,5-triethoxy-benzamide (Compound 23);
- N-[[[4-fluoro-3-(benzoylamino)phenyl]amino]thiaxomethy]-3,4,5-trimethoxy-benzamide (Compound 24);
- N-[[[4-chloro-3-(phenylamino)carbonyl]amino]thioxomethyl]-3,4,5-trimethoxy-benzamide (Compound 25);
- N-[[[4-chloro-3-(benzoylamino)phenyl]amino]thioxomethyl]-3,4,5-trimethoxy-benzamide (Compound 34);
- N-[[[3-(benzoylamino)phenyl]amino]thioxomethyl]-3,4,5-trimethoxy-benzamide (Compound 38);
- 3,4,5-trimethoxy-*N*-(3-(3-phenylureido)phenylcarbamothioyl)benzamide (I-b) (Compound 51),
- *N*-(3-(3-biphenyl-4-ylureido)phenylcarbamothioyl)-3,4,5-trimethoxybenzamide (I-b) (Compound 52);

14. Pharmaceutical composition **characterised in that** it comprises, as an active ingredient, at least one compound such as defined according to one of claims 11 to 13, and at least one pharmaceutically acceptable excipient.

15. Use of compounds such as defined according to one of claims 11 to 13 for the manufacture of a drug for the treatment of pathologies of the neurodegenerative type.

16. Use according to claims 15, for the treatment of Parkinson's disease, Huntington's disease, Alzheimer's disease, multiple sclerosis and motor neuron disease.

17. Use of compounds such as defined according to one of claims 11 to 13 for the manufacture of a drug for the treatment of diabetes.

18. Compounds such as defined according to one of claims 11 to 13 for their use in the treatment of pathologies of the neurodegenerative type.

19. Compounds according to claim 18, for their use in the treatment of Parkinson's disease, Huntington's disease, Alzheimer's disease, multiple sclerosis and motor neuron disease.

20. Compounds such as defined according to one of claims 11 to 13 for their use in the treatment of diabetes.
